(19) Europäisches Patentamt
European Patent Office
Office européen des brevets

(11) **EP 4 537 845 A1**

(12) **EUROPEAN PATENT APPLICATION**
published in accordance with Art. 153(4) EPC

(43) Date of publication:
**16.04.2025 Bulletin 2025/16**

(21) Application number: **23819935.0**

(22) Date of filing: **09.06.2023**

(51) International Patent Classification (IPC):
**A61K 45/00** $^{(2006.01)}$

(52) Cooperative Patent Classification (CPC):
**A61K 31/192; A61K 31/198; A61K 31/216;**
**A61K 31/428; A61K 31/70; A61K 31/7004;**
**A61K 31/7016; A61K 31/7076; A61K 31/708;**
**A61K 38/05; A61K 39/395; A61K 45/00;**
**A61P 9/00; A61P 21/02; A61P 25/00;** (Cont.)

(86) International application number:
**PCT/JP2023/021634**

(87) International publication number:
**WO 2023/238955 (14.12.2023 Gazette 2023/50)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **09.06.2022 JP 2022093746**

(71) Applicants:
• **Ion Chat Research Corporate**
  **Tokorozawa-shi, Saitama 359-1146 (JP)**
• **Saito, Mitsuyoshi**
  **Tokorozawa-shi, Saitama 359-1146 (JP)**

(72) Inventors:
• **SAITO, Mariko**
  **Tokorozawa-shi, Saitama 359-1146 (JP)**
• **SAITO, Mitsuyoshi**
  **Tokorozawa-shi, Saitama 359-1146 (JP)**

(74) Representative: **Kraus & Lederer PartGmbB**
**Thomas-Wimmer-Ring 15**
**80539 München (DE)**

Remarks:
The complete document including Reference
Table(s) and the Sequence Listing(s) can be
downloaded from the EPO website

(54) **METHOD FOR CONTROLLING MEMBRANE POTENTIAL-DEPENDENT ION CHANNEL THROUGH TYPE I TASTE RECEPTORS (T1RS)**

(57)    The present invention pertains to a method for controlling a membrane potential-dependent ion channel (VGSC or the like) through a type I taste receptor present in a nerve cell or the like. In the present invention, it has been found that an A $\beta$ peptide, or a sweet amino acid or an umami substance specifically binds to a type I taste receptor on the surface of a nerve cell to exert an agonist-like or antagonist-like action, thereby amplifying or suppressing a VGSC active current.

Moreover, with the binding of an Aβ peptide or the like to a type I taste receptor, the amplification of a VGSC active current occurs, the overactivity of nerve cells causing epileptiform attack occurs, and a large number of substances, which can effectively suppress the amplification of the VGSC active current, among ligand substances that specifically bind to the type I taste receptor, can be found.

The present invention provides: a type I taste receptor-specific ligand substance that can control the amplification or suppression of a VGSC active current; and a pharmaceutical composition for preventing or treating various neurodegenerative diseases, such as Alzheimer's disease (AD), due to the amplification of a VGSC active current caused by the binding of an Aβ peptide or the like to a type I taste receptor. Moreover, a method for using, as a target receptor, a type I taste receptor present in a nerve cell or the like to screen a ligand substance for controlling a VGSC or the like in the cell is also provided.

Furthermore, the present invention provides a method in which a solution that reproduces the type and concentration of an Aβ peptide contained in the cerebrospinal fluid and serum of a patient with Alzheimer-type dementia is used, and the Aβ peptide contained in the CFS and serum of the patient assesses pathology. Specifically, provided is: a method for assessing the form and dynamic state of cells and the activity of a neurite by using

EP 4 537 845 A1

an optical microscope; or a method for assessing the impedance change in a vascular epithelial cell.

(52) Cooperative Patent Classification (CPC): (Cont.)
**A61P 25/04; A61P 25/08; A61P 25/14;
A61P 25/16; A61P 25/28; A61P 43/00;
C07K 16/28; C12Q 1/02; G01N 33/15**

**Description**

Technical Field

The present invention is based on the discovery that the umami and sweet taste receptor, Type I taste receptor, is not only involved in taste perception but also related to the control of cellular cytoskeleton polymerization/depolymerization and ion channels such as voltage-gated sodium channels (VGSC) and voltage-gated potassium channels (VGKC). Specifically, the invention relates to methods for controlling VGSC and VGKC via the umami and sweet GPCR receptors (Type I taste receptors, "T1Rs") present in neuronal cells and cardiac myocytes. Furthermore, ligand substances that specifically bind to Type I taste receptors, such as sweet amino acids or umami compounds, can regulate VGSC activity currents amplified by binding to Type I taste receptors (e.g., T1R3) on the neuronal cell surface. The invention also encompasses preventive and/or therapeutic pharmaceutical compositions for neurological disorders resulting from the amplification of VGSC activity currents induced by sweet amino acids or umami compounds acting as effective ingredients, along with screening methods for such substances.

Furthermore, this invention is based on the discovery that Type I taste receptors are activated not only by amino acids and sweet substances but also by amyloid β peptide, and are involved in the control of cytoskeletal polymerization and the regulation of VGSC and VGKC. This strongly suggests that the neuronal dysfunction caused by amyloid β peptide, which has been widely reported in neurodegenerative diseases such as Alzheimer's disease, indicates that Type I taste receptors (T1R1, T1R2, T1R3) act as receptors for amyloid β peptide, potentially affecting the function and morphology of neurons.

In other words, this invention relates to a method for controlling VGSC (voltage-gated sodium channels) via Type I taste receptors (the "T1Rs" gene family) present in neurons and other cells. Specifically, it concerns a method for inhibiting VGSC using an amyloid β peptide binding inhibitor, which acts on the taste GPCR receptor "T1R3" in neurons. Additionally, it relates to the use of these amyloid β peptide binding inhibitors as therapeutic agents for Alzheimer's disease (AD) and amyotrophic lateral sclerosis (ALS), and their screening methods.

Furthermore, in cancer treatment, there is neuropathic pain that occurs when chemotherapy drugs (such as Paclitaxel) are used. To address this, there are pain treatment formulations that utilize inhibitors of voltage-gated sodium channels (VGSC) mediated by the sweet taste GPCR receptors T1R2/T1R3. Additionally, these formulations incorporate agonists or antagonists for the sweet taste GPCR receptors (T1R2/T1R3) that act as binding inhibitors for Aβ peptides. These approaches aim to alleviate neuropathic pain associated with cancer treatment.

[Background Technology]

In the context of pain and epileptic seizures, high-frequency action potentials are observed in neuronal cells. This phenomenon arises from neuronal hyperactivity. Consequently, suppressing these frequent action potentials is a key effect of analgesics and antiepileptic drugs. Neurodegenerative diseases, particularly Alzheimer's disease (AD), have been associated with seizure-like symptoms due to neuronal hyperactivity in brain regions such as the hippocampus. It is believed that after this hyperactive phase, neuronal cell death occurs. Therefore, the development of drugs that inhibit neuronal hyperactivity holds promise as a therapeutic approach to improve symptoms in AD patients.

In Alzheimer's disease (AD), Lewy body dementia (DLB), and other neurodegenerative cognitive disorders (Bibl et al., 2007), changes in amyloid β peptide levels have been observed in cerebrospinal fluid (CSF). Amyloid β peptides in CSF are used as biomarkers for conditions like AD.

Excessive expression of amyloid precursor protein (APP) has been reported to enhance the activity of sodium channels. Specifically, experiments using neuronal cells derived from induced pluripotent stem cells (iPSCs) have demonstrated that amyloid β peptides induce neuronal hyperactivity and amplify sodium channel activity. This hyperactivity can be inhibited by cleaving APP, which produces amyloid β peptides, and by suppressing β-secretase 1 (BACE1), the enzyme responsible for their production (Non-Patent Document 1). However, the exact mechanism by which amyloid β peptides enhance sodium channels remains unclear.

Furthermore, voltage-gated sodium channels (VGSCs) exist in various types with structurally similar features. They are widely distributed beyond brain regions. VGSCs play a crucial role in functions such as heart contraction and neural activity. However, indiscriminate blocking of VGSCs can be life-threatening. Consequently, selectively inhibiting the target VGSC using drug development that targets the VGSC molecules themselves is challenging.

On the other hand, voltage-gated sodium channels (VGSCs) are known to have their activity (kinetics) controlled by the activation of G proteins. When G proteins are activated, the magnitude of VGSC currents and their activation threshold are influenced, resulting in the modulation of the ease of action potential generation (threshold). Many G protein-coupled receptors (GPCRs) on the cell membrane activate serine/threonine kinases (PKA/PKC) via G proteins, which in turn modify VGSC activity.

Activation of Gαi and Gαo, which are sensitive to pertussis toxin, shifts the VGSC S-S curve in the hyperpolarizing direction. Furthermore, thrombin enhances this shift when it activates Gαi and Gαo (Ma et al. ,1994).

Moreover, in taste and sweet GPCR receptors (Type I taste receptors, "T1Rs" gene family) present in neuronal cells, Gαi has been shown to bind to T1R1/T1R3 (Husted et al., 2017) and T1R2/T1R3 (Yang et al., 2020). This suggests that the action of T1R GPCRs on VGSCs may also involve mechanisms mediated by Gαi. In addition to VGSC modulation by G protein α subunits, the effects of Gβγ have also been reported. Ma et al. (1997) reported that binding of Gβ2γ3, Gβ1γ3, or Gβ5γ3 to the carboxyl terminus of VGSC leads to delayed inactivation. However, this delayed inactivation effect depends on the Gβγ subtype, as Gβ1γ1 does not exhibit such an effect (Ma et al., 1997). This action is believed to directly affect VGSC via Gβ2γ3. Furthermore, Gβγ can directly modulate ion channels. For instance, GPCRs coupled with Gi/Go activation result in Gβγ directly acting on G-protein-activated inwardly rectifying K+ channels (GIRK), controlling their activity (Olivera et al., 2019).

Mantegazza et al. (2005) investigated the delayed inactivation of voltage-gated sodium channel (VGSC) currents by Gβγ in different VGSC isoforms (Nav1.1, Nav1.2, Nav1.4, Nav1.5). Their findings indicate that Gβγ-induced delayed inactivation occurs specifically in Nav1.1 and Nav1.2, while Nav1.4 and Nav1.5 do not exhibit this effect. Therefore, the delayed VGSC inactivation caused by Aβ1-42 may be attributed to different Gβγ subtypes or distinct underlying mechanisms.

As an additional note, T1R3 GPCR couples with Gαs and induces microtubule depolymerization. As a result, activation of the Rho/ROCK pathway via Gα12/13 occurs (Masubuchi et al., 2017). Additionally, T1R1/T1R3 (Husted et al., 2017) and T1R2/T1R3 (Yang et al., 2020) have also been reported to bind to Gαi.

On the other hand, in the central nervous system, there are numerous reports that the activation of PKA/PKC leads to phosphorylation of VGSC (voltage-gated sodium channels), resulting in the inhibition of VGSC activity. For instance, in hippocampal pyramidal neurons, muscarinic acetylcholine receptors (Cantrell et al., 1996), 5-HT2a/c receptors in cortical pyramidal neurons (Carr et al., 2002), and D1-class dopamine receptors in hippocampal pyramidal neurons and medium spiny neurons of the striatum (Cantrell et al., 1997; Surmeier et al., 1992), PKA activation has been reported to suppress VGSC. Additionally, PKC activation due to D2 dopamine receptor activation in medium spiny neurons of the striatum and cholinergic interneurons also inhibits VGSC (Carr et al., 2003).

Additionally, voltage-gated sodium channels (VGSC) play a crucial role in action potential generation in both peripheral and central nervous systems. Changes in VGSC activation thresholds significantly impact neuronal excitability, and they are deeply involved in the pathogenesis of neuropathic pain.

As a typical example of neuropathic pain, chemotherapy (paclitaxel)-induced neuropathic pain occurs when the anticancer drug paclitaxel is used for cancer treatment. It has been reported that paclitaxel increases the expression of voltage-gated sodium channels (VGSCs) in the dorsal root ganglion (DRG) neurons, which are specifically inhibited by the Nav1.7 VGSC blocker ProTx II. As a result, the frequency of spontaneous firing of action potentials in sensory neurons of the peripheral nervous system increases, leading to neuropathic pain (Non-Patent Literature 3).

Neuropathic pain is estimated to affect 6% to 17% of the general population and is associated with decreased quality of life (QOL) and functional impairment (North, R.Y, et al, (2018)). In 2016, approximately 20.4% of adults in the United States reported chronic pain, with 8.0% experiencing severe chronic pain that significantly impacted their daily lives (Dahlhamer J., et al.(2016)). According to Japanese statistics, the prevalence of chronic pain was 16.6%, and the prevalence of chronic pain with decreased QOL was 3.2% (Inoue, S., et al. (2017)).

Conditions such as Failed Back Surgery Syndrome (FBSS), Complex Regional Pain Syndrome (CRPS), and diabetic polyneuropathy are known to cause chronic neuropathic pain (Huygen, Frank, et al. (2019)). Peripheral nerve disorders, including diabetic neuropathy and post-herpetic neuralgia, are common causes of neuropathic pain. Peripheral nerve (primary sensory neuron) damage leads to increased excitability of primary sensory neurons, contributing to chronic pain. This heightened excitability also affects central excitability, making pain management challenging.

Chronic pain and neuropathy resulting from chemotherapy significantly impact patients' quality of life (QOL) (Xiao et al. (2008), Authier et al. (2009), Quintao, et al. (2019)). Chemotherapy-induced peripheral neuropathic pain is one of the most severe side effects of anticancer drugs, including platinum-based agents and taxanes (such as oxaliplatin, cisplatin, carboplatin, and paclitaxel). Due to its severity, chemotherapy-induced peripheral neuropathic pain can even lead to treatment discontinuation and an increased risk of mortality (Quintao, et al. (2019)).

Electrophysiological studies suggest that chronic pain and neuropathy, including chemotherapy-induced pain with peripheral nerve damage, arise from abnormal spontaneous activity in sensory neurons.

In recent years, there has been an increasing emphasis on patients' quality of life (QOL) in the medical field, and there is a general trend toward actively controlling pain. Voltage-gated sodium channels (VGSC), which are present in the cell membranes of sensory neurons, play a crucial role in generating action potentials. VGSC channels have high selectivity for sodium ions (Na+), making them important drug targets for pain management.

VGSCs have a significant impact on neuronal excitability in the peripheral and central nervous systems. They influence not only the generation of action potentials but also the suppression of excitability when the VGSC activation threshold is controlled. This makes them deeply involved in the pathogenesis of neuropathic pain. Since nerve cells use electrical pulses called action potentials to transmit pain signals to the central nervous system, inhibiting VGSC can block pain. Therefore, Na+ channel (VGSC) blockers are important candidates for analgesics.

However, VGSC (voltage-gated sodium channels) play a crucial role in supporting heart contractions and nerve function. Indiscriminate blocking of VGSC can pose a risk to life. Additionally, because there are many types of Na+ channels with similar structures, developing drugs that selectively inhibit the target Na+ channels at the molecular level are challenging. Specifically, achieving subtle adjustments-such as restoring slightly overactive responses to normal-rather than complete inhibition of Na$^+$ channels is extremely difficult. Therefore, a system capable of providing sustained substances that selectively act on peripheral nerve cell VGSC and appropriately regulate their ease of activation is necessary.

Furthermore, considering that amyloid β peptides are overexpressed in brain regions affected by Alzheimer's disease (AD) and that excessive expression of amyloid precursor protein (APP) enhances voltage-gated sodium channel (VGSC) activity, it is expected that elucidating the receptor through which amyloid β peptides are expressed in brain regions and understanding the mechanism by which they enhance VGSC activity could lead to the discovery of molecules that inhibit the binding of amyloid β peptides to receptors responsible for VGSC activation. Such discoveries may contribute to the development of drugs for treating or preventing AD.

However, according to Shakoor et al. (2017), although more than 500 genetically engineered mouse models of Alzheimer's disease have been created and their symptoms treated, the results from mouse models have not directly translated to human dementia treatment. This highlights the challenge in Alzheimer's disease research and drug development, as it remains unclear how amyloid β peptide affects or inhibits human (neuronal) cell function and which receptors are involved.

Therefore, for the effective treatment and prevention of Alzheimer's Disease (AD), it is urgent to identify the GPCR-type receptors on the surface of nerve cells that interact with amyloid β peptides in human cerebrospinal fluid (CSF) as ligands, activating VGSC currents and causing central nervous system hyperactivity.

[prior art literature]

[non-patent literature]

[non-patent literature 1] Ghatak, et al., ELife 8 (2019) https://doi.org/10.7554/eLife.50333.

[non-patent literature 2] Li, et al. (2002) Proceedings of the National Academy of Sciences 99, no. 7: 4692-96. https://doi.org/10.1073/pnas.072090199.

[non-patent literature 3] Li, et al. (2018) The Journal of Neuroscience 38, no. 5: 1124-36. https://doi.org/10.1523/JNEUROSCI.0899-17.2017.

[non-patent literature 4] Maillet, et al. (2015) Chemical Senses 40, no. 8: 577-86. https://doi.org/10.1093/chemse/bjv045.

[non-patent literature 5] Martin, et al. (2017) Journal of Biological Chemistry 292, no. 27: 11508-30. https://doi.org/10.1074/jbc.M116.773820.

[non-patent literature 6] Martinez, et al. (1993) Journal of Neural Transmission - Parkinson's Disease and Dementia Section 6, no. 1: 1-9. https://doi.org/10.1007/BF02252617.

[non-patent literature 7] Mattheisen, et al. (2018) Cell Reports 23, no. 9 (May 2018): 2770-81. https://doi.org/10.1016/j.celrep.2018.04.109.

[non-patent literature 8] Vetter, et al. (2012) Biochemical Pharmacology 83, no. 11: 1562-71. https://doi.org/10.1016/j.bcp.2012.02.022.

[non-patent literature 9] Xu, et al. (2015) Journal of Biological Chemistry 290, no. 27 (July 3, 2015): 16619-32. https://doi.org/10.1074/jbc.M115.638932.

[Outline of the invention]

[The problem that the invention seeks to solve]

The present invention provides a method for effectively controlling voltage-gated sodium channel (VGSC) activity in nerve cells by indirectly enhancing or inhibiting VGSC activity through taste receptors, such as sweet taste receptors (T1R2/T1R3), which are G protein-coupled receptors (GPCRs). Additionally, the invention aims to indirectly and efficiently suppress central nervous system hyperactivity caused by enhanced VGSC activity due to overexpression of amyloid β peptide (Aβ) and/or amyloid precursor protein (APP) in brain regions of patients with Alzheimer's disease (AD) and amyotrophic lateral sclerosis (ALS). Furthermore, the aim is to treat or prevent neuropathic pain caused by the enhanced activity of VGSC in peripheral nerves by indirectly suppressing VGSC activity through T1R receptors, thereby restoring the excessive neuronal activity to normal levels. To achieve this, it is necessary to identify the GPCR-type receptors that use amyloid β peptides as ligands, which enhance VGSC currents in nerve cells and cause neuronal hyperactivity.

[Means for solving the problem]

Type I taste receptors composed of T1R1, T1R2, and T1R3 GPCRs, which perceive umami and sweetness, are known to be expressed not only in the oral cavity responsible for taste perception but also in various tissues, including nerves. In addition to T1R2/T1R3, which perceive sweetness, these receptors also function as amino acid sensors (T1R1/T1R3) for umami taste. The inventors of this study discovered a novel biological phenomenon: Type I taste receptors are activated by amino acids containing umami and sweet substances, controlling not taste perception but rather the polymerization/depolymerization of cellular skeletons and the voltage-dependent sodium channels (VGSC) and potassium channels (VGKC). Furthermore, Type I taste receptors are activated not only by amino acids and sweet substances but also by amyloid β peptides, suggesting a strong possibility that these receptors play a role in the functional impairment of neurons caused by amyloid β peptides, which has been widely reported in neurodegenerative diseases such as Alzheimer's disease. In summary, this invention is based on these discoveries and is specifically described as follows.

<Alzheimer's disease and the taste receptor>

Alzheimer's disease, Parkinson's disease, and other neurodegenerative disorders, including ADHD and schizophrenia, have been associated with sensory abnormalities such as taste and smell perception (Field et al., 2015). In patients with mild cognitive impairment, olfactory identification deficits, particularly related to impaired smell recognition, may serve as a potential early diagnostic marker for Alzheimer's disease. Olfactory impairment has also been observed in amyotrophic lateral sclerosis (ALS), while both smell and taste dysfunction have been reported in the early stages of Parkinson's disease (Field et al., 2015).

Li et al. (2002) discovered that T1R2/T1R3 receptors recognize sweetness, including artificial sweeteners, while T1R1/T1R3 receptors detect umami taste (monosodium glutamate; MSG). They also found that the T1R1/T1R3 response to MSG is enhanced by other umami substances such as 5'-ribonucleotides (IMP, GMP) (non-patent literature 2)."

On the other hand, Martin et al. analyzed the functional role of T1R3 using T1R3-KO mice. They confirmed that T1R3 subunits are widely expressed throughout the brain, including the hypothalamus, hippocampus, and cortex (non-patent literature 5). This suggests that the sweet taste receptor system plays an important role in central nervous tissues beyond the tongue, supporting synaptic function, memory acquisition, and social behavior.

Furthermore, the inventors focused on the relationship between taste receptors, particularly the sweet or umami receptors containing the T1R3 subunit, which are widely expressed in the brain and nervous system, including the hippocampus and cortex. They used human neuroblastoma cells (SH-SY5Y) that functionally express Nav1.2, Nav1.3, and Nav1.7 (as reported in non-patent literature 8), as well as HEK cells stably expressing Nav1.5 (with endogenous expression of T1R1, T1R2, and T1R3). They observed the effects of taste stimuli on sodium channel activation. By reducing the expression of T1R1 and T1R3 genes using silencing vectors, they confirmed that the effects on sodium channel currents induced by taste stimuli act through taste receptor GPCRs. Notably, in SH-SY5Y cells, Nav1.7 mRNA is most abundant, followed by Nav1.3 >> Nav1.2 >> Nav1.4 >> Nav1.5. However, the functionally expressed genes generating voltage-dependent sodium channel (VGSC) currents are Nav1.7, Nav1.2, and Nav1.3, which exhibit TTX sensitivity (non-patent literature 8).

Next, to elucidate the mechanism by which amyloid β peptide, which is overexpressed in the hippocampus of patients with Alzheimer's disease (AD) and ALS, enhances voltage-gated sodium channels (VGSC), detailed experiments were conducted using Aβ25-35 peptide (ANASPEC). Experiments on SH-SY5Y cells and HEK cells demonstrated that Aβ25-35 peptide indirectly enhances the activity of VGSC by binding to T1R3.

Specifically, when Aβ25-35 peptide is administered to SH-SY5Y cells, it enhances the activity of Nav1.3 and Nav1.7 VGSCs. On the other hand, when T1R3-KO vector is expressed in HEK Nav1.5 expressing cells to reduce T1R3 expression, the enhancing effect of Aβ25-35 peptide on Nav1.5 activity is no longer observed.

This indicates that the binding of Aβ25-35 peptide to T1R3 receptors expressed in neurons bathed in the cerebrospinal fluid (CSF) activates VGSC. Therefore, substances that inhibit binding to T1R3, such as T1R3 antagonists that competitively bind to T1R3, strongly suggest the potential to suppress the enhancement of VGSC by Aβ25-35 peptide.

In this invention, it has been observed that other Aβ peptides besides Aβ25-35 peptide, such as Aβ1-38 (Peptide Institute), Aβ1-40 (+Wako Pure Chemical Industries), and Aβ1-42 (Wako Pure Chemical Industries), also regulate the activity of voltage-gated sodium channels (VGSC) through sweet taste receptors (T1R2/T1R3, T1R3/T1R3) or umami taste receptors (T1R1/T1R1, T1R1/T1R3).

Furthermore, the inventors recently demonstrated that the effects of these Aβ peptides on VGSC do not occur in cells where T1R3 expression has been silenced (data not shown). This indicates that the regulation of VGSC activity by Aβ peptides, including Aβ25-35, is due to their binding to T1R3, or at least that the presence of T1R3 is essential for the regulation of VGSC activity by Aβ peptides. For example, even if the action of Aβ peptides (such as Aβ1-42) on umami taste receptors (T1R1/T1R1, T1R1/T1R3) is due to binding to T1R1, the loss of action from T1R3 to the umami taste receptor would result in the inability of Aβ peptides to bind to T1R1, or the failure of signal transmission from the bound T1R1 to VGSC, thereby inhibiting the regulation of VGSC activity by Aβ peptides.

Since all four Aβ peptides used in the above experiments contain the Aβ25-35 sequence (Gly-Ser-Asn-Lys-Gly-Ala-Ile-Ile-Gly-Leu-Met: Sequence No. 1), it can be concluded that the Aβ25-35 sequence in Aβ peptides is the binding active region to T1R3. Therefore, Aβ peptides containing this sequence are highly likely to have binding activity to T1R3. Understanding the physical relationship between this amino acid sequence structure and T1R3 can provide important information for elucidating the mechanism of action of Aβ peptides on T1R3 and for searching for compounds or peptides that inhibit the action of Aβ peptides on T1R3. Specifically, the latter half of the Aβ25-35 sequence (Ala-Ile-Ile-Gly-Leu-Met) has hydrophobic side chains, except for Gly, and does not participate in hydrogen bonding that binds ligands and receptor (O'Hara et al. (1993), Hampson et al. (1999)). Therefore, the first half of the Aβ25-35 sequence, Gly-Ser-Asn-Lys-Gly, particularly Ser-Asn-Lys, is considered important for the binding of Aβ25-35 to T1R3.

As described above, this invention has, for the first time, discovered that "Type I taste receptors (T1R1, T1R2, T1R3) serve as receptors for amyloid β peptides," making it possible to develop drugs targeting amyloid β peptide receptors.

Previously, one of the major reasons why mouse models of Alzheimer's disease were not useful for developing human dementia treatments was the species difference in sensitivity to umami and sweet ligands of Type I taste receptors. In fact, mice have significantly different sensitivities to umami and sweet tastes compared to humans, while species with similar sensitivities to humans are limited to primates such as chimpanzees, gorillas, orangutans, macaques, and baboons (Li et al. (2011), Toda et al. (2021)). Therefore, it is impossible to conduct in vivo drug development using animals other than the aforementioned primates for drug screening without replacing the Type I taste receptors with human-type receptors.

Thus, in this invention, the cells used in in vitro experiments are limited to human-derived cells, animal-derived cells that endogenously express human-type Type I taste receptors, or cells that have been genetically modified to express Type I taste receptors with human-specific amino acid sequences.

<Sweet taste receptor (T1R2/T1R3 or T1R3/T1R3) binding substances>

In investigating potential inhibitors of Aβ25-35 peptide binding to T1R3, known T1R3 antagonists with sweet taste-suppressing properties-such as lactisole and its structurally similar compound clofibrate-were tested for their VGSC (voltage-gated sodium channel) activity using T1R3-expressing cells. It was confirmed that both lactisole and clofibrate inhibit VGSC activity, with dichlorprop-2-(2,4-dichlorophenoxy) propanoic acid being the most effective inhibitor compared to lactisole and clofibrate.

"When examining the $IC_{50}$ of these compounds along with their sweet taste-suppressing effects, the order of potency is as follows: lactisole > clofibrate > dichlorprop. dichlorprop was found to be the most effective inhibitor of sodium channels at the lowest concentration. These compound groups exhibit VGSC inhibitory effects due to their T1R3 antagonist-like action. Similar results were observed in an experimental system that applied sucralose-induced response inhibition using T1R2/T1R3 and $G_{16}$-gust44 overexpressing cells, as demonstrated in non-patent literature 1.

Jiang et al. (2005) reported that lactisole binds to $TMD_3$, $TMD_5$, and $TMD_6$ of T1R3. This suggests the possibility of limiting changes in the activation structure of T1R3 induced by amyloid β, which is an agonist of T1R3, similar to sweet-tasting substances.

Next, we investigated the impact of sweet taste-sensing T1R2/T1R3 receptors on the enhancement or inhibition VGSC (voltage-gated sodium channel) activity. We discovered that sweet taste-sensing T1R2/T1R3 receptors, when exposed to sweet-tasting amino acids found in cerebrospinal fluid (CSF), such as L-glutamine (L-Gln) and L-serine (L-Ser), enhance VGSC activity.

Martinez et al. reported variations in amino acid concentrations in the cerebrospinal fluid (CSF) of patients with Alzheimer's-type and vascular-type dementia. Specifically, Smith et al. measured amino acid levels in the CSF of patients with epilepsy, including those with dementia (12 patients) and those with epilepsy alone (10 patients), compared to healthy individuals (23 people). They found that L-glutamine concentrations were statistically significantly elevated in patients with epilepsy alone (715±145 nmol/ml) and in patients with epilepsy complicated by dementia (733 ± 165 nmol/ml) compared to healthy individuals (497±147 nmol/ml). This report suggests that changes in amino acid concentrations in CSF may influence neuronal hyperactivity in conditions such as epilepsy and dementia.

In summary, for sweet-tasting amino acids like glutamine and serine, contrary to T1R3 antagonists like lactisole mentioned earlier, they exhibit VGSC-enhancing effects through T1R2/ T1R3 receptors, similar to Aβ25-35 peptide. However, they also hint at the possibility of being T1R1/ T1R3 agonists.

The sweet taste receptors T1R2/T1R3, which belong to the T1R taste receptor family in class C of G protein-coupled receptors, play a role in enhancing or inhibiting VGSC (voltage-gated sodium channel) activity through interactions with substances that act on these receptors. In this context, we can categorize a group of substances, including sweet-tasting amino acids like glutamine and serine, as 'Group 1.' Within 'Group 1,' we observe agonist-like effects on VGSC currents, amplifying them, not only for sweet amino acids like serine and glutamine but also for sodium lactate (Lactate).

According to Shirahata et al. (2005), the strength of the interaction between Ankyrin G and VGSC, which binds to VGSC, may control the leftward or rightward shift of the I-V curve. Based on this, it is suggested that glutamine, serine, and other substances acting through T1R2/T1R3 may promote microfilament polymerization via Ankyrin G, resulting in enhanced VGSC activity.

"Group 2": This group consists of peptides that act on sweet taste receptors (T1R2/T1R3), including the amyloid β (Aβ)25-35 peptide, which is considered one of the causative agents of dementia. Similar to 'Group 1', these peptides exhibit agonist-like effects, amplifying VGSC (voltage-gated sodium channel) currents.

"Group 3": Artificial sweeteners that act on the sweet taste receptors (T1R2/T1R3) demonstrate an antagonist-like effect on VGSC (voltage-gated sodium channels) currents. Among artificial sweeteners, saccharin and aspartame specifically act on T1R2, while sucralose, which has a similar structure to sucrose, affects both T1R2 and T1R3 (Neiers et al., 2016).

Overexpression of T1R2 has been shown to produce effects similar to those caused by artificial sweetener stimulation in VGSC. In summary, in the T1R2/T1R3 heterodimer, stimulating T1R3 enhances VGSC activity, while stimulating T1R2 leads to inhibition. Therefore, it is highly likely that substances belonging to 'Group 1' and 'Group 2' stimulate T1R3 within the T1R2/T1R3 complex, while substances in 'Group 3' stimulate T1R2.

"Group 4": Substances that act on the umami taste receptors (T1R1/T1R3) include glutamic acid, known as an amino acid seasoning, and monosodium glutamate (MSG), as well as inosine monophosphate (IMP) and guanosine monophosphate (GMP), which are known as nucleotide seasonings. Among these, MSG delays the inactivation of VGSC (voltage-gated sodium channels) currents, while IMP transiently enhances VGSC currents. Subsequently, over time, the I-V curve shifts by approximately 10 mV in the negative direction. Similar changes in VGSC are observed even in MSG/IMP mixtures. GMP alone enhances VGSC without affecting the inactivation process or the properties of the I-V curve.

Note that when using IMP and GMP as nucleotide-based umami seasonings, their structures have a phosphorylated group at the 5' position. To avoid confusion, this document refers to them as inosine monophosphate and guanosine monophosphate in Japanese.

The amyloid β (Aβ) 1-42 peptide (ranging from 12.5 nM to 1 μM) acts on the umami taste receptor T1R1/T1R3, unlike the Aβ25-35 peptide, which functions as an agonist for T1R3. It belongs to 'Group 4' and exhibits similar effects to MSG (monosodium glutamate) and IMP (inosine monophosphate). Specifically, it delays the inactivation of voltage-gated sodium channels (VGSC) and shifts the activation threshold in the hyperpolarizing direction by 5-10 mV. In experiments using Nav1.5, the effect of Aβ1-42 on inactivation processes is weaker than that of MSG but closely resembles the effect of IMP. Additionally, its impact on SH-SY5Y cells is comparable to MSG.

Among the artificial sweeteners that act on the sweet taste receptors T1R2/T1R3, the effects of saccharin and aspartame on VGSC (voltage-gated sodium channels) were observed. The degree of VGSC reduction varied with each artificial sweetener, and there was significant variability between cells.

The mechanism of VGSC (voltage-gated sodium channel) inhibition by artificial sweeteners differs from the direct inhibition of VGSC channels by substances like lidocaine. Instead, it involves shifting the steady-state (S-S) inactivation curve to the left (towards a more negative potential), thereby reducing VGSC availability (the number of VGSCs that can be activated by voltage stimulation) and decreasing the number of activated VGSCs. Experimental results showed variability in VGSC inhibition by artificial sweeteners between cells, suggesting possible variability in the expression levels of T1R2/T1R3 receptors between cells. Attempts to overexpress T1R2/T1R3 receptors did not reduce this variability. However, overexpression of T1R2 alone resulted in VGSC current inhibition based on a leftward shift in the S-S inactivation curve, similar to the previously mentioned experimental results. From this, it was confirmed that in cells with high expression of T1R2, T1R2 can be a drug target for VGSC inhibition. These T1R2-binding inhibitors are classified as 'Group 3'.

Unlike sweet substances, umami substances, including monosodium glutamate (MSG), are detected by the umami receptor (T1R1/T1R3), which is an amino acid sensor. Similar to monosodium glutamate (MSG), which is an amino acid umami substance, IMP (inosine monophosphate) and GMP (guanosine monophosphate), known as umami substances, are known to enhance the umami effect of monosodium glutamate (MSG) and also stimulate the umami receptor (T1R1/T1R3) on their own. In this invention, to investigate the possibility that the umami receptor (T1R1/T1R3) functions as an amino acid sensor in the CSF and controls the activity of VGSC, the effect on VGSC activity of the umami receptor (T1R1/T1R3) endogenously expressed in HEK cells or SH-SY5Y cells activated by monosodium glutamate (MSG), IMP (inosine monophosphate), or GMP (guanosine monophosphate) was confirmed using the patch-clamp method.

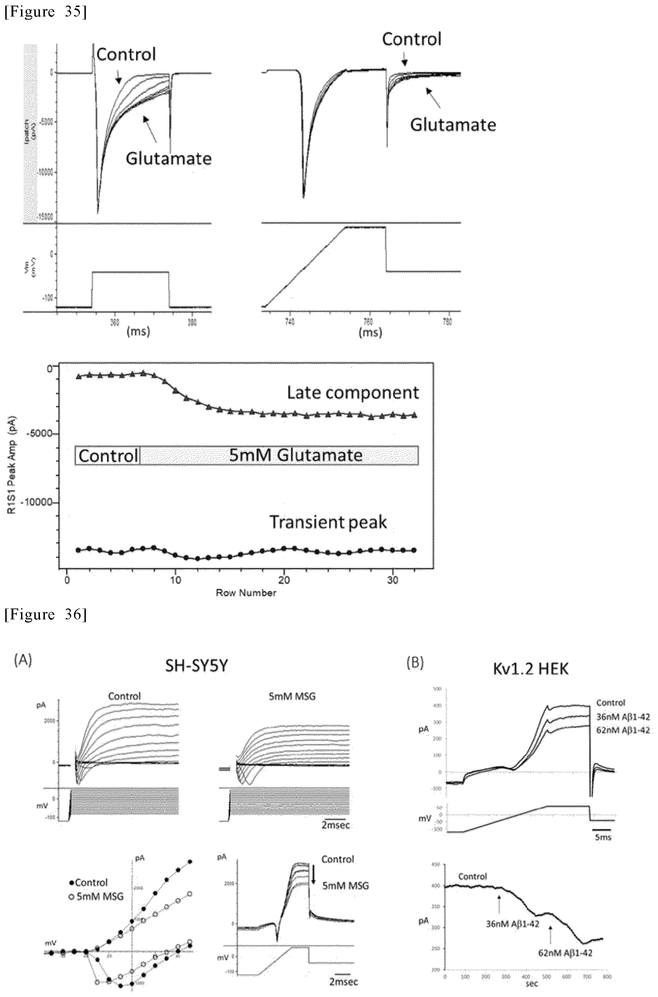

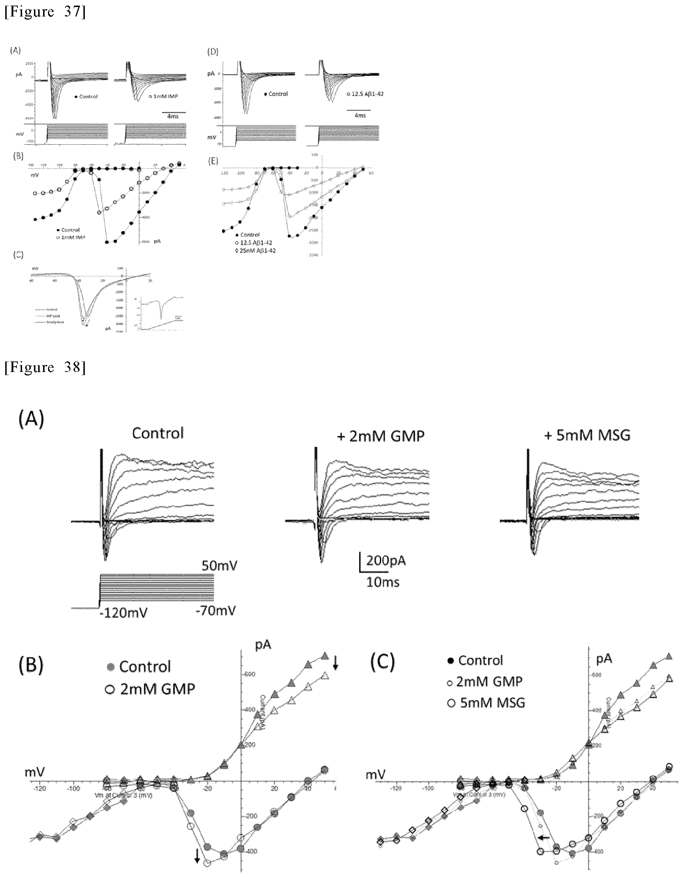

As a result, it was discovered that umami substances, depending on their type, enhance VGSC currents via T1R1, shift the threshold potential for VGSC activation in the hyperpolarizing direction (about 10 mV), and slow the inactivation rate of VGSC currents. This demonstrates for the first time that umami substances control VGSC activity, making VGSCs downstream effectors similar to taste recognition (Implementation Example 4, Figures 35, 36A, B, 36C). Additionally, the effect of amyloid β (Aβ) 1-42 peptide on VGSCs differs from that of amyloid β (Aβ) 25-35 peptide, as it also acts on umami receptors (T1R1/T1R3), and this effect is similar to that of MSG and IMP (Figure 37). Furthermore, when examining the effect of lysine and aspartic acid, whose concentrations are elevated in the CSF of Alzheimer's disease patients compared to healthy individuals, it was confirmed that, like MSG, they delay VGSC inactivation (Data not shown).

Cells expressing umami receptors (T1R1/T1R3) were pre-treated with artificial sweeteners such as aspartame to inhibit VGSC (voltage-gated sodium channels). When monosodium glutamate (MSG) or MSG2.5 (a mixture of MSG, IMP, and GMP: Ajinomoto) was applied, it was observed that the inhibition of VGSC currents by aspartame was partially reversed (shifting the VGSC I-V curve to the right). This effect of monosodium glutamate on T1R1 occurs independently of the action of 'Group 3' substances like aspartame, which inactivate VGSC through T1R2. Therefore, it was found that monosodium glutamate antagonizes the inactivation effect of VGSC mediated through T1R2.

In other words, the enhancement effect of VGSC through T1R1 by umami substances like monosodium glutamate is considered to be independent of the action on VGSC through T1R2 and T1R3. This is supported by experiments using HEK-Nav1.5 cells with silenced T1R3, where the effect of MSG2.5 on T1R1 was observed similarly to control cells. A group of substances that target such umami receptors (T1R1/T1R3) is called 'Group 4'.

As mentioned later, Aβ25-35 peptide inhibits VGSC currents at high concentrations. In other words, the enhancement effect of VGSC currents observed at low concentrations of Aβ25-35 peptide disappears at high concentrations, returning to control levels. This phenomenon is the same as that observed when T1R2 is activated by stimulation with artificial sweeteners. While it is necessary to suppress the enhancement of VGSC currents (neuronal hyperactivity) caused by low concentrations of Aβ25-35 peptide, the inhibition of VGSC currents caused by high concentrations of Aβ25-35 peptide corresponds to a state where neurons can no longer transmit information. In such cases, the action of monosodium glutamate and other substances on VGSC through T1R1 (shifting the activation threshold of VGSC in the negative direction) moves the membrane potential threshold required for VGSC activation in the hyperpolarizing direction compared to before T1R1 activation, bringing it closer to the resting membrane potential. This allows VGSC to be activated with a smaller depolarization from the resting membrane potential. As a result, action potentials are more likely to occur, potentially playing an important role in partially restoring function.

In the action of Aβ peptides, Aβ25-35 and Aβ1-42 exhibit completely different behaviors. Even focusing only on Aβ25-35, at high concentrations, it causes desensitization, and a consistent VGSC (voltage-gated sodium channel) enhancement activity is not always observed. Therefore, it is essential to identify the type and concentration of Aβ peptides. Aβ1-42 has been reported as a major peptide in the central nervous system (Agarwal et al., 2011; Lue et al., 1999; Mo et al., 2015), and is considered a major cause of disease symptoms such as Alzheimer's disease (AD). Aβ1-42, like MSG, delays the inactivation of VGSC, and this effect becomes more pronounced in a concentration-dependent manner, causing VGSC to remain persistently open.

As a diagnostic marker for Alzheimer's disease, the ratio of Aβ1-40 to Aβ1-42 has been reported. According to this, the level of Aβ1-42 in the CSF of Alzheimer's disease patients is reported to be lower than that of healthy individuals (Agarwal et al., 2011, Lue et al., 1999, Mo et al., 2015). In such cases, Aβ1-40 is thought to shift the VGSC's S-S curve in the hyperpolarizing direction, thereby reducing neuronal activity. It is also clinically known that as AD progresses, the ratio of Aβ1-40/Aβ1-42 in the CSF changes, with Aβ1-40 becoming more prevalent (Lehmann et al., 2020).

Lue et al. (1999) measured the concentrations of soluble and insoluble Aβ1-40 and Aβ1-42 in the entorhinal cortex, a component of the medial temporal lobe memory system where Alzheimer's disease lesions are observed from the early stages, and in the superior frontal gyrus, which occupies about one-third of the lateral surface of the frontal lobe responsible for higher cognitive functions. They compared these concentrations between healthy individuals and those with Alzheimer's-type dementia. The results showed that in the cerebrospinal fluid of Alzheimer's patients, Aβ1-42 was decreased. However, in the entorhinal cortex and superior frontal gyrus, the concentrations of both soluble and insoluble Aβ1-42, similar to Aβ1-40, were increased in the brains of Alzheimer's patients. Notably, soluble Aβ1-42, which was not detected in the entorhinal cortex and superior frontal gyrus of healthy individuals, was detected in these regions in Alzheimer's patients.

The following summarizes the comparison of the concentrations of soluble and insoluble Aβ1-40 and Aβ1-42 measured in the entorhinal cortex and superior frontal gyrus of healthy individuals and AD patients as shown in Lue et al. (1999) in Table 1 below.

[Table 1]

|  |  | Control | AD |
|---|---|---|---|
| CSF Aβ42 (pg/ml) |  | 232±25 | 162±2.9 |
| Entorhinal Cortex |  |  |  |
| Insuoluble | AβB42 (μg/g) | 0.8±0.3 | 53.7±25.9 |
|  | Aβ40 (μg/g) | 8.3±4.7 | 117±18.1 |
| Soluble | Aβ42 (μg/g) | 1.9±0.6 | 66.5±18.7 |
|  | Aβ40 (μg/g) | 0 | 15.5±5.9 |
| Superior frontal Gyrus |  |  |  |
| Insuoluble | Aβ42 (μg/g) | 1.1±0.3 | 105.4±40.2 |
|  | Aβ40 (μg/g) | 11.9±5.6 | 142.1±15.6 |
| Soluble | Aβ42 (μg/g) | 2.5±1.5 | 103.8±18.4 |
|  | Aβ40 (μg/g) | 0 | 6.7±3.7 |
| Lue et al. (1999) |  |  |  |

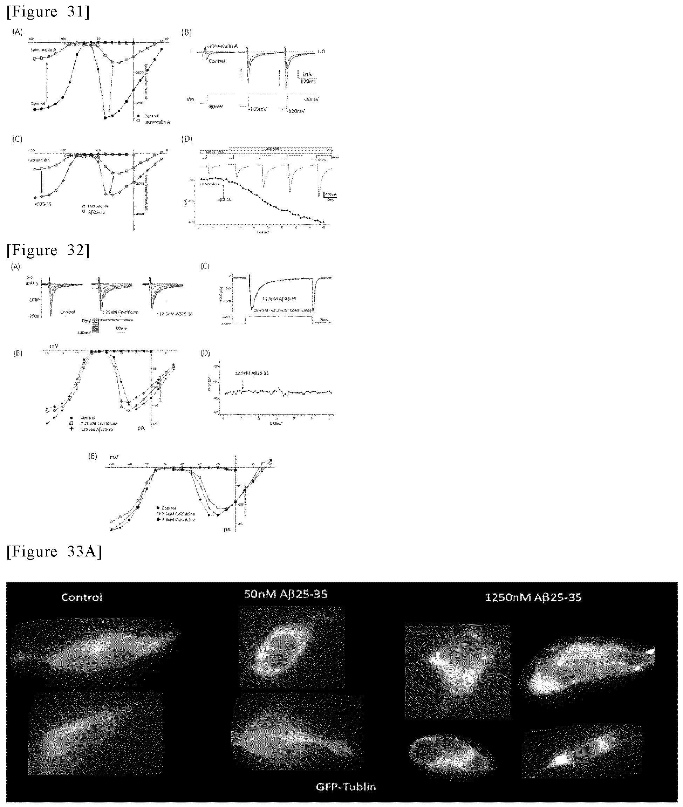

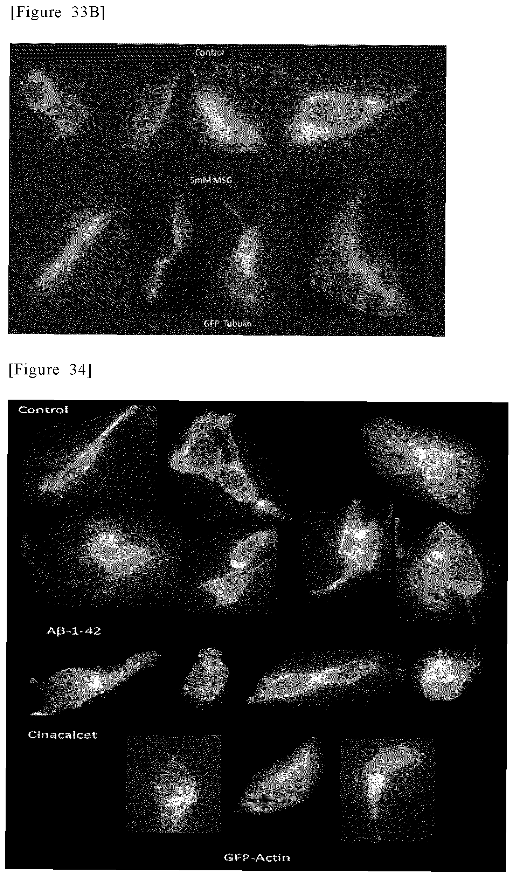

In addition, Aβ1-42 tends to inhibit outward potassium currents, as seen in a series of compounds in 'Group 4,' which may suppress activity not only in neurons but also in cardiomyocytes. In such cases, membrane potential-dependent calcium channels are activated in cardiomyocytes, and the sodium/calcium (Na+/Ca2+) exchange mechanism (NCX) reverses to expel excess intracellular sodium, transporting calcium into the cell, leading to calcium-dependent cell death. Administration of Aβ1-42 causes microtubules to depolymerize (Pianu et al. (2014)) (Figure 33), and Aβ25-35 causes microfilaments to depolymerize (Figure 34). The loss of the cytoskeleton is also thought to contribute to the progression of cell death.

The effect of a solution containing MSG or a mixture of MSG and IMP or GMP on VGSC cannot be confirmed in experiments using cells with silenced T1R1. Therefore, it can be confirmed that the effect is mediated not by the glutamate GPCR receptor (metabotropic glutamate receptor), but by the umami receptor (T1R1/T1R3).

The effect of MSG on slowing the inactivation rate of VGSC via the umami receptor is considered to have significant physiological importance. Specifically, this property of inducing delayed inactivation is crucial for the expression of high-frequency action potentials, burst firing of action potentials, and the integration of multiple synaptic inputs. Therefore, T1R1/T1R3 or T1R1 homodimers are effective targets for drug development when treating neural activity and synaptic integration. Vanderstichele et al. (2016) reported a concentration of Aβ1-40 in the cerebrospinal fluid of Alzheimer's disease (dementia) patients to be 1.9 μM, and Lehmann et al. (2020) also reported a concentration of Aβ1-40 in the cerebrospinal fluid to be 2.85 μM.

According to the report by Mattheisen et al. (2018), the calcium-sensing receptor (CaSR) agonist cinacalcet and the CaSR antagonist NPS-2143 have inhibitory effects on VGSC (voltage-gated sodium channels). It is stated that this inhibitory effect is mediated by an unknown GPCR (G-protein-coupled receptor) different from CaSR. The inventors considered that the VGSC inhibitory effect of cinacalcet might also be mediated by a sweet taste receptor including 'T1R3'. Using HEK-Nav1.5 expressing cells with silenced T1R3, they observed the effects of cinacalcet and NPS-2143 on VGSC, but no VGSC inhibitory effect was observed. This strongly suggests that cinacalcet and NPS-2143 induce VGSC inhibitory effects via a sweet taste receptor including T1R3. Furthermore, experiments with SH-SY5Y cells confirmed that sodium channel currents were inhibited similarly to the experiments with HEK-Nav1.5 expressing cells.

Based on the above, it can be said that the calcium-sensing receptor (CaSR) agonists and antagonists, such as cinacalcet and NPS-2143, also belong to 'Group 3' as they are a type of binding inhibitor of sweet taste receptors, including T1R3.

As mentioned above, excessive brain nerve activity, such as epileptic seizures, has been observed in AD patients (especially in the early stages) through EEG and other methods. One of the main causes of this is reported to be VGSC (voltage-gated sodium channels). For the first time in this invention, it has been elucidated that the inhibition of VGSC (Na+ channels), which are widely expressed in nerve cells, plays an important role in suppressing epileptic-like neural hyperactivity through sweet taste GPCR receptors, particularly 'T1R3'. Furthermore, among the sweet taste inhibitors (lactisole, clofibrate, dichlorprop), dichlorprop was found to be the most potent in inhibiting Na+ channels (neural hyperactivity suppression effect) (dichlorprop >> clofibrate > lactisole).

Furthermore, using a potent T1R3 binding inhibitor, the anti-T1R3 (229-258) antibody (anti-N-terminal T1R3 Venus Flytrap Domain (VFD, 229-258 aa) polyclonal antibody (AP16368a, Abcepta, Inc.)), we observed the effect on the enhancement of VGSC activity by Aβ peptides. As a result, it was observed that in neurons pre-treated with the anti-T1R3 antibody, the enhancement of VGSC activity by Aβ25-35 peptides was suppressed, and the binding of Aβ peptides to neurons was also prevented. Additionally, it was confirmed that the VGSC current enhanced by Aβ25-35 peptides was restored to control levels by the aforementioned T1R3 antibody (Data not shown).

<Various T1R3 antibodies and the Aβ peptide binding sites on the T1R3 protein>

The three antibodies used in this invention, anti-T1R3 (229-258) antibody, anti-T1R3 (303-396) antibody, and anti-T1R3 (400-570) antibody, are polyclonal antibodies made using the 229-258 aa, 303-396 aa, and 400-570 aa regions of the T1R3 protein as antigens, respectively. Experimental results show that the anti-T1R3 (229-258) antibody inhibits the binding of Aβ25-35 peptide, the anti-T1R3 (303-396) antibody inhibits the binding of Aβ1-40 peptide, and the anti-T1R3 (400-570) antibody inhibits the binding of Aβ1-42 peptide to T1R3.

As a result, it was found that the enhancement of VGSC currents mediated by T1R3 caused by each Aβ peptide was suppressed, and the changes in cell morphology and dynamics were improved. This indicates that each of these anti-T1R3 antibodies, or anti-T1R3 antibodies using peptides containing at least eight consecutive amino acids from the amino acid sequences of the peptides used as epitopes for antibody production, act as inhibitors of the binding of each Aβ peptide to neurons and as suppressors of the pathophysiological effects caused by each Aβ peptide in neurons. Therefore, they are also promising as treatments for Alzheimer's disease (AD).

<Inhibitory Mechanism of 'Group 3' T1R3 Binding Inhibitors on VGSC>

The action and inhibitory mechanism of dichlorprop on VGSC is very similar to the inhibitory mechanism of the casein kinase inhibitor TBB (4,5,6,7-tetrabromobenzotriazole), which inhibits casein kinase 2 (CK2). Therefore, it is considered that the inhibitory action of dichlorprop via T1R3 has an inhibitory mechanism similar to that of casein kinase 2.

The inhibition of CK2 by TBB suppresses the phosphorylation of VGSC, which is essential for its binding to Ankyrin G, thereby inhibiting its binding to Ankyrin G. Protein phosphatase also dephosphorylates VGSC in a manner similar to TBB.

It has been reported that the KCNQ2 channel, which binds to Ankyrin G like VGSC, anchors both CK2 and protein phosphatase 1 to the channel itself (Kang et al., 2014). Furthermore, Pradhan et al. (2017) reported that Gβ1 binds to the catalytic subunit of protein phosphatase 1 in platelets. From this, it is possible that the delayed inactivation of VGSC by the umami receptor T1R (T1R GPCR) occurs through a mechanism that regulates the degree of phosphorylation at the Ankyrin G binding site of VGSC via Gβ.

As mentioned earlier, when the umami substances well-known for their effects on the umami receptor (T1R1/T1R3) such as MSG (monosodium glutamate), IMP (inosine monophosphate), GMP (guanosine monophosphate), and MSG2.5 (a mixture containing 97.5% MSG and 1.25% IMP, GMP as found in "Ajinomoto") are applied, the sodium channel current of VGSC is activated, contrary to the sweet substances acting on the sweet receptor (T1R2/T1R3). However, when MSG is further administered to VGSC, which has been inhibited by more than 80% due to the action of dichlorprop, it was observed that the inactivation curve of VGSC, which had shifted in the negative direction due to the action of dichlorprop, partially recovered. This indicates that MSG, acting through T1R1, inhibits VGSC via a different pathway than dichlorprop, which acts through T1R3.

<On the Regulatory Mechanism of T1R3 Binding Inhibitors on VGSC Activity>

Paclitaxel (Taxol), which stabilizes microtubules, is used as an anticancer drug for breast cancer treatment. However, it is known to cause neuropathic pain as a side effect. This pain is strongly suggested to be due to nerve overactivity, so the presence of VGSC enhancement by Paclitaxel (Taxol) was investigated. As a result, it was discovered that VGSC enhancement occurs even when Paclitaxel (Taxol) is applied to VGSC-expressing cells.

This suggests that neuropathic pain, which occurs as a side effect of the anticancer drug Paclitaxel (Taxol), is also due to VGSC enhancement, similar to nerve overactivity. Therefore, it is expected that VGSC inhibitors mediated by T1R3 will have analgesic effects on neuropathic pain. In other words, T1R3 binding inhibitors such as dichlorprop are suggested to be effective in treating neuropathic pain.

<Consideration of the Regulatory Mechanism of T1R3 Binding Inhibitors on VGSC Activity>

Based on the above, the regulatory mechanism of T1R3 binding inhibitors on VGSC activity can be considered as follows. However, the regulatory mechanism of T1R3 binding inhibitors on VGSC activity in this invention is not limited to the restriction mechanisms considered below.

When the sweet taste receptor (T1R2/T1R3) binds and is activated by a ligand, it modifies the cytoskeletal structure, and this structural change modifies the activity of VGSC. It is known that VGSC binds to the cytoskeleton via ankyrin G and spectrin. Specifically, the binding of ankyrin G and VGSC requires the phosphorylation of the ankyrin binding site, which is preserved in many voltage-dependent sodium channels in the intracellular loop between domains II and III of VGSC, by CK2 (Casein kinase 2) (Brechet et al. (2008), Non-Patent Literature 9). Specifically, the cytoskeleton binds to VGSC via ankyrin G/spectrin, and the binding of ankyrin G and VGSC is necessary for the localization of VGSC to the cell membrane surface. This binding of ankyrin G and VGSC plays an important role in the dense expression of VGSC at the axon initial segment of neurons and the nodes of Ranvier.

At that time, it was reported that knocking out ankyrin-G reduced the magnitude of VGSC currents (Lowe et al. (2008)), and this was attributed to a decrease in the expression density of VGSCs. However, in the present invention, it was found that activation of T1R2/T1R3 does not reduce the number of VGSCs themselves, but rather inactivates VGSCs. In other words, while the report on ankyrin-G knockout concluded that the reduction in VGSC currents was due to a decrease in VGSC expression density, the control mechanism by T1R2/T1R3 receptors was found to control VGSC currents not only by the number of VGSCs on the cell membrane surface but also by inactivating VGSCs, thereby reducing the number of active VGSCs. In the present invention, it was discovered that dephosphorylation of VGSCs by CK2 inhibitors (such as TBB) not only affects the extent of VGSC expression on the cell membrane but also acts on the properties of VGSCs functioning on the cell membrane, exerting a functional inhibitory effect.

In VGSC, the binding site with ankyrin G has been identified, and it is known that the phosphorylation by casein kinase 2 (CK2) at this site controls the expression level of VGSC in the cell membrane. By administering TBB, an inhibitor of CK2, and conducting experiments, it was discovered that the inhibition of CK2 by TBB suppresses VGSC. Thus, it was found that CK2 inhibitors also suppress VGSC, indicating their analgesic effect. Considering the enhancing effect of paclitaxel (Taxol), a microtubule stabilizer that constitutes the cytoskeleton, on VGSC, and the regulation of VGSC activity by CK2 inhibition, which controls the binding strength between ankyrin G and VGSC, it was found that taste receptors control VGSC through the cytoskeleton (mainly microtubule-ankyrin G). From these findings, the mechanism by which T1R3 binding inhibitors enhance or suppress VGSC was also confirmed.

As mentioned above, it has been confirmed that compounds binding to sweet and umami GPCR receptors (T1R3, T1R2, and T1R1), including T1R3 binding inhibitors, are all substances involved in the regulation (inhibition or enhancement) of VGSC activity via sweet and umami GPCR receptors. Table 2 shows these compounds, categorized by their agonist-like and antagonist-like effects on the target T1Rs and VGSC currents.

[Table 2]

| Target T1Rs | | Compound Group | Group | Effects on VGSCs |
|---|---|---|---|---|
| Umami taste | T1R1 | L-Gln, L-Glu (MSG), Aβ42 peptide | Group 4 | Delay in VGSC inactivation Inhibition of Voltage gated K+ channel (Kv1.2) |
| | | Mono Sodium-Glutamic acid (MSG) Nucleic acid seasoning (IMP, MSG2.5) | | Transient enhancement of VGSC Left shift of the I-V curve |
| | | Nucleic acid seasoning (GMP) | | Enhancement of VGSC |
| | T1R2 | Sucrose, Artificail sweeteners (Aspartate, Saccharin) Gymnema Tea extract | Group 3 | Inhibition of VGSCs |

(continued)

| Target T1Rs | | Compound Group | Group | Effects on VGSCs |
|---|---|---|---|---|
| Sweet taste | T1R2/T1R3 | Saccharides (Glucose) <10mM | Group 1 | Enhancement of VGSC |
| | | Sweet amino acids (L-Gln, L-Serin) | | |
| | | Aβ25-35 peptides | Group 2 | Enhancement of VGSC |
| | | | | Enhancement of VGSC by Ab25-35 is suppressed at high concentration by desensitization |
| | | Glucose (>20mM), Sucrose Artifical sweetener (Scuralose) | Group 3 | Inhibition of VGSCs |
| | T1R3 | DichrolProp, Clofibrate, Lactisole | | |
| | | Cinacalcet, NPS-2143 | | |
| | | Anit T1R3 Antibodies | | |

In these compounds [Table 2], particularly dichlorprop, an inhibitor of the T1R3 sweet taste receptor, and anti-T1R3 antibodies, it was confirmed that they can prevent the binding of Aβ peptides to neurons in the brains of AD patients, thereby preventing neuronal hyperexcitation via VGSC. This suggests potential therapeutic and preventive effects for AD, as well as potential efficacy as a treatment for neuropathic pain, leading to the completion of this invention.

Additionally, artificial sweeteners such as aspartame, which bind to T1R2 or T1R2/T1R3 and inhibit VGSC currents, are also expected to suppress neuronal hyperexcitation, suggesting similar therapeutic effects.

Furthermore, while the main binding site of the Aβ1-42 peptide is on T1R3, it also partially acts on T1R1. Therefore, it was confirmed that glutamic acid, monosodium glutamate, and nucleotide seasonings, which bind to T1R1, could potentially be used as therapeutic agents or for screening by utilizing the inhibitory activity on the VGSC action mediated by T1R1.

< Effects of Artificial Sweetener Aspartame on Cell Morphology >

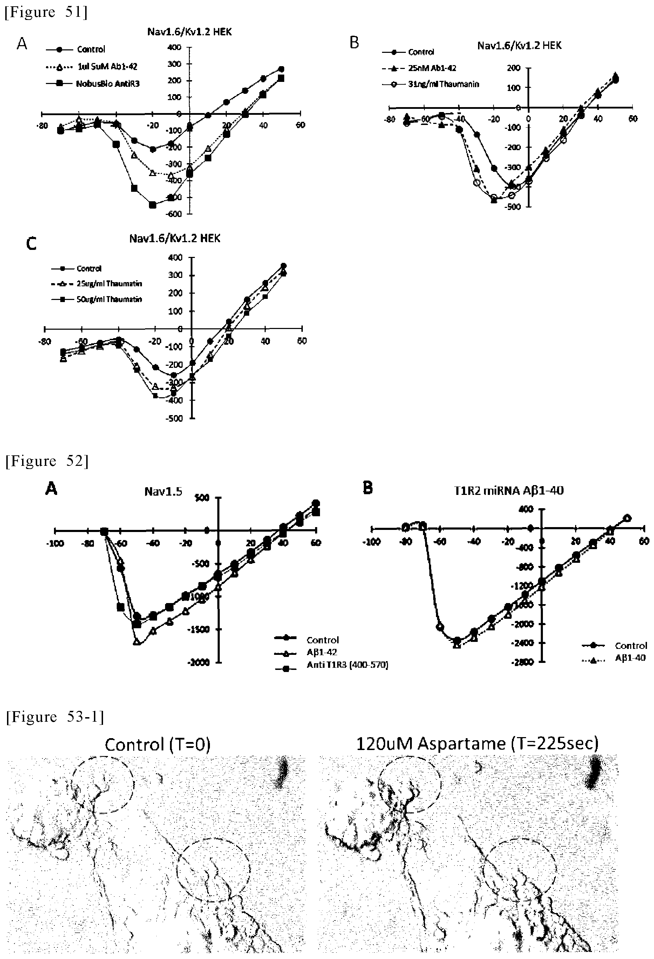

We investigated the effects of the artificial sweetener aspartame, an agonist of T1R2/T1R3, on cell morphology (Figure 53-1). Similar to the example of MSG stimulation shown in (Figure 33B), not only Aβ peptides but also taste substances control the polymerization state of cytoskeletal fibers. In this implementation example, undifferentiated SH-SY5Y cells were treated with 120 μM aspartame, and changes in cell morphology were observed. As a result, it was observed that stimulation of T1R2/T1R3 by the artificial sweetener affected and contracted cell morphology. Considering this alongside the results of patch-clamp experiments, it is suggested that umami and sweet substances control cell morphology through T1R receptors.

< Effects of Aβ42/Aβ40 Concentration Ratio on Cell Morphology >

Aβ1-42 and Aβ1-40 are both present in cerebrospinal fluid or blood, and it is known that the concentration ratio of Aβ42/Aβ40 differs depending on the presence or absence of cognitive impairment in Alzheimer's disease. A correlation has been observed between a ratio of Aβ1-42 below 10% and the presence of cognitive impairment.

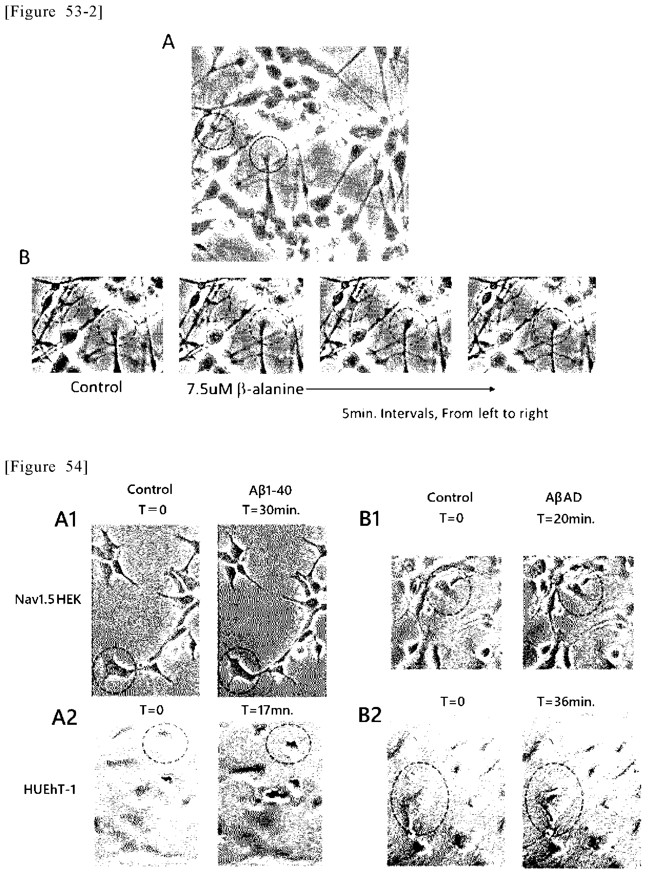

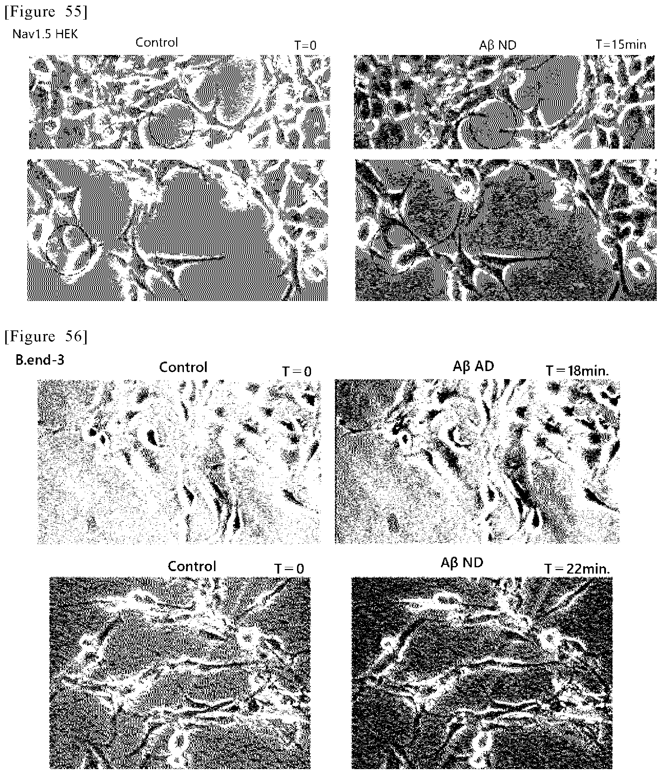

In Alzheimer's disease with cognitive impairment (AD-D), cells contract, and the motility of neurites is impaired and regresses. On the other hand, when the Aβ42/Aβ40 ratio is high and the concentration of Aβ1-42 is high, no contraction is observed except for physiological morphological changes, and ruffling movements are observed at the cell periphery (Figure 55). Similar ruffling-like changes in neurites (lamellipodia) were observed with β-alanine (Figure 53-2).

In other words, in healthy individuals with a normal Aβ42/Aβ40 ratio, cells are activated and do not suffer from contraction-related issues, maintaining cell junctions (capillaries, blood-brain barrier, renal glomerular membrane) and enhancing the motility of cell processes, thereby preserving or improving neuronal plasticity. Conversely, in Alzheimer's disease with cognitive impairment and a low Aβ42/Aβ40 ratio, cells contract, weakening cell junctions and leading to fundamental changes in many diseases, including chronic nephritis. Similarly, in neurons, cell body and neurite contraction, along with reduced neurite motility, decrease neuronal plasticity.

Thus, the present invention includes the following embodiments.

(1) One embodiment of the present invention relates to the use of Type I taste receptors present on the cell surface (particularly on the surface of nerve cells, cardiomyocytes, or skeletal muscle cells) as target receptors for controlling the active currents of voltage-gated ion channels (e.g., voltage-gated sodium channels (VGSC), voltage-gated potassium channels (VGKC), or voltage-gated calcium channels (VGCC)). This is described as a method invention as follows.

The target cells here are human-derived cells or cells expressing human-type Type I taste receptors (e.g., primate-derived cells or humanized experimental animal-derived cells) that require control of the active currents of voltage-gated ion channels on the cell membrane surface.

In addition to typical nerve cells and cardiomyocytes, all cells that fire VGSC-dependent action potentials are targeted. Particularly, skeletal muscle cells have been found to have more T1R3 RNA messages than nerves and cardiomyocytes. The normalized transcripts per million (nTPM) for RNA expression frequency averaged across samples are as follows: skeletal muscle cells 1.1, cardiomyocytes 0.9, neurons (cerebellum 0.9, cerebral cortex 0.7, hippocampus 0.4), and kidney 0.4. Additionally, the presence of $A\beta$ peptide in skeletal muscle cells has been confirmed (Kuo et al. 2000), and muscle weakness and disuse atrophy in diabetes have been reported (Perry et al. 2016). Therefore, it is considered that Type I taste receptors are present in skeletal muscle-type VGSC (Nav 1.4), similar to nerves and cardiomyocytes. For these reasons, they are listed alongside nerve cells and cardiomyocytes.

Other cells that fire VGSC-dependent action potentials, such as neurosecretory cells and $\beta$ cells of the islets of Langerhans, are also considered to be applicable to the technology of this invention.

This invention includes newly added measurement methods (including assays using cell morphology/motility, impedance assays, and assays using changes in intracellular calcium concentration as indicators). Additionally, it is important to use humanized mice (experimental animals) in research on many diseases, such as Alzheimer's disease and chronic nephritis, due to species differences. Therefore, the commercial use of T1R genes in humanized mice, the binding region of $A\beta$ peptide, and the commercial use of the extracellular region of T1R2 (targeted drug discovery) are also included.

Specifically, since T1R2/T1R3 GPCR proteins are $A\beta$ peptide-binding proteins, drugs that can inhibit the binding of $A\beta$ peptides to T1R2/T1R3 GPCR proteins are highly effective in treating $A\beta$ peptide-related diseases (such as Alzheimer's disease and chronic nephritis). Therefore, in drug discovery aimed at treating the above-mentioned diseases, T1R2/T1R3 GPCR proteins, which have $A\beta$ peptide binding sites, are important drug discovery targets. Furthermore, the regions of the amino acid sequence of the VFD region where $A\beta25-35$ binds (229-258, LB2), the region where $A\beta1-40$ binds (303-396, LB1), and the region that inhibits the action of VGSC currents of $A\beta1-42$ (400-570) are important drug discovery targets. Thus, in addition to T1R2/T1R3 GPCR proteins, the T1R3 sites indicated by the above amino acid sequences are included as drug discovery targets.

Regarding the enhancing effect of $A\beta40$ mixed peptide on VGSC currents in human and mouse cells, and its significantly different effects on cell morphology, future research must use proteins derived from human genes T1R1, T1R2, and T1R3. Otherwise, experiments using mice will not yield research results applicable to human clinical applications or the development of clinically applicable therapeutic drugs. Therefore, it is necessary to replace the T1R GPCR genes in the host mouse with human-derived genes. This includes the use of humanized mice with T1R genes for drug discovery research and development by commercial enterprises.

When conducting drug discovery screening at the cellular level, it has been shown that methods using changes in intracellular calcium concentration as an indicator, and methods observing changes in cell sheet impedance, are effective for primary screening. Therefore, these screening methods are included.

Depending on the type of $A\beta$ peptide, particularly the $A\beta42/A\beta40$ ratio in Alzheimer's disease patients compared to healthy individuals, significant changes occur in cell morphology/dynamics, especially when observed using neurons, affecting the morphology and motility of neurites. This phenomenon can be easily measured under a microscope, making it effective for screening drugs that restore the activity of neurites inactivated by the $A\beta42/A\beta40$ ratio in Alzheimer's disease patients, such as $\beta$-alanine, or for functional diagnosis using patient CSF and serum. Therefore, this measurement method is included.

[1-1] A method for controlling the activation current of voltage-dependent ion channels in nerve cells, cardiac muscle cells, or skeletal muscle cells, by acting on a specific Type I taste receptor present on the cell surface with a ligand that specifically binds to the Type I taste receptor and exerts an antagonist-like or agonist effect.

[1-2] The method described in [1-1], wherein the voltage-dependent ion channel is a voltage-gated sodium channel (VGSC), a voltage-gated potassium channel (VGKC), or a voltage-gated calcium channel (VGCC).

[1-3] The method described in [1-1] or [1-2], wherein the Type I taste receptor is a sweet taste receptor or umami taste receptor, and is T1R1, T1R2, or T1R3, or a homodimer or heterodimer thereof.

[1-4] The method according to [1-3], wherein the ligand specifically binds to T1R2 and/or T1R3 to suppress the active current of VGSC.

[1-5] The method according to [1-4], wherein the ligand specifically binds to T1R3 to exert an antagonist-like effect to suppress the active current of VGSC or to suppress the active current of VGCC, and the ligand is selected from anti-human T1R3 antibody, lactisole, clofibrate, and dichlorprop.

Here, the anti-human T1R3 antibody typically includes the following three types of antibodies:

1. A monoclonal or polyclonal antibody that specifically binds to an epitope containing at least a continuous 3-5 amino acid sequence, preferably 5-8 amino acid sequence, more preferably 8-10 amino acid sequence, even more preferably 11-13 amino acid sequence, and most preferably 13-15 amino acid sequence in the 229-258 aa region of human T1R3 protein, and inhibits the binding of Aβ25-35 peptide to human T1R3, thereby suppressing the active current of VGSC amplified by Aβ25-35 peptide.

2. A monoclonal or polyclonal antibody that specifically binds to an epitope containing at least a continuous 3-5 amino acid sequence, preferably a 5-8 amino acid sequence, more preferably an 8-10 amino acid sequence, even more preferably an 11-13 amino acid sequence, and most preferably a 13-15 amino acid sequence in the 303-396 aa region of the human T1R3 protein, which inhibits the binding of Aβ1-40 peptide to human T1R3 and suppresses the VGSC active current amplified by Aβ1-40 peptide.

3. A monoclonal or polyclonal antibody that specifically binds to an epitope containing at least a continuous 3-5 amino acid sequence, preferably a 5-8 amino acid sequence, more preferably an 8-10 amino acid sequence, even more preferably an 11-13 amino acid sequence, and most preferably a 13-15 amino acid sequence in the 400-570 aa region of the human T1R3 protein, which inhibits the binding of Aβ1-42 peptide to human T1R3 and suppresses the VGSC active current amplified by Aβ1-42 peptide.

[1-6] The method according to [1-4], wherein the ligand specifically binds to T1R2 or T1R2/T1R3 and exerts an antagonist-like effect to inhibit the active current of VGSC or VGCC, and is selected from artificial sweeteners such as saccharin, aspartame, sucralose, and structural analogs such as Gymnema tea extract.

[1-7] The method according to [1-3], wherein the ligand specifically binds to T1R3, T1R2/T1R3, or T1R1/T1R3 and exerts an agonist-like effect to amplify the active current of VGSC.

[1-8] The method according to [1-7], wherein the ligand is a sugar or a sweet-tasting amino acid.

[1-9] glutamine, serine, and mixtures thereof.

[1-10] The method according to [1-3], wherein the ligand specifically binds to T1R1 and exerts an agonist-like or antagonist-like effect to control the active current of VGSC and/or VGKC within cells.

[1-11] The method according to [1-10], wherein the ligand is selected from glutamic acid (L-Glu), monosodium glutamate (MSG), inosine monophosphate, guanosine monophosphate, and mixtures thereof.

[2] Another embodiment of the present invention is the invention of the "ligand (substance)" itself used in the invention of the "method" described above. The invention regarding the "ligand substance" is described as follows.

[2-1] A ligand substance for controlling the activation current of voltage-dependent ion channels in nerve cells, cardiomyocytes, or skeletal muscle cells, characterized by specifically binding to Type I taste receptors present on the cell surface and exerting agonist-like or antagonist-like effects as a Type I taste receptor ligand.

[2-2] The voltage-dependent ion channel is a membrane potential-dependent sodium channel (VGSC), membrane potential-dependent potassium channel (VGKC), or membrane potential-dependent calcium channel (VGCC), and the Type I taste receptor is a sweet taste receptor or umami taste receptor, which is T1R1, T1R2, or T1R3, or their homodimers or heterodimers, as described in (2-1)

[2-3] The ligand described in [2-2], characterized by specifically binding to T1R2 and/or T1R3 to inhibit the activation current of VGSC.

[2-4] The ligand described in [2-3], characterized by specifically binding to T1R3 and exerting antagonist-like effects to inhibit the activation current of VGSC, selected from anti-human T1R3 antibody, lactisole, clofibrate, and dichlorprop.

[2-5] The ligand is selected from saccharin, aspartame, sucralose, and gymnema tea extract, as well as their structural analogs, and is a T1R2 ligand or T1R2/T1R3 ligand that specifically binds to T1R2 or T1R2/T1R3 and exerts an antagonist-like effect to suppress the active current of VGSC, as described in (2-3).

[2-6] The ligand is a ligand substance described in [2-2], which specifically binds to T1R3, T1R2/T1R3, or T1R1/T1R3 and exerts an agonist-like effect to amplify the active current of VGSC or VGCC.

[2-7] The ligand is a ligand substance described in [2-6], which is a sugar or a sweet-tasting amino acid.

[2-8] The ligand is a ligand substance described in [2-7], selected from glucose, sucrose, fructose, glutamine, serine, and mixtures thereof.

[2-9] The ligand is a T1R1 ligand described in [2-2], which specifically binds to T1R1 or T1R1/T1R3 and exerts an agonist-like effect to shift the I-V curve of VGSC active current to the left or delay inactivation, or to suppress VGKC active current.

[2-10] The T1R1 or T1R1/T1R3 ligand is a ligand substance described in [2-9], selected from glutamic acid (L-Glu), monosodium glutamate (MSG), inosine monophosphate (IMP), guanosine monophosphate (GMP), and mixtures thereof.

[3] The present invention relates to a pharmaceutical composition for the prevention or treatment of various diseases caused by the amplification of VGSC active currents and/or the suppression of VGKC active currents, which occurs when sweet-tasting amino acids, which are ligands (agonists) of T1R2/T1R3, or umami substances, which are ligands (agonists) of T1R1/T1R3, bind to T1R2/T1R3 or T1R1/T1R3 present on the cell surface (particularly on the surface of nerve cells, cardiomyocytes, or skeletal muscle cells). Specifically, it targets neurological diseases associated with the overactivity of nerve cells caused by the amplification of VGSC active currents due to the binding of sweet-tasting amino acids such as glutamine and serine, and umami substances such as glutamic acid and its sodium salt (MSG), as well as nucleic acid seasonings such as IMP and GMP, which have been confirmed to be present in CSF.

[3-1] A pharmaceutical composition for the prevention and/or treatment of diseases caused by the amplification of VGSC currents and/or the suppression of VGKC currents, which occurs when sweet-tasting amino acids or umami substances bind to T1R2/T1R3 or T1R1/T1R3 on the surface of nerve cells, cardiomyocytes, or skeletal muscle cells, comprising a ligand that specifically binds to T1R2 or T1R3, or their homodimers or heterodimers, and exerts an antagonist-like effect as an active ingredient.

[3-2] The pharmaceutical composition according to [3-1], wherein the disease is a neurological disease and/or neuropathic pain condition in which an epilepsy-like state of neuronal hyperactivity is observed.

[3-3] The pharmaceutical composition according to [3-2], wherein the neurological disease in which an epilepsy-like state of neuronal hyperactivity is observed is selected from the group consisting of Alzheimer's disease (AD), amyotrophic lateral sclerosis (ALS), Lewy body dementia, focal neurological symptoms of cerebrovascular disorders, epilepsy, Parkinson's disease, progressive supranuclear palsy, multiple system atrophy, focal neurological symptoms of areas damaged by traumatic brain injury, Huntington's disease, spinocerebellar degeneration, and multiple sclerosis.

[3-4] The pharmaceutical composition according to [3-2], wherein the neuropathic pain condition is neuropathic pain associated with neuronal hyperactivity based on the amplification of VGSC currents, selected from the group consisting of peripheral neuropathy caused by anticancer drug administration, failed back surgery syndrome (FBSS), complex regional pain syndrome (CRPS), and diabetic polyneuropathy.

[3-5] The pharmaceutical composition according to [3-1] or [3-2], wherein the ligand specifically binds to T1R3 and exerts an antagonist-like effect, selected from the group consisting of anti-human T1R3 antibodies, cinacalcet, NPS-2143, lactisole, clofibrate, and dichloprop, as well as their structural analogs.

Here, the candidate anti-human T1R3 antibodies for the pharmaceutical composition typically include the following three types of antibodies:

(1) A monoclonal or polyclonal antibody that specifically binds to an epitope containing at least a continuous 3-5 amino acid sequence, preferably a 5-8 amino acid sequence, more preferably an 8-10 amino acid sequence, even more preferably an 11-13 amino acid sequence, and most preferably a 13-15 amino acid sequence in the 229-258 aa region of the human T1R3 protein, and inhibits the binding of sweet-tasting amino acids or umami substances to human T1R3, thereby suppressing the VGSC active currents amplified by sweet-tasting amino acids or umami substances.

(2) A monoclonal or polyclonal antibody that specifically binds to an epitope containing at least a continuous 3-5 amino acid sequence, preferably a 5-8 amino acid sequence, more preferably an 8-10 amino acid sequence, even more preferably an 11-13 amino acid sequence, and most preferably a 13-15 amino acid sequence in the 303-396 aa region of the human T1R3 protein, and inhibits the binding of sweet-tasting amino acids or umami substances to human T1R3, thereby suppressing the VGSC active currents amplified by sweet-tasting amino acids or umami substances.

(3) A monoclonal or polyclonal antibody that specifically binds to an epitope containing at least a continuous 3-5 amino acid sequence, preferably a 5-8 amino acid sequence, more preferably an 8-10 amino acid sequence, even more preferably an 11-13 amino acid sequence, and most preferably a 13-15 amino acid sequence in the 400-570 aa region of the human T1R3 protein, and inhibits the binding of sweet-tasting amino acids or umami substances to human T1R3, thereby suppressing the VGSC active currents amplified by sweet-tasting amino acids or umami substances.

[3-6] The pharmaceutical composition according to [3-1] or [3-2], wherein the ligand specifically binds to T1R2 or T1R2/T1R3 and exerts an antagonist-like effect, selected from the group consisting of saccharin, aspartame, sucralose, and Gymnema tea extract, as well as their structural analogs.

[3-5] Another aspect of the present invention relates to a pharmaceutical composition for the prevention or treatment of various diseases caused by the amplification of VGSC active currents and/or the inhibition of VGKC active currents, which occurs when Aβ peptides, ligands (agonists) of Type I taste receptors, bind to Type I taste receptors (particularly T1R2/T1R3 or T1R3) present on the cell surface (especially on the surface of nerve cells, cardiomyocytes, or skeletal muscle cells). The shortest Aβ peptide is Aβ25-35 peptide (Sequence No. 1), and it includes Aβ1-38 (Sequence No. 2), Aβ1-40 (Sequence No. 3), and the typical Aβ1-42 (Sequence No. 4), which extend the amino acid sequence of Aβ25-35. In other words, the Aβ peptide of the present invention can also be expressed as "Aβ peptide containing the amino acid sequence shown in 'Sequence No. 1'." In particular, the pharmaceutical composition of the present invention is for the prevention or treatment of neurological diseases characterized by the hyperactivity of nerve cells, which is caused by the amplification of VGSC active currents when Aβ25-35 peptide, a ligand (agonist) of T1R2/T1R3, binds to T1R2/T1R3 present on the surface of nerve cells.

[4-1] A pharmaceutical composition for the treatment and/or prevention of diseases caused by the hyperexcitation of nerve cells, which occurs when Aβ peptides containing the amino acid sequence shown in "Sequence Number 1" bind to Type I taste receptors on the surface of nerve cells. The composition contains a ligand that specifically binds to T1R1, T1R2, or T1R3, or their homodimers or heterodimers, and acts as an antagonist.

[4-2] A pharmaceutical composition for the treatment and/or prevention of diseases caused by the hyperexcitation of nerve cells mediated by T1R2/T1R3 due to Aβ peptides selected from Aβ25-35 peptide, Aβ1-38 peptide, and Aβ1-40 peptide. The composition contains a ligand that specifically binds to T1R2 or T1R3, or their homodimers or heterodimers, and acts as an antagonist.

[4-3] The pharmaceutical composition according to [4-1] or [4-2], wherein the disease is a neurological disease and/or neuropathic pain condition characterized by epilepsy-like neural hyperactivity.

[4-4] The pharmaceutical composition according to [4-3], wherein the neurological disease characterized by epilepsy-like neural hyperactivity is selected from the group consisting of Alzheimer's disease (AD), amyotrophic lateral sclerosis (ALS), Lewy body dementia, focal neurological symptoms of cerebrovascular disorders, epilepsy, Parkinson's disease, progressive supranuclear palsy, multiple system atrophy, focal neurological symptoms of brain regions damaged by traumatic brain injury, Huntington's disease, spinocerebellar degeneration, and multiple sclerosis.

[4-5] The neuropathic pain is a neuropathic pain associated with nerve hyperactivity based on the amplification of VGSC currents, and is selected from the group consisting of peripheral neuropathy caused by anticancer drug administration, failed back surgery syndrome (FBSS), complex regional pain syndrome (CRPS), and diabetic polyneuropathy. The pharmaceutical composition according to [4-3].

[4-6] The ligand specifically binds to T1R3 and acts as an antagonist, and is selected from the group consisting of anti-human T1R3 antibody, cinacalcet, NPS-2143, lactisole, clofibrate, and dichloprop, as well as structural analogs thereof. The pharmaceutical composition according to [4-1] or [4-2].

Here, the anti-human T1R3 antibody, which is a candidate for the pharmaceutical composition, typically includes the following three types of antibodies:

(1) A monoclonal or polyclonal antibody that specifically binds to an epitope containing at least a continuous 3-5 amino acid sequence, preferably 5-8 amino acid sequence, more preferably 8-10 amino acid sequence, even more preferably 11-13 amino acid sequence, and most preferably 13-15 amino acid sequence in the 229-258 aa region of the human T1R3 protein, and inhibits the binding of Aβ25-35 peptide to human T1R3 and suppresses the VGSC active current amplified by Aβ25-35 peptide.

(2) A monoclonal or polyclonal antibody that specifically binds to an epitope containing at least a continuous 3-5 amino acid sequence, preferably 5-8 amino acid sequence, more preferably 8-10 amino acid sequence, even more preferably 11-13 amino acid sequence, and most preferably 13-15 amino acid sequence in the 303-396 aa region of the human T1R3 protein, and inhibits the binding of Aβ1-40 peptide to human T1R3 and suppresses the VGSC active current amplified by Aβ1-40 peptide.

(3) A monoclonal or polyclonal antibody that specifically binds to an epitope containing at least a continuous 3-5 amino acid sequence, preferably 5-8 amino acid sequence, more preferably 8-10 amino acid sequence, even more preferably 11-13 amino acid sequence, and most preferably 13-15 amino acid sequence in the 400-570 aa region of the human T1R3 protein, and inhibits the binding of Aβ1-42 peptide to human T1R3 and suppresses the VGSC active current amplified by Aβ1-42 peptide.

[4-7] The ligand specifically binds to T1R2 or T1R2/T1R3 and acts as an antagonist, and is selected from the group consisting of saccharin, aspartame, sucralose, and gymnema tea extract, as well as structural analogs thereof. The pharmaceutical composition according to [4-1] or [4-2].

[5] In another embodiment of the present invention, a screening method is provided for substances capable of controlling the amplification or inhibition of voltage-dependent ion channel activation currents mediated through Type I taste receptors on the surface of human neurons, cardiomyocytes, or skeletal muscle cells. Here, neurons, cardiomyocytes, or skeletal muscle cells express voltage-dependent ion channels along with Type I taste receptors on their cell surface, with voltage-dependent sodium channels (VGSC), voltage-dependent potassium channels (VGKC), or voltage-gated calcium channels (VGCC) being typical.

[5-1] In another embodiment of the present invention, a screening method is provided for substances Method for Screening Substances Controlling Amplification or Inhibition of Voltage-Dependent Ion Channel Currents Mediated by Type I Taste Receptors on the Surface of Neurons, Cardiomyocytes, or Skeletal Muscle Cells:

(1) Cultivate human-derived cells expressing voltage-dependent ion channels on the cell surface along with Type I taste receptors.

(2) Administer a fluorescently labeled known Type I taste receptor ligand to the culture medium of these cells.

(3) Simultaneously or sequentially, expose the culture medium to the test substance with or without administration. Compare the fluorescence intensity of the cultured cell surface when the test substance is administered to when it is not. If a significant increase or decrease is observed or measured, select the test substance as a candidate for controlling voltage-dependent ion channel currents on the surface of neurons, cardiomyocytes, or skeletal muscle cells.

[5-2] The method described in [5-1] is characterized by meeting the following conditions: The control of amplification or inhibition of the activation current of the aforementioned voltage-gated ion channel, which involves the amplification of VGSC current and/or inhibition of VGKC current, where the Type I taste receptor includes T1R1, T1R2, or T1R3, or their homodimers or heterodimers. Additionally, the human-derived cultured cells used in step (1) are cultured human nerve cells, cultured human cardiomyocytes, or human-derived cultured cells forcibly expressing VGSC and/or VGKC. The human-derived cultured cells that forcibly express VGSC and/or VGKC refer to cells with endogenous Type I taste receptors, such as human HEK cells. The same applies hereafter

[5-3] In the method described in [5-1], the Type I taste receptor is composed of T1R2/T1R3, and the cultured human-derived cells used in step (1) are either cultured human neuronal cells or human-derived cultured cells in which VGSC (voltage-gated sodium channels) are forcibly expressed. Additionally, the known Type I taste receptor ligand administered to the culture medium in step (1) is the Aβ25-35 peptide.

[5-4] A screening method for substances capable of controlling the amplification of VGSC current and/or inhibition of VGKC current, which is caused by the binding of Aβ peptide or sweet amino acids or umami substances to Type I taste receptors on the surface of human nerve cells, cardiomyocytes, or skeletal muscle cells, including the following steps (1) to (4):

(1) Using the patch-clamp technique (voltage clamping), measure VGSC and/or VGKC currents in cultured human neuronal cells, cultured human cardiac muscle cells, or human-derived cultured cells in which VGSC and/or VGKC are forcibly expressed. Establish control values.

(2) Administer Aβ peptides, sweet amino acids, or umami substances to the cell culture medium and measure VGSC and/or VGKC currents using the same method as in step 1. Measure the amplification of VGSC currents and/or the inhibition of VGKC currents resulting from the binding of these substances to Type I taste receptors.

(3) Subsequently, administer the test substance to the cell culture medium and measure VGSC and/or VGKC currents using the same method as in step (1).

(4) Evaluate the test substance as a controller of VGSC current amplification and/or VGKC current inhibition mediated by Type I taste receptors for Aβ peptides, sweet amino acids, or umami substances in human neuronal cells or cardiac muscle cells if the measured VGSC and/or VGKC currents in step 3 significantly inhibit either or both the amplification of VGSC currents measured in step (2) or the inhibition of VGKC currents.

[5-5] In a method for screening substances capable of controlling the properties of action potentials resulting from the amplification of voltage-gated sodium channel (VGSC) currents and/or inhibition of voltage-gated potassium channel (VGKC) currents, which occur due to the binding of Aβ peptides or sweet-tasting amino acids or umami substances to Type I taste receptors on human neurons, cardiomyocytes, or skeletal muscle cells, the method includes the following (1)-(4):

(1) Using patch-clamp technique (current clamp mode), NanoTouch method, or MEA (multi-electrode array) method, measure and/or record spontaneous action potentials from cultured human neurons, cultured human cardiomyocytes, or human-derived cultured cells expressing VGSC and/or VGKC on their cell surfaces, establishing control values.

(2) Administer Aβ peptides or sweet-tasting amino acids or umami substances to the culture medium of the cells and measure and/or record induced action potentials using the same methods as in step (1).

(3) Next, administer the test substance to the culture medium from step (2) and measure and/or record the action potentials induced by the test substance using the same methods as in step (1).

(4) If the measured firing rate of action potentials or waveform shape of action potentials in step (3) shows significant changes compared to those measured in step (2), the test substance is evaluated as a candidate for controlling the amplification of voltage-gated sodium channel (VGSC) currents and/or inhibition of voltage-gated potassium channel (VGKC) currents via Type I taste receptors for Aβ peptides, sweet-tasting amino acids, or umami substances in human neurons or cardiomyocytes.

[5-6] A screening method for substances that can control the modification of the cytoskeleton in Type I taste receptors of human neurons, caused by the binding of Aβ peptides or sweet amino acids or umami substances, comprising the following steps (1) to (4):

(1) Culturing human neurons or Type I taste receptors expressed on the cell surface of human-derived cultured cells in a culture medium containing a fluorescent reagent. The intracellular calcium concentration is measured using a fluorescence reagent and control values are established.

(2) Administering Aβ peptides or sweet-tasting amino acids or umami substances to the culture medium of the cells and measuring the increased intracellular calcium concentration using the same method as in step (1).

(3) Subsequently, administering test substances to the culture medium from step (2) and measuring the intracellular calcium concentration using the same method as in step (1).

(4) If the intracellular calcium concentration measured in step (3) is significantly lower than that measured in step (2), the test substance is evaluated as a substance that activates Type I taste receptors in human neurons, causing changes in intracellular calcium concentration ([Ca$^{2+}$] i). It is selected as a candidate substance capable of controlling the modification of the cellular cytoskeleton mediated by Aβ peptides or sweet-tasting amino acids or umami substances in human neurons.

[5-7] Method for screening substances capable of controlling the modification of cellular cytoskeleton induced by the binding of Aβ peptides or sweet-tasting amino acids or umami substances to Type I taste receptors in human neurons or cardiomyocytes, including the following steps (1) to (4).

(1) Form a cell sheet using cultured human neurons, cultured human cardiac myocytes, or human-derived cultured cells that forcibly express Type I taste receptors (TAS1R) on the cell surface, as well as VGSIC and/or VGKIC. Measure changes in cell skeleton and cell adhesion using impedance methods to establish control values.

(2) Administer Aβ peptides, sweet-tasting amino acids, or umami substances to the cell culture medium. Using a method similar to step (1), measure changes in cell skeleton and cell adhesion caused by these substances binding to Type I taste receptors.

(3) Next, administer test substances to the cell culture medium and measure the resistance on the cell sheet using the same method as in step (1).

(4) If the change in resistance in the cell sheet is significantly greater when the test substance is administered compared to when it is not, select the test substance as a candidate that can control the amplification of VGSIC currents and/or inhibition of VGKIC currents due to structural changes in cell skeletons (microfilaments) mediated by Type I taste receptors for Aβ peptides, sweet-tasting amino acids, or umami substances.

Additionally, the following screening methods are included.
[5-8] Method for Screening Compounds Modulating/Inhibiting Activity on VGSC and VGKC via TAS 1R-Mediated Aβ Peptides, Sweet-Tasting Amino Acids, or Umami Substances in CSF:

**EP 4 537 845 A1**

(1) Cultivate human epithelial cells in a sheet format, where Type I taste receptors (TAS1R) are expressed. Apply Aβ peptides, sweet-tasting amino acids, or umami substances to the cells and measure impedance (resistance) values at both ends of the cell sheet.

(2) Next, measure impedance values after exposing the cells to the test substance.

(3) If a decrease in impedance is observed due to the test substance's action, evaluate it as a candidate compound capable of controlling the amplification of VGSIC currents and/or inhibition of VGKIC currents mediated by TAS1R for Aβ peptides, sweet-tasting amino acids, or umami substances.

(5-9) A method for evaluating cell barrier function (degree of cell-cell adhesion) mediated by T1Rs due to the difference in the ratio of Aβ42/40 contained in CSF or serum, comprising:

(1) Measuring the impedance (resistance) values at both ends of a cell sheet cultured in a sheet form of human vascular endothelial cells,

(2) Then applying a test substance such as Aβ peptide, sweet-tasting amino acids, or umami substances to the Type I taste receptors of the cells, and measuring the impedance values of the cell sheet at that time,

(3) Evaluating whether a decrease in impedance values occurs due to the action of the test substance, and assessing whether there is a pathological effect on the barrier function in the cell sheet.

[6] Another aspect of the present invention relates to a method for screening candidate compounds for active ingredients of pharmaceutical compositions intended to prevent and/or treat diseases caused by the amplification or inhibition of activation currents of voltage-dependent ion channels, which are triggered via Type I taste receptors on the surface of human nerve cells, cardiomyocytes, or skeletal muscle cells.

[6-1] A method for screening candidate compounds for active ingredients of a pharmaceutical composition to prevent and/or treat diseases caused by the amplification of VGSC currents and/or the inhibition of VGKC currents, which occur due to the binding of Aβ peptides or sweet-tasting amino acids or umami substances to Type I taste receptors in human nerve cells or cardiomyocytes, comprising the following steps (1) to (4):

(1) Using the patch-clamp method (voltage-clamp method), measure the VGSC and/or VGKC currents of cultured human nerve cells, cultured human cardiomyocytes, or human-derived cultured cells that forcibly express VGSC and/or VGKC, which express Type I taste receptors on the cell surface, and establish control values.

(2) Administer Aβ peptide or sweet-tasting amino acids or umami substances to the culture medium, measure the VGSC and/or VGKC currents of the cells using the same method as in step (1), and measure the amplification of VGSC currents and/or the suppression of VGKC currents from the control values.

(3) Next, administer the test substance to the culture medium of the cells, and measure the VGSC and/or VGKC currents of the cells using the same method as in step (1).

(4) If the measured values of VGSC and/or VGKC currents in step (3) significantly inhibit either or both the amplification of VGSC currents and/or the suppression of VGKC currents measured in step (2), evaluate the test substance as a substance capable of controlling the amplification of VGSC currents and/or the suppression of VGKC currents mediated by Type I taste receptors of Aβ peptide or sweet-tasting amino acids or umami substances in human nerve cells or cardiomyocytes, and select it as a candidate compound for the active ingredient of the pharmaceutical composition.

[6-2] A method for screening candidate compounds for active ingredients in a pharmaceutical composition for the prevention and/or treatment of diseases caused by the amplification of VGSC currents and/or the suppression of VGKC currents, which occur when Aβ peptides or sweet-tasting amino acids or umami substances bind to Type I taste receptors in human nerve cells or cardiomyocytes, comprising the following steps (1) to (4):

(1) Using the patch-clamp method (current-clamp mode), NanoTouch method, or MEA method, measure the action potentials of cultured human nerve cells, cultured human cardiomyocytes, or cultured human cells that spontaneously or by external stimulation express Type I taste receptors on the cell surface and can fire action potentials, or cultured human cells that have been forcibly expressed with VGSC and/or VGKC, and record the magnitude, width, and firing frequency of their waveforms to establish control values.

(2) Administer Aβ peptide or sweet-tasting amino acids or umami substances to the culture medium of the cells, and using the same method as in step (1), measure and/or record the action potentials induced by the binding of the Aβ peptide or sweet-tasting amino acids or umami substances to the Type I taste receptors, and record their waveform widths.

(3) Next, administer the test substance to the culture medium in step (2), and using the same method as in step (1), measure and/or record the action potentials induced by the administration of the test substance and record their waveform widths.

(4) If the measured values of the frequency of occurrence or the shape of the waveform width of the action potentials measured in step (3) show a significant change compared to the frequency of occurrence or the shape of the waveform width of the action potentials measured in step (2), evaluate the test substance as a substance capable of controlling the amplification of VGSC currents and/or the suppression of VGKC currents mediated by the Type I taste receptors of Aβ peptide or sweet-tasting amino acids or umami substances in human nerve cells or cardiomyocytes, and select it as a candidate compound for the active ingredient of the pharmaceutical composition.

[6-3] A method for screening candidate compounds for active ingredients of a pharmaceutical composition for the prevention and/or treatment of diseases caused by cytoskeletal modifications resulting from the binding of Aβ peptides or sweet-tasting amino acids or umami substances to Type I taste receptors in human nerve cells, comprising the following steps (1) to (4):

(1) Culturing human nerve cells or human-derived cultured cells (e.g., HEK cells) that express Type I taste receptors on their cell surface in a culture medium containing a fluorescent reagent, and measuring the intracellular calcium concentration using a fluorescent reagent to establish control values.

(2) Adding the Aβ peptide or sweet-tasting amino acids or umami substances to the culture medium of the cells, and using the same method as in step (1) to measure the increased intracellular calcium concentration due to the binding of these substances to the Type I taste receptors using a fluorescent reagent.

(3) Subsequently, adding the test substance to the culture medium from step (2), and measuring the intracellular calcium concentration using a fluorescent reagent by the same method as in step (1).

(4) If the intracellular calcium concentration measured in step (3) is significantly reduced compared to the calcium concentration measured in step (2), evaluating the test substance as a substance capable of activating the Type I taste receptors of nerve cells and causing changes in intracellular calcium concentration ($[Ca^{2+}]i$), and as a substance capable of controlling the cytoskeletal modifications mediated by the Type I taste receptors due to the Aβ peptide or sweet-tasting amino acids or umami substances in human nerve cells, and selecting it as a candidate compound for the active ingredient of the pharmaceutical composition.

Additionally, since the experiments measuring changes in intracellular calcium or staining the cytoskeleton are reactions that occur before the control of VGSC, human-derived cultured cells (HEK cells) expressing Type I taste receptors on their cell surface that do not express VGSC can be used.

[6-4] A method for screening candidate compounds for active ingredients of a pharmaceutical composition for the prevention and/or treatment of diseases caused by the amplification of VGSC currents and/or the inhibitory effect on VGKC currents, which occur due to the binding of Aβ peptides consisting of Aβ25-35, Aβ1-38, Aβ1-40, or Aβ1-42 to Type I taste receptors in human nerve cells or cardiomyocytes, comprising the following steps (1) to (3):

(1) Cultured human nerve cells, cultured human cardiomyocytes, or human-derived cultured cells that forcibly express VGSC and/or VGKC, which express Type I taste receptors on the cell surface, are made to express fluorescently labeled actin or tubulin in advance. By administering one of the Aβ peptides selected from Aβ25-35, Aβ1-38, Aβ1-40, and Aβ1-42 to the cell culture medium, it is confirmed that the Aβ peptide binds to the Type I taste receptor, causing the cell cytoskeleton to polymerize and emit fluorescence in a fibrous form.

(2) In parallel with Step 1, the Aβ peptide is administered to the cell culture medium that has been pre-treated with the test substance, and the degree of depolymerization of the fluorescently labeled fibrous cytoskeleton caused by the Aβ peptide is observed.

(3) If the degree of depolymerization of the cytoskeleton observed in step (2) is significantly suppressed compared to the control value in Step 1, the test substance is evaluated as a candidate compound with high inhibitory activity against the amplification of VGSC current and/or the inhibitory effect on VGKC current caused by the Aβ peptide, and selected as a candidate compound for the active ingredient of the pharmaceutical composition.

Additionally, instead of expressing fluorescently labeled actin or tubulin, methods such as staining actin with rhodamine phalloidin or using an anti-tubulin antibody as a primary antibody and a fluorescently labeled secondary antibody can also be used.

[6-5] A method for screening substances capable of controlling the amplification of VGSC current and/or the inhibition of VGKC current caused by the binding of Aβ peptides or sweet-tasting amino acids or umami substances to Type I taste receptors in human nerve cells or cardiomyocytes, comprising the following steps (1) to (4):

(1) Forming a cell sheet with cultured human nerve cells, cultured human cardiomyocytes, or human-derived cultured cells that forcibly express VGSC and/or VGKC, which express Type I taste receptors on the cell surface. Measuring changes in the cytoskeleton and cell adhesion of the cultured cells as resistance values on the cell sheet using the impedance method, and establishing control values.

(2) Administering Aβ peptide, sweet-tasting amino acids, or umami substances to the cell culture medium, and measuring changes in the cytoskeleton and cell adhesion caused by the binding of these substances to Type I taste receptors as resistance values on the cell sheet using the same method as in step (1).

(3) Administering the test substance to the cell culture medium and measuring the resistance on the cell sheet using the same method as in step (1).

(4) If the change in resistance of the cell sheet is significantly greater when the test substance is administered compared to when it is not, selecting the test substance as a candidate substance capable of controlling the amplification of VGSC currents and/or the suppression of VGKC currents caused by structural changes in the cytoskeleton (microfilaments) and/or changes in intracellular calcium via Type I taste receptors of Aβ peptide, sweet-tasting amino acids, or umami substances.

In the same way as with the NanoTouch method, the impedance method completely covers the electrode surface in a sheet form. This allows the measurement of the impedance of the cell sheet by observing changes when a current is applied between the ground placed in the extracellular fluid and the electrode covered by the cells (a material with conductivity, such as conductive glass). When a constant current (I) is applied, according to Ohm's law ($V = IR$), if the impedance (resistance R) increases, the voltage (V) will increase. Conversely, if R decreases, since I is constant, the output voltage (V) will decrease.

[6-6] A method for screening candidate compounds for active ingredients of a pharmaceutical composition to prevent and/or treat diseases caused by the amplification of VGSC currents and/or the suppression of VGKC currents, which occur due to the binding of Aβ peptides or sweet-tasting amino acids or umami substances to Type I taste receptors in human nerve cells or cardiomyocytes, comprising the following steps (1) to (4):

(1) Using the patch-clamp method (voltage-clamp method), measure the VGSC and/or VGKC currents of cultured human nerve cells, cultured human cardiomyocytes, or human-derived cultured cells that forcibly express VGSC and/or VGKC, which express Type I taste receptors on the cell surface, and establish control values.

(2) Administer Aβ peptide or sweet-tasting amino acids or umami substances to the culture medium, measure the VGSC and/or VGKC currents of the cells using the same method as in step (1), and measure the amplification of VGSC currents and/or the suppression of VGKC currents from the control values.

(3) Next, administer the test substance to the culture medium of the cells, and measure the VGSC and/or VGKC currents of the cells using the same method as in step (1).

(4) If the measured values of VGSC and/or VGKC currents in step (3) significantly inhibit either or both the amplification of VGSC currents and/or the suppression of VGKC currents measured in step (2), evaluate the test substance as a substance capable of controlling the amplification of VGSC currents and/or the suppression of VGKC currents mediated by Type I taste receptors of Aβ peptide or sweet-tasting amino acids or umami substances in human nerve cells or cardiomyocytes, and select it as a candidate compound for the active ingredient of the pharmaceutical composition.

[6-7] Method for Screening Candidate Compounds for Active Ingredients in Pharmaceutical Compositions to Prevent and/or Treat Diseases Caused by Amplification of VGSC Currents and/or Inhibition of VGKC Currents Due to Binding of Aβ Peptides or Sweet Amino Acids or Umami Substances to Type I Taste Receptors in Human Neurons or Cardiomyocytes, Including the Following Steps:

(1) Establishing Control Values: Measure and/or record the action potentials and waveform widths of cultured human neurons, cultured human cardiomyocytes, or cultured human cells forcibly expressing VGSC and/or VGKC, which express Type I taste receptors on the cell surface and can fire action potentials spontaneously or in response to external stimuli, using methods such as patch-clamp (current-clamp mode), NanoTouch, MEA, or fluorescent membrane potential-sensitive dyes or fluorescent intracellular calcium-sensitive dyes.

(2) Inducing Action Potentials: Administer Aβ peptides or sweet amino acids or umami substances to the culture medium of the cells, and measure and/or record the action potentials and waveform widths induced by the binding of Aβ peptides or sweet amino acids or umami substances to Type I taste receptors using the same methods as in step (1).

(3) Administering Test Substances: Administer test substances to the culture medium from step (2), and measure and/or record the action potentials and waveform widths induced by the test substances using the same methods as in step (1).

(4) Evaluating Test Substances: If the measured frequency of action potential expression or waveform width shape in step (3) shows a significant change compared to the measured frequency of action potential expression or waveform width shape in step (2), evaluate the test substance as a substance capable of controlling the amplification of VGSC currents and/or inhibition of VGKC currents mediated by Type I taste receptors of Aβ peptides or sweet amino acids or umami substances in human neurons or cardiomyocytes, and select it as a candidate compound for the active ingredient of the pharmaceutical composition.

[6-8] Method for Screening Candidate Compounds as Active Ingredients of Pharmaceutical Compositions for Preventing and/or Treating Diseases Caused by Amplification of VGSC Currents or Modification of the Cytoskeleton Induced by the Binding of Aβ Peptides or Sweet Amino Acids or Umami Substances to Type I Taste Receptors in Human Neurons

This method includes the following steps:

(1) Culturing human neurons expressing Type I taste receptors on the cell surface, or human-derived cultured cells forcibly expressing VGSC, in a culture medium containing a fluorescent reagent, and measuring the intracellular calcium concentration using a calcium measurement method to establish a control value.

(2) Administering Aβ peptides or sweet amino acids or umami substances to the culture medium of the cells, and measuring the increased intracellular calcium concentration using a fluorescent reagent, similar to step (1), caused by the binding of these substances to Type I taste receptors.

(3) Administering a test substance to the culture medium from step (2), and measuring the intracellular calcium concentration using a fluorescent reagent, similar to step (1).

(4) If the intracellular calcium concentration measured in step (3) is significantly reduced compared to the calcium concentration measured in step (2), evaluating the test substance as a substance capable of activating Type I taste receptors in neurons, causing changes in intracellular calcium concentration ($[Ca^{2+}]i$), and controlling the modification of the cytoskeleton or amplification of VGSC currents mediated by Type I taste receptors in human neurons by Aβ peptides or sweet amino acids or umami substances, and selecting it as a candidate compound for the active ingredient of the pharmaceutical composition.

Additionally, when measuring intracellular calcium concentration, high-throughput screening using a plate reader can be performed, and procedures such as using control wells where only Aβ peptides or sweet amino acids or umami substances are applied, or administering a solution mixed with the test substance and Aβ peptides or sweet amino acids or umami substances simultaneously, can also be used.

[6-9] Method for screening candidate compounds for active ingredients of a pharmaceutical composition for preventing and/or treating diseases caused by the amplification of VGSC currents and/or suppression of VGKC currents due to the binding of Aβ peptides (Aβ25-35, Aβ1-38, Aβ1-40, or Aβ1-42) to Type I taste receptors in human nerve cells or cardiomyocytes, comprising the following steps:

(1) Expressing Type I taste receptors on the cell surface of cultured human nerve cells, cultured human cardiomyocytes, or human-derived cultured cells forcibly expressing VGSC and/or VGKC, and pre-expressing fluorescently labeled actin or tubulin, then administering any of the Aβ peptides selected from Aβ25-35, Aβ1-38, Aβ1-40, and Aβ1-42 to the cell culture medium, confirming that the Aβ peptide binds to the Type I taste receptor, causing the cell cytoskeleton to polymerize and emit fluorescence in a fibrous form.

(2) In parallel with step (1), administering the Aβ peptide to the cell culture medium pre-treated with the test substance, and observing the degree of depolymerization of the fluorescently labeled fibrous cytoskeleton caused by the Aβ peptide.

(3) Comparing the degree of cytoskeleton depolymerization observed in step (2) with the control value in step (1), and if the degree of cytoskeleton depolymerization is significantly suppressed, evaluating the test substance as a candidate compound with high inhibitory activity against the amplification of VGSC currents and/or suppression of VGKC currents caused by the Aβ peptide, and selecting it as a candidate compound for the active ingredient of the pharmaceutical composition.

[6-10] Screening Method for Candidate Compounds as Active Ingredients of Pharmaceutical Compositions for Preventing and/or Treating Diseases Caused by Amplification of VGSC Currents and/or Inhibition of VGKC Currents Due to the Binding of Aβ Peptides or Sweet Amino Acids or Umami Substances to Type I Taste Receptors in Human Nerve Cells or Cardiomyocytes, Including the Following Steps (1) to (4):
Screening Method for Controllable Substances, Including the Following Steps (1) to (4):

(1) Forming a cell sheet with cultured human nerve cells, cultured human cardiomyocytes, or human-derived cultured cells forcibly expressing VGSC and/or VGKC, which are cells expressing Type I taste receptors on the cell surface, and measuring changes in the cytoskeleton and cell adhesion of the cultured cells on the cell sheet as resistance values using the impedance method to establish control values.

(2) Administering the Aβ peptide or sweet amino acid or umami substance to the cell culture medium, and measuring changes in the cytoskeleton and cell adhesion of the cultured cells on the cell sheet as resistance values using the same method as in step (1), due to the binding of the substance to the Type I taste receptor.

(3) Administering the test substance to the cell culture medium, and measuring the resistance values on the cell sheet using the same method as in step (1).

(4) If the change in resistance of the cell sheet when the test substance is administered is significantly greater than when it is not administered, evaluating the test substance as a candidate substance capable of controlling the amplification of VGSC currents and/or inhibition of VGKC currents due to changes in the cytoskeleton (microfibers) and/or intracellular calcium caused by the Type I taste receptor of the Aβ peptide or sweet amino acid or umami substance, and selecting it as a candidate compound for the active ingredient of the pharmaceutical composition.

[6-11] A screening method for candidate compounds as active ingredients of pharmaceutical compositions for preventing and/or treating diseases caused by the amplification of VGSC currents due to the binding of Aβ peptides to Type I taste receptors in human nerve cells or humanized experimental animal-derived cells, including a step of observing changes in the area or volume of cultured human nerve cells or humanized mouse nerve cells.

[6-12] A screening method for active ingredients of pharmaceutical compositions, where VGSC currents enhanced by the action of the Aβ42/40 ratio (AD-D) observed in the cerebrospinal fluid of AD patients on Type I taste receptors of human nerve cells or humanized experimental animal-derived cells are reduced by the subsequently administered test substance, and the test substance is evaluated as a candidate compound for the active ingredient of the pharmaceutical composition for preventing and/or treating diseases caused by the amplification of VGSC currents due to the binding of Aβ peptides.

Effects of the Invention

In this invention, it was discovered for the first time that Type I taste receptors (T1R1, T1R2, T1R3) control the properties and magnitude of membrane potential-dependent sodium channel currents (VGSC) and membrane potential-dependent potassium channel currents (VGKC) through the cytoskeleton. While the mechanism of memory formation in the hippocampus of the brain was considered to be primarily driven by the action of glutamate receptors (AMPA type, NMDA type), this discovery suggests that, in addition to the dynamics of glutamate receptors, membrane potential-dependent ion channels also play an important role in neural plasticity and the memory formation process. This indicates that Type I taste receptors are effective targets for drug development in the treatment of memory disorders such as dementia.

Firstly, T1R1, which functions as a receptor for umami substances such as glutamate (partially also acting on Aβ1-42), can be identified as a candidate for therapeutic drugs by exploring its agonists and positive allosteric modulators (PAM). Next, T1R2 acts as a receptor for artificial sweeteners and suppresses membrane potential-dependent sodium channel currents through microfilaments, making it an effective drug development target for analgesics and antiepileptic drugs. Furthermore, T1R3 was identified as a receptor for amyloid β peptide (Aβ25-35), which is considered most important in the clinical findings of Alzheimer's disease. Since Aβ25-35 amplifies VGSC currents and causes hyperactivity in neurons or cardiac muscle, antagonists of T1R3 or inhibitors of Aβ25-35 binding to T1R3 are promising candidates for the treatment of Alzheimer's disease.

As described above, in this invention, it was found that Type I taste receptors (T1Rs) are receptors for amyloid β peptides (Aβ25-35 receptor is T1R3, Aβ1-42 receptor is T1R1 and T1R3), and that Aβ peptides control VGSC and VGKC currents through T1Rs, at least in neuronal cells. This suggests that T1Rs are drug development targets for preventing or inhibiting the binding/deposition of amyloid β peptides, which cause functional decline in Alzheimer's disease, ALS, and other tissues.

Additionally, the therapeutic drugs for neuropathic pain provided by this invention act on VGSC through sweet taste receptors, making them effective even under inflammatory acidic conditions, and thus highly effective for use as local anesthetics.

Additionally, in this invention, we elucidated which regions of T1R3 or T1R2/T1R3 the highly toxic amyloid β peptides Aβ25-35, Aβ1-42, and Aβ1-40 act upon to enhance VGSC currents using various anti-T1R3 antibodies. Furthermore, we compared the effects on VGSC currents using a mixed solution adjusted to the concentration ratios of Aβ1-42/Aβ1-40 (Aβ42/40) in the cerebrospinal fluid of Alzheimer's disease (AD) patients (AD-D) and healthy individuals (NDC). As a result, no significant changes were observed with NDC, but a remarkable enhancement of VGSC currents was noted with AD-D. This suggests that the epilepsy-like symptoms observed in Alzheimer's patients may be due to neuronal hyperactivity caused by the enhancement of VGSC currents by Aβ42/40 (ADD).

This enhancement was reversed by β-alanine. Additionally, when AD-D was applied to neuron-like cells derived from mice/rats, the effect of AD-D was found to decrease, contrary to human cells. This indicates a fundamental reason why research results from mouse models are not effective in human clinical studies in Alzheimer's drug discovery and research.

Similarly, we observed morphological changes in human neuron-like cells and confirmed that the morphological changes induced by AD-D concentration are significantly involved in the onset of AD. Furthermore, we found that these morphological changes were reversed by β-alanine. Thus, observing the morphological/dynamic changes in human cultured neuron-like cells caused by the cerebrospinal fluid or serum of AD patients can enable the determination of the disease progression state, and β-alanine or its structural analogs strongly suggest potential as a promising therapeutic agent for AD.

We also demonstrated species differences in the effects of Aβ peptides on T1Rs using electrophysiological methods.

Brief Description of the Drawings

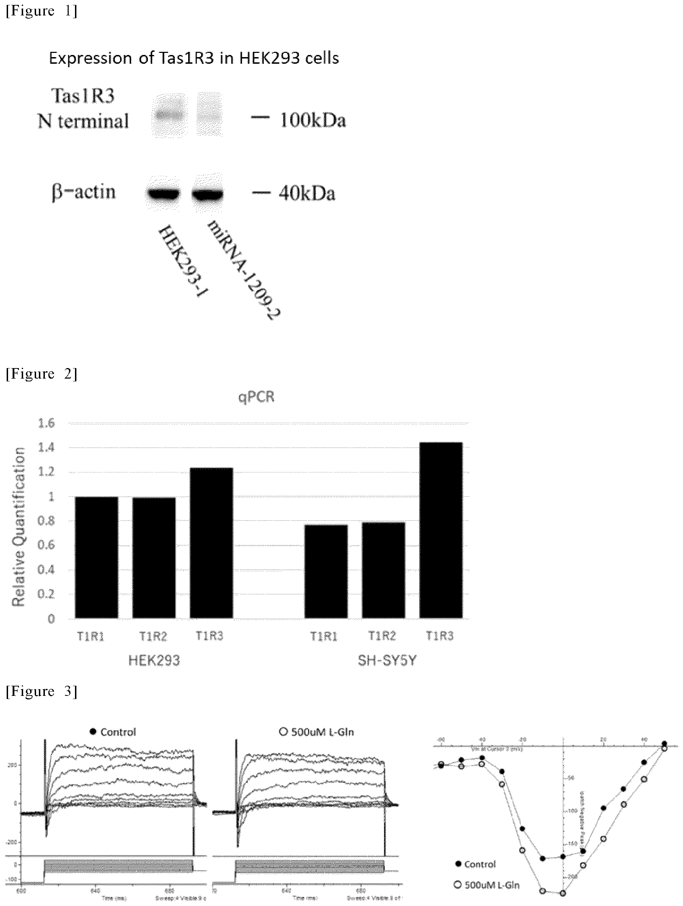

(Figure1) Western blot showing the silencing of endogenous T1R3 in HEK293 cells: The silencing effect on T1R3 protein expression was confirmed using a T1R3 antibody. Left lane: HEK293 Control, right lane: HEK293 with silencing vector introduced.

(Figure 2) Quantitative PCR confirming the expression levels of endogenously expressed T1R1, T1R2, and T1R3 in HEK and SH-SY5Y cells.

(Figure 3) Enhancement effect of glutamine on VGSC: The effect of 500μM glutamine on VGSC current in SH-SY5Y was measured using the patch-clamp method. Left figure: Patch-clamp recordings before and after the application of 500μM glutamine, showing an enhancement of transient downward VGSC current. Right figure: Analysis of the results from the left figure using an I-V plot.

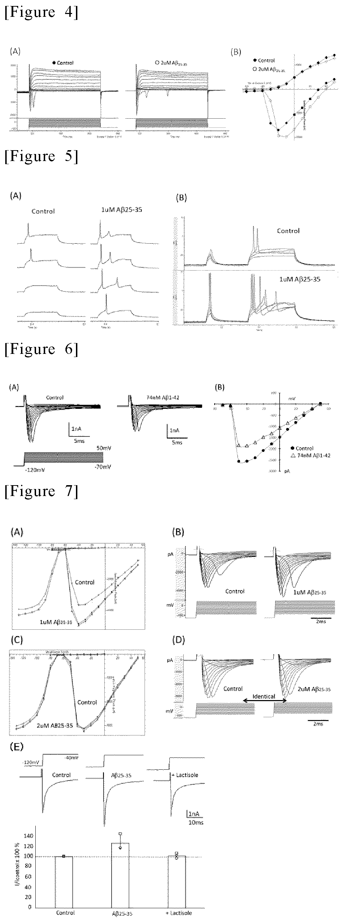

(Figure 4) Effect of Aβ25-35 on VGSC current in SH-SY5Y The effect of 2μM Aβ25-35 peptide on VGSC current in SH-SY5Y was measured using the patch-clamp method. (A) Patch-clamp recordings before and after the application of 2μM Aβ25-35 peptide, showing an enhancement of transient downward VGSC current after application. (B) Analysis of the results from the left figure using an I-V plot.

(Figure 5) Aβ25-35 peptide, like glutamine, increased the excitability of SH-SY5Y cells. Current-clamp recordings were performed using SH-SY5Y cells to confirm the effect of Aβ25-35 peptide on action potentials. The cells were stimulated using the following current patterns: short stimulus (25ms), followed by a 200ms interval, and then a long stimulus (250ms). The current intensity was increased sequentially to 6, 8, 10, and 12pA. The cell membrane potential was maintained at -80mV by appropriate current injection. (A) Responses to each stimulus were displayed at 125mV intervals. (B) Four traces were overlaid for display.

(Figure 6) Effect of Aβ1-42 on HEK Nav1.5 VGSC Current The effect of 74nM Aβ1-42 peptide on HEK Nav1.5 VGSC current was measured using the patch-clamp method. (A) Patch-clamp recordings before and after the application of 74nM Aβ1-42 peptide showed a transient downward VGSC that did not exhibit the enhancing effect seen with Aβ25-35, but rather showed a decreasing trend (n=4). The I-V plot peak tended to shift 5-10mV in the hyperpolarizing direction, indicating a delay in VGSC inactivation (Figure 37). (B) shows the results of (A) analyzed in an I-V plot.

(Figure 7) Comparison of the Effect of Aβ25-35 Peptide on VGSC in Control HEK Nav1.5 Cells and T1R3-Silenced HEK Nav1.5 Cells (A) and (B) show a comparison of VGSC recordings from control HEK Nav1.5 cells before and after the application of 1μM Aβ25-35 using voltage clamp. An enhancing effect on VGSC was confirmed with 1μM Aβ25-35. © and (D) show VGSC recordings from T1R3-silenced HEK Nav1.5 cells. Even with the application of 2μM Aβ25-35, which is double the amount used in the control, no change in VGSC was observed. (E) shows that lactisole, which specifically binds to the T1R3 TM (TransMembrane) domain, inhibits the enhancing effect of Aβ25-35 on VGSC. The upper figure shows VGSC currents in patch-clamp recordings. From left to right, it shows the control, the enhancement of VGSC by Aβ25-35, and the inhibition of this enhancement by 1mM lactisole. The lower bar graph shows the changes in peak current as a percentage of the control in three experiments, with symbols representing individual experiments and the bar graph showing the relative average change.

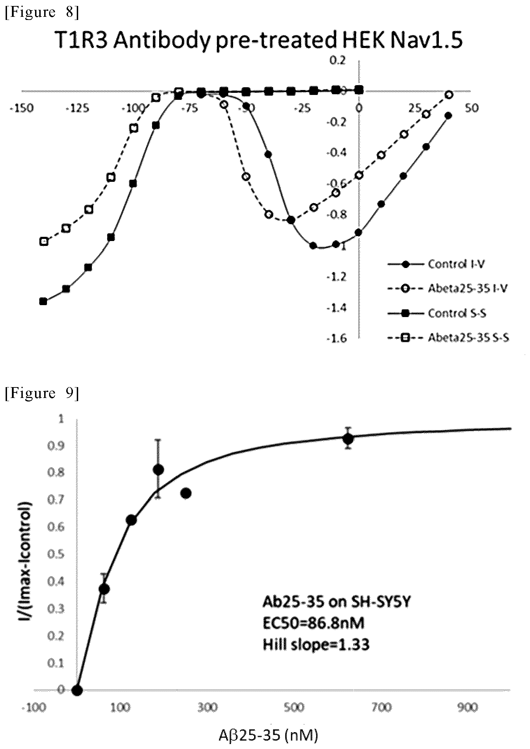

(Figure 8) HEK Nav1.5 cells cultured on cover glass were maintained at room temperature for 10 to 30 minutes in a culture medium diluted 1000 times with anti-T1R3 antibody (Abcepta, Inc.). The cover glass was then transferred to HBPS solution, and the membrane potential-dependent activation process and steady-state inactivation properties of VGSC (Nav1.5) currents were measured using the voltage-clamp method, serving as the control. Subsequently, Aβ25-35 peptide was added to the recording solution, and changes in VGSC currents were measured using the same experimental method, observing changes before and after Aβ25-35 peptide administration. In cells not pretreated with T1R3 antibody, Aβ25-35 peptide enhanced the maximum VGSC current, but this enhancement was not observed in cells pretreated with T1R3 antibody. The changes in the I-V curve of VGSC caused by Aβ25-35 peptide were similar to those observed when T1R2 was stimulated with artificial sweeteners, resulting in a hyperpolarizing shift in the steady-state inactivation curve. This suggests that the enhancement of VGSC currents by Aβ25-35 peptide occurs through T1R3. This experiment indicates that when Aβ25-35 peptide cannot act on T1R3 due to T1R3 antibody, it may act on T1R2. This suggests that T1R2 may act as a secondary low-affinity binding site for Aβ25-35 peptide. At present, it is possible that Aβ25-35 peptide acts only on T1R3 or on both T1R2 and T1R3, similar to sucrose and sucralose.

(Figure 9) The concentration-dependent amplification effect of VGSC currents by Aβ25-35 peptide, with an EC50 value of 86.8 nM.

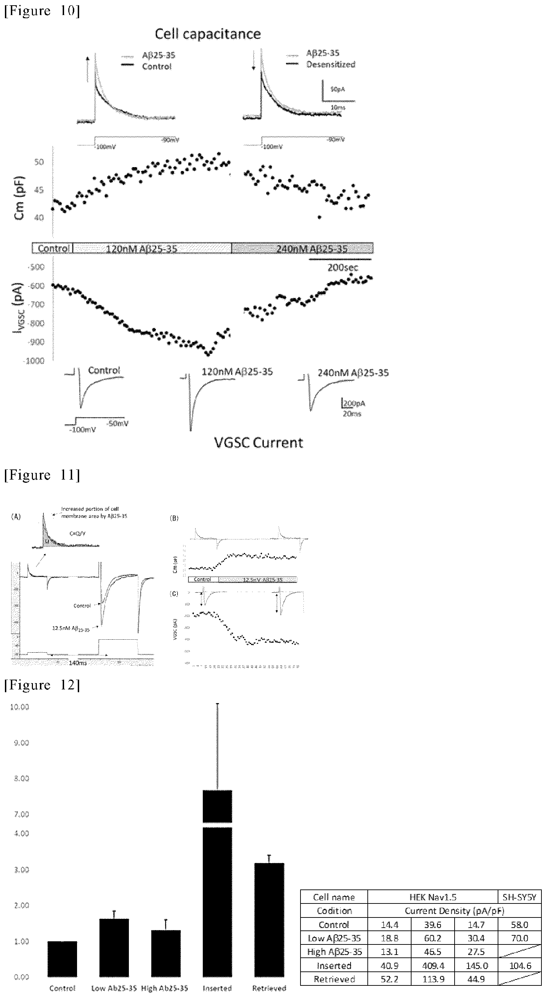

(Figure 10) Using the perforated patch-clamp method, the concentration-dependent amplification effect of VGSC currents and membrane capacitance by Aβ25-35 peptide through T1R3, and the desensitization process. The upper half of the figure shows changes in cell membrane capacitance, and the lower half shows the magnitude of VGSC currents. Both cell membrane capacitance and VGSC currents increased with 120 nM Aβ25-35, but decreased to control levels (before Aβ25-35 action) when the concentration of Aβ25-35 was increased to 240 nM.

(Figure 11) Similar to (Figure 10) using the perforated patch-clamp recording method, we reproduced the concentration-dependent VGSC current and membrane capacitance amplification effect by Aβ25-35 peptide via T1R3. (A) Shows the increase in cell membrane capacitance and VGSC current (HEKNav1.5) after control and 12.5 nM Aβ treatment. The membrane capacitance is obtained by integrating the current waveform changes caused by depolarizing from the holding voltage to 10 mV while the membrane potential is fixed at the top of the figure. The dotted pattern part is the control capacitance, and the white part above it is the increase after Aβ treatment. As the capacitance increases, the VGSC current also increases. (B) Plots the change in membrane capacitance due to Aβ treatment over time. The waveform on the plot represents the membrane capacitance in the inactive state of VGSC. (Left, control, right after Aβ treatment) © Simultaneously with the capacitance measurement in (B), the change (increase) in VGSC current after Aβ treatment was measured and plotted against the time axis.

(Figure 12) Comparison of VGSC current density due to Aβ25-35 peptide action (including experimental results from Figures 10 and 11). Bar graph: The current density (pA/pF) was obtained by dividing the peak current value of VGSC by the cell membrane capacitance measured in the control. From left to right, the bars show control, low concentration Aβ25-35, and high concentration Aβ25-35. Following that, Inserted and Retrieved show the current density of intracellular vesicles obtained by dividing the increase in VGSC current by the difference in membrane capacitance measured under each experimental condition and the control. This indicates the increase in membrane area due to the fusion of intracellular vesicle membrane components containing VGSC into the cell membrane. The VGSC current density of vesicles inserted into the cell membrane by low concentration Aβ25-35 and the VGSC current density of vesicles endocytosed and taken up from the cell membrane due to receptor desensitization by high concentration Aβ25-35 were almost the same. This suggests that the VGSC derived from the inserted vesicles remains within the vesicle-derived membrane without diffusing into the surrounding cell membrane in a short period like this experiment. The table on the right shows the individual experimental measurements used to create the bar graph on the left.

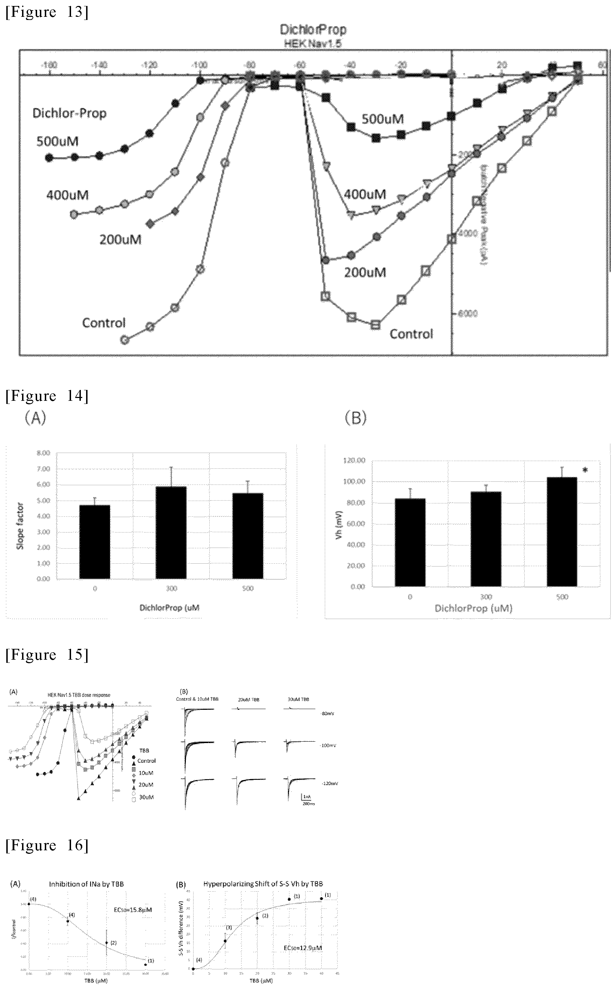

(Figure 13) Effect of dichlorprop on VGSC current: dichlorprop reduced the amplitude of VGSC current. The activity of voltage-gated sodium channels (VGSC) was recorded by applying depolarizing stimuli at 10 mV intervals from -80 mV to 50 mV following a prepulse of -120 mV. The peak of the inward current decreased in a concentration-dependent manner with dichlorprop. The curve on the left side of the figure shows steady-state inactivation, and the size of the VGSC current activated by the test pulse added after all pulses from -160 mV to -120 mV to 0 mV was plotted against the membrane potential. It gradually shifted to the left with dichlorprop.

(Figure 14) Effect of dichlorprop on the slope and Vh of the steady-state inactivation curve: Using the Boltzmann equation (1/(1+exp((V50-x)/slope))), Vh and slope were determined. The analysis software used was CurveExpert Professional (Hyams Development) (n=4). (A) Comparison of the slopes of the curves for Control, 300, and 500 μM dichlorprop. There was no significant difference in the t-test for these three conditions. (B) Comparison of the effect on Vh. A statistically significant difference was confirmed from the measurements of 500 μM dichlorprop. It was found that the Vh value shifted to a more negative potential with high concentration dichlorprop.

(Figure 15) Effect of TBB on VGSC Current (A) TBB reduced the amplitude of VGSC current. The activity of voltage-gated sodium channels (VGSC) was recorded by applying depolarizing stimuli from -80mV to 50mV in 10mV increments, following a prepulse of -120mV. The peak inward current decreased in a TBB concentration-dependent manner. The left side of the figure shows the steady-state inactivation curve, plotting the current of VGSC activated by a test pulse of -20mV following pulses from -170mV to -120mV to 0mV against the membrane potential. This shifted gradually to the left with TBB. (B) The prepulse potentials are shown from top to bottom as -80mV (top), -100mV (middle), and -120mV (bottom). The VGSC current generated by a test pulse of -20mV from the prepulse potential was recorded. The concentration of TBB increased from right to left. From left to right, the changes are shown for Control and 10μM TBB, middle for 20μM TBB, and right for 30μM TBB. At -80mV (top), VGSC current was almost completely suppressed by 10μM TBB, at -100mV (middle) it was about half suppressed, and at -120mV (bottom) it was more than 80% unaffected. At 10μM TBB, VGSC current was almost completely suppressed except at -120mV.

(Figure 16) (A) The IC50 of the concentration-dependent inhibitory effect of TBB on VGSC current and VGSC activated by stepping from prepulse -80mV to -20mV was almost completely suppressed at 10μM, and at -120mV stimulation, it was only about 50% suppressed at the used TBB concentration. Using the measurement results with -100mV as the prepulse, the IC50 (15.8μM) of TBB was calculated using a Hill plot. INa activated by stepping from -100mV to -20mV was plotted against TBB concentration. The vertical axis represents INa control as 1, and the amplitude of INa after TBB action was averaged. (B) The steady-state inactivation curve was calculated using the Boltzmann equation, plotting the difference in Vh before (control) and after TBB administration against TBB concentration. This plot was further analyzed using the Hill equation, calculating an IC50 (12.9μM). Although different methods were used in (A) and (B) to analyze the inhibitory effect of TBB, almost the same IC50 values were obtained.

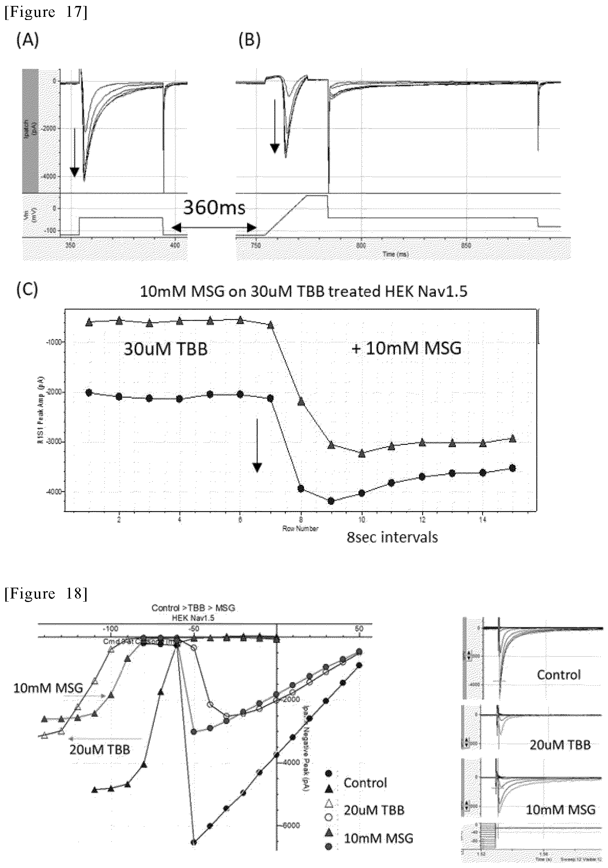

(Figure 17) VGSC current recordings were observed using the Pulse & Ramp Protocol. The membrane potential was fixed at -120mV, and initially, a rectangular pulse of -20mV was applied to activate the transient VGSC current component (A). Then, a ramp waveform stimulus from -120mV to +60mV was applied to induce the slowly inactivating VGSC current component (B). The application of the CK2 inhibitor TBB suppressed the amplitude of both the transient component (A) and the slowly inactivating component (B). The effect of 10mM MSG was observed in this suppressed state. The VGSC current induced by the rectangular pulse (A) and the ramp (B) partially recovered from TBB suppression. © The enhancement process of VGSC current by MSG was plotted against the time axis. The enhancement of VGSC current by MSG suggests a mechanism independent of the suppressive mechanism of TBB. The VGSC current induced by the rectangular pulse is indicated by (•), and the VGSC current induced by the ramp is indicated by (▲).

(Figure 18) The delaying effect of MSG on VGSC inactivation in the presence of TBB: The S-S curve on the left shows the VGSC membrane potential changing stepwise to -20mV from different steady-state holding potentials, stimulating the VGSC. Inhibition of CK2 by TBB caused a significant hyperpolarizing shift in the S-S curve (leftward arrow). Subsequently, the application of MSG partially reversed the leftward shift caused by TBB, resulting in a rightward shift (rightward arrow). The right figure shows the actual VGSC current traces measured for the S-S curve. The action of MSG confirmed a delay in the inactivation process of VGSC current treated with TBB. Comparing the current waveform after MSG application with the control waveform, it can be interpreted that the slowly inactivating component observed in the control waveform is enhanced.

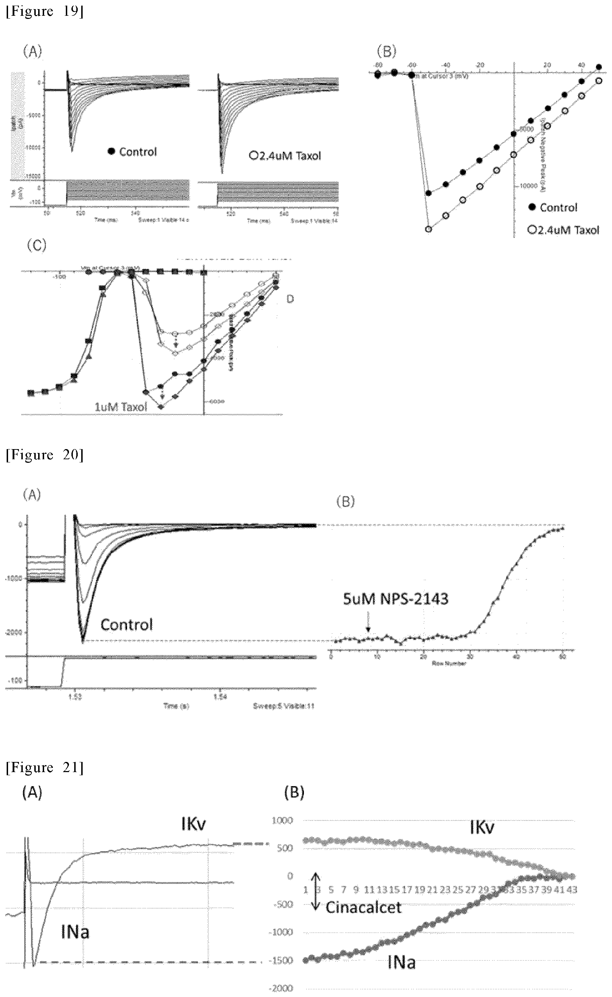

(Figure 19) Paclitaxel (Taxol) also enhances HEK Nav1.5 cell VGSC, similar to glutamine and Aβ25-35 peptide. (A) VGSC currents before (left control) and after (right) the application of 2.4μM Taxol under voltage-clamp conditions. (B) I-V plot of VGSC peak currents shown in (A). © Similar experiments were conducted using 1μM Taxol. The I-V curves were created for cases where the prepulse was applied from -120mV (•) and from -80mV (O). In both cases, the peak current amplitude was increased by Taxol. However, while the measurements at -70mV and -80mV prepulse slightly increased, the overall impact on the S-S curve was limited. Detailed investigation confirmed that the action of Taxol (which stabilizes microtubules) on VGSC is similar to that of glutamine and Aβ25-35 peptide, enhancing HEK Nav1.5 cell VGSC.

(Figure 20) Inhibitory effect of 5μM NPS-2143 on HEK Nav1.5 VGSC. (A) shows the process of VGSC current being inhibited by NPS-2143. (B) is an I-T plot showing the decrease in current amplitude over time due to the action of NPS-2143. A stimulus pulse from -120mV to -20mV was given every 8 seconds.

(Figure 21) Inhibitory effect of 5μM Cinacalcet on inward VGSC current and outward potassium current in SH-SY5Y cells. (A) shows the waveform before and after the complete inhibition by Cinacalcet. (B) is an I-T plot showing the decrease in current amplitude over time due to the action of Cinacalcet. A stimulus pulse from -120mV to -20mV was given every 8 seconds. Both inward VGSC current and outward potassium current were inhibited by Cinacalcet.

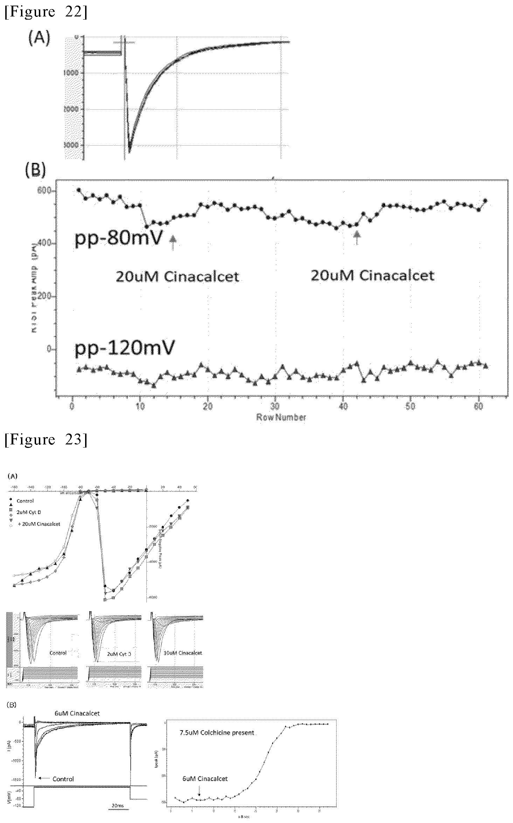

(Figure 22) The inhibitory effect of Cinacalcet on VGSC occurs via T1R3. In HEK Nav1.5 cells where T1R3 expression was silenced, the inhibitory effect of Cinacalcet was nullified. (A) shows VGSC current recordings before and after Cinacalcet administration. In cells with suppressed T1R3 expression, no change in VGSC current was observed due to Cinacalcet. The pre-pulse was -120mV, and the test pulse was -20mV. (B) is an I-T plot showing the amplitude and time change of VGSC current. Cinacalcet was applied twice at the points indicated by arrows. The amplitude of VGSC was measured with a test pulse of -20mV from pre-pulses of -80mV and -120mV, showing the changes.

(Figure 23) The inhibitory effect of the CaSR agonist Cinacalcet on VGSC via T1R3 is hindered by the polymerization state of the cytoskeleton. (A) When actin polymerization was inhibited by Cytochalasin D, the inhibitory effect of Cinacalcet on VGSC was lost. The I-V curve and steady-state inactivation curve of VGSC current under control, Cytochalasin D, and high concentration Cinacalcet conditions are shown. (B) When tubulin polymerization was inhibited by Colchicine, unlike the actin polymerization inhibitor Cytochalasin D, the inhibitory effect of Cinacalcet on VGSC via T1R3 was observed, and no effect of Colchicine was seen. It was concluded that Cinacalcet inhibits VGSC via T1R3 by acting on actin.

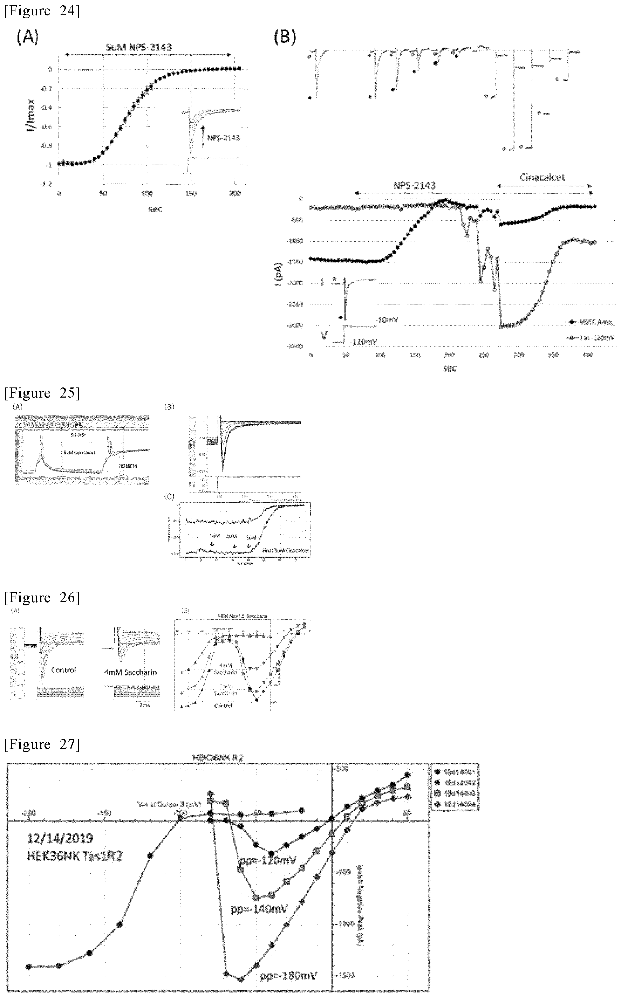

(Figure 24) Inhibitory effect of CaSR antagonist NPS-2143 on VGSC: (A) Shows that when 5μM NPS-2143 is applied, VGSC current is slowly inhibited, similar to cinacalcet. The vertical axis represents the control current magnitude as 1, showing the inhibition process. The horizontal axis represents time (SEC). (B) Shows the inhibition process of VGSC current by NPS-2143. • indicates the VGSC current peak, and ∘ indicates the baseline level. The time course changes from left to right, similar to (A). When the inhibition of VGSC current by NPS-2143 is almost complete, the baseline (leak) current increases. However, when the CaR agonist cinacalcet is applied, the baseline-leak current decreases (upper part of B). The lower part of (B) shows the measured values of peak current and baseline plotted from actual experimental data.

(Figure 25) Inhibitory effect of cinacalcet on VGSC under acidic conditions: (A) Cinacalcet inhibited VGSC-dependent action potentials in SH-SY5Y cells under acidic conditions of pH6 (Current-clamp). (B) The inhibitory effect on HEK Nav1.5 VGSC current using the voltage-clamp method was also similar to that at normal pH7.4. The upper part of (B) shows the voltage-clamp trace of Na+ current. Na+ current was activated by repeating voltage steps from -120mV to -20mV every 8 seconds. When 5μM cinacalcet was applied, Na+ current was gradually inhibited. The results are shown in the I-T relationship in ©. The initial low trace amplitude corresponds to the size of the Na+ current in the raw recording trace on the left side.

(Figure 26) Effect of saccharin on VGSC current: (A) Saccharin reduced the amplitude of VGSC current in a concentration-dependent manner. The activity of VGSC, which is membrane potential-dependent, was recorded by applying depolarizing stimuli from -80mV to 50mV at 10mV intervals following a pre-pulse of -120mV. (B) The plot shows that the peak current of the inward current decreases depending on the concentration of saccharin. The curve on the right side of the figure shows the steady-state inactivation curve, plotting the size of VGSC current activated by a test pulse of - 20mV following a full pulse from -120mV to 0mV against the membrane potential.

(Figure 27) Overexpression of T1R2 in HEK Nav1.5 Cells and Its Impact on VGSC Currents. Investigating the activation properties, an I-V curve was examined for T1R2 overexpression in HEK Nav1.5 cells. When a prepulse was applied from the usual -120 mV, the peak current of VGSC was small. However, expanding the membrane potential range for steady-state inactivation curve measurement to a negative range of -200 mV resulted in approximately a fivefold increase in VGSC peak current amplitude. Further experiments with prepulses set at -140 mV and -180 mV, similar to the -120 mV experiment, also showed an increase in VGSC peak current maximum values. Based on this, it is suggested that T1R2 overexpression, similar to artificial sweetener stimulation, affects VGSC inactivation[1].

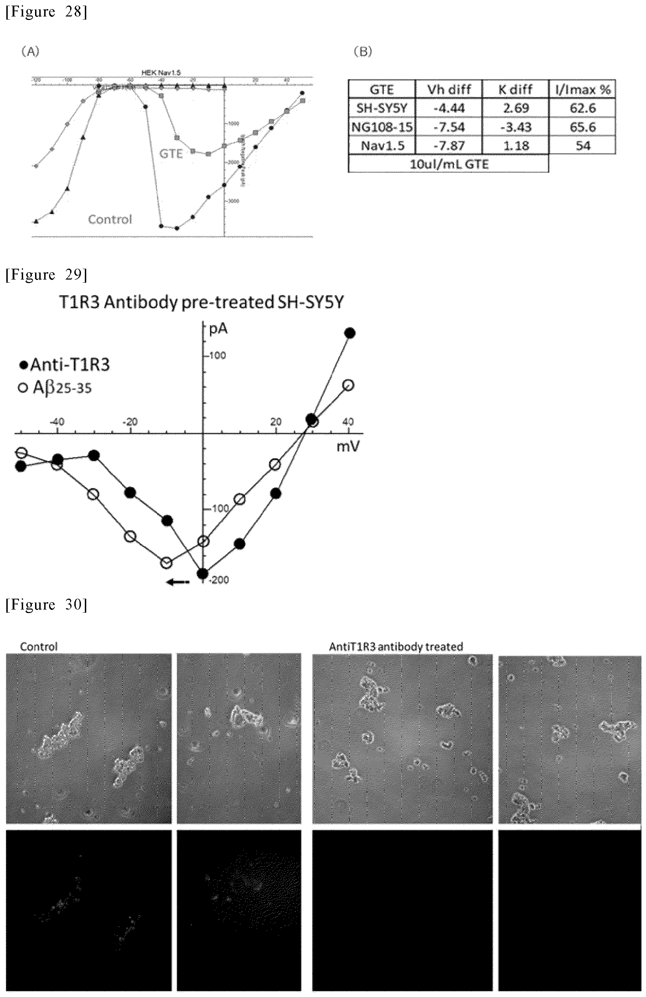

(Figure 28) Effect of Gymnema Tea Extract (GTE) on VGSC Currents: (A) GTE reduced the amplitude of VGSC currents. The voltage-dependent VGSC activity was recorded by applying depolarizing stimuli from -80 mV to 50 mV in 10 mV intervals following a prepulse at -120 mV. The peak current in the I-V curve decreased by 40-50% after GTE treatment. The left curve represents steady-state inactivation, plotting the size of VGSC currents activated by a test pulse at -20 mV following all pulses from -120 mV to 0 mV. GTE caused a leftward shift in this curve. (B) VGSC currents were recorded from SH-SY5Y, Nav1.5-HEK, and NG108-15 cells, and steady-state inactivation curves were plotted. Using the Botzman equation, the half-maximal membrane potential (Vh) for VGSC current was calculated. After GTE treatment (10 μl/ml), Vh was determined and the differences in Vh (Vhdiff) and slope (K) (Kdiff) were summarized in the table. In all tested cells, Vh shifted toward hyperpolarization, and VGSC peak current was reduced (inhibited) by approximately 60%[1].

(Figure 29) Changes in Sodium Current Enhancement by Pre-treatment with Anti-T1R3 Antibodies in SH-SY5Y Cells: Similar to the experiment in HEK Nav1.5 cells (Figure 8), pre-treatment with anti-T1R3 antibodies was performed in SH-SY5Y cells. The membrane potential-dependent VGSC was activated by applying stimulus pulses from a holding voltage of -120 mV, and an I-V curve was created and compared to the control. Although a slight leftward shift in the I-V curve was observed upon treatment with Aβ25-35 peptide (similar to Figure 8), there was no increase in VGSC current as seen in the recordings from SH-SY5Y cells without anti-T1R3 antibody treatment. This suggests that T1R3GPCR involvement is essential for the enhancement of VGSC current by Aβ25-35 peptide[1].

(Figure 30) Binding of Fluorescently Labeled Aβ25-35 Peptide (FL-Aβ25-35) to SH-SY5Y Cells. The left panel shows the results of positive control where Aβ25-35 peptide was applied to SH-SY5Y cells. Fluorescence microscopy confirms binding of Aβ25-35 peptide to the cells. The right panel shows cells pre-treated with anti-T1R3 antibodies. Despite applying FL-Aβ25-35 at the same concentration and duration as the control, fluorescence from FL-Aβ25-35 was barely detected on the cell surface.

(Figure 31) Enhancement of VGSC Current by Aβ25-35 in the Presence of Latrunculin A (LatA) (A) LatA's effect on VGSC current. The plot shows VGSC current before (•) and after (□) treatment with 12.6 μM LatA. (B) LatA's voltage-dependent inhibition. At -80 mV holding potential, LatA significantly inhibits VGSC current compared to -120 mV. (C) Observing the effect of Aβ25-35 under LatA-induced inhibition of actin polymerization. VGSC current was plotted against membrane potential (□ before Aβ25-35, ◇ after Aβ25-35), confirming VGSC amplification by Aβ25-35 in the presence of LatA. (D) Time-series plot of VGSC current enhancement by Aβ25-35 in the presence of LatA.

(Figure 32) Lack of VGSC Current Enhancement by Aβ25-35 in the Presence of Colchicine, (A) VGSC current was stimulated by varying the voltage of the pre-pulse before -20 mV test pulses (S-S inactivation protocol). Control traces (left) show VGSC current after Colchicine treatment, and further Aβ25-35 application (right). (B) Plot of VGSC current against stimulus pulse voltage for control, Colchicine-treated, and Aβ25-35-treated conditions. Colchicine inhibits Aβ25-35-induced enhancement. (C) No change in VGSC current waveform due to Aβ25-35 under Colchicine treatment. (D) Plotting peak current during measurements (as shown in C) against time, demonstrating Aβ25-35 inhibition. (E) is an example of an increase in VGSC current by colchicine in cells different from the cells used in (A) to (D) above.

(Figure 33A) Disruption of Microtubule Fibrous Structure by Aβ25-35 Peptide, GFP-Tubulin labeling shows disappearance of microtubule-like structures upon Aβ25-35 treatment.

(Figure 33B) Similar Disruption of Microtubule Fibrous Structure by MSG (Umami Receptor Agonist), MSG treatment also leads to the disappearance of microtubule-like structures similar to Aβ25-35 treatment (GFP-Tubulin labeling).

(Figure 34) Changes/Disappearance of Microfilament Fibrous Structure by Aβ1-42 Peptide and Cinacalcet, GFP-Actin labeling reveals alterations in microfilament structure due to Aβ1-42 peptide and Cinacalcet treatment.

(Figure 35) Delay in Inactivation Process of VGSC Current by Glutamate (MSG), a T1R1 Agonist (HEK NaV1.5 Cells): When MSG is applied, the transient inactivation process of VGSC current is significantly delayed. As a result, the transient VGSC current component decreases, but simultaneously, the amplitude of a small sustained VGSC current following the transient VGSC current is enhanced. This was investigated using rectangular pulse stimulation for transient activation current (upper left panel) and ramp waveform for sustained current (upper right panel). The measured values from the experiment using rectangular pulse stimulation are plotted against time (current amplitude vs. time) in the lower diagram.

(Figure 36) Effects of MSG (Glutamate) and Aβ1-42 on Voltage-Dependent Potassium Channels (A) Using the voltage clamp technique with SH-SY5Y cells, MSG treatment delays the inactivation process of VGSC current and reduces the voltage-dependent potassium channel current. The upper left panel shows results before MSG administration, and the upper right panel shows results after administration. The lower right panel displays an I-V curve beneath outward potassium current traces. In the lower right diagram of (A), after a ramp waveform stimulation, the membrane potential is held at 40 mV, confirming gradual inhibition/reduction of potassium current. (B) Using HEK cells expressing Kv1.2, the effects of Aβ1-42 on potassium channels, causing a delay in VGSC inactivation similar to MSG, were investigated using the same ramp waveform. Aβ1-42 reduced Kv1.2 potassium current as observed in SH-SY5Y cell recordings. The upper section shows actual experimental traces, and the lower section plots the Aβ1-42-induced inhibition process of potassium channel current over time.

(Figure 37) Inhibitory Effects of IMP and Aβ1-42 on VGSC Current and Delayed Inactivation Process of VGSC Current: (A) The upper panel shows the reduction in VGSC current observed after treatment with 1 mM IMP compared to control. (B) Experimental results from (A) display the Steady-state inactivation curve and I-V plot. IMP decreases VGSC current but also exhibits a transient enhancement shortly after application. (C) Evaluation of IMP's effect on VGSC current using a voltage clamp experiment with ramp waveforms (downward arrow indicates VGSC current increase, followed by an upward arrow indicating VGSC current decrease). (D) and (E) analyze the effects of Aβ1-42 using the same voltage clamp method as in (A) and (B). Increasing concentrations of Aβ1-42 (12.5 nM and 25 nM) lead to concentration-dependent reductions in VGSC current, similar to MSG and IMP. Delayed inactivation of VGSC, as seen with IMP, is also confirmed.

(Figure 38) Effects of GMP on Inward VGSC Current and Outward Potassium Current in SH-SY5Y Cells. (A) Total current observed via voltage clamp before (left), after 2 mM GMP application (middle), and further after 5 mM MSG administration. (B) Creation of the I-V curve for inward VGSC current reveals enhancement due to GMP treatment. Potassium current (voltage-dependent) is also plotted. Steady-state inactivation curve is included. White symbols represent changes after GMP application. VGSC current shows slight increase and leftward shift in the I-V curve (downward arrow at -20 mV), while peak outward potassium current decreases (downward arrow at +50 mV). No change is observed in the Steady-state inactivation curve. GMP-induced enhancement of VGSC current is smaller than IMP and varies among recorded cells. GMP's effect persists over time, unlike MSG, and has minimal impact on inactivation processes. (C) As shown in (A), no MSG-like changes in inactivation processes are observed before and after GMP administration. Therefore, we administered MSG after GMP treatment and examined its effects on VGSC current using I-V plots. Under GMP presence, MSG causes approximately 10 mV leftward shift in the I-V curve (leftward arrow).

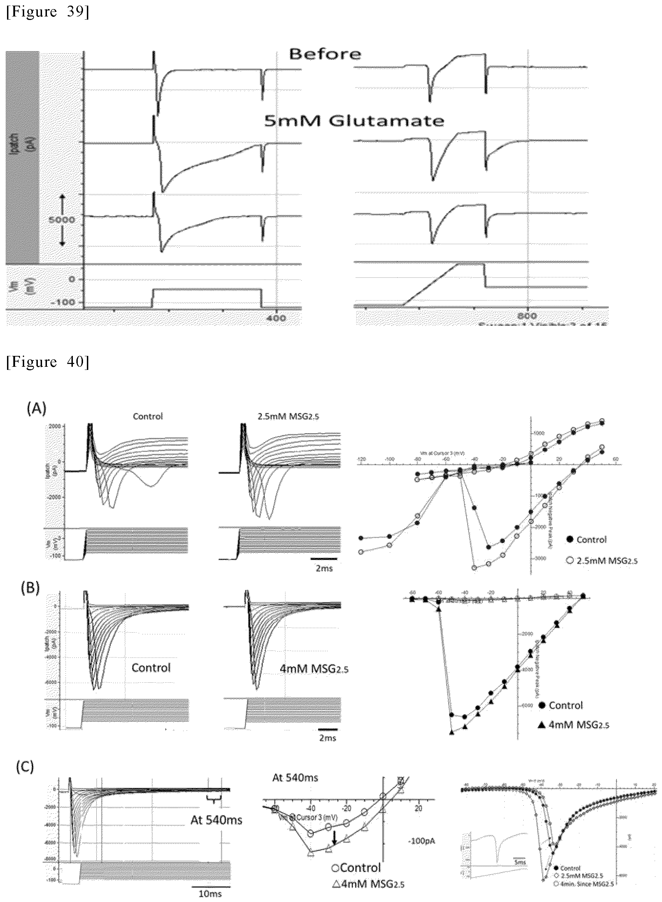

(Figure 39) Effect of Artificial Sweetener (Saccharin) on MSG's VGSC Current Inactivation Rate: Using voltage clamp experiments with rectangular pulse stimulation (left) for transiently activated VGSC current and ramp waveforms (right) for sustained current, we investigated the impact of 5 mM glutamic acid (MSG) on VGSC current kinetics in HEK Nav1.5 cells activated by T1R2 via saccharin. The top to bottom panels represents current waveforms before treatment (control), 30 seconds after MSG administration, and 10 minutes after MSG administration. The inactivation process of transient VGSC current was significantly delayed after MSG treatment. Although the effect weakened over time, it persisted even after 10 minutes. The bottommost panel shows the voltage waveforms used for stimulation.

(Figure 40) Investigated the effect of MSG2.5 on VGSC (voltage-gated sodium channel) currents in SH-SY5Y cells (human neuroblastoma cells) and Nav1.5 HEK cells. MSG2.5 amplified peak VGSC currents, but its impact on the inactivation process was limited. The upper diagrams labeled (A) and (B) show VGSC currents measured before and after MSG2.5 treatment using voltage clamp techniques. The right side of each diagram displays the I-V relationship (peak current vs. membrane potential). In the lower diagram labeled (C), the sustained VGSC current component after 540 ms of stimulation (not just the transient peak current) was analyzed using Nav1.5 HEK cells. The I-V relationship for this sustained current is shown in the center of diagram (C). The experiment used a ramp waveform (not a rectangular pulse) as shown on the right side of diagram (C). After MSG2.5 treatment, the peak current temporarily shifted in the hyperpolarizing direction at 1 minute and 12 seconds, but returned to a waveform similar to pre-treatment after 4 minutes.

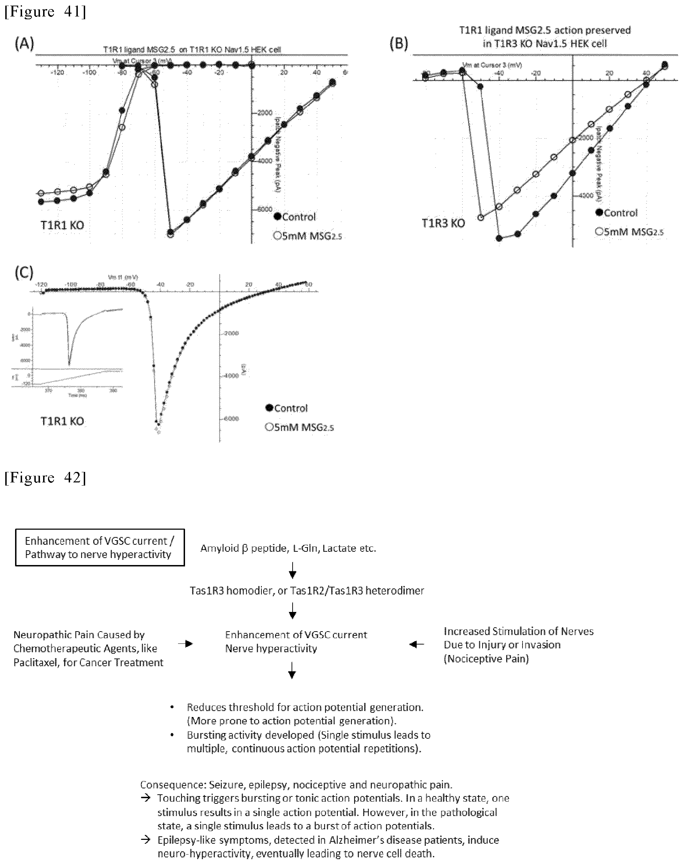

(Figure 41) Explored the effect of MSG2.5 on VGSC currents in HEK Nav1.5 cells with silenced T1R1 expression. In T1R1-silenced cells, the change in VGSC current induced by 5 mM MSG2.5 was inhibited. The lower diagram labeled © shows the observation and analysis of MSG2.5's effect on sustained VGSC currents before and after MSG2.5 treatment (stimulated every 8 seconds) in the same cell. However, rectangular pulse stimulation did not confirm MSG2.5's effect, similar to the results from cells without T1R3 silencing. This suggests that T1R1 expression is essential for MSG2.5's effect on VGSC currents, while the presence or absence of T1R3 does not influence MSG2.5's action.

(Figure 42) Mechanism of Pain and Seizure-Like Symptoms Due to Neuronal Hyperactivity The occurrence mechanism of pain and seizure-like symptoms due to neuronal hyperactivity involves several factors. These include sweet-tasting amino acids such as amyloid $\beta$-peptides and glutamine (L-Gln), as well as neuropathic pain resulting from chemotherapy. Chemotherapy drugs like paclitaxel increase voltage-gated sodium channel (VGSC) currents by polymerizing microtubules, leading to neuronal hyperactivity. Colchicine, which stabilizes microtubules but inhibits their polymerization, also increases VGSC currents. In this state, VGSC currents per neuron are enhanced, the action potential threshold decreases, and single action potentials can trigger burst-like activity involving multiple spikes.

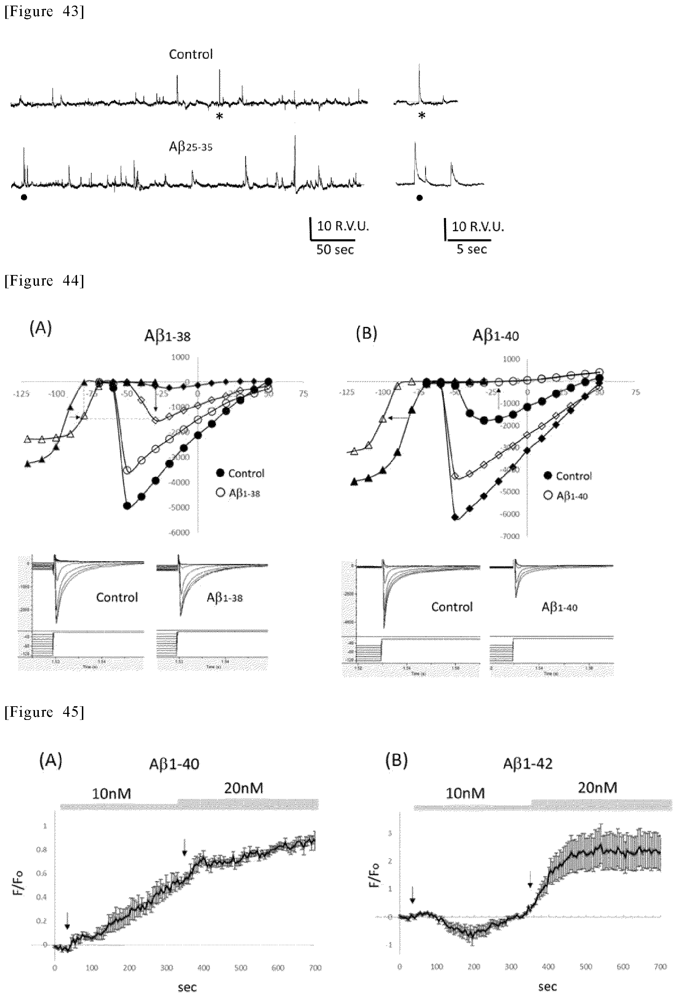

(Figure 43) Measurement of VGSC Enhancement by A$\beta$25-35 Peptide via T1R3 Using the Nano Charge Method: In differentiated SH-SY5Y cell cultures, A$\beta$25-35 peptide was added, and changes in intracellular membrane potential were measured. While the control group showed a low occurrence rate of action potentials, the A$\beta$25-35 peptide led to an increase in spontaneous action potential frequency.

(Figure 44) Changes in VGSC Currents upon Exposure to A$\beta$1-38 and A$\beta$1-40 in HEK Nav1.5: Cardiomyocytes in HEK Nav1.5 cardiomyocytes, exposure to A$\beta$1-38 and A$\beta$1-40 resulted in distinct effects on VGSC currents: For A$\beta$1-38 (Panel A), the Steady-state inactivation curve shifted to the right, enhancing currents originating from a smaller prepulse potential of -80 mV. However, currents from -120 mV prepulse decreased. Conversely, A$\beta$1-40 (Panel B) shifted the Steady-state inactivation curve to the left, resulting in reduced VGSC currents for both prepulse potentials.

(Figure 45) Effects of A$\beta$1-40 and A$\beta$1-42 on Intracellular Calcium. Using the fluorescent dye Cal-520 (AAT Bioquest), we measured changes in intracellular calcium concentration induced by A$\beta$1-40 and A$\beta$1-42 peptide administration in HEK cells expressing endogenous T1R GPCRs. Fluorescence microscopy was used for measurements. In panel (A), A$\beta$1-40 increased intracellular calcium levels, while panel (B) shows the dynamic response of intracellular calcium to A$\beta$1-42. A$\beta$1-42 exhibited a biphasic response, initially decreasing and then increasing intracellular calcium. Previously, studying taste responses to T1R GPCRs relied on overexpressing T1R GPCR genes and G16 or Gustducin/G16 for calcium response. However, this implementation example demonstrates that measurements and screenings using intracellular calcium response as an indicator can be achieved solely with the endogenous T1R GPCR expressed in HEK cells and the G proteins that couple with its receptor

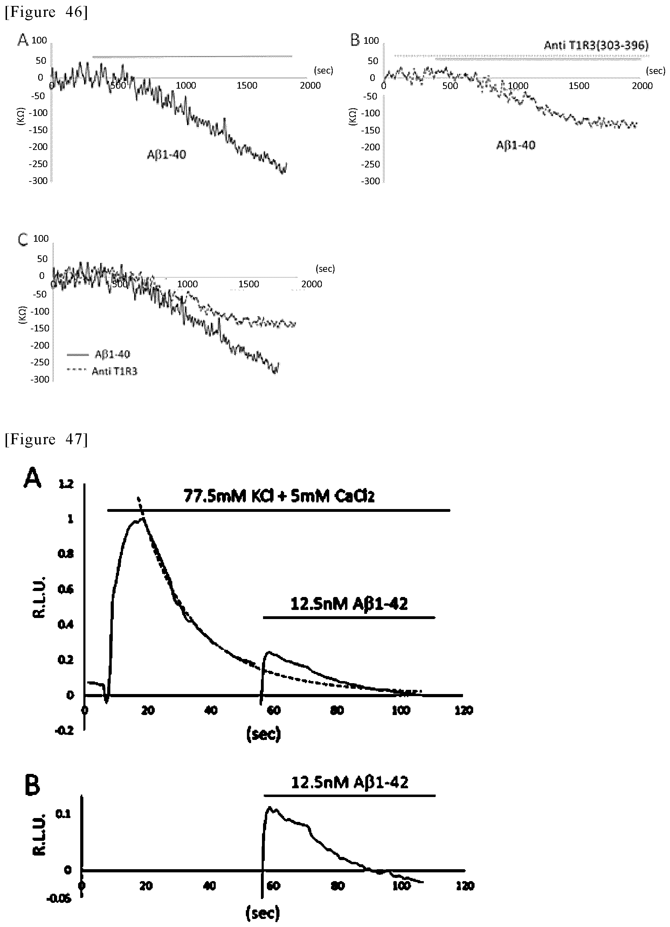

(Figure 46) Ligand Effects of A$\beta$1-40 and T1R3 Inhibition. Panel (A) shows the ligand effects of A$\beta$1-40 using HEK cells stably expressing Nav1.5. Panel (B) demonstrates the inhibitory effect of anti-T1R3(303-396) antibodies against A$\beta$1-40 peptide action at the ligand-binding site 2 (303-396aa) identified from patch-clamp experiments. Panel ©

compares antibody inhibition with Aβ1-40 peptide alone on the same scale. The vertical axis represents impedance (resistance, kilohms), and the horizontal axis represents time (seconds).

(Figure 47) Measuring Cav1.2 Activity via Intracellular Calcium Changes. In panel (A), we measured Cav1.2 activity by monitoring intracellular calcium concentration changes. The vertical axis (R.L.U., Relative Light Unit) is normalized to the strongest response (set as 1). External KCl (77.5 mM) depolarized the cell membrane, activating Cav1.2 channels. By increasing extracellular calcium to 5 mM, calcium influx through Cav1.2 channels was enhanced, facilitating measurement of intracellular calcium changes. Following KCl-induced Cav1.2 channel activation, Aβ1-42 was applied. Panel (B) shows the specific activation of Cav1.2 channels induced by Aβ1-42, fitted using the Hyperbolic Decline model for the unaffected response portion.

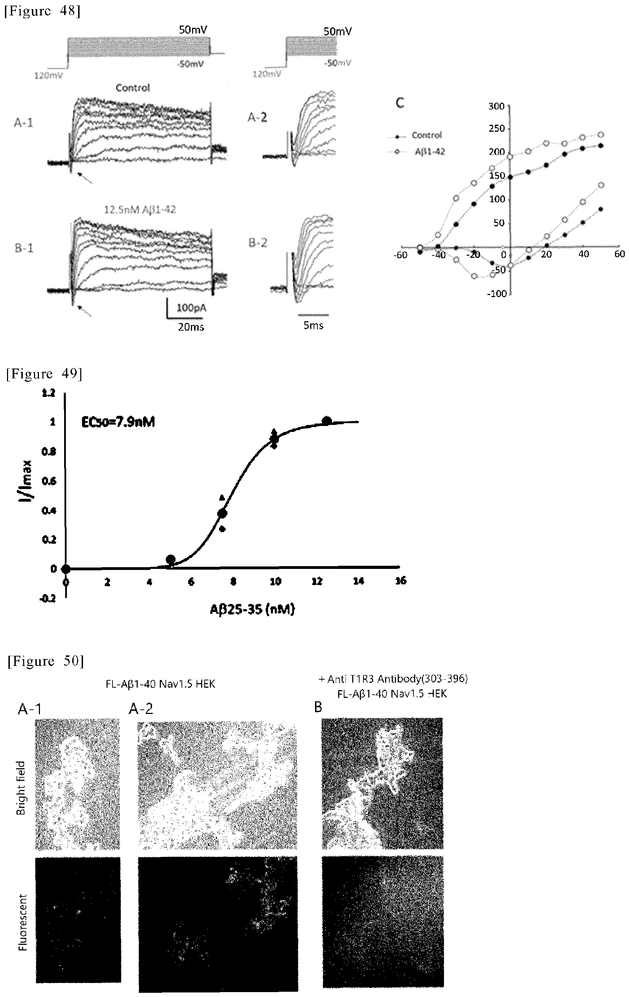

(Figure 48) Whole-Cell Current from Jurkat Cells. Panel (A) shows the whole-cell current from Jurkat cells. (A-1) and (B-1) indicate downward signals marked by arrows, which correspond to VGSC (voltage-gated sodium channel) currents. In (A-2) and (B-2), the time axis is expanded to facilitate observation of changes induced by Aβ1-42. The upper part of the current response shows the membrane potential changes used in the voltage-clamp method. The membrane potential was set to - 120 mV and stepped from -50 mV to +50 mV in 10 mV increments. In (C), inward VGSC currents (downward) and outward currents measured during the last 10 ms of voltage pulses are plotted in an X-Y plot. The horizontal axis represents the stimulus voltage, and the vertical axis represents the current magnitude.

(Figure 49) Concentration-Dependent Enhancement of Aβ25-35 on Nav1.5 Current. By using 10 mM NaOH as the solvent for Aβ25-35 to maintain it in a monomeric state, the EC50 value was calculated to be approximately 10 times lower (7.9 nM).

(Figure 50) The upper panel captures bright-field images, while the lower panel shows fluorescence images of Aβ1-40 peptide binding to cell surfaces. In (A), the labeled Aβ1-40 peptide binding is visible as a fluorescence signal. In (B), when cells treated with fluorescent Aβ1-40 were exposed to anti-T1R3 (303-396) antibody, Aβ1-40 binding nearly disappeared. To avoid fluorescence bleaching effects, unexposed regions were observed.

(Figure 51) In (A), Nav1.6 VGSC currents were recorded using the voltage-clamp method in membrane potential fixed mode. (•) represents control, △ represents after Aβ1-42 treatment, and (■) represents after T1R3 antibody treatment. VGSC currents were amplified at each step. In (B), a similar experiment was performed using thaumatin instead of antibodies. Unlike with T1R3 antibodies, further amplification of VGSC currents was not observed after Aβ1-42 treatment. However, thaumatin similarly amplified VGSC currents as Aβ1-42. Increasing the concentration did not lead to additional VGSC current amplification.

(Figure 52) In this Figure, the voltage clamp method was used to measure Nav1.5 VGSC (voltage-gated sodium channel) currents, and an I-V plot was created. Panel (A): The (•) represents the control condition before Aβ1-42 treatment. The VGSC current enhanced by 12.5nM Aβ1-42 is indicated by (△), while the VGSC current attenuated by the anti-T1R3 (400-570) antibody (Epitope 400-570) is shown as ( ▪ ). Panel (B): When T1R2 expression was suppressed using miRNA vectors, the effect of Aβ1-40 on VGSC currents disappeared. (•) represents the condition before Aβ1-42 treatment, and (△) represents the condition after treatment.

(Figure 53) (53-1): To investigate the effects on cell morphology, undifferentiated SH-SY5Y cells were treated with 120μM aspartame, an artificial sweetener and T1R2/T1R3 agonist. The results confirmed that T1R2/T1R3 stimulation by artificial sweeteners influenced cell morphology, causing cell contraction. (53-2): Neuronal-like differentiated SH-SY5Y cells were used to study the effects of β-alanine on cell morphology. These cells were differentiated in the presence of 1% FBS and retinoic acid. Lamellipodia changes over time were observed under an optical microscope (Panel A). The circular region highlighted in white showed morphological changes. Neurite terminal structures (initially fan-shaped) contracted and then expanded.

(Figure 54) HEK cells (A1) and HUEhT-1 cells (A2) were observed for changes in cell morphology due to Aβ1-40 treatment. The left column shows the control condition, while the right column compares cell morphology after Aβ1-40 treatment within the same field of view. Both (A1) and (A2) show individual cells undergoing contraction. In (B1) and (B2), a mixture of Aβ1-42 and Aβ1-40 (AD-D ratio observed in AD dementia) was applied to the same cells as in (A). Cell contraction was observed similarly to the (A) case, but the effect was weaker when Aβ1-40 acted alone in HUEhT-1 cells.

(Figure 55) In this figure, the Aβ42/40 ratio, measured in healthy individuals (Aβ ND), was applied to observe changes in cell morphology. While cell contraction was not prominent compared to patients showing dementia symptoms, ruffling at the cell periphery was observed.

(Figure 56) Mouse-derived brain microvascular endothelial cells (βEnd.3) were used to observe the impact of AβAD and AβND on cell morphology. Unlike human cells, most cells did not exhibit significant morphological changes except for a few exceptions.

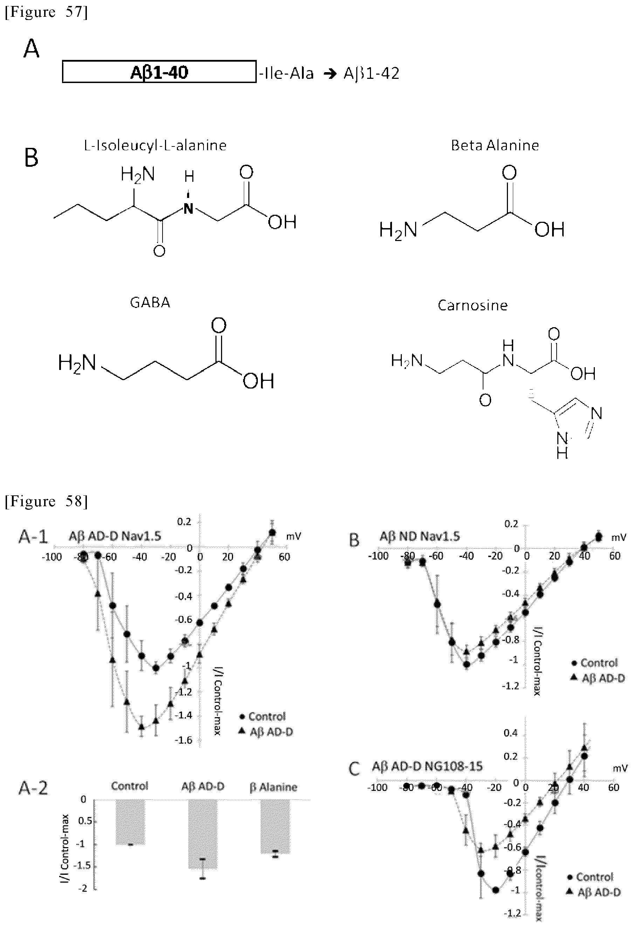

(Figure 57) When the C-terminal Ile-Ala (Isoleucyl-alanine) is cleaved from Aβ1-42, it becomes Aβ1-40. Isoleucyl-alanine has a similar structural formula to β-alanine. Panel (A): A diagram illustrating that Aβ1-42 has Ile-Ala added to the C-terminus of Aβ1-40. Panel (B): A representation of structurally similar compounds containing Isoleucyl-alanine and β-alanine. Carnosine, composed of histidine and β-alanine, is also shown with amino groups attached to the α, β, and γ carbons.

(Figure 58) Using whole-cell patch-clamp recording, VGSC currents were recorded from human-derived cells expressing human Nav1.5 (HEK, SH-SY5Y) or mouse/rat hybridoma cells (NG108-15) differentiated into neuron-like cells through forskolin and low FBS treatment. The effects of Aβ peptides (a mixture of Aβ40 and Aβ42) were observed. Two types of mixtures were used: one with the Aβ42/40 ratio observed in cerebrospinal fluid from healthy individuals (NDC) and the other with the ratio observed in AD patients with cognitive impairment (AD-D). (A-1) VGSC currents were enhanced by AD-D ratio Aβ42/40 compared to baseline (n=6). This enhancement was greater than when other Aβ peptides were applied individually. It qualitatively resembles the effect observed when Aβ25-35 was applied after actin depolymerization by latrunculin A (as shown in Figure 31). In the figure, (•) represents control, and (A) represents the effect of Aβ peptide mixture. VGSC current recordings were performed using voltage clamp at a membrane potential of -120 mV, with 80 ms stimulus pulses ranging from -80 mV to 50 mV in 10 mV increments. The maximum VGSC current from control recordings was averaged, and the effects of Aβ peptides were compared across multiple cells. I-V plots show mean values with error bars representing standard error. (A-2) When observing cell morphology, β-alanine activated lamellipodia and induced ruffling, suggesting increased cell motility and enhanced synaptic plasticity. Application of β-alanine to VGSC currents enhanced by AD-D ratio Aβ42/40 reduced the increase, nearly restoring it to control levels (n=2). The bar graph control is set to 1, showing the increase and recovery levels on average, with horizontal lines indicating actual experimental measurements. (B) Peptide mixtures with the Aβ41/40 ratio observed in healthy individuals were applied. Unlike AD-D, VGSC current changes were almost negligible (n=5). © When AD-D Aβ42/40, which significantly enhanced VGSC currents in human cells (human T1R2/T1R3), was applied to VGSC currents expressed in mouse/rat hybridoma cells (NG108-15), the mouse/rat-type T1R2/T1R3 showed a decrease rather than an increase (n=3).

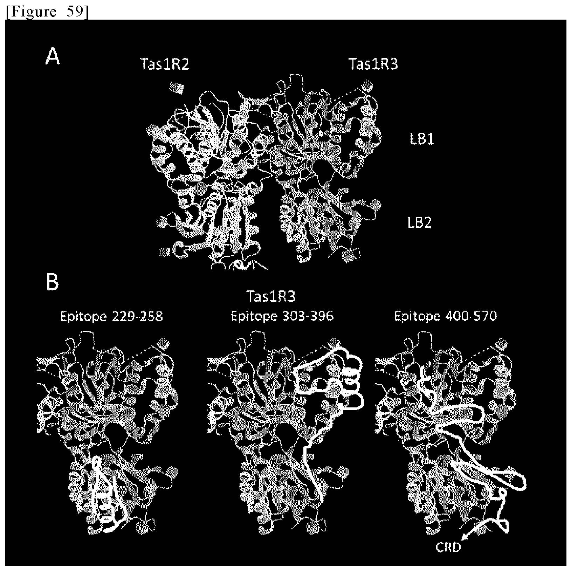

(Figure 59) The positions of peptides used for creating anti-T1R3 antibodies are indicated on the crystal structure according to Nuemket et al. (2017). Panel (A) shows the crystal structure of the T1R2/T1R3 heterodimer (PDB 5X2M) in complex with L-glutamine. Panel (B) displays the crystal structure of T1R3 alone, with white bars indicating the positions of the peptide sequences used as antigens. The amino acid sequences corresponding to LB2 (229-258), LB1 (303-396), and an intermediate region between LB1 and LB2 (400-570) containing the CRD can be confirmed.

[Embodiment for carrying out the invention]

1. Definition of terms

(1) In the present invention, it was discovered that the sweet taste G-protein-coupled receptor (GPCR) (T1R2/T1R3) is the first to regulate VGSC (voltage-gated sodium channel). Additionally, T1R2/T1R3 is believed to play a regulatory role in VGSC in various tissues, including neural and excitable cells such as muscles. Note that in this invention, when referring to "sodium channels" or "Na+ channels," it specifically refers to "voltage-dependent sodium channels (VGSC)."

It is known that G proteins regulate voltage-dependent sodium channels (VGSC). The action of G proteins also affects the threshold for action potentials and the ease of expression of action potentials. Furthermore, in neurons, G proteins influence the propensity for repetitive action potentials and their sustainability. This is recognized as a key regulatory factor determining the degree of membrane potential depolarization in dendritic spines, which in turn affects how action potentials are generated and the extent of neurotransmitter release at synapses (Gonzalez-Burgos and Barrionuevo, 2001; Schwindt and Crill, 1995; Williams, 1999).

Many GPCRs have been reported to phosphorylate VGSC (voltage-gated sodium channels) through the action of serine/threonine kinases (PKA, PKC), thereby modulating their activity. Activation of PKA has been documented to inhibit VGSC in various studies. For instance, muscarinic acetylcholine receptors in hippocampal pyramidal neurons (Cantrell et al., 1996), 5-HT2a/c receptors in cortical pyramidal neurons (Carr et al., 2002), and D1-class dopamine receptors in hippocampal pyramidal neurons and medium spiny neurons of the striatum (Cantrell et al., 1997; Surmeier et al., 1992) are examples where PKA activation leads to VGSC inhibition. Additionally, PKC activation has been associated with VGSC inhibition, such as the activation of D2 dopamine receptors in medium spiny neurons of the striatum and cholinergic interneurons (Carr et al., 2003).

(2) Substances acting on T1Rs (T1R1, T1R2, and T1R3) and their control of VGSC currents Below, I've summarized the effects of compound groups acting on T1R3 (T1R3/T1R3) in [Table 3], those acting on T1R2 (T1R2/T1R2 or T1R2/T1R3) in [Table 4], and those acting on T1R1 (T1R1/T1R1 or T1R1/T1R3) in [Table 5]. Notably, sweet-tasting amino acids like glutamine and serine found in cerebrospinal fluid (CSF) may enhance VGSC similarly to Aβ25-35, potentially leading to neuronal hyperexcitability and contributing to conditions like epilepsy or neuropathic pain. Furthermore, both glutamic acid (MSG) and inosinic acid (IMP), which act as umami substances on T1R1, are present in CSF (Barnett et al., 2020). These compounds could individually or cooperatively enhance VGSC via T1R1, potentially playing a role in epilepsy-like neurological disorders and/or neuropathic pain. (Note that glutamic acid and inosinic acid are sometimes referred to as "umami substances," commonly found in flavor enhancers.)

[Table3]

| | | | |
|---|---|---|---|
| T1R3 | L-Gln | L-Gln enhances VGSC current between 500uM and 1mM but not at higher concentrations. | Group 1 |
| | L-Serin | Enhances VGSC currentat 2.5mM | Enhances VGSC |
| | Aβ25-35 | Ab25-35 (125nM to 2μM) enhances VGSC current but not at higher concentrations. | Group 1 Enhances VGSC |
| | DichlorProp | Inhibit VGSC current in a concentration-dependent manner. | Group 1 Inhibits VGSC |
| | Clofibrate | | |
| | Lactisole | | |
| | Microtubules | Taxol and Colchicine mimic T1R3 agonists on microtubules. MSG's effect on T1R1 is independent of the microtubules' polymerization state | Enhances VGSC |
| T1R3 silencing | | T1R3 silencing nullifies ab25-35's enhancement of VGSC and cinacalcet's inhibition of VGSC. Eliminating T1R3 agonist/antagonist effects on VGSC. | Nullifies the effects on VGSC |

[Table4]

| | | | |
|---|---|---|---|
| T1R2 (T1R2/T1R3) | Aspartame | 50μM Aspartame inhibited VGSC by over 90%. Inhibition is due to a parallel shift of the S-S inactivation towards the hyperpolarizing direction, similar to the effects of DichlorProp and TBB. | |
| | Sucralose | IC50=37μM (SH-SY5Y) | Contrary to the amplifying effect of L-Gln and Amyloid beta peptide on VGSC currents, Sucralose and Saccharin purely inhibit VGSC. |
| | Saccharin | IC50=4mM | No significant impact on S-S inactivation. |
| | T1R2 Overexpression | S-S inactivation curve shifted to hyperpolarizing direction. | Effects similar to those observed with artificial sweeteners and TBB. |
| | T1R2 silencing | The S-S inactivation curve shifted to the hyperpolarizing direction even at control recordings. | |
| CK2 inhibitor (TBB) | | Similar to T1R2 overexpression, the S-S inactivation curve shifted to the hyperpolarizing direction even at control recordings. | |

[Table5]

| T1R1 | MSG | 5 mM MSG shifts the VGSC I-V curve to a more hyperpolarized direction. The inactivation speed of VGSC slows in 2 out of 3 SH-SY5Y cells. This delay in inactivation speed more pronounced in Nav1.5 cells. This delaying effect even more evident at 10 mM MSG. | Goup 4 VGSC enhancement |
|---|---|---|---|
| | | MSG 2.5 delays the inactivation process and can reverse and shift rightward the leftward shifted S-S inactivation curve caused by artificial sweeteners. | |
| | | MSG had no effect on VGSC enhancement in T1R1 silenced cells. | |
| | MSG2.5 | When applied together with IMP and GMP (2.5% combined as MSG2.5), the effect of MSG is enhanced. Within an MSG2.5 concentration range of 2.5 to 5 mM, VGSC is enhanced by 10-20%, and the I-V curve shifts 10 mV negatively. After 3-5 min., VGSC enhancement returns to control levels, but the I-V curve remains hyperpolarized. | |
| | IMP | 1mM IMP transiently enhanced VGSC currents by 10-20% and caused a 10mV leftward shift in the I-V curve (activation threshold became more negative). Within 2-3 min., the peak current reversed and eventually decreased by about 10%. However, the negative shift in the I-V curve persisted, resulting in VGSC activation at more negative membrane potentials than control. | |
| | GMP | 1mM GMP, much like L-Gln, primarily enhances VGSC without altering other kinetics. Its effect is limited to VGSC enhancement. | |
| | Aβ1-42 | The effect of Aβ1-42 (12.5nM to 1μM) was similar to IMP, delaying inactivation and shifting the activation threshold by 5-10mV towards hyperpolarization. In Nav1.5, the delay in inactivation was weaker than MSG but comparable to IMP. In SH-SY5Y cells, Aβ1-42's effect mirrored that of MSG. | |

2 . Disease to be treated in this invention/Control of VGSC current mediated through T1Rs

(1) Central nervous system disorders

(1-1) Pain and epilepsy-like hyperactivity symptoms due to excessive VGSC activity.

One of the main target diseases in this invention is believed to be caused by excessive VGSC activity resulting in pain and ultimately leading to neuronal cell death, which is the root cause of clinical symptoms. When sodium channels are activated, a transient influx of sodium ions occurs into the cell, playing a crucial role in generating action potentials. During this process, sodium channels rapidly inactivate within a few milliseconds after activation, and the subsequent activation of potassium channels allows potassium ions to flow out, mitigating the accumulation of intracellular cations caused by sodium influx and restoring the cell membrane potential to the resting membrane potential. The interplay between sodium and potassium channels is essential for efficiently and safely transmitting electrical signals from peripheral to central or vice versa. Delayed inactivation of VGSCs can lead to neuronal hyperactivity, arrhythmias, or epileptic-like seizures, ultimately resulting in neuronal cell death.

(1-2) Mechanism of Action of Aβ Peptide (Aβ25-35) related to the Onset of Alzheimer's Disease (AD) and AD Therapeutics

In Alzheimer's disease, neuronal hyperactivity (resembling epileptic seizures) is observed. While this mechanism is not limited to the following, it can be explained as follows, including the results of the present invention.
Specifically, amyloid β peptide (Aβ25-35) binds to T1R3, recruiting voltage-gated sodium channels (VGSC) derived from intracellular endosomes to the cell membrane, thereby enhancing neuronal activity. This process is believed to contribute to the epileptic-like neuronal hyperactivity observed in Alzheimer's disease. In cells where T1R3 is silenced, the VGSC enhancement mentioned above does not occur. Therefore, drugs that inhibit the binding of Aβ25-35 to T1R3 or antibodies against T1R3 could serve as therapeutic targets for treating neuronal hyperactivity. Additionally, in neurons where hyperactivity has already occurred, VGSC activity can be suppressed via T1R2/T1R3 using substances (drugs) that depolymerize the cell cytoskeleton (microfilaments), such as dichlorprop or artificial sweeteners (aspartame).

Antagonists (or T1R2 agonists) belonging to 'Group 3', including dichlorprop and artificial sweeteners (aspartame), are effective as potential treatments for Alzheimer's disease (AD), where Aβ25-35 is considered a causative factor due to its inhibitory effect on binding to T1R3.

Furthermore, anti-T1R3 antibodies completely block the binding of Aβ25-35 peptide to T1R3 when observed by competing with fluorescent Aβ25-35 peptide on the cell surface. Therefore, anti-T1R3 antibodies hold promise as potent AD therapeutics.

As a symptomatic therapy to suppress VGSC hyperactivity, substances such as aspartame, Gymnema tea extract (containing gymnemic acid), and agonists of the calcium-sensing receptor (CaSR) like Cinacalcet, as well as CaSR antagonists (such as NPS-2143), are effective in inhibiting VGSC activity.

(1-3) Neurological Disorders with Epileptic-Like Activity Similar to Alzheimer's Disease (AD)

In addition to Alzheimer's disease, there are numerous neurological disorders that often exhibit epileptic-like neural hyperactivity. These conditions are presumed to develop through mechanisms similar to those observed in Alzheimer's disease. Some of the disorders that may develop via such mechanisms include:

- Alzheimer's Disease: Characterized by memory impairment.

- Lewy Body Dementia: Presents with cognitive impairment and hallucinations.

- Cerebrovascular Disorders: Localized brain neurologic symptoms occur due to cerebral infarction (ischemic or hemorrhagic).

- Epilepsy: Manifests as partial seizures or complex seizures, with varying symptoms based on seizure type.

- Parkinson's Disease: Essential features include bradykinesia (slowness of movement), along with rigidity or resting tremor. Other symptoms include postural reflex impairment, non-motor symptoms like constipation, REM sleep behavior disorder, and reduced sense of smell.

- Progressive Supranuclear Palsy: Features parkinsonism (bradykinesia, rigidity, gait disturbance), dysphagia, instability during walking, and vertical gaze abnormalities.

- Multiple System Atrophy: Presents with parkinsonism, dysphagia, cerebellar symptoms (unsteadiness during walking), falls, orthostatic hypotension, and autonomic dysfunction affecting the bladder and rectum.

- Amyotrophic Lateral Sclerosis (ALS): Progressive weakness, atrophy, dysphagia, and tongue atrophy in the limbs.

- Traumatic Brain Injury: Symptoms vary based on the site of injury. Localized brain neurologic symptoms occur in areas affected by trauma.

- Huntington's Disease: Characterized by choreiform movements (involuntary dance-like motions), psychiatric symptoms, and dementia.

- Spinal Cerebellar Ataxia: Symptoms include unsteadiness, difficulty walking, impaired hand function, and loss of smooth movements.

- Multiple Sclerosis: Recurrent motor paralysis, visual impairment, sensory deficits, and demyelination characterize this disorder.

(1-4) Pathological conditions induced by delayed inactivation of VGSC

The delayed inactivation of voltage-gated sodium channels (VGSC) by monosodium glutamate (MSG) leads to the pathological conditions described in [Table 4]. While MSG-induced neuronal hyperactivity was previously thought to be solely due to glutamate receptor activation, the delayed inactivation of VGSC currents through T1R1 and T1R1/T1R3 also contributes to neuronal hyperexcitability. Inhibitors targeting T1R1 and T1R1/T1R3 (preventing binding to MSG's T1R1 receptor) are highly effective as drug targets for treating the mentioned diseases. When both enhancement of VGSC currents via T1R2/T1R3 mediated by sweet-tasting amino acids such as glutamine and serine, and MSG-induced delayed inactivation of VGSC occur simultaneously, neuronal hyperexcitation leading to neuronal cell death is easily anticipated. This phenomenon is particularly relevant in conditions like stroke and spinal cord injury, where neurons and astrocytes may undergo necrotic cell death due to mechanical trauma, resulting in the release of intracellular amino acids into the extracellular space.

(1-5) Physiological Roles of Aβ25-35 and Aβ1-42

Aβ peptides are found in cerebrospinal fluid (CSF) and are also known to be released from synapses (Lehmann et al., 2020; Groemer et al., 2011). Let's discuss the physiological functions of Aβ peptides in long-term potentiation (LTP) at synaptic cells.

When synaptic plasticity is induced in neurons by high-frequency repetitive stimulation (tetanic stimulation), LTP occurs. During this process, it is believed that Aβ peptides are released simultaneously with the neurotransmitter glutamate from the presynaptic terminal into the synaptic cleft.

The prevailing theory suggests that massive glutamate release leads to activation of NMDA-type glutamate receptors, causing calcium influx into the postsynaptic terminal. This influx results in the insertion of AMPA-type glutamate receptors from intracellular endosomes to the cell surface (synaptic cleft), leading to increased efficiency in synaptic signal transmission.

In this context, the present study discovered that AMPA-type glutamate receptors (Park et al., 2004) are inserted into the cell membrane (postsynaptic membrane) from intracellular endosomes during LTP. Similarly, voltage-gated sodium channels (VGSCs) are inserted into the cell membrane via the T1R3 receptor by Aβ25-35, increasing current density.

Furthermore, glutamate itself delays VGSC inactivation through the T1R1 receptor, enhancing sustained inward VGSC currents and facilitating burst-like activity in cells. Additionally, glutamate was found to suppress membrane potential-dependent potassium channel currents (e.g., Kv1.2) through the T1R1 receptor. This effect creates a situation where cells are more prone to excitability.

Interestingly, Aβ1-42, even at nanomolar concentrations, exerts effects similar to glutamate on VGSCs and voltage-gated potassium channels (VGKCs) via the T1R1/T1R3 receptors.

These findings highlight the essential role of Aβ peptides not only in Alzheimer's disease but also in other conditions involving cell death and disease symptoms related to amyloidosis. Exploring positive allosteric modulation (PAM) of T1R1 as a potential drug target could improve symptoms such as memory impairment.

(2) Neuropathic Pain

(2-1) Mechanism of Action of Neuropathic Pain and Scope of Therapeutic Agents According to Said Mechanism The present invention also targets the effective treatment of neuropathic pain associated with neuronal hyperactivity based on amplification of VGSC (voltage-gated sodium channel) currents. The typical mechanism of action for such neuropathic pain is illustrated in (Figure 42).

The neuropathic pain targeted by the present invention includes not only pain resulting from chemotherapy administration (such as taxanes) but also chronic neuropathic pain syndromes caused by conditions like failed back surgery syndrome (FBSS), complex regional pain syndrome (CRPS), and diabetic polyneuropathy. Other neuropathic conditions that may arise from similar mechanisms include:

- Cancer-Related Neuropathy (Paraneoplastic Peripheral Nerve Disorders): Neuropathy beyond direct nerve compression by tumors.

- Peripheral Neuropathy Due to Chemotherapy (Neurotoxicity): Numbness, pain, and reduced sensation in the extremities caused by agents like paclitaxel.

Furthermore, in neurological disorders where seizure-like neuronal hyperactivity (as described in (1)) is observed, VGSC action potentials rapidly increase in all nerve cells, including those in the central nervous system (CNS) and peripheral nervous system (PNS). Consequently, it effectively prevents and treats neuropathic pain associated with the conditions mentioned in (1).

In the context of neuropathic pain, one of the mechanisms involves the enhancement of VGSC (voltage-gated sodium channel) currents on the cell membrane, leading to neuronal hyperactivity and pain. Various substances may contribute to this process, including chemotherapy drugs used in cancer treatment, as well as compounds like amyloid β peptides, sweet-tasting amino acids such as glutamine (L-Gln), and umami substances like inosine. (Figure 42) illustrates one of the proposed mechanisms for neuropathic pain development related to VGSC currents.

Specifically, chemotherapy agents like paclitaxel increase VGSC currents by promoting microtubule polymerization. Colchicine, which inhibits microtubule polymerization while stabilizing them, also leads to increased VGSC currents. In this state, VGSC currents per nerve cell are enhanced, the action potential threshold decreases, and action potentials become more likely to occur. Consequently, stimuli that induce single action potentials can trigger bursts of sustained activity potentials.

As shown in (Figure 42), the mechanism involving VGSC current enhancement due to sweet-tasting amino acids (such as amyloid β peptide, Aβ25-35 and glutamine, L-Gln) within the cerebrospinal fluid (CSF) shares commonality with the neuropathic pain development caused by chemotherapy agents.

(3) Other Diseases Associated with Amyloid β Peptide

(3-1) Cardiac Diseases Involving Abnormal VGSC (or VGKC) Currents Mediated by Amyloid β Peptide via T1Rs

Heart failure, like Alzheimer's disease, is a significant modern ailment that not only affects quality of life but also poses a danger to life itself. Troncone et al. (2016) used echocardiography to examine cardiac function and found that patients with Alzheimer's disease exhibited impaired cardiac diastolic function in addition to memory deficits. The expression levels of Aβ1-40 and Aβ1-42 within the myocardium were elevated in Alzheimer's patients compared to healthy individuals.

Given that Type I taste receptors (T1R1, T1R2, and especially T1R3) serve as receptors for amyloid β peptides, they are believed to play a crucial role in the pathogenesis of various neuronal disorders. In neurons, amyloid β peptides insert into ion channels such as VGSC and VGKC from intracellular pools, inducing hyperactivity. This neuronal hyperactivity resembles epileptic activity and ultimately leads to neuronal cell death (as seen in Alzheimer's disease and ALS). Specifically, amyloid β25-35 peptide acts on T1R3, increasing ion channel activity on the cell membrane, while amyloid β1-42 peptide interacts with T1R1 and T1R1/T1R3 umami receptors. The latter delays VGSC inactivation, contributing to sustained VGSC currents and burst-like action potentials. However, when amyloid B1-42 peptide levels rise, it may decrease VGSC activity, similar to MSG (glutamate), which could also apply to cardiomyocytes expressing high levels of T1R3.

Kim et al. (2011) reported that Cav 1.2 currents, equivalent to voltage-gated L-type calcium channels (VGCCs) expressed in the myocardium, are amplified by the action of amyloid β25-35 peptide, a phenomenon that can be explained by the T1R3 receptor identified in this study functioning as the receptor for amyloid β25-35 peptide.

Troncone et al. (2016) reported that amyloid β1-40 peptide is present in the myocardium of patients with Alzheimer's-type dementia who develop cardiac diastolic dysfunction. Additionally, Shibata et al. (2006) reported that NaV1.5, CaV1.2a, and KV1.5 coexist in caveolae within the recycling endosomes of cardiomyocytes. Palygin et al. (2008) further suggested that the α subunit of G proteins (Gs alpha) binds to caveolin 3 (Caveolin3), a membrane protein localized in recycling endosomes, facilitating its translocation to the cell membrane. Considering this, amyloid β1-40 peptide may contribute to myocardial hyperactivity and could potentially be a cause of arrhythmias such as tachycardia.

The observed effects of Aβ1-40 peptide deposition in various diseases and its correlation with these conditions imply that Aβ1-40 peptide may act as a ligand for T1R GPCRs, influencing cytoskeletal polymerization downstream. Further research in this area could lead to valuable discoveries for disease treatment and drug development.

(3-2) Other Cardiac Disorders Potentially Involving Amyloid β Peptides

Troncone et al. (2016) suggested that Alzheimer's-type dementia, characterized by myocardial amyloid β peptide deposition and heart dysfunction, may be a systemic disorder causing multi-organ failure, including heart disease or metastatic conditions.

Numerous studies have explored the correlation between amyloid β peptides and heart disease. While amyloid β peptide localized in brain neurons (Aβ1-42) differs in size from platelet-derived amyloid β peptide found in non-neuronal tissues like the heart (A β 1-40), the onset of heart disease due to amyloid β peptides is not limited to Alzheimer's-type dementia patients (Troncone et al., 2016).

If this holds true, myocardial deposition and heart dysfunction related to amyloid β peptides may be attributed to Aβ1-40 derived from platelets rather than Aβ1-42 found in brain neurons. Notably, Aβ1-40's effect on VGSC differs from that of Aβ1-42. In cardiac expression of Nav1.5, Aβ1-40 inhibits VGSC by shifting the S-S curve towards depolarization. This effect resembles the action of artificial sweeteners targeting T1R2/T1R3 sweet taste receptors, unlike the results observed when Aβ1-42 interacts with T1R1/T1R3 umami receptors. Given that the heart muscle functions as a syncytium, inhibition of action potential generation by Aβ1-40 in some cardiomyocytes could disrupt periodic conduction, potentially leading to arrhythmias.

Inyushin et al. (2020) have pointed out that platelet-derived amyloid β peptide is involved in the deposition of Aβ in the brain, cancer, glaucoma, and skin, as well as in pre-eclampsia, leading to Alzheimer's-type dementia and late-stage Parkinson's disease. Specifically, in glaucoma, amyloid β peptide deposits in the retinal ganglion cell layer, causing cell death in the retina, and also affects the surrounding microvasculature. The therapeutic effect of anti-amyloid β antibodies has been confirmed in glaucoma. T1R and T3R are common GPCR-type receptors expressed in all tissues (The Human Protein Atlas: (https://ww.proteinatlas.org/), suggesting that amyloid β peptide deposition on T1R or T3R may be involved in retinal ganglion cell death.

According to Stamatelopoulos et al. (2017), amyloid β1-40 (Aβ40) primarily accumulates in brain blood vessels (not neurons) and contributes to cerebral amyloid angiopathy. Aβ40 is also present in the myocardial tissue of Alzheimer's disease and heart failure patients, promoting atherosclerosis and exerting harmful effects on the cardiovascular system. Circulating Aβ1-40 levels correlate with the presence and severity of atherosclerosis and predict cardiovascular mortality in patients with coronary artery disease. Plasma-derived Aβ1-40 levels are elevated in heart failure patients compared to controls and are associated with risk factors for heart failure, including renal dysfunction, diabetes, and coronary artery disease.

Similarly, Bayes-Genis et al. (2017) report that circulating Aβ1-40 is elevated in all cases of outpatient heart failure, serving as a predictive factor for heart failure etiology. Notably, unlike Aβ1-42, plasma Aβ1-40 concentration is not associated with cognitive impairment.

These findings suggest that Aβ1-40 binding to T1Rs in the cardiovascular system may contribute to heart failure.

A history of atherosclerotic arterial disease has been reported to promote the occurrence of arrhythmias through electrical remodeling after acute myocardial infarction, similar to hypertension (Guvenc et al. (2010), De Jesus et al. (2015)). It is believed that Type I taste receptors (T1Rs), which are considered receptors for amyloid β peptides (Aβ), may influence the polymerization state of cardiac myocyte cytoskeletal fibers and affect ion channel function, thereby contributing to arrhythmia development.

In this context, Taxol, which enhances VGSC (voltage-gated sodium channel) activity, has been used in the present invention. Besides stabilizing microtubules, Taxol increases the proportion of depolymerized microtubules along with cell cytoskeletal density. As a result, cell rigidity is enhanced (Kerr et al., 2015).

The relationship between myocardial function and cytoskeletal structure has been elucidated in clinical studies. Increased rigidity of the cytoskeleton due to depolymerization of microtubules occurs similarly in cardiac muscle. When many microtubules (polymerized tubulin) undergo depolymerization, muscle mechanical resistance (stiffness) increases, making it difficult for muscles, including cardiac muscle, to contract (Chen et al., 2018). Chen and colleagues also reported that proteomic analysis consistently showed highly depolymerized microtubule networks, characteristic of heart failure, in the left ventricle of individuals with heart failure. This finding correlates with increased rigidity of cardiac myocytes and impaired contractility.

Considering these factors, it is plausible that amyloid β peptides may act on cardiac myocyte cytoskeleton via Type I taste receptors (T1Rs), potentially triggering cardiac dysfunction.

(3-3) Other diseases possibly related to amyloid β1-40 peptide

Regarding Aβ1-40 peptide, the concentration of Aβ1-40 in the blood is positively correlated with kidney dysfunction and diabetes. Considering that Type I taste receptors (T1Rs) function as receptors for Aβ1-40 peptide, similar to the heart, they may also contribute to cellular dysfunction in the kidneys and pancreas. For example, when we examine kidney disorders such as nephrotic syndrome and renal failure, we find that amyloid β25-35 and amyloid β1-42 have an effect on microtubules and microfilaments in nerve cells (Pianu et al., 2014). Similarly, Aβ1-40 may also influence microtubules and microfilaments in any of the kidney cells. The kidneys play a crucial role in filtering waste products from the blood and excreting them as urine. When kidney function declines, waste accumulates in the blood, affecting various organ functions. Podocytes, which are essential for kidney function, can develop nephrotic syndrome, characterized by symptoms like proteinuria, when their cellular skeleton is inhibited. Therefore, chronic stimulation of Type I taste receptors by amyloid β1-40 peptide on podocytes could lead to the disassembly of the cellular skeleton (especially microfilaments), resulting in impaired kidney function (Schell et al., 2014). This suggests that alterations in cellular skeleton assembly/-disassembly may impact cell morphology.

In diseases potentially caused by Aβ1-40, Type I taste receptors may lead to functional impairments in various organs due to their effects on ion channels (especially VGSC and VGKC) or cellular skeleton structures (promoting or inhibiting polymerization/disassembly). Particularly, if structural effects on microtubules and microfilaments are considered as the cause of the disease, measuring the polymerization state of microtubules and microfilaments using markers like GFP could be an effective diagnostic and drug screening method.

(4) Administration method and dosage of therapeutic pharmaceutical compositions according to the present invention. The substances that act on Type I taste receptors (T1Rs), particularly those stimulating T1R2/T1R3, provided by the present invention can suppress excessive activity of VGSC, which causes epilepsy-like hyperactivity in cerebrospinal fluid (CSF) and inflammatory pain in nerve cells. Therefore, they can be used as preventive or therapeutic pharmaceutical compositions for Alzheimer's disease (AD) and other conditions. Additionally, they may be used in conjunction with existing treatments for AD, amyotrophic lateral sclerosis (ALS), and other diseases. Furthermore, substances stimulating T1R2/T1R3 can serve as effective components in pharmaceutical compositions for preventing or treating neuropathic pain. They can be used in combination with existing analgesics such as opioid formulations or other drugs like epinephrine (a vasoconstrictor) to minimize side effects on other organs. The administration routes for these pharmaceutical compositions include oral, nasal, rectal, and non-oral methods. For oral administration, various dosage forms such as powders, granules, capsules, tablets, elixirs, suspensions, emulsions, and syrups can be chosen and modified for sustained release, stability, disintegration, enteric coating, easy absorption, and other properties. For intravenous, intramuscular, or subcutaneous administration, injection solutions or drip solutions (including reconstituted dry products) can be selected as appropriate. Depending on the dosage form (oral or various non-oral forms), pharmaceutical compositions according to the present invention can incorporate carriers and additives that are pharmaceutically acceptable. These include solvents, excipients, coating agents, bases, binders, lubricants, disintegrants, dissolution enhancers, suspending agents, thickeners, emulsifiers, stabilizers, buffers, isotonic agents, pain relievers, preservatives, flavor enhancers, fragrances, and colorants.

When using the pharmaceutical composition of the present invention as a preventive or therapeutic agent for diseases related to VGSC activation due to binding to Aβ peptides (such as Aβ25-35 peptide) or sweet-tasting amino acids in CSF, umami substances, etc. (for example, Alzheimer's disease, epilepsy, ALS, and prevention or treatment of neuropathic pain), the oral dosage should preferably be in the range of 0.03 to 300 mg/kg body weight, and more preferably 0.1 to 50 mg/kg body weight, converted from the amount of the compounds of the present invention. For intravenous administration, an effective blood concentration range of 0.2 to 50 μg/mL, and more preferably 0.5 to 20 μg/mL, can be used. The same applies when using it for the prevention or treatment of neuropathic pain.

Please note that these dosages may vary based on factors such as age, gender, weight, severity of the condition, and route of administration.

Among the compound groups belonging to 'Group 3', which can inhibit VGSC activity by stimulating typical T1R2/T1R3, artificial sweeteners such as aspartame, dichlorprop, and cinacalcet are included. Although I'll provide detailed dosages and usage examples below, it's important to note that the effective compounds for the pharmaceutical composition of the present invention are not limited solely to these specific compounds or their numerical ranges.

For instance, in the case of the artificial sweetener aspartame, it inhibits VGSC at an IC50 value of 15-17 μM. Since aspartame is fully digested in the small intestine, non-oral administration (such as intravenous injection) is preferable. The goal of administration is not complete VGSC inhibition but rather reducing firing frequency and preventing burst generation. Therefore, the recommended concentration for intravenous administration is 4.5-10 mg/kg. Aspartame's impact on cardiac VGSC (NaV1.5) is considered low at membrane potentials near -100 mV.

Given that aspartame's effects tend to weaken in the presence of Aβ peptides, it may be more effective as a pain-relieving agent for inflammation or damage-related pain than for AD treatment.

In the case of dichlorprop, it acts on VGSC (Nav1.7, Nav1.6) expressed in neurons (SH-SY5Y) at lower concentrations, significantly increasing membrane potential before action potential expression. Particularly in neural activities showing burst-like patterns (such as pain, epileptic seizures, and AD), it effectively operates at lower concentrations (IC50 values ranging from 9 μM to 17.3 μM). Whether administered orally or non-orally (e.g., intravenously), doses of 2-10 mg/kg are likely effective. Higher concentrations may be necessary if needed, but the upper limit of dosage concentration is determined by not inhibiting cardiac VGSC (NaV1.5) at the resting membrane potential.

For cinacalcet, at 5 μM, it completely inhibits VGSC activity. However, if administered systemically, significant side effects are expected. Therefore, its role as a local anesthetic pharmaceutical composition is recommended. The administration method involves local injection for local anesthesia. The preferred concentration is 1-2 mg/kg (including 1:100,000 epinephrine).

In cases where pain sites require relief due to inflammation or other factors (such as end-stage cancer pain or acute apical periodontitis during dental treatment), conventional local anesthetics (like lidocaine) may not be effective due to pH dependence. In such situations, including epinephrine (as with typical local anesthetics) can constrict local blood vessels, mitigate systemic side effects, and ensure safe administration, making it an effective analgesic.

3. Screening Method

(1) "Type I taste receptors (T1Rs)" as drug targets related to the increase of amyloid β peptides and sweet amino acids (such as serine, glutamine, etc.) and umami substances (such as glutamic acid, inosinic acid) in cerebrospinal fluid (CSF) In this invention, among Aβ peptides, Aβ25-35 peptide enhances VGCS through T1R3, while Aβ1-42 peptide at nanomolar concentrations delays inactivation of VGSC through T1R3 and inhibits membrane potential-dependent potassium channels (such as Kv1.2), thereby enhancing neuronal (myocardial) hyperactivity. On the other hand, glutamic acid, known as an agonist for glutamate receptors, has an excitatory neurotransmitter effect. It delays VGSC inactivation through T1R1, inhibits membrane potential-dependent potassium channels (such as Kv1.2), and enhances neuronal (myocardial) hyperactivity (data not shown).

Considering that enhanced VGSC can lead to neuronal cell death and contribute to conditions like Alzheimer's disease (AD), it's worth noting that not only Aβ25-35 or Aβ1-42 peptides with agonist-like effects but also sweet amino acids (such as glutamine, serine) present in cerebrospinal fluid (CSF) and umami substances (such as glutamic acid, aspartic acid, and inosinic acid) could be potential drug targets for therapeutic purposes. Specifically, substances inhibiting the binding of glutamine (L-Gln), an amino acid that shows elevated levels in CSF in conditions like Alzheimer's disease, epilepsy, and ALS, may have therapeutic potential. Here, "inhibitory substances" for T1Rs refer to compounds that either directly inhibit Aβ peptide binding to T1Rs or interfere with the signal transmission after binding to T1Rs.

This invention provides an efficient method for screening such "T1R" inhibitory substances.

(2) In vitro screening method for substances that inhibit the function of Aβ peptides or sweet amino acids in cerebrospinal fluid (CSF) from binding to T1Rs: Aβ peptides or sweet amino acids in CSF are believed to enhance voltage-gated calcium channels (VGSCs) or voltage-gated potassium channels (VGKCs) on the surface of target cells by binding to T1Rs. Consequently, this mechanism leads to cell death in the target cells. To search for substances that inhibit the function of Aβ peptides or sweet amino acids binding to T1Rs or their complexes, effective screening methods are needed.

The effect of Aβ peptides or sweet amino acids binding to T1R3, a Type I taste receptor (T1R) that includes VGSCs, is thought to involve multiple steps:

i) First, the process of Aβ peptides or sweet amino acids binding to T1Rs (T1R1/T1R3, T1R2/T1R3, GPCR).

ii) Activation of G proteins resulting from the binding of Aβ peptides or sweet amino acids, leading to T1R activation.

iii) Subsequent polymerization or depolymerization of microfilaments and microtubules.

iv) Finally, control mechanisms that enhance or inhibit VGSC or VGKC currents.

Additionally, activation of T1R GPCRs leads to the activation of G protein β and γ subunits, which in turn activate adenylate cyclase and phospholipase C. This activation affects IP3 channels and Ryanodine receptor channels, releasing calcium from the endoplasmic reticulum (ER) and increasing intracellular calcium concentration.

Based on the above, several points may serve as drug targets to inhibit or modulate the effects of Aβ peptides or sweet amino acids on VGSC or VGKC currents in cells. These include:

(1) Preventing the binding of Aβ peptides or sweet amino acids to T1Rs (e.g., T1R3) themselves: - For instance, searching for compounds such as anti-T1R3 antibodies or equivalents.

(2) Exploring compounds that inhibit the activation of T1Rs resulting from the binding of Aβ peptides or sweet amino acids to T1Rs: - For T1R3, compounds like lactisole or dichlorprop that specifically bind to T1R3 and prevent the conformational changes induced by Aβ peptide or sweet amino acid binding, thereby inhibiting downstream G protein signaling.

(3) For taste receptors (e.g., T1R2/T1R3), exploring compounds that activate T1R2 (agonists) and have inhibitory effects on VGSC currents: - These compounds could suppress the amplification of VGSC currents induced by Aβ peptides (especially Aβ25-35) or sweet amino acids (e.g., glutamine) binding to T1R3.

The approaches described in (2) and (3) could also be applied to the search for substances based on changes in intracellular calcium concentration as an output.

In the method for measuring the effects of Aβ peptides or sweet-tasting amino acids in cerebrospinal fluid (CSF) using the voltage clamp technique (patch-clamp method) described in (2-3) below, the most accurate and useful approach is employed. However, measurements using the voltage clamp technique require skilled and intricate operations performed under high-magnification microscopy. To simplify and improve screening efficiency, the observation methods based on fluorescent labeling described in (2-1) and (2-2) are effective.

For instance, by applying drug screening principles from (2-1), changes in the cell cytoskeleton resulting from the binding of fluorescently labeled Aβ peptides or sweet-tasting amino acids in CSF to the T1R3 receptor can be observed using fluorescence microscopy. This allows the confirmation of changes caused by the binding of Aβ peptides or sweet-tasting amino acids, aiding in the search for substances that prevent such changes.

Similarly, when conducting screening based on points (2) and (3), measuring the polymerization state of microfilaments and microtubules visualized by fluorescently labeled actin and tubulin using the method described in (2-2) enables the observation of cell cytoskeleton polymerization states resulting from the binding of Aβ peptides or sweet-tasting amino acids to the Type I taste receptor (T1R3). This facilitates the screening of substances that inhibit changes in voltage-gated sodium channels (VGSC) or voltage-gated potassium channels (VGKC) caused by the binding of Aβ peptides to the Type I taste receptor.

Additionally, similar screening can be achieved by monitoring intracellular calcium concentration.

(2-1) Method for Observing Fluorescent Labeling on the Surface of Target Cells

In a typical scenario, the screening method for inhibitory substances binding to the T1R3 receptor of Aβ peptides (Aβ25-35) is described as follows. It's important to note that the specific combination of Aβ peptides, T1Rs, and cells is not limited to the examples provided.

As previously mentioned, when anti-T1R3 antibodies are added to cultured neuronal cells (such as SH-SY5Y cells differentiated with retinoic acid) along with fluorescently labeled Aβ peptides (Aβ25-35) or cells expressing Na+ channels (e.g., Nav1.5) forcibly, the binding of fluorescent Aβ peptides to the cell surface T1R3 can be completely inhibited. By observing the fluorescence labeling on the cell surface, the degree of competitive inhibition regarding the binding to T1R3 can be visually evaluated.

To apply this method, one can use cells with high T1R3 expression (e.g., SH-SY5Y cells differentiated with retinoic acid) and administer the test substance along with fluorescently labeled Aβ peptides in the cell culture medium. By observing the fluorescence on the cell surface, it's possible to determine whether the binding of fluorescent Aβ peptides is inhibited. This approach allows for screening potential AD therapeutic agents from test substances. If anti-T1R3 antibodies are the test substance, it's also possible to screen for more effective neutralizing antibodies among them.

Specifically, by administering pre-labeled Aβ peptides (Aβ25-35 or Aβ1-42) to cultured neuronal cells expressing T1R3 (or T1R3) in high quantities (e.g., SH-SY5Y cells differentiated with retinoic acid) or human-derived cultured cells expressing sodium channels (such as Nav1.5), one can confirm fluorescence emission on the cell surface through imaging. Subsequently, by checking whether the fluorescence intensity decreases upon administration of the test substance, it becomes possible to assess the presence and strength of inhibitory activity of the test substance against Aβ peptide binding to T1R3.

(2-2) Other methods for observing fluorescent labeling: In this invention, G protein-coupled receptors (GPCRs) expressed on the cell membrane can modulate the function of effectors such as voltage-gated sodium channels (VGSC) through changes in the polymerization state of the cell cytoskeleton, including actin and tubulin, induced by agonists. This characteristic can be utilized. By expressing fluorescently labeled actin or tubulin in cells that endogenously express T1R GPCRs (such as HEK cells) and observing changes in the cell cytoskeleton using fluorescence microscopy, it is possible to screen for inhibitors of the action of Aβ peptides or sweet-tasting amino acids in cerebrospinal fluid (CSF). Similarly, this method is effective even when the final effector of sweet-tasting amino acids or umami substances in CSF is not an ion channel, similar to the impedance-based method described in (2-6). As a control experiment to distinguish direct effects on the cytoskeleton from effects mediated by other GPCRs, one can use GFP-labeled T1R GPCR silencing vectors and RFP-labeled actin or tubulin.

(2-3) Method using patch-clamp: The patch-clamp technique records intracellular membrane potentials and allows simultaneous injection of current to stimulate cells. There are two recording modes in patch-clamp: Voltage-clamp (membrane potential fixed) and Current-clamp. In the voltage-clamp mode, ion channel currents such as VGSC are recorded. In the current-clamp mode, changes in intracellular membrane potential are recorded. Specifically, action potentials, which are changes in cell membrane potential, can be recorded, including their width, amplitude, and frequency. Additionally, in patch-clamp experiments, recordings from the same cell are taken for control conditions and responses after drug application. By comparing the results before and after drug application, it is possible to determine the presence or absence of drug effects. The use of single-cell electrophysiology (patch-clamp) allows for specific observations by recording changes over time from the same cell, which distinguishes it from other experimental methods. The experimental results obtained through patch-clamp enable direct measurement of drug changes over time and represent the most accurate experimental method. The delayed inactivation phenomenon of VGSC mediated by T1R1 through monosodium glutamate (MSG) could be applied to antiarrhythmic drug discovery screening. The increase in persistent sodium current resulting from delayed inactivation plays an important pathophysiological role in heart diseases, including arrhythmias (Horvath et al., 2020). As described in this invention, the major VGSC in cardiac muscle, Nav1.5, generates persistent sodium current due to MSG-induced delayed inactivation.

In conventional drug screening, the pharmacological confirmation of sustained sodium currents was achieved using Nav1.5 mutants, veratridine, and sea anemone toxin (ATX-II). However, in this invention, it was discovered that umami stimulation delays the inactivation of Nav1.5. As a result, safety pharmacological testing for Nav1.5 can now be conducted using umami stimulation alone, without the need for special mutants or expensive drugs. Specifically, by administering MSG to cultured cardiac myocytes, delaying the inactivation of voltage-gated sodium channels (VGSC) via the umami receptor (T1R1), and inducing sustained sodium currents (VGSC) in these cells, drug screening based on VGSC current changes can be performed.

The effects of Aβ peptides or umami substances on sustained potassium currents (VGKC) in cardiac myocytes can be studied using HEK cells expressing VGKC (such as Kv1.2, Kv3.2, Kv3.3) as well as SH-SY5Y cells expressing endogenous T1R GPCRs. For instance, as shown in the implementation example in (Figure 36B), Aβ peptides were applied to VGKC-expressing human cultured cells, and VGKC currents were recorded using ramp-like or rectangular voltage waveforms via the patch-clamp technique. This allowed measurement of the changes (inhibition) in VGKC currents caused by Aβ peptides. Similar inhibition studies on VGKC can be conducted using umami substance L-glutamate sodium (MSG) (Figure 36A).

The method used to demonstrate that the control effect of VGSC current by the aforementioned Aβ peptides (Aβ25-35, Aβ1-38, Aβ1-40, Aβ1-42) is lost by silencing TR3 was the voltage-clamp method (patch-clamp method).

(2-4) Method for measuring membrane potential changes in target cells using the "NanoTouch" technique

As an alternative method, the membrane potential changes in target cells can be measured using the "NanoTouch" method previously developed by the present inventors (Saito, 2019) (International Publication Number: WO2018/199334). The "NanoTouch" method involves penetrating conductive nanoparticles through the cell membrane to use them as intracellular electrodes. Among the variations of this method, the one combined with a capacitive voltage measurement device (NanoCharge) is used to record spontaneous action potentials in cultured neurons or cardiomyocytes. Specifically, the procedure involves culturing the aforementioned cells on conductive glass, mixing polyethyleneimine and conductive magnetic nanoparticles (gold magnetic nanoparticles), applying this mixture to the cells previously washed with PBS (-), and allowing it to act for 15 minutes in a 37°C incubator. Next, the mixture is replaced with physiological saline or culture medium, and the conductive glass where the cells are cultured is connected to the positive pole. A metal film (such as aluminum foil) is placed underneath the conductive glass where no cells are cultured, and it is connected to the negative electrode. The conductive glass is then placed on a magnet with a magnetic field strength of 180-200 millitesla. This magnetic force attracts the conductive magnetic nanoparticles already introduced into the cells toward the cell membrane direction in contact with the glass surface, allowing them to penetrate the cell membrane. As the method utilizes the principles of capacitive voltage measurement devices, capacitors are formed between the conductive material installed on the upper and lower surfaces of the conductive glass. This allows the detection of changes in intracellular potential as charges, and the opposite conductive material on the conductive glass side detects these charges. Since charge (Q) is related to $Q = CV$, the potential (V) can be measured proportionally to the capacitance (C) of the electrode glass. By using this method, it is possible to record intracellular potentials without using techniques such as the patch-clamp method, and it enables the measurement of the effects of Aβ peptides or sweet amino acids in cerebrospinal fluid (CSF) on neuronal or cardiac activity, as well as the effects of inhibitors.

(2-5) Method for observing the frequency of action potentials in neuronal cultured cells using extracellular recording

In the case of multielectrode arrays (such as Multielectrode Array (MEA)), the frequency of cell action potentials can be observed. However, unlike the "NanoTouch" method mentioned earlier, the waveform of action potentials or slow synaptic potential changes cannot be observed. When using MEA, each target cell is seeded and cultured on a probe embedded with recording electrodes specific to MEA, and extracellular recordings are performed using the MEA device. The effects of Aβ peptides on neurons or inhibitors can be studied similarly to the experiments described above, using either fluorescent membrane potential-sensitive reagents or fluorescent intracellular calcium-sensitive reagents. For cells that spontaneously generate action potentials (referred to as "spontaneous firing," which occurs independently of external stimuli), such as neurons and cardiomyocytes (with neurons having particularly high natural firing activity), they are seeded and cultured. In the case of calcium-sensitive reagents, the reagent mixture is administered and cultured at 37°C in an incubator for 60-90 minutes. For fluorescent membrane potential reagents, similar measurement equipment is used. The specific fluorescent wavelength depends on the reagent used, so it should adhere to the appropriate specifications.

(2-6) Method for screening compounds that modify/inhibit changes induced by Aβ peptides or sweet-tasting amino acids in cerebrospinal fluid (CSF):

Measuring cell impedance (resistance). The measured impedance of cell sheets fluctuates, reflecting the polymerization state of ATP and actin. This change is linked to alterations in cell morphology. Actin cytoskeleton, a downstream effector of important GPCRs (G protein-coupled receptors), allows sensitive measurement of impedance changes based on the adhesive state of cells seeded on electrode substrates and the spreading of adhesive areas. In GPCR pathways that activate Gαi, an increase in impedance is observed upon activation. For pathways that activate Gαq, a transient impedance phenomenon is initially observed, followed by a significant increase. Finally, in pathways that activate Gαs, impedance decreases have been reported (Scott and Matthew, 2010).

When measuring the changes in cell morphology caused by the activation of G protein-coupled receptors (GPCRs) through alterations in the cellular cytoskeleton, impedance values of cell sheets are typically measured using instruments such as the xCELLigence System (ACEA Biosciences) or CellKey (Molecular Devices).

Specifically, cells are cultured in a sheet-like arrangement on electrode plates made of materials like gold foil or conductive glass. By applying electrical current between the conductive glass surface covered by cells and the extracellular solution, changes in impedance values of the cell sheet can be measured. Culturing cells in a sheet format ensures that the electrode surface is completely covered by cells, isolating it from the extracellular fluid. Consequently, by passing current (I) between the extracellular fluid and the electrode plate and measuring the resulting potential difference (V), the overall resistance ® of the cell sheet can be calculated using Ohm's law ($V = IR \Rightarrow R = V/I$). Impedance is the reciprocal of resistance, so 1/R represents the measured impedance.

In this context, GPCR activation leads to changes in the cell cytoskeleton, alterations in cell morphology, and modifications in gap junctions connecting neighboring cells. Weaker cell adhesion due to these changes allows current to flow more easily, resulting in decreased resistance (and increased impedance).

To investigate further, ligands such as sweet or umami taste receptors (T1Rs), which are agonists for type I taste receptors, can be applied to cells in the presence of Aβ peptides (or amino acids related to sweet taste found in cerebrospinal fluid) to measure impedance changes. Additionally, identifying which G protein ($G\alpha i$, $G\alpha q$, or Gas) is being stimulated provides valuable insights.

Using the T1R GPCRs, various Aβ peptides (or amino acids related to sweet taste found in cerebrospinal fluid) can be tested to analyze the resulting impedance changes. This experimental system allows for screening compounds that modify or inhibit changes in cell cytoskeleton induced by Aβ peptides (or amino acids related to sweet taste found in cerebrospinal fluid).

The invention also demonstrates that T1R GPCRs expressed on the cell membrane can modulate the function of effectors such as VGSC (voltage-gated sodium channels) through changes in the polymerization state of cytoskeletal components like actin and tubulin.

In addition to impedance measurements, another effective method for temporary screening involves monitoring intracellular calcium concentrations using cells that endogenously express T1R GPCRs, such as HEK cells. In this discovery, Aβ1-40 and Aβ1-42 exhibit distinct patterns of intracellular calcium changes. Accumulating response patterns from other lengths of Aβ peptides, umami, and sweet taste substances can help correlate specific T1R GPCR subunits with response patterns, facilitating precise drug discovery when combined with the screening methods described above.

This approach can be used as an initial screening step, followed by more detailed analyses (such as fluorescence-labeled actin and tubulin effects, "NanoTouch" assays, MEA-based assessments of cell excitability, and membrane potential fixation methods).

It's worth noting that the measurable impedance changes are known to be influenced by the activity of GPCRs expressed on the cell surface.

(3) In vivo screening method: (Using experimentally modified animals with humanized T1Rs)

For in vivo screening, it is necessary to use experimental animals such as mice with humanized T1R genes. Compounds that inhibit the conformational change of T1R3 by ligands, represented by lactisole, respond differently in mice and rats compared to human genes. Lactisole selectively suppresses umami and sweet taste perception in humans but does not exhibit the same effect in mice or rats (Jiang et al., 2005).

Additionally, Toda et al. (2018) reported allosteric modulation of T1R1/T1R3 by methional. While this compound acts as a positive allosteric modulator (PAM) in humans, it functions as a negative allosteric modulator (NAM) in mice.

Thaumatin, another sweet taste protein, is detected in humans but not in mouse T1R3. Similarly, gymnemic acid is effective for human T1R2 but not for mouse T1R2.

The above-mentioned findings highlight the need to use humanized mice, achieved through knockout (KO) of mouse-derived related genes and insertion of human T1R genes, for in vivo drug screening and functional analysis of taste receptors.

[Implementation Example]

Next, I will provide examples to further explain the present invention, but the invention is not limited to these examples.

Other terms and concepts used in the present invention are based on meanings commonly used in the field. Various techniques used to implement the invention can be readily and reliably carried out by those skilled in the art based on well-known literature, except for techniques explicitly cited as sources. Additionally, various analyses were performed following the methods described in the handling instructions for the analysis equipment, reagents, kits, catalogs, etc. Furthermore, the content cited from technical literature, patent publications, and patent application documents in this specification is considered part of the disclosure of the present invention. Moreover, in the context of the present invention, when referring to "cells," it specifically denotes human-derived cells. However, all animal-derived cells that intrinsically express human-type Type I taste receptors or cells expressing human-type Type I taste receptors with specific amino acid sequences through gene introduction can also be used similarly. Animals possessing cells that intrinsically express human-type Type I taste receptors typically include chimpanzees, gorillas, orangutans, macaques, baboons, and other primates. Nevertheless, cells derived from animals with similar properties are not limited to these examples.

< Experimental Method Used in This Example >

1. Electrophysiological Experiments

In this example, electrophysiological experiments were conducted using the "patch-clamp method (voltage clamp, current clamp). Patch-clamp experiments were conducted using the Axopatch 200A amplifier (Axon Instruments Inc., Union City, CA) and digitized with the Data Digidata 1332a (Axon Instruments Inc.). Voltage control and data acquisition were performed using the Clampex software (pClamp 9.0, Axon Instruments Inc.). The digitally recorded data from Clampex were analyzed using Clampfit 10.6.2.2 (Molecular Devices, San Jose, CA).
Statistical processing was carried out using Microsoft Excel. The Boltzmann equation and Hill plot analysis were performed using Curve Expert Professional 2.6.0 (Hyams Development).
Specifically, a patch electrode containing intracellular solution was attached to the cell of interest under a microscope connected to the Axopatch amplifier. The opening of the patch electrode tip was used to disrupt the cell membrane, allowing recording of intracellular voltage. Stimulation was repeated at 8- to 10-second intervals until recording stability was achieved. I-V curves and S-S curves were measured and used as the baseline control. Subsequently, the test substance was applied while repeating stimulation at the same intervals. The stability of the test substance's effect was observed, or measurements were taken again after 5 minutes or more to compare the results with the control and determine the presence or absence of the test substance's effect. If narrowing of the patch electrode's cell membrane opening occurred during the experiment, resulting in decreased accuracy, those data points were excluded from the analysis.
For patch-clamp experiments, the solutions were prepared as follows:

Extracellular solution (in mM):
NaCl: 126 mM, KCl: 4 mM, CaCl2: 1.8 mM, MgCl2: 1 mM, HEPES: 24 mM, Glucose: 10 mM, pH adjusted to 7.4 with NaOH.

Intracellular solution (electrode internal solution) (in mM):
KCl: 130 mM. MgCl2: 5 mM. EGTA: 5 mM. Tris-ATP: 4 mM. HEPES: 10 mM. pH adjusted to 7.2 with KOH.

The chemicals used were purchased from NACALAI TESQUE, INC, Sigma-Aldrich, and Tokyo Chemical Industry Co., Ltd.

2. Cell lines and culture conditions used in the present invention:

(1) Creation of stable Nav1.5-expressing HEK cell line (HEK293):
HEK293 cells (from JCRB bank) were transfected with Nav1.5-pcDNA3.1 (hygromycin, Invitrogen, USA) using Superfect transfection reagent (Qiagen, DE) to create a hygromycin-resistant cell line. The HEK293 cell culture medium consisted of "DMEM (Sigma-Aldrich, St. Louis, MO, USA)" and "10% FBS (BioWest, FR)." In this document, "HEK293 cells" refer to the stable Nav1.5-expressing HEK293 cell line.

(2) Culture and differentiation of SH-SY5Y cell line into neuronal-like cells:
The SH-SY5Y cell culture medium was prepared by mixing DMEM (Sigma-Aldrich) and F-12 (Sigma-Aldrich) in equal proportions. To induce neuronal-like differentiation, the medium contained 1% FBS (BioWest), 5% 40 mM L-Glutamine (Fuji Film), and 1% MEM non-essential amino acids (Wako Pure Chemical Industries). During differentiation, SH-SY5Y cells were cultured for at least 5 days with the addition of 10 μM/ml retinoic acid (Fuji Film). In this document, "SH-SY5Y cells" refer to the SH-SY5Y cell line differentiated into neuronal-like cells.

3. Electrophysiological Measurement Protocol:

(1) Activation Protocol for Voltage-Gated Sodium Channels (VGSC): - Set the membrane potential to -80 mV. - Apply a pre-pulse at -120 mV for 500 ms. - Finally, apply a depolarization pulse of 200 ms duration from -80 mV to 40 mV in 10 mV increments. Double Pulse Protocol: - Similar to the activation protocol above, but after the initial multiple pulses, apply a prepulse at -120 mV before giving the pulse group again.

(2) Protocol for Investigating VGSC Steady-State Inactivation:
Set the membrane potential to -80 mV. - First, apply a pulse at -20 mV (200 ms). - Then, vary the potential from 0 mV to -120 mV (1 sec) in 10 mV increments to induce inactivation. - Finally, examine how the size of VGSC activated by the test pulse (at -20 mV, 200 ms) is affected by the membrane potential before the test pulse. Note that different test pulses may be used for Nav1.5 expressed in HEK cells and Nav1.7/Nav1.3 expressed in SH-SY5Y cells.

(3) Pulse and Ramp Protocol

Start with a pre-pulse at -120 mV for 320 ms, followed by a 20 ms step to -20 mV for depolarization. - Maintain the membrane potential at -120 mV for 360 ms as the second pre-pulse. - Apply a ramp waveform from -120 mV to 60 mV over 20 ms. - Subsequently, hold the membrane potential at 60 mV for 10 ms, followed by a 100 ms hold at -40 mV.

Implementation Example 1) Confirmation of the effectiveness of the T1R3 silencing vector:

The effectiveness of the T1R3 silencing vector was investigated by comparing protein levels of endogenously expressed T1R3 in control HEK cells and HEK cells expressing the T1R3 knockout vector. Western blot analysis was used to examine the silencing effect of T1R3 (Figure 1). The antibodies used for Western blotting were T1R3 antibody from LifeSpan Biosciences, Inc. (LSP) (catalog number LS-C496868-100) and anti-T1R3/AS1R3 Human (Rabbit) antibody. Specifically, cells were pelleted at 6000 rpm for 3 minutes at 4°C. The resulting pellet was suspended in 100 μL of lysis buffer (20 mM/L Tri-HCl, 150 mM/L NaCl, 2 mM/EDTA, 1% Nonidet P-40) containing protease inhibitor cocktail (Complete Mini; Roche Applied Science) and phosphatase inhibitor cocktail (PhosSTOP; Roche Applied Science). After incubating on ice for 20 minutes, the cellcontaining lysate was centrifuged at 15,000 rpm for 20 minutes at 4°C, and the supernatant concentration was measured. The resulting 10 μg of protein was subjected to electrophoresis (7.5% Tris-HCl polyacrylamide gel) for Western blotting. The separated proteins were transferred to a polyvinylidene difluoride membrane (Immobilon P; Millipore, Burlington, MA, USA). Primary antibody used was Tas1R3 antibody (LifeSpan Biosciences, Inc. (LSP) LS-C496868), and secondary antibody was sheep anti-mouse HRP-conjugated secondary antibody (Amersham Pharmacia Biotech, Buckinghamshire, UK). Visualization was achieved using enhanced chemiluminescence (ECL; Amersham Pharmacia Biotech).

(Implementation Example 2) Creation of expression vectors for hT1R1, hT1R2, and hT1R3, and measurement of the expression levels of T1R1, T1R2, and T1R3 endogenously expressed in HEK cells and SH-SY5Y cells using quantitative PCR.

The cDNAs used and their Accession numbers are hTAS1R1 (BC136515), hTAS1R2 (BC141437), and hTAS1R3 (BC152912) (Kabushiki Kaisha DNAFORM). The purchased cDNAs were inserted into expression vectors using the following method. The cDNA cloning was performed using Thermo's cloning kit (Gateway® cloning technology). First, the cDNAs were ligated into the BamH1/Xho1 site of the pENTR1.0A (Entry Clone) vector, and then cloned into the Vivid Color™ pcDNA™6.2/EmGEFP-Bsd/V5-DEST Vector (Catalog number: V36620) using the LR reaction (Gateway™ LR Clonase™ II Enzyme mix: Catalog number 11791100).

Using quantitative PCR (qPCR), the expression levels of T1R1, T1R2, and T1R3 endogenously expressed in HEK cells and SH-SY5Y cells were examined, confirming sufficient expression levels. The results of the quantitative PCR from HEK cells are shown in (Figure 2). It was found that T1R3 is significantly more abundantly expressed than T1R1 and T1R2.

(Implementation Example 3) Creation of Knockdown Vector

The miRNAs Oligo (Thermofisher) used for silencing were annealed to form double-stranded DNA, which was then linked to the miR RNAi Expression Vector using T4 DNA ligase to create the knockdown vector. Specifically, the procedure followed the miR RNAi Expression Vector Kit manual (BLOCK-iTM Pol II miR RNAi Expression Vector Kit with EmGFP (Catalog number: K493600)).

(Implementation Example 1) Activation Effect of VGSC via the Sweet GPCR Receptor T1R2/T1R3 by Glutamine (Gln)
Glutamine is a sweet amino acid recognized as a taste through the T1R2/T1R3 receptor.

It is known to be significantly elevated in the cerebrospinal fluid of AD patients compared to healthy controls. Therefore, the potential effect of glutamine on VGSC was hypothesized and investigated. An increase in VGSC current (10-20%) was observed with the administration of 0.5-1 mM glutamine. No further enhancement was seen with the addition of glutamine at concentrations higher than 1 mM. The enhancing effect of glutamine on VGSC was observed in both SH-SY5Y (n=8) and HEK-Nav1.5 (n=4) cells. (Figure 3) shows the recordings from SH-SY5Y cells. The amplification of VGSC inward current before and after the administration of 500 $\mu$M glutamine is confirmed. (Figure 3) left shows the actual current waveform of the experiment, and (Figure 3) right shows the I-V plot (a graph plotting the change in current magnitude corresponding to the stimulus voltage).

(Implementation Example 2) Activation of VGSC via T1R2/T1R3 by Amyloid $\beta$ Peptide (A$\beta$25-35)

(2-1) Enhancement of VGSC Inward Current by A$\beta$25-35

Amyloid $\beta$ peptide is used as a biomarker for diseases such as Alzheimer's disease (AD), dementia with Lewy bodies, and amyotrophic lateral sclerosis (ALS), as its enhancement in the brain has been reported through immunostaining using A$\beta$ antibodies (Bibl et al., 2007; Lanznaster et al., 2020).

Clinically, the relationship between amyloid $\beta$ peptide and neuronal hyperexcitability has been studied in ALS patients (Palop et al., 2009; Sperling et al., 2009). The hyperexcitability induced by amyloid $\beta$ peptide suggests that it may cause burst-like activity (seizures) in AD or ALS patients, potentially leading to neurodegeneration (Hartley et al., 1999; Brunet et al., 2020). Overexpression of amyloid $\beta$ peptide has been suggested to enhance VGSCs such as Nav1.6, resulting in neuronal hyperexcitability (Li et al., 2016; Ciccone et al., 2019; Non-Patent Literature 1).

Non-Patent Literature 1 shows that cultured cortical neurons differentiated from iPS cells derived from AD patients exhibit increased excitability and burst-like hyperactivity compared to controls from healthy individuals. This hyperactivity is supported by the amplification of VGSCs, as demonstrated by electrophysiological methods (voltage-clamp technique). Furthermore, this neuronal hyperactivity and VGSC amplification disappear when BACE1 inhibitors are applied, suggesting a causal relationship between A$\beta$ peptide and neuronal hyperactivity and seizure onset in AD patients.

Amyloid $\beta$ peptide, specifically A$\beta$1-42, is formed from amyloid precursor protein. Among amyloid $\beta$ peptides, A$\beta$25-35, formed by proteases in the brain, is reported to be the most toxic. Immunohistochemical analysis shows that A$\beta$25-35 is not found in healthy individuals but is present in senile plaques and degenerated neurons in the CA1 region of the hippocampus in AD patients (Kubo et al., 2002).

(2-2) Concentration-Dependent Amplification of VGSC Current by A$\beta$25-35 Peptide

For these reasons, this example investigates the effects of A$\beta$25-35 peptide on VGSC. Specifically, experiments similar to those with glutamine (Gln) in (Implementation Example 1) were conducted using 1-6 $\mu$M A$\beta$25-35 peptide. As shown in (Figure 4), similar to the effects of glutamine, an enhancement of VGSC inward current was observed in SH-SY5Y cells (n=5). Similar enhancement was also observed in HEK-Nav1.5 cells (n=4).

The concentration-dependent effects of various concentrations of A$\beta$25-35 peptide on VGSC current in SH-SY5Y cells were examined. At the highest concentration of 6 $\mu$M, the VGSC current was amplified by 1.65 times. Using the voltage-clamp technique (perforated patch-clamp recording method) described in detail in (2-4), the changes at 6 $\mu$M A$\beta$25-35 peptide were assumed to be the maximum change (100%), and EC50 and Hill slope were determined. The results showed an EC50 of 86.8 nM and a Hill slope of 1.33 (Figure 9).

Next, using the current-clamp mode, we applied current stimulation and recorded action potentials from differentiated SH-SY5Y cells (Figure 5, from the lower to the upper part of the figure, rectangular wave current stimulation was applied. The current strengths were 6, 8, 10, and 12 pA from the bottom). Before the application of A$\beta$25-35 peptide, action potentials were not induced at stimulation currents of 6 pA and 8 pA, but action potentials were recorded at stronger stimulation currents (10 pA and 12 pA). From this, it can be concluded that the threshold for generating action potentials was lowered by the A$\beta$25-35 peptide. Additionally, since the amplitude of the action potentials also increased, it was found that the A$\beta$25-35 peptide enhanced VGSC, making the neurons more excitable, similar to the effect of glutamine on VGSC, which lowers the excitability threshold.

Since A$\beta$25-35 peptide is said to be the most toxic, we used HEK-Nav1.5 to confirm whether A$\beta$1-42 peptide has the same effect as A$\beta$25-35 peptide on VGSC currents using the voltage clamp method (Figure 6). A$\beta$1-42 did not show the same enhancing effect as A$\beta$25-35 (n=4). Rather, unlike A$\beta$25-35 peptide, A$\beta$1-42 peptide suppressed sodium channel currents in VGSC. Vitvitsky et al. reported that when A$\beta$25-35 peptide acts on astrocytes, there is an increase in intracellular Na+ and K+, but such an effect was not observed with A$\beta$1-42 peptide (Vitvitsky et al. (2012)), which is consistent with the results of this example using VGSC. Vitvitsky et al. also described that Aβ25-35 and Aβ1-40 have similar effects, but Aβ1-42 does not, suggesting that the presence or absence of amino acid residues 40-42 may cause some important structural changes.

(2-3) The effect of Aβ25-35 peptide on enhancing VGSC activity via T1R2/T1R3

(i) Observation by silencing T1R3 or treating with anti-T1R3 antibody: The experimental results in (Implementation Example 1) indicated that the sweet amino acid glutamine acts through the sweet receptor (T1R2/T1R3), and the enhancing effect of glutamine on VGSC was interpreted as lowering the excitability threshold, making it easier to generate action potentials and increasing the amplitude of action potentials. Since Aβ25-35 peptide produced similar effects (enhancing VGSC activity, lowering the excitability threshold) as glutamine, it was considered that Aβ25-35 peptide might also act on T1R2/T1R3 receptors like glutamine. Therefore, in this implementation example, we used HEK-Nav1.5 cells with silenced T1R3 and compared the effect of Aβ25-35 peptide on VGSC with that in control HEK-Nav1.5 cells.

The comparison of VGSC recorded from control HEK Nav1.5 cells using the voltage clamp method before and after the administration of Aβ25-35 is shown in (Figure 7A and B). The enhancement effect of VGSC current by 1μM Aβ25-35 was confirmed. (B) shows the actual experimental current waveforms (left: control, right: after 1μM Aβ25-35 administration). (A) shows the I-V plot measured from experiment B. On the other hand, in HEK Nav1.5 cells where T1R3 was silenced, no change in VGSC was observed even when 2μM Aβ25-35 peptide, twice the concentration used in the control recording, was applied (n=4). (A) I-V plot, (B) VGSC current traces before and after Aβ25-35 peptide administration.

In addition to the above observations, the VGSC enhanced by Aβ25-35 peptide recovered to the size of VGSC recorded in the control before enhancement by 1-1.5mM lactisole (n=2) (Figure 7E). Given that lactisole specifically binds to T1R3, this observation also indicates that Aβ25-35 peptide is acting on T1R3. It has been suggested that lactisole, which binds to the TM domain located near the G-protein binding site rather than the extracellular ligand binding site (Venus flytrap domain), inhibits the activation of T1R3. The suppression of the enhancement effect by lactisole strongly suggests that the action of Aβ25-35 is mediated by T1R3.

(ii) Changes in the sodium current enhancement effect in HEK Nav1.5 cells treated with anti-T1R3 VFD antibody:

The changes in the sodium current enhancement effect in HEK Nav1.5 cells pre-treated with anti-T1R3 VFD antibody were observed (Figure 8). HEK Nav1.5 cells were incubated with 1000-fold diluted anti-T1R3 antibody for 10-20 minutes, and patch-clamp experiments were conducted to confirm whether the action of Aβ25-35 peptide on VGSC could be suppressed.

The I-V and S-S curves of VGSC current recorded from HEK Nav1.5 cells treated with T1R3 N-terminal recognition antibody before and after the action of Aβ25-35 peptide are shown (n=2). In experiments where T1R3 was silenced, the action of Aβ25-35 peptide disappeared. In the control without anti-T1R3 antibody pre-treatment, a reproducible 10-20% increase in VGSC current amplitude was confirmed, but no enhancement effect was observed in cells pre-treated with anti-T1R3 antibody. This antibody was created against the amino acid sequence 229-258 of the human T1R3 VFD region and specifically reacts with human cells. The fact that this antibody suppresses the action of Aβ25-35 peptide suggests the possibility of species differences (differences from mice) in the action of Aβ25-35 peptide.

(iii) Possibility of acting on both T1R2 and T1R3:

On the other hand, when T1R3 was treated with T1R3 N-terminal recognition antibody, the enhancement effect of VGSC current by Aβ25-35 peptide was suppressed, but a left shift (negative potential direction) of the I-V and S-S curves was observed (Figures 8, 29). The phenomenon of the left shift of the I-V and S-S curves is mainly observed when reagents/compounds acting on T1R2 are applied, suggesting that T1R2 and T1R3 are functionally closely involved.

This suggests that when Aβ25-35 peptide cannot act on T1R3 due to T1R3 antibody, T1R2 may function as a low-affinity binding site, indicating that Aβ25-35 peptide, like sucrose and sucralose, may act on both T1R2 and T1R3.

From the above, it was confirmed that the T1R2/T1R3 heterodimer or T1R3/T1R3 homodimer acts as a receptor for Aβ25-35 peptide, enhancing VGSC by a mechanism similar to glutamine, and potentially causing neuronal hyperactivity.

(2-4) Enhancement Effect of Aβ25-35 Peptide on VGSC Current via T1R3: Re-examination Using the Perforated Patch-clamp Method

The enhancement effect of Aβ25-35 peptide on VGSC current via T1R3 acts on the effector (VGSC) through intracellular second messengers mediated by G-proteins. In the experiment of (Implementation Example 2-2), the fixed patch electrode of the whole-cell membrane used had an opening in the inner diameter that destroyed the cell membrane, creating a hole that integrated the intracellular and patch electrode interiors. This allowed for the recording of intracellular potentials and the control of cell membrane potentials, enabling the measurement of ion currents using the voltage-clamp method. During this process, the patch electrode solution mixed with the intracellular solution, and since the volume of the patch electrode solution was much larger than the intracellular fluid, the intracellular fluid and substances were replaced by the patch electrode solution. To ensure the proper functioning of intracellular second messengers, it is essential to prevent the outflow of intracellular substances such as ATP into the patch electrode.

Therefore, the effect of Aβ25-35 peptide was re-examined using the perforated patch-clamp method, a voltage-clamp method. The perforated patch-clamp method involves mixing antibiotics such as amphotericin B and gramicidin, which form pathways for monovalent cations to pass through lipid membranes containing cholesterol in the patch electrode solution, without creating holes in the cell membrane (thus not altering the intracellular environment). This method records VGSC currents using the voltage-clamp method without creating holes in the cell membrane.

When Aβ25-35 peptide was applied, an amplification of VGSC current was observed, similar to that recorded with the conventional voltage-clamp method (patch-clamp method). Subsequently, increasing the concentration of Aβ25-35 peptide caused the amplification of VGSC current induced by Aβ25-35 peptide to disappear, returning to almost the same level as before the application of Aβ25-35 peptide (Figure 10). This phenomenon is thought to be due to the desensitization of VGSC receptors.

Notably, along with the amplification of VGSC current, an increase in cell membrane area was also observed. When a rectangular wave voltage was applied during voltage-clamp recording, the amplifier charged the capacitance generated by the cell membrane before recording the ion current. This charge amount is proportional to the size of the cell (cell membrane size) and can be measured experimentally. This value, Q, is expressed by the equation $Q = CV$.

By applying the voltage pulse value ($V = 10\,mV$) used in this experiment and the experimentally measured charge amount ($Q$) to the formula $C = Q/V$, the electrical capacitance ($C$) of the cell membrane can be determined.

As shown in (2-2) (Figure 9), the cell membrane charge amount and VGSC current magnitude were measured after the action of Aβ25-35 peptide using the membrane potential fixation method. It was observed that the VGSC current amplification was accompanied by an increase in cell membrane charge capacity. The relationship between the increase in cell membrane and the increase in VGSC current was analyzed by calculating the current density of VGSC current. Current density (pA/pF) is obtained by dividing VGSC current (pA) by cell membrane capacity (pF) (Figure 11B).

After the action of Aβ peptide, it was found that not only the VGSC current increased but also the VGSC current density (pA/pF) increased. The increase in cell membrane capacity indicates that the cell membrane area increased due to the action of Aβ peptide. This means that vesicles, such as endosomes within the cell, were added to the cell membrane. Furthermore, the increase in VGSC current density after the action of Aβ peptide indicates that the vesicles added to the cell membrane contain more VGSCs than the VGSCs already present in the cell membrane. When the concentration of Aβ peptide was further increased, both VGSC current and cell membrane capacity decreased, returning to almost control levels (Figure 11A).

Since the experiment using the membrane potential fixation method (patch-clamp method: Perforated Patch-clamp) this time is transient, it is unclear whether the VGSC derived from the intracellular vesicles with high VGSC current density caused by Aβ peptide will diffuse into the cell membrane that does not originate from intracellular vesicles. However, since the differential current density returns when the cell capacity returns to the control level, it is considered that in an experiment of about 10 minutes like this time, the membrane derived from the intracellular vesicles with high VGSC current density inserted into the membrane is endocytosed into the vesicles while maintaining the VGSC current density. The increase or decrease in VGSC current density due to exocytosis or endocytosis was confirmed not only in HEK Nav1.5 (n=3) but also in RA-differentiated SH-SY5Y neuroblastoma cells (n=1). Stimulation was given every 8 seconds. The X-axis of (Figure 11C) indicates the number of stimulations. Therefore, for example, stimulation 10 means 80 seconds after the start of the experiment.

(2-5) Comparison of changes in VGSC current density due to changes in Aβ25-35 peptide concentration

In this experiment, since the mechanism of enhancement of AMPA-type glutamate receptors on the cell membrane in LTP and the mechanism of enhancement of VGSC on the cell membrane by Aβ25-35 are very similar, the method used in LTP research was applied, and the method of evaluating the insertion of endosomes containing VGSC into the cell membrane using the membrane potential fixation method (patch-clamp method) was adopted (Figure 12).

The results of measuring VGSC current and current density via T1R3 using Perforated Patch-clamp obtained in (2-4) (Figure 10) and (Figure 11) are also illustrated in (Figure 12).

In accordance with (Figure 12), the action of Aβ25-35 peptide increased the current of VGSC. Furthermore, when the concentration of Aβ25-35 peptide was further increased, the VGSC current decreased and returned to the pre-Aβ25-35 peptide action level. The increase and decrease in VGSC current were observed to occur simultaneously with changes in cell membrane capacitance. To analyze this, the VGSC current (pA) was divided by cell capacitance (pF) to calculate current density (pA/pF). As a result, it was confirmed that the current density of VGSC increased due to the action of Aβ25-35 peptide, and the decrease in current density occurred with increasing Aβ25-35 peptide concentration.

This suggests that endosomes expressing VGSC within the cell membrane are fused with the cell surface upon Aβ25-35 peptide action (resulting in increased current density), and then taken back into the cell upon further Aβ25-35 peptide accumulation (resulting in decreased current density).

Additionally, when comparing the change in current density caused by Aβ25-35 peptide to the pre-action current density, it was found that the change was greater. The decrease in current density resulting from the increased Aβ25-35 peptide concentration was also larger compared to the pre-action current density. In summary, the VGSC current density on endosomes inserted by Aβ25-35 peptide is greater than the pre-existing VGSC current density on the cell membrane. The membrane unit area containing VGSC, both newly added by Aβ25-35peptide and absorbed from the cell membrane due to T1R3 desensitization, is also larger than the pre-action VGSC unit area.

The strengthening of synaptic currents observed in LTP (Long-term potentiation), considered important in explaining the process of initial memory formation, is reported to occur through the fusion of AMPA-type glutamate receptors expressed in intracellular vesicles with the cell membrane (Park et al., 2004, Science 24:305(5692):1972-5). This discovery is very similar to the process in which VGSC localized in vesicles in this study are fused and inserted into the cell membrane. It is believed that Rab11a and Rab5, involved in the trafficking of recycling endosomes, play a role in the fusion and retrieval of vesicles expressing AMPA-type glutamate receptors (Park et al., 2004).

Therefore, it is possible to evaluate the degree/process of vesicular fusion to the cell membrane of vesicles expressing VGSCs by measuring the current density of VGSCs in cell vesicles as shown above (2-4) and by pre-labeling the cell vesicles expressing VGSCs with FITC-dextran, FM1-43 fluorescent reagent, etc. Using the method described above, it is possible to evaluate the enhancement process/degree of VGSCs caused by the insertion of VGSCs into the cell membrane by Aβ peptide. This method is effective for screening inhibitors that suppress the hyperactivity of neurons caused by Aβ peptide. Also, this technique can be applied when studying the effects on neuronal activity of many amino acids contained in CSF (cerebrospinal fluid), particularly L-Gln (glutamine) and Lysine, which are known to increase in neurodegenerative diseases such as epilepsy and Alzheimer's-type dementia.

(Implementation Example 3) Inhibitory Effect on VGSC Targeting Sweet Receptors (T1R2/T1R3 Heterodimer or T1R3 Homodimer) and its Concentration Dependence

(3-1) Inhibitory Effect on VGSC Targeting T1R2/T1R3 or T1R3/T1R3

Mailliet and colleagues developed an experimental system that allows the observation of cellular responses to sweet stimuli as increases in intracellular calcium. They expressed G16-gust44 (Ueda et al., 2003), which has the function of converting these receptor responses into enhanced intracellular calcium reactions, along with T1R2 and T1R3 in HEK cells. They reported the observation of the effects of T1R3 inhibitors on sweet stimulus responses (Mailliet et al., 2009).

In this system, the artificial sweetener sucralose was used as the stimulant, and the response of the obtained sweet receptor (T1R2/T1R3) was used as the control. The study reported on the inhibitory effects of lactisole and its structural analogs, fibrates and phenoxy-herbicides, on this sweet stimulus response.

The results indicated that the IC50 values were 70μM for lactisole, 28μM for clofibric acid (a fibrate used for hyperlipidemia), and 5.3μM for dichlorprop (a phenoxy herbicide), with dichlorprop being reported to bind to the transmembrane region of T1R3 similarly to lactisole (Nagakita et al., 2019).

In this experiment, substances like clofibrate and dichlorprop, which were identified as sweet receptor (T1R2/T1R3) antagonists in the aforementioned experimental system by Mailliet et al., were applied to VGSC to determine whether their IC50 values show a similar trend to the IC50 values obtained in Mailliet's experiments. Additionally, the study observed whether the effects of saccharin and sucralose (non-patent literature 2), which show agonist-like action on the sweet receptor (T1R2/T1R3), and gymnema tea extract (GTE, Sanematsu et al. (2014) J. of Biol. Chem. 289(37): 25711-20), which shows antagonist action, on VGSC were activating or inhibiting.

We observed the concentration-dependent effects of dichlorprop based on the voltage-dependent activation curve of the voltage-gated sodium channel (VGSC) and the steady-state inactivation curve (Figure 13). Unlike Saccharin, Sucralose, and Gymnema tea extract (GTE), dichlorprop reduces VGSC current in a concentration-dependent manner by shifting the steady-state inactivation curve to the left (hyperpolarizing direction). Therefore, when using a depolarizing stimulus with a holding voltage of -120 mV (frequently employed in our experiments to activate Nav1.5 or maintain the resting membrane potential of typical cells around -80 mV), the steady-state inactivation curve shifts even further to the left, rendering VGSC activation impossible and acting as an inhibitor.

However, Saccharin, Sucralose, and Gymnema tea purely inhibit VGSC without affecting the steady-state inactivation curve. Consequently, dichlorprop differs significantly from Saccharin, Sucralose, and Gymnema tea extract in its ability to shift the steady-state inactivation curve parallel to the negative direction of membrane potential, similar to cases involving T1R2 overexpression, T1R2 silencing, or the use of TBB (Casein kinase 2 inhibitor). Although dichlorprop and saccharin/gymnema tea extract share the ability to concentration-dependently reduce VGSC activity by acting on the sweet taste receptor (T1R2/T1R3), their mechanisms of action differ. Specifically, Saccharin, Sucralose, and Gymnema tea do not affect kinetics such as glutamine (L-Gln) or A$\beta$ peptide inactivation kinetics, while still increasing or decreasing VGSC current.

We analyzed the steady-state inactivation curve using Boltzmann's equation and calculated the midpoint membrane potential (Vh) required for half-maximal reduction from the slope factor and maximum current (Figure 14A). Although we observed no significant changes in the slope, the Vh shifted towards hyperpolarization with increasing dichlorprop concentration (Figure 14B). The analysis results, combined with those of clofibrate, are shown in the table below [Tables 6,7].

(3-2) Concentration-dependent effects of dichlorprop on VGSC amplitude

Next, we analyzed the concentration-dependent effects of dichlorprop on VGSC amplitude. Based on experimental results of steady-state inactivation recorded at different dichlorprop concentrations, we could analyze the degree of inhibition dependent on VGSC current amplitude during preconditioning pulse (at -20 mV) relative to dichlorprop concentration. Analyzing the concentration-dependent inhibitory effects of dichlorprop at pre-pulse voltages of -80 mV, -100 mV, and -120 mV [Tables 6,7], we found that the more hyperpolarized the pre-pulse voltage, the larger the IC50. In other words, when used for therapeutic purposes to correct neuronal hyperexcitation caused by VGSC enhancement (as demonstrated in Implementation Example (2-2) with A$\beta$25-35 peptide), dichlorprop is highly effective at concentrations below 100 $\mu$M, especially at the typical resting membrane potential of -80 mV. Similar therapeutic effects can be expected for epilepsy, ALS, and neuropathic pain resulting from concomitant chemotherapy (Taxol, which similarly enhances VGSC as A$\beta$25-35 peptide, as shown in Figure 8).

We calculated the IC50 for VGSC inhibition using dichlorprop and clofibrate under different preconditioning pulse conditions, as shown in [Tables 6 and 7]. From these results, it is evident that DichlorProp is significantly more effective than clofibrate. The IC50 values are more than one order of magnitude larger than those measured by Mailiet et al. (2009) using calcium imaging (dichlorprop IC50 = 5.3 $\mu$M, clofibrate IC50 = 28 $\mu$M). However, it is clear that the degree of hyperpolarization in the preconditioning pulse, which activates VGSC in our experiments, strongly influences the IC50.

Table 6

| Prepulse (mV) | DichrolProp | | Clofibrate | |
|---|---|---|---|---|
| | IC50($\mu$M) | **Hill** Slope | IC50($\mu$M) | Hill Slope |
| -80 | 95.7 | 1.6 | 580 | 5 |
| -100 | 304.3 | 1.9 | 1200 | 3.3 |
| -120 | 373.6 | 1.7 | 1040 | 3 |

The Nav1.5 channel used in this experiment exhibits steady state inactivation positioned in the hyperpolarizing direction compared to Nav1.6, which is expressed in SH-SY5Y cells. Therefore, as mentioned earlier, the IC50 values for dichlorprop and clofibrate are expected to increase with more hyperpolarized holding voltages, which may differ from the results obtained through calcium imaging measurements. To further explore this discrepancy with the IC50 value obtained from calcium imaging, we determined the IC50 for dichlorprop using SH-SY5Y cells expressing Nav1.6. As a result, at a holding voltage of -60 mV, the IC50 value was 9 $\mu$M, which is nearly equivalent to the value of 5.3 $\mu$M obtained from calcium imaging.

This suggests that dichlorprop may be safe for application as an analgesic or antiepileptic drug, particularly in terms of cardiac function.

However, the fact that dichlorprop's IC50 value differs between HEK Nav1.5 and SH-SY5Y (Nav1.6) cells at the same holding voltage (-80 mV) indicates that differences in VGS genes (Nav1.5, Nav1.6) may significantly influence IC50 variations compared to the action of T1R genes.

[Table 7]

| Cell type | Prepulse (mV) | DichlorProp | |
|---|---|---|---|
| | | IC50($\mu$M) | Hill Slope |
| SH-SY5Y | -60 | 9 | 2.5 |
| | -80 | 17.3 | 2.27 |
| HEK Nav1.5 | -80 | 95.7 | 1.6 |
| | -100 | 304.3 | 1.9 |
| | -120 | 373.6 | 1.7 |

As described above, dichloprop, known to bind to T1R3 similarly to clofibrate and lactisole, has been confirmed to cause a shift in the voltage dependence of the steady-state inactivation curve of VGSC to a more hyperpolarized state. This phenomenon is similar to what happens when the connection between the cytoskeleton and VGSC is disrupted due to shear force or stretch in cases of brain injury, leading to a shift in the voltage dependence of the steady-state inactivation curve (Wang et al. (2009), Shcherbatko et al. (1999)). This suggests that the GPCR sweet taste receptor (T1R2/T1R3) may also influence the relationship between the cytoskeleton and VGSC, potentially controlling VGSC function. As mentioned above, the cytoskeleton binds to VGSC through Ankyrin G/Spectrin, with the phosphorylation status and degree by casein kinase 2 of the Ankyrin G binding sequence on VGSC playing a crucial role in the binding of VGSC to Ankyrin G.

Next, let's discuss the significant impact of prepulse potentials on IC50 values based on the experimental results in [Table 6] and [Table 7]. According to Nin et al. (2009), hyperpolarizing the membrane potential stabilizes the cellular cytoskeleton, particularly reinforcing adherens junctions (adhesion belts) in epithelial cells. Therefore, if the prepulse potential is hyperpolarized, the cellular cytoskeleton becomes more stable, potentially weakening the effect of T1R3 blockers (sweet taste inhibitory compounds). In other words, when neurons are hyperactive, the membrane potential is depolarized compared to the prepulse potentials used in [Table 6] and [Table 7]. Consequently, these compounds may exhibit VGSC inhibitory effects at even lower concentrations. This selective action could be advantageous for treatment, as it affects hyperactive neurons while having minimal impact on normally functioning neurons.

Considering the above, inhibiting Casein Kinase 2 (CK2) could lead to the dephosphorylation of the Ankyrin G binding site on VGSC, weakening the relationship between VGSC and the cytoskeleton. This, in turn, may cause changes such as shifting the steady-state inactivation curve membrane dependence toward hyperpolarization.

(Implementation Example 4 ) Effect of Ankyrin G Dephosphorylation on VGSC Using Casein Kinase 2 Inhibitor (TBB)

(4-1) Concentration-Dependent Inhibition of VGSC Current by Casein Kinase 2 Inhibitor (TBB)

The casein kinase 2 inhibitor known as TBB was found to reduce the amplitude of voltage-gated sodium channel (VGSC) currents. Specifically, the membrane potential-dependent activity of VGSC is influenced by the voltage of preceding pulses. When the preceding pulse depolarizes from -120 mV to -20 mV, the amplitude of VGSC current decreases compared to depolarization from -80 mV to -50 mV in 10 mV intervals. To investigate this effect, TBB was administered to Nav1.5-expressing HEK cells, and changes in VGSC current were observed. The inward peak current decreased in a TBB concentration-dependent manner. Notably, the inhibitory effect of TBB strongly depends on the membrane potential during preceding pulses. At -80 mV, even the minimum concentration of TBB *(30 $\mu$ M, as shown in Figure 15B, left side)* resulted in nearly complete VGSC current inhibition. However, at -100 mV, the degree of inhibition was weaker, and at -120 mV, inhibition was almost absent. The steady-state inactivation (S-S) curve in (Figure 15A), obtained when the pre-pulse was at -120 mV, demonstrates this behavior. The S-S curve for 10 $\mu$M TBB (white circles) shifts to the hyperpolarized direction (left) compared to the control (black circles), indicating VGSC current inhibition. Additionally, in steady-state inactivation experiments, the current size of VGSC activated by a test pulse at -20 mV following pre-pulses from -170 mV to -120 mV to 0 mV was plotted against membrane potential. As TBB concentration increased, the curve shifted gradually to the left (hyperpolarized direction) (Figure 15A). (Figure 15B) shows actual experimental data waveforms used for the plot in (Figure 15A).

Subsequently, similar experiments were conducted using SH-SY5Y cells, yielding comparable results (data not shown).

The experimental results in (Figure 15) indicate that TBB has a pronounced membrane potential-dependent inhibitory effect on VGSC. This phenomenon can be attributed to the shift of the steady-state inactivation curve (as shown in Figures 13 and 14) toward the hyperpolarized direction.

(Figure 16) displays the IC50 value for VGSC current inhibition by TBB (Figure 16A) and the shift of the steady-state inactivation curve (Figure 16B) induced by TBB. The difference between the control (pre-TBB treatment) and post-TBB treatment Vh values is shown.

The phenomenon of VGSC inhibition due to leftward shift of the S-S curve is reminiscent of the inhibition observed with aspartame, which acts as an agonist for T1R2 and demonstrates VGSC inhibition. This suggests that TBB's inhibitory action on VGSC may also involve T1R2 or other T1Rs. If the impact of TBB on VGSC IC50 is confirmed while partially inhibiting VGSC with TBB, it would strongly imply a relationship between VGSC and cellular scaffolds like Ankyrin G through T1Rs, influencing VGSC phosphorylation by CK2.

The phenomenon of VGSC inhibition due to a left shift in S-S of VGSC is very similar to the inhibition of VGSC by aspartame, which shows T1R2 overexpression and T1R2 agonist-like action, as described later. This suggests that the VGSC inhibitory effect of TBB may also share a common mechanism with the inhibition of VGSC activity mediated by T1R2 or other T1Rs. If it can be confirmed that the IC50 of dichlorprop and aspartame is affected in a partially inhibited state of VGSC by TBB, it would indicate that the inhibition of VGSC via T1R2 or T1R3 is related to the degree of inhibition of VGSC phosphorylation by CK2 by TBB. This strongly suggests that the relationship between VGSC and the cytoskeleton, such as ankyrin G, is related to the mechanism of VGSC inhibition through T1Rs.

The voltage-gated sodium channel (VGSC) is co-localized with the membrane cytoskeletal protein ankyrin G at the initial segment of axons (AIS), the Ranvier node, and the neuromuscular junction in mammals. Zhou et al. reported that ankyrin G is essential for clustering VGSC at the initial segment of axons and is crucial for VGSC activity (Zhou et al., 1998). Similarly, Brechet et al. found an ankyrin-binding motif within VGSC, and phosphorylation of a serine residue within this motif by CK2 is essential for VGSC-Ankyrin G binding. Inhibition of CK2 activity reduces sodium channel accumulation at the neuron's axon initial segment (AIS) (Brechet et al., 2008). Thus, CK2-mediated phosphorylation of VGSC regulates the degree of binding between VGSC and Ankyrin G. Xu et al. (2015) also reported that CK2-mediated phosphorylation of VGSC is crucial for binding with Ankyrin G (non-patent literature 9).

When applying TBB during patch-clamp recording of VGSC currents, the steady-state inactivation curve (abbreviated as S-S) significantly shifts in the hyperpolarizing direction (Figure 15). In other words, to activate VGSC currents, the holding potential (membrane potential set before delivering the stimulus pulse) must be significantly hyperpolarized (below -140 mV) to prevent substantial reduction (inhibition) of VGSC current.

(4-2) Delayed VGSC inactivation by MSG

Even in this inhibited state, applying MSG leads to a recovery trend in VGSC current size (Figure 17) and corrects the hyperpolarizing shift of S-S (Figure 18). Additionally, since MSG effects occur even in T1R3 knockout (KO), it suggests that the umami receptor T1R1 and the sweet receptor have distinct mechanisms for acting on VGSC while maintaining mutual coordination.

The results of (Figure 18) indicate that the action of casein kinase 2 (CK2) inhibitor TBB on the S-S curve of the voltage-gated sodium channel (VGSC) (a shift toward hyperpolarization) and the subsequent action of MSG partially restoring the TBB-induced S-S curve suggest that MSG's effect on VGSC via the T1R1 umami receptor is independent of the effect caused by CK2.

In the left panel of (Figure 18), the S-S curve was measured by stepwise changing the VGSC membrane potential from different steady-state holding voltages to -20 mV and stimulating VGSC. Inhibition of CK2 by TBB significantly shifted the S-S curve toward hyperpolarization (leftward arrow). Subsequent exposure to MSG partially restored the leftward shift induced by TBB and caused a rightward shift (rightward arrow). The right side of the S-S curve (I-V curve) was measured at a holding potential of -120 mV, which influenced the maximum current due to the effects of the reagents.

The right panel of (Figure 18) shows the actual VGSC current trace obtained from measuring the S-S curve. The vertical axis scale is consistent, allowing clear confirmation of the VGSC maximum current for each reagent. MSG treatment also revealed delayed VGSC inactivation processes.

The experimental results in (Figure 18) confirm that inhibition of casein kinase 2 (CK2) by TBB suppresses VGSC. This occurs because CK2 inhibitors shift the membrane potential conditions necessary for VGSC activity to a negative potential range within the cell's physiological tolerance, thereby promoting inactivation. This observation was made using patch-clamp techniques. Our invention represents the first report describing this phenomenon. Considering the enhancement of VGSC by paclitaxel (Taxol), a microtubule stabilizer constituting the cell cytoskeleton, and the enhancement of VGSC by colchicine, a microtubule depolymerizing agent, as well as the inhibition of VGSC current by latrunculin A, a microfilament polymerization inhibitor, we concluded that taste receptor stimulation via the cell cytoskeleton controls VGSC current.

In summary, the strong impact of the relationship between VGSC and the cell cytoskeleton on VGSC current modulation suggests that TBB, a CK2 inhibitor, may also have therapeutic implications for pain relief, epilepsy-like seizures, and other conditions.

(Implementation Example5) Effects on Paclitaxel (Taxol)-Induced Neuropathic Pain

Neuropathic pain resulting from neural hyperactivity, other than brain neurologic disorders, can occur due to agents like paclitaxel (Taxol), an anticancer drug used in breast cancer treatment. We hypothesized that this neuropathic pain is associated with amplified voltage-gated sodium channel (VGSC) currents. To investigate the impact of Taxol on VGSC currents, we used HEK Nav1.5 cells and examined VGSC currents in response to Taxol (Figure 19). VGSC currents were recorded from HEK Nav1.5 cells, and Taxol was applied at concentrations ranging from 0.4 $\mu$M to 2.4 $\mu$M. In all experiments, VGSC enhancement was observed (Figure 19, n=4). Notably, Taxol exhibited similar VGSC-enhancing effects as glutamate and A$\beta$25-35 peptide. This enhancement potentially lowers the threshold for action potential generation, leading to burst-like activity associated with pain. Additionally, the degree of microtubule polymerization may play a role in VGSC activity. These findings suggest that both direct action on the cell cytoskeleton (microtubules) and stimulation via taste receptors (similar to L-glutamate and A$\beta$25-35 peptide) enhance VGSC.

Furthermore, we observed that dichlorprop shifted the S-S curve of VGSC in the hyperpolarizing direction even in the presence of Taxol, resulting in VGSC current inhibition/reduction. This suggests that compounds with VGSC inhibitory effects mediated by T1R3 inhibitors could potentially be applied for treating neuropathic pain concurrent with Taxol.

(Implementation Example 6) Substances Targeting T1R3 with VGSC Inhibitory Effects

(6-1) The VGSC Inhibitory Effects of Cinacalcet Act via T1R3

Mattheisen et al. reported that cinacalcet (a calcium-sensing receptor allosteric modulator), calindol, calhex, and NPS2143 completely inhibit VGSC in the majority of cultured neocortical neurons in mice. Their study indicated that VGSC inhibition by these reagents occurs via GPCRs, independent of PKA and PKC, and is not affected by CaSR knockout mice (non-patent literature 7). However, the specific GPCR involved was not identified in their report.

In this implementation example, considering that sweet amino acids act on VGSC function via T1R3 or T1R2/T1R3 sweet taste (GPCR) receptors, we hypothesized that the effects of CaSR allosteric modulators used by Mattheisen et al. on VGSC involve sweet taste receptors. To test this, we used CaSR agonist Cinacalcet and antagonist NPS-2143 among the reagents mentioned. We examined their effects on VGSC expressed in human-derived HEK Nav1.5 and SH-SY5Y cells, verifying whether similar effects occur in human cells (GPCRs) as observed in mouse neurons. Both cinacalcet and NPS-2143 inhibited human VGSC (NPS-2143: n=10, Cinacalcet: n=11). Notably, the required concentration for VGSC inhibition was 5 $\mu$M, and the inhibition took 3-5 minutes to complete, consistent with the report by Mattheisen et al. (non-patent literature 7). We present an implementation example showing the inhibitory effect of 5 $\mu$M NPS-2143 on VGSC in HEK Nav1.5 (Figure 20) and the effect of 5 $\mu$M cinacalcet on SH-SY5Y (Figure 21). In (Figure 21A), intrinsic VGSC currents in SH-SY5Y and subsequent outward potassium channel currents are visible. After cinacalcet treatment, both VGSC and potassium channel currents were suppressed. (Figure 21B) shows the time course of VGSC and potassium channel current changes after cinacalcet treatment, demonstrating complete inhibition of both currents within a similar timeframe.

(6-2) Analgesic effect through VGSC inhibition via T1R3

To test the hypothesis that the above-mentioned reagents act through the sweet taste receptor, we pre-treated T1R3 with lactisole (Nakagita et al., 2019), which specifically binds to T1R3, at concentrations of 1-1.5 mM before applying cinacalcet. As a result, we confirmed that cinacalcet's action on HEK Nav1.5 VGSC was inhibited (n=3, data not shown). This experimental result strongly suggests that cinacalcet's inhibitory effect is mediated by T1R3.

To strengthen these findings, we conducted similar experiments using HEK Nav1.5 cells in which T1R3 expression was silenced (Figure 22). We measured VGSC currents in T1R3-silenced HEK Nav1.5 cells, applying stimuli every 8 seconds. Interestingly, when we applied cinacalcet at 20 $\mu$M (which completely inhibits VGSC currents in control HEK Nav1.5 cells), no observable effect was seen. This result confirms that suppressing T1R3 expression renders cinacalcet's inhibitory effect ineffective (n=7).

(6-3) Observation of cinacalcet inhibition on VGSC in cells with altered cytoskeleton polymerization state

Based on the results from section (6-2), which suggested that VGSC current inhibition by cinacalcet involves the cytoskeleton polymerization state, we tested cinacalcet's inhibitory effect on VGSC in cells treated with cytochalasin D (which affects microfilaments) or colchicine (which affects microtubules) (Figure 23). In the lower panel of (Figure 23A), we show VGSC currents after control, cytochalasin D treatment, and cinacalcet treatment from left to right. The upper plot in (Figure 23A) displays I-V plots and S-S curves for each recording condition. Cinacalcet's effect was inhibited by cytochalasin D treatment, suggesting that cinacalcet's action is regulated by microfilament polymerization. In (Figure 23B) , we treated cells with colchicine to depolymerize microtubules and observed that cinacalcet still inhibited VGSC currents similarly to untreated cells. Therefore, microtubules do not appear to be involved in cinacalcet-mediated VGSC current inhibition. Based on these results, we conclude that cinacalcet's VGSC inhibition occurs via T1R3 in HEK cells and SH-SY5Y neuroblastoma cells.

(6-4) Inhibition of VGSC by NPS-2143, a CaSR antagonist. Similarly to cinacalcet, NPS-2143, a CaSR antagonist, also suppressed VGSC currents. However, an important difference was observed between the two compounds. After administration, NPS-2143 led to non-selective leak currents (both outward and inward) in many cases when VGSC inhibition was nearly complete. This phenomenon was not observed with cinacalcet. Conversely, cinacalcet reduced leak currents (Figure 24).

(Figure 24A) shows the time course of VGSC current inhibition by NPS-2143. The upper trace in (Figure 24B) represents the actual VGSC current, with black symbols indicating peak current and white symbols representing baseline values. The lower plot in (Figure 24B) shows the VGSC current and baseline values after NPS-2143 and cinacalcet treatment. It was consistently observed that leak currents increased at the timing when VGSC current was almost completely inhibited. To test whether cinacalcet could reverse this leak current, it was administered after NPS-2143. As expected, cinacalcet reduced the leak current.

This suggests that while NPS-2143 and cinacalcet exhibit similar effects in VGSC inhibition, they have opposite effects on cell cytoskeleton and cell membrane integrity. Specifically, cinacalcet promotes microfilament polymerization, whereas NPS-2143 may inhibit it.

(Implementation Example 7) Application of cinacalcet as a local anesthetic and analgesic for neuropathic pain. Local anesthetics are known to effectively diffuse across cell (lipid) membranes only when they are uncharged (non-ionized). However, most local anesthetics have pKa values greater than pH 7.6 at 37°C. Under inflammatory acidic conditions, local anesthetics predominantly exist in a charged (ionized) form, limiting their membrane permeability and membrane activity. Consequently, the effectiveness of local anesthetics significantly decreases under inflammatory acidic conditions (Tsuchiya, 2008).

Therefore, instead of directly targeting VGSC, compounds like cinacalcet that target extracellular sweet taste receptors (GPCRs) were considered effective in inhibiting VGSC even under inflammatory acidic conditions. To measure the VGSC inhibition effect of cinacalcet, cell culture medium adjusted to pH 6 (acidic conditions) was used (Figure 25). The results confirmed that cinacalcet exhibited VGSC inhibition even at a concentration of 5 $\mu$M under acidic conditions (pH 6), making it effective as a local anesthetic.

Additionally, the pain associated with late-stage cancer metastasis is believed to be caused by the release of H+ from invasive cancer cells, resulting in an acidic environment. Therefore, cinacalcet's analgesic application in late-stage cancer is plausible.

When cinacalcet is used for the treatment of hypercalcemia, the plasma concentration is reported to be 50 nM. The concentration of 5 $\mu$M used in this experiment is approximately 100 times higher. However, local anesthetics are typically administered locally along with vasoconstrictors such as epinephrine. As a result, their effects are limited to the injection site, and widespread diffusion into the surrounding tissues is unlikely. Based on this, cinacalcet appears to be a promising candidate for local anesthesia.

(Implementation Example 8) Analgesic effects of artificial sweeteners. In this implementation example, we investigated the impact of artificial sweeteners-saccharin, aspartame, and sucraloseon VGSC (voltage-gated sodium channel) activity. The results showed that all the artificial sweeteners tested shifted steady-state inactivation of VGSC in a concentration-dependent manner toward the negative direction. (Figure 26) illustrates the experimental results after administering saccharin. Compared to the control, 2 mM and 4 mM saccharin caused a significant reduction in VGSC amplitude without altering the waveform. This suggests that proper concentrations of saccharin can inhibit overactivity and normalize neural function.

This phenomenon occurs because VGSC becomes transiently inactivated shortly after activation, preventing current flow. If the membrane potential is not sufficiently restored from inactivation (e.g., maintaining a negative value like -120 mV), the number of VGSC channels available for activation decreases. Consequently, the VGSC current activated by depolarizing stimuli becomes smaller. The reduction in VGSC current due to saccharin and the leftward shift in the steady-state inactivation curve at -120 mV indicate that VGSC may not fully recover. Similar changes were observed with aspartame, although the concentration required for a comparable shift was approximately 1/5 of that for saccharin, suggesting that aspartame has a stronger effect.

[Table 8]

| Cell type | Prepulse (mV) | Aspartame | |
|---|---|---|---|
| | | IC50($\mu$M) | Hill Slope |
| HEK Nav1.5 | -70 | 15.3 | 1.9 |
| | -100 | 49.7 | 4.2 |
| HEK Nav1.6 | -80 | 17 | n.d. |
| SH-SY5Y | -100 | 17$\mu$M Blocked | |

We calculated the effective concentration of aspartame using VGSC inhibition as an indicator. The IC50 value for aspartame, based on the analysis results, was <17 $\mu$M (for Nav1.5, with a membrane potential set at -100 mV, it was 49.7 $\mu$M). This IC50 value, obtained using a calcium assay for T1R2/T1R3 overexpressed by Masuda et al. (2012), reflects the concentration at which aspartame induces an increase in intracellular calcium (EC50) rather than VGSC inhibition. Notably, this value was more than 10 times higher than the value obtained using VGSC as an indicator. The mechanism of VGSC inhibition by artificial sweeteners is believed to differ from the pathway through which they are recognized as sweet compounds.

We simultaneously introduced the T1R2 and T1R3 genes to test whether stimulation by artificial sweeteners enhances the response of endogenous sweet receptors. However, no significant changes were observed in the response of endogenous sweet receptors. Therefore, when only T1R2 was expressed, reproducible results were obtained, showing a significant leftward shift in the steady-state inactivation curve toward hyperpolarization. (Figure 27) shows recordings from cells expressing T1R2 in HEK Nav1.5. Although VGSC current was very small in these cells, overexpression of T1R2 resulted in a large leftward shift in the steady-state inactivation curve, similar to the effect observed when artificial sweeteners acting as T1R2 antagonists were applied. Specifically, when a holding voltage of -100 mV was set and a stimulus pulse was applied, VGSC was completely inhibited. However, when the preceding pulse holding voltage was set to -120 mV, -140 mV, and -180 mV (hyperpolarization), the amplitude of VGSC current in response to the stimulus significantly increased and recovered (n=3). The loss of inhibitory effects when the holding voltage was set in the hyperpolarizing direction suggests that the principle of VGSC inhibition via the T1R2 sweet receptor by artificial sweeteners occurs by promoting VGSC's inactive state during steady-state conditions.

(Implementation Example 9) Inhibition of hyperactivity by Gymnema tea extract.

Next, we investigated Gymnema tea extract (GTE), a traditional folk medicine used in India for over 2,000 years to treat diabetes and obesity. Gymnema tea is derived from Gymnema sylvestre, a tropical plant known for its anti-sweet taste properties. The active component of Gymnema tea is gymnemic acid. In Western countries, Gymnema and other plant-derived anti-sweet compounds are increasingly recognized as alternative therapies for lowering high blood glucose levels and treating obesity (Mailiet et al., 2009). Similar to saccharin, the results of experiments using Gymnema tea extract (GTE) showed a leftward shift in the steady-state inactivation curve. We conducted this experiment not only in HEK Nav1.5 cells but also in SH-SY5Y cells and NG108-15 cells. Analyzing the shift toward hyperpolarization of the membrane potential between the peak of the steady-state inactivation curve expressed by Vh and zero response current before and after GTE administration, we summarized the values in the table in (Figure 28) (n=2). These results suggest potential efficacy of GTE for AD treatment, similar to dichlorprop.

(Implementation Example 10) Inhibition of VGSC Hyperactivity Induced by A$\beta$25-35 Peptide Using Anti-T1R3 Antibodies

(10-1) Inhibition of A$\beta$ Peptide-Induced Enhancement of VGSC Inward Current by Anti-T1R3 Antibodies

HEK Nav1.5 cells seeded on cover glass were pre-incubated for at least 10 minutes in a culture medium containing a 1,000-fold diluted anti-T1R3 antibody. Subsequently, the HEK Nav1.5 cells were transferred to recording extracellular solution (HBPS) using patch-clamp techniques to record VGSC currents. After the VGSC current amplitude stabilized, A$\beta$ peptide was administered, and its effects were measured. In cells treated with anti-T1R3 antibodies, the VGSC current amplitude did not increase at A$\beta$ peptide concentrations that typically amplify VGSC currents. Instead, a leftward shift occurred in the threshold for VGSC activation (hyperpolarization). The cells were cultured in a medium containing A$\beta$25-35 peptide, and the inward currents of VGSC were measured in the same manner as in Implementation Example 2-1 (Figures 4, 6, and 7).

The same experiment was conducted with SH-SY5Y cells, and similar inhibition of the A$\beta$25-35-induced VGSC

current enhancement was observed (Figure 29).

Interestingly, when the incubation time with anti-T1R3 antibodies was 5 or 7 minutes, the inhibitory effect on Aβ25-35-induced VGSC enhancement was insufficient in both SH-SY5Y and HEK Nav1.5 cells.

(10-2) Inhibition of APB Peptide Binding to Neurons by Anti-T1R3 Antibodies

Fluorescently labeled Aβ25-35 peptide (FL-Aβ25-35) was applied to SH-SY5Y cells for 20-30 minutes, resulting in fluorescence labeling of the cells. Subsequently, we investigated whether the binding of FL-Aβ25-35 peptide itself to SH-SY5Y cells changed when treated with T1R3 antibodies. Interestingly, pre-treatment with T1R3 antibodies almost completely prevented the fluorescence induced by FL-Aβ25-35 peptide (Figure 30, left), compared to the control (Figure 30, right).

The anti-T1R3 antibodies not only inhibited the Aβ peptide-induced VGSC current enhancement but also showed a tendency to reduce the binding of FL-Aβ25-35 peptide to SH-SY5Y cells. These findings suggest that anti-T1R3 antibodies could be a potential therapeutic candidate to prevent Aβ peptide binding to neurons and mitigate neuronal hyperexcitability mediated by VGSC.

(10-3) Enhancement of VGSC Current by Colchicine with Microtubule Disassembly and Reduction of VGSC Current by Latrunculin A with Microfilament Disassembly

Colchicine, which disassembles microtubules, enhances VGSC, while Latrunculin A, which disassembles microfilaments, reduces VGSC.

Interestingly, even in the presence of Latrunculin A, Aβ25-35-induced VGSC enhancement was observed. Similar to TBB, artificial sweeteners, and dichlorprop, 12.6 μM latrunculin a shifted the VGSC current toward hyperpolarization and inhibited it. Additionally, the overall VGSC current amplitude was reduced. When 12.5 μM Aβ25-35 was applied under these conditions, the VGSC current amplitude, previously decreased by Latrunculin A, doubled (Figure 31).

These results suggest that the polymerization state of microfilaments does not play a crucial role in the mechanism of Aβ25-35-induced VGSC enhancement. Particularly in cells where the enhancement effect of Aβ25-35 is not influenced by the polymerization state of microfilaments (i.e., under normal conditions), the enhancement via Aβ25-35 through microtubules is likely controlled.

Investigation of Microtubule Polymerization State and Aβ25-35-Induced VGSC Enhancement

To explore how the polymerization state of microtubules affects VGSC (voltage-gated sodium channel) kinetics, we examined whether Aβ25-35 peptide-induced VGSC currents are enhanced under the presence of the microtubule polymerization inhibitor colchicine (2.25 μM). In these cells, colchicine caused a slight shift in the I-V curve toward hyperpolarization. At -20 mV, the VGSC current change was limited, but at -40 mV, the VGSC current amplitude nearly doubled. However, when we applied 12.5 nM Aβ25-35, no further enhancement of VGSC current was observed (Figure 32). Although colchicine's VGSC current enhancement effect was relatively small in these cells, the Aβ25-35-induced enhancement was completely inhibited.

From these results, we conclude that Aβ25-35-induced VGSC current enhancement depends on the polymerization state of microtubules. While this is from experiments using different cells, (Figure 32E) illustrates an example of VGSC current enhancement due to colchicine.

(Implementation Example 11) Screening Method Using GFP-Tubulin to Observe Microtubule Fiber Changes in Aβ Peptide-Induced VGSC Effects

Through patch-clamp experiments, we observed that Aβ peptide (Aβ25-35) still affects VGSC currents even after microfilament disassembly using latrunculin A. This suggests that the polymerization state of cellular cytoskeletal fibers, including microfilaments (which contain microtubules), may influence Aβ peptide effects. We expressed GFP-Tubulin in HEK Nav1.5 cells and observed the microtubule polymerization state upon Aβ peptide treatment. While the control condition clearly showed fibrous microtubule structures, Aβ peptide treatment led to the disappearance of these structures. Therefore, observing the microtubule fiber structure using GFP-Tubulin can be a useful screening method for inhibitors of Aβ peptide effects on VGSC-containing cellular proteins (Figure 33A).

To confirm that the structural changes in microfilaments are not due to direct Aβ peptide effects but rather mediated through Type I taste receptors, we treated cells with MSG (a ligand for taste receptors). (Figure 33B) demonstrates that MSG induced structural changes in microfilaments. After 5 mM MSG treatment, the microtubule structure disappeared due to depolymerization.

Influence of Microfilament Structure on VGSC Currents and Screening Method Using GFP-Actin

Similar to TBB, artificial sweeteners, and dichlorprop, latrunculin A inhibits VGSC currents by shifting the S-S curve toward hyperpolarization. Additionally, the compound cytochalasin D, which affects microfilament polymerization, diminishes the VGSC current inhibition caused by cinacalcet. To observe the impact of cinacalcet and Aβ1-42 peptide on microfilament (actin) fiber structures, we labeled cells with GFP-Actin (Figure 34). Although GFP-Actin did not clearly show fibrous structures compared to GFP-Tubulin, both cinacalcet and Aβ1-42 peptide treatments resulted in condensed GFP-Actin structures. These findings suggest that VGSC current kinetics are influenced by the polymerization state of microfilaments and microtubules. This experimental approach, using GFP-Tubulin, GFP-Actin, or other fluorescent proteins to label microtubules and microfilaments, can be used to screen compounds that regulate the activity of ion channels and neuronal function-controlling proteins. Additionally, simultaneous labeling of tubulin and actin can be achieved using fluorescent antibodies or phalloidin after cell fixation.

In this invention, it was demonstrated that changes in the fiber structure of the cytoskeleton enhance or inhibit ion channels. Therefore, a screening method using cells labeled with GFP-Tublin, GFP-Actin, and similar markers is effective for excluding substances that directly or indirectly affect the cytoskeletal structure to ensure safety in drug development. Particularly, this method is considered useful for screening cardiac safety tests where variations in the function of voltage-gated sodium channels (VGSC) have a significant impact.

(Implementation Example 12) Screening Method for Developing Safe Artificial Sweeteners-Regarding Artificial Sweeteners and Migraine

The relationship between migraine and Nav1.1 VGSC mutants has been reported. Nav1.1 migraine mutants are associated with seizure-like hyperactivity and are categorized into types that induce or decrease Nav1.1 expression. Aspartame inhibits VGSC, which raises concerns that excessive consumption of aspartame in diet drinks, especially for individuals with a genetic predisposition to migraines, could trigger seizures. Other artificial sweeteners like sucralose and saccharin also inhibit VGSC, suggesting a similar risk of seizure induction.

Given the importance of safety in artificial sweetener development (where the IC50 for VGSC inhibition dissociates from the EC50 for taste recognition), assessing VGSC inhibitory effects becomes essential when investigating taste recognition. The results comparing the EC50 for T1R2/T1R3 responses obtained from Masuda et al.'s (2012) calcium imaging method with the IC50 required for VGSC inhibition in this study are shown in [Table 9] below.

[Table 9]

| Compound names | T1R2/T1R3 Receptor Down stream effectors | Inhibition of VGSC (IC50) | Calcium Imaging (G16-gust44 coepxression) EC50 |
|---|---|---|---|
| | Aspartame | <50μM | 750±110μM* |
| | Scuralose | ~27μM | 80±20μM |
| | Saccharin | ~4mM | 190±70μM* |

*Matsuda et al. (2012) PlosOne7, no.4:e35380

Based on this table, substances with low EC50 concentrations for taste recognition compared to the IC50 concentrations that inhibit VGSC suggest that saccharin has the lowest EC50 among the compounds tested, and at the concentrations required for taste recognition, VGSC inhibition does not occur. Other compounds, especially aspartame, exhibit VGSC inhibition at concentrations one order of magnitude lower than taste recognition.

Therefore, when developing safe artificial sweeteners, it is essential to incorporate a screening method that not only assesses VGSC inhibition but also examines the impact of cytoskeletal structures using GFP-Actin and GFP-Tubulin.

Additionally, for the development of analgesics and antiepileptic drugs, substances like aspartame, which have minimal impact on taste, can be effective for VGSC inhibition.

(Implementation Example 13) Consideration of Screening Methods

T1Rs proteins (particularly T1R3 and T1R2) function as receptors for amyloid β peptides and affect VGSC properties such as current magnitude and kinetics. Therefore, in drug screening methods, it is crucial to investigate substances that prevent binding of amyloid β peptides to T1Rs (e.g., T1R3 antibodies), inhibit VGSC functional changes resulting from amyloid β peptide binding, and hinder amyloid β peptide binding to T1Rs (e.g., amyloid β peptide antibodies). Methods such as patch-clamp recording to observe functional changes in VGSC currents and measuring changes in cell membrane capacitance due to amyloid β peptide (at low concentrations <100 nM, VGSC current enhancement occurs via exocytosis, leading to increased cell membrane area and capacitance; at high concentrations, receptor desensitization reverses this process, reducing cell membrane area and capacitance), NanoTouch, and MEA using cultured neurons as indicators of excitability can be applied as screening methods using the present invention.

Furthermore, in this patent invention, it was discovered that ion channels, particularly VGSC, are regulated by the polymerization state of the cytoskeleton. For example, substances that stabilize microfilaments (such as cytochalasin D) inhibit cinacalcet action, while Latrunculin A inhibits Actin polymerization, promoting VGSC inactivation by hindering endosome insertion (via exocytosis) and reducing the number of active VGSCs in the physiological membrane potential range. Conversely, substances like colchicine (which inhibits microtubule polymerization) and taxol (which enhances polymerization) both enhance VGSC. Therefore, assessing the effects of drugs on cytoskeletal polymerization or depolymerization using fluorescently labeled cells as indicators is crucial for safety screening during early drug development, as substances that modify the cytoskeleton may have harmful side effects. Reports on cytoskeletal control of ion channel activity extend beyond neurons and include examples like epithelial sodium channels expressed in epithelial cells (Chifflet et al., 2012).

(Implementation Example 14) Delay in the Inactivation Process of VGSC Current by Monosodium Glutamate (MSG)

Monosodium glutamate (MSG) acts on Nav1.5 expressed in HEK cells via the T1R1/T1R3 receptor and on Nav1.2 and Nav1.7 endogenously expressed in SH-SY5Y cells, affecting the kinetics of VGSC. Notably, the effect is more pronounced in HEK Nav1.5 VGSC compared to SH-SY5Y Nav1.7 VGSC.

MSG also affects the metabotropic glutamate receptors, similar to the T1R1 receptor. However, only T1R1 responds to purine nucleotide monophosphates, specifically inosine 5'-monophosphate (5'IMP) and guanosine 5'-monophosphate (5'GMP). Furthermore, the response to MSG is synergistically enhanced by IMP and GMP, as demonstrated by measuring intracellular calcium changes using a specific method (Zhao et al., 2003).

Based on this, we can conclude that the action of MSG on VGSC is mediated not by metabotropic glutamate receptors but through the T1R1 Type I taste receptor.

In this implementation example, we investigated the effect of 5 mM monosodium glutamate (MSG) on VGSC current kinetics using the voltage clamp technique in HEK Nav1.5 cells. The inactivation process of transient currents was significantly delayed, enhancing the amplitude of sustained VGSC currents (Figure 35). Under ramp pulse stimulation, the impact on the inactivation process was observed during the -40mV pulse following the ramp wave. Specifically, a slow recovery tail current, not seen in the control, was observed after MSG administration. This suggests delayed closure of VGSC at -40 mV, in addition to the deceleration of the inactivation process (n=3).

Specifically, voltage clamp experiments were conducted to investigate the effect of 5 mM monosodium glutamate (MSG) on VGSC current kinetics in HEK Nav1.5 cells. The transiently activating portion was examined using rectangular pulse stimulation (Figure 35, upper left), while the sustained current was studied using a ramp waveform (Figure 35, upper right). Under each experimental condition, the changes in current waveform due to MSG administration were observed. The inactivation process of transient currents was significantly delayed, but the response to sustained currents following ramp stimulation was not markedly affected by MSG.

From this, we can conclude that MSG delays the inactivation process of transient currents, but its impact on sustained currents, which already exhibit inactivation properties, is not evident. Interestingly, tail currents were observed after ramp waveform stimulation that induced sustained currents, following -40 mV pulses after MSG administration. This suggests delayed closure of VGSC at -40 mV, in addition to the deceleration of the inactivation process (Figure 35, lower diagram). The experimental measurements using rectangular pulse stimulation (Figure 35, upper left) are plotted as I-T (current amplitude vs. time) in the lower diagram. In this experiment, MSG did not affect the amplitude of transient currents but caused delayed inactivation and amplification of sustained current amplitudes over time. The x-axis represents the number of stimuli, with stimulation occurring every 8 seconds.

The inactivation process of VGSC currents in SH-SY5Y cells, induced by MSG administration, was delayed, although to a lesser extent compared to HEK Nav1.5 cells. Notably, SH-SY5Y cells differentiate and express action potentials similar to neurons. Therefore, in addition to inward VGSC currents, MSG effects can also be observed on outward potassium currents. As a result, in SH-SY5Y neuronal differentiated cells, MSG was confirmed to decrease both VGSC channel currents and outward potassium currents (Figure 36A).

As shown in (Figure 37), Aβ1-42 peptide, unlike Aβ25-35 targeting T1R3, interacts with T1R1 and inhibits VGSC currents (Figures 37D, E). Consequently, to explore the possibility that MSG's inhibitory effect on potassium currents is also mediated through the T1R1 umami taste receptor, we expressed Kv1.2, predominantly found in neurons, in HEK cells and observed the effects of Aβ1-42. The results confirmed that Aβ1-42 concentration-dependently inhibits Kv1.2 potassium currents (Figure 36B). The upper panel illustrates the process of Kv1.2 current inhibition by Aβ1-42, while the lower panel shows the temporal progression of this inhibition.

Using SH-SY5Y cells differentiated by retinoic acid (RA), we present the results of actual voltage-clamp experiments before and after administering 5 mM monosodium glutamate (MSG) (Figure 36A, upper panel). The membrane potential was fixed at -120 mV (holding voltage), and voltage stimuli were applied from -70 mV to 60 mV in 10 mV intervals. Since intracellular solutions contain potassium ions as monovalent cations, outward potassium currents are also recorded (Figure 36A, upper panel). The recordings after MSG administration were obtained more than 5 minutes later, suggesting that the effect has concluded. While the inactivation process of VGSC currents was delayed by MSG, the extent was smaller compared to HEK Nav1.5 cells (as shown in Figure 35). Notably, in addition to inward VGSC currents, outward currents were also reduced by MSG. The I-V curve is shown below the current trace (Figure 36A, lower left). The inward current represents the transient peak observed immediately after stimulation, while the outward current measures the average value during the last 50 ms of rectangular pulse stimulation. This plot confirms that MSG induces a decrease in outward currents (Figure 36A). The temporal changes in experimental results using ramp waveform stimulation are shown on the right side of (Figure 36A). This figure further confirms MSG's reduction of outward currents (n=2).

Similar inhibition of outward currents was observed with 1 mM GTP (Figure 38), suggesting that the effect is mediated through T1R1 rather than metabotropic glutamate receptors (mGluRs).

The inactivation process of VGSC currents induced by MSG administration in SH-SY5Y cells was delayed, although to a lesser extent compared to HEK Nav1.5 cells. In SH-SY5Y neuronal differentiated cells, which express action potentials similar to neurons, MSG affects both inward VGSC currents and outward potassium currents. MSG was confirmed to decrease outward potassium currents in addition to VGSC channel currents. To explore the possibility that MSG's inhibitory effect on potassium currents is also mediated through the T1R1 umami taste receptor, we expressed Kv1.2, predominantly found in neurons, in HEK cells and observed the effects of Aβ1-42. The results confirmed that Aβ1-42 concentration-dependently inhibits Kv1.2 potassium currents.

(Implementation Example 15) Effects on VGSC via Umami Receptors (T1R1/T1R3)

(15-1) Activation of Umami Receptors (T1R1/T1R3) and Enhancement of VGSC Currents by Umami Substances

Umami substances, including monosodium glutamate (MSG), are sensed by umami receptors (T1R1/T1R3), which function as amino acid sensors distinct from sweet receptors. MSG, an amino acid umami substance, and other known umami substances such as inosine 5'-monophosphate (IMP) and guanosine 5'-monophosphate (GMP) enhance the umami effect of MSG and can independently stimulate umami receptors (T1R1/T1R3). In this study, we investigated how umami receptors (T1R1/T1R3) function as amino acid sensors in cerebrospinal fluid (CSF) and how they activate VGSC. Using HEK cells or SH-SY5Y cells, we activated endogenously expressed umami receptors (T1R1/T1R3) by MSG (Figure 37), IMP (Figure 37), and GMP (Figure 38).

As a result, umami substances, depending on their type, shift the threshold potential for VGSC enhancement and activation in the hyperpolarizing direction (approximately 10 mV) via T1R1. Additionally, they slow down VGSC inactivation rates. This finding demonstrates that umami substances control VGSC activity, providing evidence that VGSC, like taste perception, is a downstream effector. The strong evidence against the involvement of metabotropic glutamate receptors (mGluRs) in the VGSC activation by umami substances lies in the fact that umami receptors (T1R1/T1R3) respond to MSG enhancement by IMP and GMP alone, whereas mGluRs do not respond to IMP and GMP.

(15-2) Effects of Nucleotide Seasonings such as Inosinic Acid (IMP) and Aβ1-42 on VGSC or VGKC via Umami Receptors (T1R1/T1R3)

As mentioned in the previous section (15-1), the changes in the VGSC due to MSG also occur with IMP, which is specific to T1R1/T1R3 receptors. Since IMP, which only acts on T1R1/T1R3, induces the same changes as MSG, it was concluded that the observed changes in the VGSC are due to MSG acting on T1R1/T1R3 receptors rather than on metabotropic glutamate receptors.

The results evaluating IMP's effect on VGSC currents using voltage-clamp experiments are shown in (Figure 37).

IMP led to a reduction in VGSC currents (Figure 37A). The upper panel displays the results before (left) and after (right) 1 mM IMP application. Analyzing these results, we plotted the S-S curve and I-V curve (Figure 37B). In addition to reducing VGSC currents, IMP induced a transient enhancement within approximately 5 minutes after application. This evaluation was based on VGSC current responses using ramp waveform stimulation (Figure 37C).

Specifically, when 1 mM IMP was administered to HEK cells or SH-SY5Y cells expressing endogenous umami receptors (T1R1/T1R3), VGSC current amplification occurred, accompanied by a shift in peak current toward hyperpolarization by approximately 10 mV (Figure 37C). This enhancement was transient, concluding within 30 seconds of IMP administration, and despite IMP's presence, the current size returned to control levels. Occasionally, VGSC currents exceeded the enhancement observed, becoming even smaller than control values. The current traces in (Figure 37A) represent the results after the transient changes due to control and IMP have concluded.

Compared to the effect observed with MSG alone (Figure 35), the delay in the inactivation process of VGSC induced by 1 mM IMP was not significantly more pronounced. However, the current traces in (Figure 37A) show that the delay in inactivation is still evident compared to control.

Next, the effects of Aβ1-42 shown in Fig. 6 in the previous section (Example 2-2) were also investigated in detail (Fig. 37B). When similar voltage-clamp method experiments as in Fig. 37A were conducted and analyzed, it was found that Aβ1-42 caused a decrease in VGSC currents, similar to MSG and IMP. From these results, it is suggested that, unlike Aβ25-35 which acts solely on T1R3, Aβ1-42 prominently causes changes in VGSC currents by acting on T1R1.

We further observed the effects of the second purine nucleotide, guanosine 5'-monophosphate (GMP), on VGSC (Figure 38). Although 2 mM GMP led to a slight increase in VGSC currents, the effect was transient. Unlike IMP, GMP exhibited sustained effects. GMP also reduced outward potassium currents similarly to IMP (Figure 38A). The graph in (Figure 38B) shows VGSC and VGKC currents obtained from SH-SY5Y cells using voltage clamp experiments. From left to right, it displays control, post-GMP application, and further post-MSG application (5 mM). (Figure 38C) presents a similar graph based on the current traces obtained after GMP application, with the size of the white symbols (representing GMP) reduced and the results after MSG application (following GMP) plotted using larger white symbols. Similar to MSG and IMP, GMP caused a leftward shift of approximately 10 mV in the I-V curve. Unlike MSG and IMP, GMP exhibited variability between cells, with VGSC enhancement ranging from 10% to 20% and some cells showing minimal changes.

(15-3) The Effect of Umami Substances on T1R1/T1R3 with VGSC Suppressed by Artificial Sweeteners

Cells expressing the umami receptor (T1R1/T1R3) were pre-treated with artificial sweeteners such as aspartame and saccharin to suppress VGSC. When monosodium glutamate (MSG) or MSG2.5 (a mixture of MSG, IMP, and GMP: Ajinomoto) was applied, a phenomenon was observed where the suppression of VGSC currents by saccharin and aspartame was partially reversed. This was due to the delay in the inactivation process of VGSC, which increased the total influx of sodium ions into the cells during activation (Figure 39). Additionally, when TBB, a casein kinase II inhibitor shown in (Figure 18), was used instead of artificial sweeteners, a similar shift in the VGSC I-V curve to the right (positive direction) was observed after shifting the VGSC S-S curve to the left (negative potential) and then applying MSG (data not shown).

In Figure 39, voltage-clamp experiments were conducted using HEK Nav1.5 cells where T1R2 was activated by saccharin. Initially, transient VGSC currents were activated by applying rectangular pulses (Figure 39, left). This was followed by a ramp waveform stimulus to measure the sustained currents (Figure 39, right). The effect of 5 mM MSG on the kinetics of VGSC currents was investigated. The current waveforms before application (control), 30 seconds after application (middle of Figure 39), and 10 minutes after application (bottom of Figure 39) are shown. The inactivation process of the transient current was significantly delayed, and an increase in the peak transient current was observed in response to ramp wave stimulation. The effect on the inactivation process was also observed as tail currents at -40 mV pulses following the ramp wave after MSG application. This indicates that, in addition to the delay in the inactivation process, the closure process of VGSC at -40 mV was also delayed. The kinetic delay effect of MSG on VGSC was initially very large but partially recovered over time, reaching a steady state 5 minutes after application (n=3)

(Figure 39).

The effect of monosodium glutamate (MSG) on T1R1 occurs independently of the action of 'Group 3' substances, such as aspartame, which inactivate VGSC via T1R2. Therefore, it was found that MSG functions antagonistically to the VGSC inactivation effect mediated by T1R2.

In other words, the effect of umami substances like MSG on VGSC via T1R1 is considered independent of the effects mediated through T1R2 and T1R3. This is supported by experiments using HEK-Nav1.5 cells with silenced T1R3, where the effect of MSG2.5 on T1R1 was observed similarly to control cells.

As shown in (Figure 10), high concentrations of Aβ25-35 peptide desensitize T1R3, and the mechanism suggests that the enhancement of VGSC currents induced by low concentrations of the peptide (through the fusion of intracellular reserve endosomes containing VGSC with the cell membrane) is nullified by re-incorporating the fused endosome-derived cell membrane back into the cell as endosomes.

Additionally, Aβ1-42 peptide, unlike Aβ25-35 peptide, tended to decrease VGSC (Figure 6). This suggests that Aβ1-42 peptide may act not only on T1R2/T1R3 but also on T1R1, unlike Aβ25-35 peptide, which targets T1R3.

On the other hand, in cells treated with T1R3 antibodies, Aβ25-35 peptide caused a decrease in VGSC rather than an enhancement (Figure 8). This phenomenon is similar to the VGSC suppression observed when T1R2 is activated by artificial sweeteners (saccharin, Figure 26) or Gymnema tea extract (Figure 28).

This suggests that the VGSC current enhancement (neuronal hyperactivity) caused by low concentrations of Aβ25-35 peptide occurs through binding to T1R3, while at high concentrations, it acts on T1R2/T1R3, causing VGSC current suppression. This implies the existence of a secondary binding site for Aβ peptides with low affinity to T1R2 compared to T1R3. Therefore, using substances/compounds that act on T1R1, such as MSG, which functions independently of T1R2/T1R3, is expected to promote the recovery of VGSC function.

Such substances targeting the umami receptors (T1R1/T1R3) are classified as 'Group 4' substances in [Tables 2 and 5].

The action of VGSC by a solution of MSG or a mixture of MSG and IMP or GMP cannot be confirmed in experiments using cells with silenced T1R1, indicating that the action is not mediated by the glutamate GPCR receptor (metabotropic glutamate receptor) but by the umami receptor (T1R1/T1R3). The action of MSG in slowing the inactivation rate of VGSC through the umami receptor is considered to have important physiological functions. Specifically, this property of inducing delayed inactivation is important for the expression of high-frequency action potentials, burst action potentials, and the integration of multiple synaptic inputs. Therefore, T1R1/T1R3 or T1R1 homodimers are effective targets for drug development in treating neural activity and synaptic integration.

(Implementation Example 16) Action of various umami substances on VGSC currents

(16-1) Action of GMP

In the previous (Implementation Example15-2), the effects of GMP on inward VGSC currents and outward potassium currents were investigated using SH-SY5Y cells (Figure 38). Specifically, when 2 mM GMP was administered to SH-SY5Y cells, followed by 5 mM MSG, the total current was observed using the voltage clamp method (Figures 38A, B). The I-V curve of the inward current by VGSC was created, and it was confirmed that the VGSC current was enhanced by GMP administration. In (Figure 38), not only VGSC currents but also voltage-dependent potassium currents were plotted. Additionally, the steady-state inactivation curve was included in the same plot. Black symbols indicate control, and white symbols indicate changes after GMP action. VGSC currents showed a slight increase and a left shift in the I-V curve, while potassium currents showed a decrease in peak current. No changes were observed in the steady-state inactivation curve. The enhancement of VGSC currents by GMP alone was smaller compared to IMP, with variability depending on the recorded cells. In the figure, downward arrows indicate the direction of changes caused by GMP.

The action of GMP is similar to MSG in that it has a sustained effect, but differences were observed in its action on the inactivation process. As shown in (Figure 38A), no changes in the inactivation process were observed before and after GMP administration, unlike MSG. When MSG was administered after GMP, a left shift of about 10 mV was observed in both the I-V curve and the steady-state inactivation curve compared to the control (Figure 38C). However, the delay in VGSC current inactivation observed with MSG alone was not observed in the presence of GMP.

(16-2) Effects of MSG2.5 on VGSC Currents

In this implementation example, we tested the effects of MSG2.5, which contains 1.25% IMP and 1.25% GMP (Ajinomoto, MSG 97.5%, IMP 1.25%, GMP 1.25%). MSG2.5 at concentrations of 2.5-5 mM resulted in an increase in the peak current of VGSC and a leftward (hyperpolarizing) shift in the I-V curve. This effect is similar to that of 1 mM IMP, but in the case of 5 mM MSG2.5, despite the very low concentration of IMP (62.5 μM), the delay in the inactivation process caused by MSG was suppressed by IMP. Additionally, the transient enhancement of sustained VGSC currents and the leftward shift of the I-V peak current, which are specific effects of IMP, were more pronounced with MSG2.5 than with MSG alone (Figure 40).

SH-SY5Y Cells: The effect of MSG2.5 on SH-SY5Y currents using the voltage-clamp method showed that the peak VGSC current was enhanced by MSG2.5. No changes were observed in the VGSC current inactivation process or the magnitude of the outward current with MSG2.5 administration (Figure 40A). The figure shows the current traces from left to right: control and after MSG2.5 administration. The right side plots the changes in current before and after MSG2.5 administration.

HEK Nav1.5 Cells: Similar to SH-SY5Y cells, MSG2.5 amplified the peak VGSC current, but unlike MSG alone, the degree of current enhancement and the impact on the inactivation process were limited (Figure 40B). Next, we investigated how MSG2.5 affects the sustained VGSC current with slow inactivation, rather than the transient component of the VGSC current. An I-V curve was created at 540 ms after the start of the pulse, when sufficient time had passed for inactivation to progress after the rectangular pulse action, and the changes before and after MSG2.5 administration were compared. An enhancement of the VGSC current was observed (Figure 40C left). Although the analysis of the I-V plot suggests that the effect of MSG2.5 on Nav1.5 recordings is significantly smaller compared to SH-SY5Y recordings, a clear effect was observed. The results of the I-V plot indicate that the receptor was activated and desensitization occurred. In the continuous voltage-clamp experiments using ramp waveforms before and after MSG2.5 administration, the transient increase in the peak VGSC current and the leftward shift were observed, but the effect decreased over time and returned to control values by the end of the recording (Figure 40C right).

(16-3) Effects of MSG2.5 on T1R1-Silenced HEK Nav1.5 Cells

Next, we investigated the role of T1R1 in the response to umami (MSG) stimulation using HEK Nav1.5 cells transiently expressing the T1R1 silencing vector. The experiment was conducted using cells that had been transfected with the T1R1 silencing vector for more than three days, using MSG2.5. Since the silencing vector co-expresses eGFP, cells that fluoresce were considered T1R1-silenced cells, and experiments were conducted using the voltage-clamp method (patch-clamp). Similar to the experiment shown in (Figure 40) of (Implementation Example16-2), experiments were conducted using MSG2.5, but no changes were observed in VGSC currents before and after MSG2.5 administration with rectangular pulse and ramp wave stimulation (Figure 41). Since MSG2.5 contains IMP, it is characterized by a leftward (hyperpolarizing) shift in the peak current with ramp wave stimulation, but no such changes were observed in experiments using T1R1-silenced cells (n=4). From the above experimental results, it was concluded that MSG affects VGSC kinetics via T1R1 GPCR. As will be described later, the effects of MSG2.5 are also observed in T1R3-silenced cells, confirming that MSG2.5 (MSG) acts through T1R1 and that the involvement of T1R3 is not essential.

(16-4) Functional Significance of Umami Receptor Effects on VGSC Currents:

Delayed inactivation of persistent VGSC currents can cause burst-like action potentials and high-frequency repetitive action potentials in neurons (Alzheimer et al. (1993)). Persistent VGSC currents are considered important during the integration and enhancement of synaptic inputs in dendrites, potentially playing a crucial role in determining the input-output relationship of neurons.

In HEK Nav1.5 cells with suppressed T1R1 expression, the effect of MSG2.5 on VGSC currents disappeared (Figure 41A). Using rectangular waves and voltage-clamp methods, VGSC currents were recorded from HEK Nav1.5 cells with silenced T1R1, showing the I-V curve before and after MG2.5 administration (• before administration, ○ after MG2.5 administration). Using ramp waves and voltage-clamp methods, the effect of MG2.5 on persistent VGSC currents was measured. In HEK Nav1.5 cells with silenced T1R1, no changes in VGSC current waveforms were observed before and after MG2.5 administration (Figure 41C). The role of T1R3 in the effect of MG2.5 on VGSC currents was investigated. In HEK Nav1.5 cells with silenced T1R3, the effect of MG2.5 on VGSC currents was confirmed to be similar to that in control cells. This confirmed that the presence of T1R3 is not involved in the effect of MG2.5 on VGSC currents. Although further research is needed to draw conclusions due to the incomplete suppression of T1R3 expression by the T1R3 silencing vector, this experiment suggested that T1R1 might function without forming a heterodimer with T1R3 (Figure 41B).

Next, similar to the observations with IMP in (13-2) and (14-2) (Figures 37A-C, 40C), the effect of persistent VGSC currents by MSG2.5 was observed and analyzed using voltage-clamp methods and ramp waveforms. The VGSC peak current transiently shifted in the hyperpolarizing direction and tended to recover. However, in Nav1.5-expressing cells with silenced T1R1 expression, no effects of MSG2.5 were observed. This concludes that MSG and its agonists act through the T1R1/umami receptor.

(Implementation Example 17) Measurement of VGSC Enhancement by Aβ Peptide via T1R3 Using the "NanoCharge Method"

In this implementation example, the spontaneous action potentials of differentiated SH-SY5Y cells were measured using the "NanoCharge Method" (WO2019/208828) previously developed by the inventors, and changes in VGSC currents by Aβ25-35 peptide were measured.

The "NanoCharge Method" developed by the inventors is a technique that enables the recording of intracellular potentials without using conventional electrodes, using a capacitive potential detection device. The effects of many T1Rs receptor agonists and antagonists, including Aβ25-35, on action potentials can be measured and observed using the "NanoCharge Method." By using spontaneously firing cells such as differentiated SH-SY5Y cells and iPS cell-derived cardiomyocytes, the changes in intracellular potentials mediated by sweet and umami receptors (T1Rs) on the cell surface can be measured and observed. This allows for the rapid and simple observation of the effects of test substances on neuronal action potentials, making it applicable as a screening method for AD treatment drugs

By using magnetic conductive nanoparticles as intracellular electrodes for measuring the neuronal action potentials of differentiated SH-SY5Y cells, and combining this with a capacitive potential detection device to measure changes in intracellular potentials, the spontaneous action potentials of these cells were measured. Specifically, 6.25 nM of the T1R3 agonist Aβ25-35 peptide was added to 400 µl of SH-SY5Y cell culture medium, and the spontaneous action potentials before and after the addition of A β 25-35 peptide were measured to observe the changes.

This membrane potential recording method focuses on the magnetic conductive nanoparticles penetrating the cell membrane, the cover glass on which the cells are seeded, and the electrode placed below the cover glass forming a capacitor. The charge (Q) charged to this capacitor is recorded as the membrane potential in terms of charge rather than voltage changes. Since the charge (Q) is obtained from Q = CV, where C is the capacitance and V is the potential, the charge changes measured here are influenced by the capacitor (C) (Figure 43).

In the upper control panel of (Figure 43), the occurrence rate of action potentials is low, but in the lower panel after the action of Aβ25-35 peptide, an increase in the frequency of spontaneous action potentials was confirmed. The action potentials marked with (*) in the upper panel and (•) in the lower panel are shown on the right side of the figure with a compressed time axis. Calibration Bars: X-axis - Time (Second); Y-axis - Relative Voltage Unit (R.V.U.). Since the intracellular potential is measured as charge, it was measured as a relative potential (Figure 43).

(Implementation Example 18) Effects of Aβ1-38 and Aβ1-40 Peptides on VGSC (HEK Nav1.5 Cells)

(18-1) Experiments Using Cardiac-Type VGSC (HEK Nav1.5 Cells)

In previous implementation examples, the effects of Aβ peptides, particularly the highly toxic Aβ25-35 peptide and Aβ1-42, on VGSC via T1Rs were examined. In this implementation example, the effects of Aβ1-38 and Aβ1-40 peptides on VGSC were further investigated using cardiac-type VGSC (Nav1.5) cells.

Aβ25-35 is thought to increase VGSC currents by increasing the number of VGSCs on the cell membrane by moving them from intracellular reservoirs via T1R3, without affecting properties such as S-S and inactivation. On the other hand, the effects of Aβ1-42 were similar to changes induced by T1R1 umami receptor stimulation by MSG (delayed inactivation, suppression of VGSC currents, and suppression of VGKC).

(Figure 44A) shows an implementation example where Aβ1-38 was applied to HEK Nav1.5 cells. After holding the membrane potential at -80 mV, depolarizing stimuli were applied from -70 mV to +50 mV in 10 mV increments. Subsequently, the membrane potential was held at -120 mV, and similar depolarizing stimuli were applied, plotting the VGSC current magnitude (vertical axis) against the membrane potential (horizontal axis). In the control, the VGSC current response at -80 mV holding potential was very small, but after the action of Aβ1-38, the VGSC current magnitude was significantly amplified. This amplification is thought to be due to the S-S curve (control, post-treatment) shifting to the depolarizing side (right side). The VGSC current at the holding potential of -120 mV (• control, ○ post-treatment) slightly decreased, but this change suggests that action potentials are more likely to occur even at shallower membrane potentials (depolarized), indicating increased excitability in the presence of Aβ1-38 in cardiac cells. Similar to Aβ1-42, Aβ1-38 also showed a delayed inactivation effect on VGSC (Figure 44A lower panel), suggesting that this change makes cells more excitable.

In Figure 44B, the effects of Aβ1-40 were measured using the same experimental protocol as in (Figure 44A). Unlike Aβ1-38, Aβ1-40 shifted the S-S curve (control, post-treatment) more towards the hyperpolarizing direction. The I-V plots for holding voltages of -80 mV (• control, ○ post-treatment) and -120 mV (control, post-treatment) are shown in the upper part of (Figure 44B).

From this experiment, it is suggested that in cardiac-type VGSC cells, Aβ1-40, unlike other Aβ peptides, tends to suppress VGSC currents more and prevent the occurrence of action potentials. If Aβ1-40 exhibits similar effects in cardiac cells in vivo, cells affected by Aβ1-40 in areas such as the ventricular wall where amyloid deposition occurs may find it difficult to generate action potentials. This could hinder the uniform and periodic transmission of action potentials generated in surrounding normal cells, potentially leading to arrhythmias and premature contractions.

(18-2) Follow-up Experiments Using Neuronal VGSC (Nav1.1 or Nav1.6) Cells

The cells used in the previous (18-1) experiment were cardiac-type VGSC cells. In this experiment, HEK (Nav1.1) and HEK (Nav1.6) cells, which are considered neuronal VGSC cells, were created. The effects of four peptides-Aβ25-35, Aβ1-38, Aβ1-40, and Aβ1-42-were examined using the same experimental protocol as in (18-1).

As a result, Aβ25-35, Aβ1-38, and Aβ1-40, except for Aβ1-42, all showed VGSC enhancement similar to that observed in cardiac-type VGSC cells. Although no effects on the S-S curve were confirmed, the I-V curve rise shifted 10 mV to the left (negative direction) for all peptides, albeit to varying degrees (Data not shown).

Considering these results, in neuronal VGSC cells with Nav1.1 or Nav1.6, the S-S curve is originally positioned to the right, unlike Nav1.5. Therefore, the right shift of the S-S curve observed with Aβ1-38 in (18-1) did not occur. The reason for the lack of a left shift effect on the S-S curve with Aβ1-40 is unknown, but if the S-S curve shift is determined by the relationship between VGSC and ankyrin, the difference in the amino acid sequence that binds to ankyrin in cardiac-type VGSC (Nav1.5) cells compared to neuronal VGSC cells might be a contributing factor.

Furthermore, in the intracellular calcium fluctuation experiments using HEK (Nav1.1) cells described in (Implementation Example 19), Aβ25-35, Aβ1-38, and Aβ1-40, excluding Aβ1-42, all showed similar time-dependent increases in intracellular calcium.

Considering all these points, the similarity of Aβ1-38 to Aβ1-42 and the opposite trend of Aβ1-40 observed in (18-1) are interpreted as phenomena specific to cardiac-type VGSC (Nav1.5) cells. It is highly likely that Aβ peptides other than Aβ1-42, such as Aβ25-35, Aβ1-38, and Aβ1-40, can also cause diseases related to "overexcitation of neurons mediated by T1R2/T1R3." At the very least, it can be said that Aβ1-42 peptide, including other Aβ peptides, are substances that cause diseases related to "overexcitation of neurons mediated by Type I taste receptors."

Meanwhile, although different trends were observed in the effects of Aβ peptides due to structural differences between cardiac-type VGSC (Nav1.5) cells and neuronal VGSC (Nav1.1, Nav1.6) cells, the inhibitory effect of VGSC currents by latrunculin A, a microfilament polymerization inhibitor, was observed in neuronal VGSC cells, including SH-SY5Y cells, similar to cardiac-type VGSC. Therefore, it is considered that the inhibitory effect on VGSC via T1R2 is not influenced by structural differences in VGSC, suggesting a different mechanism from the action of Aβ peptides.

(Implementation Example 19) Screening Method for Inhibitors of Voltage-Dependent Ion Channel Activation Current via Type I Taste Receptors by Aβ Peptide or Sweet Amino Acids or Umami Substances

In this implementation example, we demonstrate that the effects of Aβ peptide, sweet amino acids, or umami substances in CSF on the activation current of voltage-dependent ion channels in cells endogenously expressing Type I taste receptors (T1R GPCR), as well as the effects of their inhibitors, can be observed using a fluorescent intracellular calcium-sensitive reagent.

Specifically, experiments were conducted as follows using HEK cells stably expressing Nav1.1 (Figure 45). The calcium-sensitive reagent (Cal-520, AAT Bioquest) was incubated at 37°C for 60 minutes, followed by incubation at room temperature for 30 minutes, and then changes in intracellular calcium were measured using a fluorescence microscope. (Figure 45A) shows the experimental results when Aβ1-40 was applied. Initially, 10 nM, followed by 20 nM of Aβ1-40 was applied after 5 minutes (arrow). After application, intracellular calcium continued to slowly increase for more than 10 minutes. A total of 6 cell groups from two independent experiments were measured, averaged by the maximum fluorescence response value, and the mean and standard error (stderr) were calculated and plotted. (Figure 45B) shows the experimental results when Aβ1-42 was applied. 10 nM, followed by 20 nM of Aβ1-42 was applied after 5 minutes (arrow). After the application of 10 nM, unlike the case of Aβ1-40, intracellular calcium transiently decreased, slowly recovered, and then began to increase. When an additional 10 nM (total 20 nM) of Aβ1-42 was applied, intracellular calcium slowly increased similarly to the case of Aβ1-40. A total of 12 cell groups from two independent experiments were measured, averaged by the minimum fluorescence response value, and the mean and standard error (stderr) were calculated and plotted. Similar to the experimental results using the patch-clamp method when Aβ peptide was applied to Nav1.5, it was confirmed from intracellular calcium measurements that the effects of Aβ1-40 and Aβ1-42 were different.

It is noteworthy that when overexpressing Type I taste receptors (T1R GPCR) and measuring their response by changes in intracellular calcium concentration, it has been traditionally necessary to co-express Gα15, Gα16 (which convert GPCR activation to changes in intracellular calcium by activating PLC), and Gα16 and gustducin chimeric G16-gust44 (a G protein chimera containing the C-terminal 44 amino acids of gustducin) (Jiang et al. (2005)). However, when measuring the function of endogenous Type I taste receptors (T1R GPCR), it is not necessary to co-express Gα15, and it is possible to measure biphasic intracellular changes that transition from a decrease to an increase in intracellular calcium.

Therefore, from these points, this method is considered to accelerate the study of the function of Type I taste receptors more physiologically. This is extremely important for the study of the effects of Aβ peptides and the search for their binding inhibitors, as well as for responses to umami and sweet stimuli.

Furthermore, using the same HEK (Nav1.1) cells, when Aβ25-35 and Aβ1-38 peptides were administered and changes in intracellular calcium were measured, an increase in intracellular calcium was observed with a similar time course to that of Aβ1-40 (Data not shown).

Type I taste receptors (T1R GPCR) couple with G protein Gα (Gq, Gs, Gi/o, G12/13) and βγ subunits. The following summarizes what is known about each G protein:

Gq produces IP3 and DG through the activation of PLC. IP3 releases Ca2+ from the ER, and DG activates PKC.

Gs activates adenylate cyclase (AC), producing cAMP and activating PKA, while Gi inhibits AC (Schappi et al., 2014).

The effect of G proteins on cytoskeletal structures: The promotion of microfilament formation occurs through the activation of Rho kinase by G12/13. Conversely, microfilaments are inactivated and degraded when phosphorylated by PKA activated by Gs (Newell-Litwa and Horwitz, 2011).

Microtubule formation is promoted by tubulin polymerization by $G\beta\gamma$, while Gs activation promotes microtubule GTPase activity, leading to tubulin depolymerization and degradation (Roychowdhury and Rasenick, 2008).

The fibrous structures of the cytoskeleton play an important role in maintaining cell morphology. Therefore, the depolymerization of cytoskeletal fibers, particularly microfilaments, caused by T1R GPCR stimulation significantly impacts cell morphology. The inventors observed that stimulation of T1R2/T1R3 by artificial sweeteners, which are T1R2 agonists, causes transient increases in intracellular calcium concentration and cell contraction (Data not shown). Considering that morphological changes in vascular endothelial cells and vascular smooth muscle cells control many barriers formed by endothelial cells (blood-brain barrier function, glomerular filtration barrier function, pulmonary endothelial barrier, capillaries), the search for substances that inhibit or control cell morphological changes caused by T1R2 agonists and antagonists is important as potential therapeutic candidates for endothelial barrier dysfunction. In particular, in cases where hyperglycemia persists, such as the three major complications of diabetes (diabetic retinopathy, diabetic nephropathy, diabetic neuropathy), it can be considered that T1R2 is constantly stimulated, leading to capillary barrier dysfunction due to morphological control dysfunction of endothelial effects. Additionally, in cerebral small vessel diseases such as vascular dementia (cerebral amyloid angiopathy), the accumulation of immunoreactive protein $\beta$-amyloid in the walls of arteries and arterioles and the observation of functional impairment in cerebral vascular endothelial cells suggest that the search for substances that can control the effects of Type I taste receptors (T1R GPCR) on cell morphology by blood sweet and umami substances and $A\beta$ peptides is expected to be an important drug discovery target.

(Implementation Example 20) Screening Method for Candidate Compounds of Active Ingredients in Pharmaceutical Compositions

(20-1) Primary Screening

(i) Method for Monitoring Changes in Intracellular Calcium Concentration ($[Ca^{2+}]i$)

In this implementation example, $A\beta$ peptides ($A\beta$25-35, 1-38, 1-40, 1-42) induce changes in $[Ca^{2+}]i$ in HEK cells that endogenously express Type I taste receptors (T1R GPCR). This method screens for test substances (compounds, peptides, antibodies) that inhibit the $[Ca^{2+}]i$ changes induced by $A\beta$ peptides.

Specifically, test substances are administered to the HEK cell culture medium before or after the action of $A\beta$ peptides, and changes in intracellular calcium concentration are observed. By evaluating the effects of these substances, inhibitors of the action of $A\beta$ peptides are identified.

Next, the test reagents that showed changes in intracellular calcium concentration are administered alone to the culture medium to confirm whether the concentration changes are dependent on $A\beta$ peptides.

Furthermore, to demonstrate which specific Type I taste receptor (T1R GPCR) is affected, experiments are conducted using cells with reduced T1R3 expression via silencing vectors or cells with T1R3 knocked out by CRISPR as controls. This shows that the screening results are specific to a particular Type I taste receptor (T1R GPCR). If necessary, T1R2 KO cells and T1R1 KO cells are also used to evaluate the specificity of the action on T1R GPCRs.

Additionally, substances that inhibit the increase in intracellular calcium concentration and cell contraction induced by T1R2 stimulation are explored. Substances identified as specific are considered candidates for treating endothelial cell barrier dysfunction.

Substances that inhibit the enhancement of intracellular calcium concentration mediated by Type I taste receptors (T1R GPCR) due to $A\beta$ peptides are candidates for preventing and treating the adverse effects caused by $A\beta$ peptide binding to cells.

Moreover, substances that independently increase $[Ca^{2+}]i$ without binding to $A\beta$ peptides are candidates for therapeutic agents for functional recovery.

(ii) The concentration of test substances deemed effective in the primary screening (i) is increased to approximately 3-4 times, and similar experiments are conducted. Based on the response to this concentration, IC50 and EC50 are calculated.

The same primary screening can also be conducted using the impedance method. The choice of method depends on the available equipment and other factors.

(iii) Screening of substances that can control the activation current of voltage-gated ion channels (VGSC, VGKC) in nerve cells or cardiomyocytes induced by sweet-tasting amino acids or umami substances. Using the same method (procedure) as in (i), replace $A\beta$ peptide with sweet-tasting amino acids or umami substances, observe changes in the enhancement effect of $[Ca^{2+}]i$ due to the administration of the test substance, and select the inhibitory substance. Additionally, by exploring positive allosteric modulators (PAM) that are effective as therapeutic agents for functional recovery in long-term dementia, candidate therapeutic agents can be identified. By applying at least one or more of the secondary to quaternary screening methods shown below, and further adding other screening methods, more reliable identification of candidate therapeutic agents can be achieved. Ultimately, confirmation experiments using humanized experimental animals, in which the T1Rs gene is replaced with the human T1Rs gene, are necessary.

(20-2) Secondary Screening

As secondary screening, determine by screening whether the test substance selected in (1) affects the cytoskeletal structure (polymerization state of microtubules and microfilaments). Since the structure of the cytoskeleton, the polymerization state of actin and tubulin, is directly linked to the function of VGSC and VGKC, and thus to cell morphology, it is important as supplementary material for interpreting/judging the screening results. Direct action on VGSC through G$\beta\gamma$ is also conceivable, so the screening results at this stage are not considered in the decision to proceed to tertiary screening.

(20-3) Tertiary Screening

As tertiary screening, administer fluorescently labeled A$\beta$ peptide to the culture medium of human cells expressing Type I taste receptors (T1R GPCR), and screen for test substances that inhibit the binding of this fluorescent peptide to T1R GPCR.

(20-4) Quaternary Screening

As quaternary screening, measure the effect on VGSC and VGKC using the voltage clamp method. The manual patch-clamp method used in this discovery follows the techniques used in this specification. To improve the efficiency of patch-clamping, automated patch-clamping can also be used. In this case, it is necessary to use suspension cells (such as Jurkat cells) expressing Type I taste receptors (T1R GPCR). This is because cells cultured on culture dishes, like normal HEK cells, need to be detached from the dish using cell detachment solutions such as trypsin. During this process, the cells become spherical, and the cytoskeletal structure is destroyed. Therefore, it is necessary to use cells overexpressing the target ion channels (such as VGSC) in suspension cells expressing T1R GPCR for the experiments.

(20-5) Others

(i) Using computer simulations based on the results of the voltage clamp method, predict the effects of the test substance on the waveform of action potentials.

(ii) Screening of test drugs using the waveform, frequency, and pattern of action potentials as indicators.

(iii) Using current clamp (patch-clamp method), NanoTouch method, intracellular calcium concentration/membrane potential-sensitive dyes, and MEA method, record action potentials from cells to observe how the enhancement or inhibition of VGSC and VGKC manifests in the actual function of cellular action potentials, and evaluate the effects of the test drugs.

(Implementation Example 21) Method for screening compounds that modify/inhibit VGSC activity mediated by T1Rs of A$\beta$ peptides using a method for measuring cell impedance

(21-1) Principle of the impedance method

The impedance method is used in drug discovery assays targeting GPCRs. Products such as the xCELLigence System (ACEA Biosciences) and CellKey (trademark) (Molecular Devices) are well-known for using the principle of the impedance method. Generally, assays targeting GPCRs required labeling the target cells in some way, such as expressing reporter genes or measuring intracellular calcium changes. The impedance method, known as a label-free system, does not require labeling cells. In the impedance (resistance) method, cells cultured in a sheet are used, electrodes are placed on both sides of the cell sheet, and the impedance (resistance) of the cell sheet is measured. The measured values reflect the polymerization state of ATP and actin, and changes in these values are linked to changes in cell morphology, causing fluctuations (changes) in impedance values. Since the actin cytoskeleton is an important downstream effector of GPCRs, the active state of GPCRs can be sensitively measured as changes in impedance due to changes in cell adhesion state or adhesion area on the electrode substrate. In the pathway activating G protein G$\alpha$i, an increase in impedance is observed when GPCRs are activated. In the pathway activating G$\alpha$q, a transient decrease in impedance is observed initially, followed by a significant increase. In the pathway activating Gas, a decrease in impedance is reported (Scott and Matthew, 2010). This implementation example demonstrates that the impedance method is effective for screening compounds that modify/inhibit VGSC and VGKC activity mediated by T1Rs in CSF due to A$\beta$ peptides or sweet/umami substances.

(21-2) Measurement of the effect on VGSC mediated by TIR3 inherent in HEK cells by A$\beta$1-40 and the inhibitory effect by T1R3 antibody

In this implementation example, A$\beta$1-40 peptide was used as a ligand, and the change in impedance value by T1R3 antibody, which inhibits the effect of A$\beta$1-40 peptide identified from patch-clamp experiments, was verified. The T1R3 antibody is an antibody that epitopes the ligand-binding site 2 (303-396aa: sequence number 6) of T1R3.

Specifically, cells were cultured on a conductive glass (FTO glass, thickness 2.2 mm) electrode plate to form a sheet, and the change in impedance value of the cell sheet was measured by applying current stimulation between the conductive glass covered with cells and the extracellular solution. The measurement experiment was conducted using the voltage-clamp method. A voltage pulse of 1 mV (1 Hz, 220 ms width) was applied between the silver chloride electrode placed in the extracellular solution and the electrode plate, and the change caused by the amyloid peptide was measured as a change in current (I).

By measuring the current response (I) occurring there, the resistance ® of the entire cell sheet to the applied voltage (1 mV) can be calculated from Ohm's law (V = IR > R = V/I). As shown in (Figure 46), it was confirmed that the T1R3 antibody inhibits the effect of A$\beta$1-40 peptide. Furthermore, since a decrease in impedance was observed, it was suggested that A$\beta$1-40 peptide stimulates G$\alpha$s-G protein, as described by Scott and Matthew (2010).

From these results, it was confirmed that T1R GPCRs expressed on the cell membrane change the function of effectors such as VGSC through changes in the polymerization state of cytoskeletons such as actin and tubulin by the given agonist. This indicates that the impedance method using cells expressing human T1R GPCRs endogenously, such as HEK cells, is an effective method for primary screening, similar to the method of monitoring intracellular calcium concentration.

(Implementation Example 22) Effect of A$\beta$1-42 peptide on Cav1.2 calcium channel (VGCC)

According to the report by Kim and Rhim (2011), it was suggested that A$\beta$ peptide enhances Cav1.2 calcium channel, but it was concluded that the effect is mediated by the $\beta$ subunit that forms a complex with Cav1.2. However, it has been shown by Shibata's group that Cav1.2 shows the same localization as Nav1.5 within cells. Therefore, this implementation example suggests that VGCC, like VGSC, is also regulated in its activity via T1Rs GPCR.

Specifically, experiments were conducted using a method to measure intracellular calcium concentration in HEK cells stably expressing Cav1.2. The results of these experiments are shown in (Figure 47A). High concentrations of KCl depolarize the cell membrane potential by depolarizing the potassium equilibrium potential from the resting potential, activating voltage-gated calcium channels (VGCC, Cav1.2) that are activated by depolarization, and allowing extracellular calcium to flow into the cell through these calcium channels. (Figure 47A) measures the activation process of intracellular calcium increase caused by the activation of these VGCCs. This increase in intracellular calcium is inhibited by nifedipine, which inhibits Cav1.2 (data not shown), indicating that this increase in intracellular calcium is mediated by Cav1.2 VGCCs.

Next, when 12.5 nM A$\beta$1-42 peptide was applied, a further increase in intracellular calcium was observed (Figures 47A, B). This confirmed that A$\beta$1-42 peptide activates Cav1.2 VGCCs. These results suggest that the activation of VGCC currents, like VGSC, is highly likely to be regulated via T1Rs GPCR.

The calcium response to high concentrations of potassium decays with a certain regularity, so the process was approximated using the Hyperbolic Decline formula

$$(eq.1)$$

$$y = q_0(1+bx/a)^{(-1/b)}$$

and the difference between the curve obtained (dashed line in Figure 47A) and the experimental values was calculated to derive the enhancement of intracellular calcium by A$\beta$1-42 peptide. The degree of enhancement by A$\beta$1-42 peptide was about 11% of the response to high concentrations of potassium (n=3).

Using the above method, it is highly likely that not only the effect of A$\beta$1-42 peptide on Cav1.2 but also the inhibitory and enhancing effects of A$\beta$ peptides on other VGCCs can be measured by pre-administering the test drug, and this method can be applied to drug discovery screening

(Implementation Example 23) Using floating cells (Jurkat cells) to measure the inhibitory and enhancing effects of Aβ peptides using the patch-clamp method by pre-administering the test drug:

[23-1] Measurement of membrane potential changes of Aβ1-42 peptide on endogenous VGSC in Jurkat cells

Modification of VGSC function by T1R GPCR involves structural changes in the cytoskeleton. Therefore, when measuring with the patch-clamp method, it is necessary to maintain the normal structure of the cytoskeleton, so the test cells must be stably adhered to the culture dish during the experiment. However, the process of detaching cells with trypsin, EDTA, etc., which is essential for screening measurements with auto-patch, is expected to cause cytoskeletal reconstruction, posing a significant hurdle when measuring ion channel control via GPCR.

Therefore, the inventors considered that if the experiment is conducted using floating cells, the cytoskeleton would not be affected by cell detachment, making it effective for screening the effects of Aβ peptides on VGSC function via T1R GPCR, as well as their agonists and antagonists.

Specifically, a proof-of-concept experiment was conducted using Jurkat cells, a human leukemia T-cell-derived cell line reported to abundantly express T1R3 (Thiel et al. 2017). Approximately 10% of Jurkat cells endogenously express VGSC, with Nav1.5 being functionally expressed (Fraser et al., 2004). The results of the action of Aβ1-42 peptide on endogenous VGSC in Jurkat cells are shown in (Figure 48).

(23-2) Re-evaluation of the concentration-dependent enhancing effect of Aβ25-35

In the initial experiments, Aβ1-42 was dissolved in ddH2O, and the stock was stored at -20°C for use. After thawing, reproducible results were confirmed by treating the stock tube with an ultrasonic cleaner for 10 seconds, but over time, data variability that could not be improved by ultrasonic cleaning alone began to appear. According to the websites of many peptide manufacturers, Aβ1-42 is described as monomeric in alkaline solution (https://www.merckmillipore.com/JP/ja/product/- Amyloid-1-42-a-ultra-pure-NaOH-recombinant-human,MM_NF-AG970-1MG), so the solvent was changed from ddH2O to 10 mM NaOH. As a result, Aβ1-42, including Aβ25-35, showed significant changes at concentrations of 6 to 12.5 nM. Taylor et al. (2023) also reported that Aβ peptides are monomeric in 10 mM NaOH, so the test results are interpreted as the action of monomeric peptides.

When the concentration-dependent response of Aβ25-35 to Nav1.5 was re-evaluated, the EC50 was calculated to be 7.9 nM compared to 86.8 nM when prepared with ddH2O, approximately 10 times lower.

The Hill coefficient was calculated based on two experiments. In the figure, (•) indicates the average value, (▲), (♦) indicate the individual experimental measurements. (Figure 49).

Curve fitting was performed using the equation "$y=1/(1+(x/IC50)^h)$" and the CurveExpert Professional Program (Hyams Development). Since Aβ25-35 was effective at a concentration of 12.5 nM, experiments were also conducted with other sizes of Aβ peptides (Aβ1-38, 140, 1-42) using 10 mM NaOH as the solvent. In the patch-clamp experiments, the effect of Aβ1-40 was stronger than that of other peptide sizes, so the concentration was set to 6.25 nM.

(Implementation Example 24) Binding sites of Aβ1-40 and Aβ1-42 on human T1Rs

[24-1] Binding site of Aβ1-40 on human T1R3

As described in (Implementation Example 10), the binding of fluorescently labeled Aβ25-35 peptide to T1R3 on the surface of HEK cells is inhibited by anti-T1R3 antibody. The anti-T1R3 antibody used here is a polyclonal antibody made against the VFD region (amino acid sequence 229-258: sequence number 5), which is the ligand-binding domain of human T1R3. Hereafter, to distinguish this antibody from other anti-T1R3 antibodies, it is referred to as "anti-T1R3 (229-258) antibody." However, the anti-T1R3 (229-258) antibody did not inhibit the binding of Aβ1-40 and Aβ1-42 to T1R3. Therefore, as described in (Implementation Example 21-2), the "anti-T1R3 (303-396) antibody" (Polyclonal Antibody against Human TAS1R3 (Atlas Antibodies AB, HPA053439)), which was created using another part of the ligand-binding domain of human T1R3, 303-396, as the epitope, was applied. It was found to inhibit the binding of fluorescent Aβ1-40 to T1R3 on the surface of HEK cells. Based on the crystal structure of the medaka T1R2/T1R3 heterodimer shown in the Protein Data Base (5X2M), the positions of the ligand-binding domains of each anti-T1R3 were determined from the corresponding human T1R3 sequence (Figure 59). From this, it is understood that Aβ1-40 acts on a different ligand-binding site 2 (region 303-396, LB1: sequence number 6) of the T1R3 protein than Aβ25-35.

From the above, it can be said that the ligand-binding domains of human T1R3, the region 229-258 (LB2) where Aβ25-35 binds, and the region 303-396 (LB1) where Aβ1-40 binds, are important.

(24-2) Examination of the binding site of Aβ1-42 on T1R3 - 1

Since the binding of Aβ1-42 to T1R3 is not inhibited by either the anti-T1R3 (229-258) antibody or the anti-T1R3 (303-396) antibody, it is strongly suggested that the binding site of Aβ1-42 on T1R3 is in a region other than the amino acid sequence regions 229-258 and 303-396 of the T1R3 protein. Therefore, first, using the "anti-T1R3 (whole extracellular region) antibody" (Novus Biologicals, NB100-98792), which is an antibody against the entire extracellular region of T1R3, the change in the effect of Aβ1-42 on VGSC current was observed. Specifically, using HEK cells expressing Nav1.6 and Kv1.2, it was confirmed that the enhancing effect of Aβ1-42 on VGSC current in T1R3 was further enhanced by the "anti-T1R3 (whole extracellular region) antibody" (Figure 5 1A). Since this antibody is against the entire extracellular region of T1R3, there is no information on where it specifically binds, such as the VFD or CRD (Cysteine Rich Domain) regions, but as shown in (Figure 51A), when the anti-T1R3 antibody was applied following Aβ1-42, it was found that the VGSC current enhanced by Aβ1-42 was further additively enhanced.

Next, the effects of Aβ1-42 and thaumatin were measured. Thaumatin alone amplified VGSC currents similarly to Aβ1-42, but further increases in concentration did not result in additional amplification of VGSC currents (Figure 51C). On the other hand, when thaumatin was applied after Aβ1-42, no further amplification of the VGSC currents enhanced by Aβ1-42 was observed, unlike with the anti-T1R3 antibody (Figure 51B). Since it is known that thaumatin binds to CRD, it is considered that CRD is an important site of action when thaumatin enhances VGSC currents via T1R3. Additionally, the lack of further enhancement when thaumatin is applied after Aβ1-42 suggests that the functional sites of action of Aβ1-42 and thaumatin on T1R3 may be the same. However, since no further enhancement is observed with increased concentrations of thaumatin in (Figure 51C), it is possible that the enhancement effect through CRD is saturated. On the other hand, the anti-T1R3 (whole extracellular region) antibody causes further enhancement of VGSC currents in addition to the enhancement by Aβ1-42, suggesting that the sites of action on T1R3 for both may be different.

(24-3) Examination of the binding site of Aβ1-42 on T1R3 - 2

Sweet-tasting proteins such as thaumatin and brazzein are known to act as sweeteners at very low concentrations compared to other artificial sweeteners, and their binding sites on T1R3 have already been identified by converting amino acid sequences (Jiang et al., 2004; Masuda et al., 2013), and are reported to be within the CRD (Cysteine Rich Domain) located between the VFD and transmembrane regions. Comparing (Figure 51A and 51B), the additive enhancement of VGSC currents by Aβ1-42 and the anti-T1R3 (whole extracellular region) antibody is observed, but no further enhancement of VGSC currents is observed with thaumatin after the action of Aβ1-42. This strongly suggests that Aβ1-42 acts on a common site of action with thaumatin. Therefore, the action of Aβ1-42 on Nav1.5 is very similar to that of thaumatin, and the antigen of the T1R3 (400-570aa) antibody that inhibits the action of Aβ1-42 includes the region from the middle of LB1 and LB2 to the CRD, which is similar to the changes caused by thaumatin, suggesting that the site of action of Aβ1-42 on T1R3 is likely to be in or around the CRD region (Cysteine Rich Domain).

(24-4) Examination of the binding site of Aβ1-42 on T1R3 - 3

As the antibody created against the VFD region inhibited the action of Aβ25-35, the anti-T1R3 antibody (anti-T1R3 (400-570) antibody) created using the amino acid sequence near the CRD region (400-570aa, sequence number 7) as an epitope was used to examine whether the antibody inhibits the enhancement effect of Aβ1-42 on VGSC currents (the epitope 400-570aa of the anti-T1R3 (400-570) antibody is Novus Bio. NBP2-93271). If the antibody inhibits the action of Aβ1-42 on VGSC, it can be inferred that the binding site on T1R3 is in or near the CRD region. This provides an important clue to predict the binding site of Aβ1-42 on T1R3. Therefore, after applying Aβ1-42 to Nav1.5, the anti-T1R3 antibody was applied, and the effect of Aβ1-42 on VGSC currents was examined. As shown in (Figure 52A), the VGSC currents enhanced by Aβ1-42 almost returned to control values. This indicates that the binding site of Aβ1-42 on T1R3 is in or at least near the CRD region (amino acid sequence 400-570aa).

In this experiment, we used the perforated patch-clamp method with Gramicidin instead of the conventional whole-cell patch-clamp method. The reason for this is that in previous experiments, while the enhancement of VGSC was observed for Nav1.1 and Nav1.6 when observing the effects of Aβ1-42 (as shown in Figure 52A), for Nav1.5, using the conventional whole-cell patch-clamp method, despite the gene being expressed on the surface of HEK cells as with Nav1.1 and Nav1.6, a phenomenon of VGSC current attenuation accompanied by an increase in resistance between the electrode and the cell interior was observed in many experiments. This attenuation of VGSC current may cause changes in the cytoskeleton due to Aβ1-42, altering the relationship between the electrode and the cell membrane, making accurate current recording difficult. Therefore, in this experiment, we used the perforated patch-clamp method. Using this method, it was possible to measure the enhancement effect of Aβ1-42 on VGSC current for Nav1.5, similar to its effect on Nav1.1 and Nav1.6.

(24-5) The receptor for Aβ1-40 peptide is a T1R3 and T1R2 heterodimer.

Next, Aβ1-40 has an effect of shifting the S-S curve to the left, similar to the effect of artificial sweeteners like saccharin. Therefore, it is possible that Aβ1-40 acts not only on T1R3 but also on T1R2. To investigate this, we used HEK cells expressing T1R2 miRNA, which silenced T1R2 (Figure 52B). The effect of Aβ1-40 on VGSC (Nav1.5) was almost eliminated in T1R2 miRNA-expressing cells (n=3). Thus, as mentioned in (24-1), Aβ1-40 binds to ligand-binding site 2 (303-396 region) on T1R3 and also binds to T1R2, causing an enhancement effect on VGSC current. This strongly suggests that the receptor for Aβ1-40 peptide is not a T1R3 homodimer but a T1R2/T1R3 heterodimer receptor.

(Implementation Example 25) Effects of Aβ peptide on cell morphology

The morphological changes of vascular endothelial cells and vascular smooth muscle, which form many barriers (blood-brain barrier function, glomerular filtration barrier function, pulmonary endothelial barrier, capillaries) controlled by endothelial cells, are important for maintaining homeostasis in the body. Considering this importance, verifying the effects of Aβ peptide on the morphology of endothelial cells via T1R GPCR and elucidating its mechanism is crucial for exploring treatments for Alzheimer's disease, chronic renal failure, etc. Furthermore, the search for substances that can control the effects of Aβ peptide on cell morphology is expected to be an important target for drug discovery. Therefore, in this implementation example, we observed the effects of Aβ peptide on cell morphology.

In a previous implementation example (Figure 54), it was shown that Aβ peptides and artificial sweeteners cause depolymerization of the cytoskeleton. When Aβ1-40 (12.5nM) was applied to human kidney HEK293 cells (A1) and human endothelial cells (A2, umbilical vein endothelial cells HUEhT-1 cells), a decrease in cell area/volume was observed in both. To easily compare and confirm the morphological changes before and after the application of Aβ1-40, the areas of interest were circled with dashed lines. Observations were made using bright-field microscopy. Images were captured with a camera (UI-3880CP-M) from IDS mounted on the microscope, and the images were imported to a PC using the software that came with the camera. Further image processing was done using ImageJ (Rasband, 1997-2018).

In a previous implementation example, it was shown that Aβ peptides cause depolymerization of the cytoskeleton using tubulin (Figure 33A) and actin (Figure 34), and it was also shown that MSG causes changes in the tubulin structure of the cytoskeleton and that artificial sweetener aspartame also causes cell contraction (additional Figure 53-1). Considering the experimental results of patch-clamp (Implementation Example 24) in parallel, it is natural to think that the effects of Aβ peptides on cell morphology are mediated through T1R3 or T1R2/T1R3. Therefore, it was concluded that the method of observing changes in cell area/volume from the above Implementation experimental results is effective as a method for screening agonists and antagonists that affect the action of Aβ peptides.

(Implementation Example 26) Effects of the concentration ratio of Aβ1-42 and Aβ1-40 (Aβ42/40) on cell morphology

Various lengths of Aβ peptides have been reported, among which Aβ1-42 and Aβ1-40 are well known. This study examines the relationship between the concentration of these peptides in cerebrospinal fluid and blood and the degree of Alzheimer's-type dementia. Although this relationship has been studied for many years, it has been reported that the concentration ratio of Aβ1-42 to Aβ1-40, rather than the absolute amount of these peptides, is most useful for diagnosing the degree of clinical symptoms (Lewczuk, Piotr et al., 2015).

Therefore, assuming Aβ42/40 = 0.16 for healthy individuals (non-demented: Aβ ND) and Aβ42/40 = 0.1 for dementia (Demented: Aβ AD), mixed solutions were prepared and the effects on cell morphology were examined. Although differences in degree were observed, a decrease in cell volume was seen in both HEK cells (A2) and HUEhT-1 cells (B2). Specifically, the effects of Aβ42/40 at the ratio found in healthy individuals (Aβ ND) on cell morphology were examined using HEK cells. Comparing before (Control) and after the application of Aβ ND, changes in the structure around the cells were observed. Cells with extended protrusions, cells with extended lamellipodia (membrane pseudopodia), and spreading cells were confirmed. In (Figure 53-2B), some cells that had contracted more than others were also observed to recover their size. The changes in VGSC currents and cell morphology based on changes in the Aβ42/40 ratio, like the effects of Aβ peptides themselves, are clearly mediated through T1Rs.

From the above, it is suggested that the Aβ42/40 ratio found in healthy individuals plays an important role in maintaining cell motility, preserving cell morphology plasticity, and maintaining barrier function between cells without causing cell contraction (atrophy). This is the first discovery demonstrating the physiological function of Aβ peptides at the cellular level.

Additionally, similar to the physiological Aβ ND, the Aβ42/40 ratio found in healthy individuals, β-alanine is also abundantly present in the serum of healthy individuals, and its decreased concentration has been reported to correlate with the degree of cognitive symptoms (Hata et al., 2019). It has been reported that a serum concentration of β-alanine in the range of 1.23-8.34 μmol/L is considered healthy. Therefore, the effect of β-alanine was measured using an intermediate value of 5 μM. As a result, it was found that β-alanine, like Aβ ND, also promotes cell motility. Furthermore, it was observed that β-alanine induces activity in cells (in this case, HEK cells) that have contracted and whose peripheral activity has ceased due to Aβ AD (Data not shown). This suggests that β-alanine may have the potential to restore the function of synapses that have lost plasticity in dementia.

[Table 10]

| Diagnosis | NDC (n=23) | | AD (n=23) | |
|---|---|---|---|---|
| | Mean | ±SD | Mean | ±SD |
| Aβ1-40 | 6.83 | 2.53 | 6.27 | 2.5 |
| Aβ1-42 | 1.37 | 0.8 | 0.65 | 0.3 |
| Aβ42/40 | 0.21 | - | 0.1 | - |

Aβ peptide levels were scanned and calculated as absolute values (ng/ml) (Bibl et al., 2006)

Here, NDC refers to the Non-Demented Cohort, which is a group of subjects without symptoms of dementia, and AD refers to a group of subjects with symptoms of Alzheimer's-type cognitive impairment.

When converting the molecular weight of Aβ1-40 (4329.8) and Aβ1-42 (4514.04) from weight to concentration, it results in the following (Table 11).

[Table 11]

| Diagnosis | Aβ1-40 | Aβ1-42 | Aβ42/40 |
|---|---|---|---|
| NDC | 1.47 | 0.30 | 0.21 |
| AD | 1.45 | 0.14 | 0.10 |
| | nM | | |

The solution (HHBS*) was adjusted to achieve the final concentration values shown in Table 11 for Aβ42/40, and the changes in cell morphology were observed. Indicators included cell body contraction, extension, or the presence of lamellipodia ruffling around the cell periphery.

*HHBS (Hank's Balanced Salt Solution with 20 mM Hepes buffer): 1.26mM CaCl2, 0.49mM NgCl2, 0.41mM MgSO4, 5.33mM KCl, 0.44mM KH2PO4, 4.1mM NaHCO3, 137.93mM NaCl, 0.34mM Na2HPO4, 5.26mM D-Glucose, 20mM HEPES

[Table 12]

| | HEK | |
|---|---|---|
| | Cell body contraction | Periferal ruffling |
| Carnosine | (+++) | (++) |
| Glycine | (+++) | (+) |
| Aβ1-40 | (+++) | (+/-) |
| Aβ AD | (+++) | (+/-) |
| GABA | (++) | (+/-) |
| Aβ25-35 | (++) | (-) |
| Thaumatin | (+) | (+/-) |
| Aβ ND | (+/-) | (++) |
| β Alanine | (+/-) | (+++) |

In the table above [Table 12], reagents that cause a greater degree of cell body contraction are listed at the top, followed by those with a weaker effect. Since β-alanine induces activity around the cells similar to Aβ ND, the effect of carnosine (His-βAla: Carnosine), which consists of β-alanine and histidine and has a similar structure, was tested. However, the same effect as β-alanine was not obtained.

According to Stamatelopoulos et al. (2017), Aβ1-40 (Aβ40) primarily accumulates in the brain's blood vessels (rather than in neurons), inducing cerebral amyloid angiopathy. Additionally, Aβ40 is present in the myocardial tissue of patients with Alzheimer's disease and heart failure, promoting atherosclerosis by forming plaques and adversely affecting the cardiovascular system. The experimental results presented here show that in human umbilical vein endothelial cells (HUEhT-1), Aβ40 and Aβ AD cause significant cell body contraction (atrophy). Considering that the morphological changes in vascular endothelial cells and vascular smooth muscle cells control many barrier functions formed by endothelial cells (such as the blood-brain barrier, glomerular filtration barrier, pulmonary endothelial barrier, and capillaries), the contraction of cells like vascular endothelial cells and glomerular endothelial cells caused by Aβ40 and Aβ AD suggests the potential to impair barrier functions.

[Table 13]

| | Cell body contraction | | | | | Cell periferal, Lamelipodia, ruffling | | | | |
|---|---|---|---|---|---|---|---|---|---|---|
| | HEK | SH-SY5Y | HUEhT-1 | Bend3 | NG108-15 | HEK | SH-SYSY | HUEhT-1 | Bend3 | NG108-15 |
| A$\beta$1-40 | (+++) | (+/-) | (+++) | | (+/-) | (+/-) | (+/-) | (-) | | (-) |
| A$\beta$ AD | (+++) | | (+) | (+/-) | (+) | (+/-) | | (-) | (-) | (+/-) |
| A$\beta$ ND | (+/-) | | | (+) | (+) | (++) | | | (-) | (-) |
| $\beta$ Alanine | (+/-) | (+/-) | | | (+/-) | (+++) | (+++) | | | (+/-) |

**From** the above, observing the morphology and degree of ruffling of cultured human-derived vascular endothelial cells using an optical microscope is effective as a diagnostic tool for Alzheimer's disease. As a simpler method, similar observations can be made using HEK cells instead of the more expensive cultured vascular endothelial cells. It has been reported that changes in the Aβ42/40 ratio can be observed not only in cerebrospinal fluid but also in serum (Fandos et al., 2017). Therefore, it is possible to diagnose by observing changes in the Aβ42/40 ratio in serum instead of cerebrospinal fluid.

(Implementation Example 27) Examination of species differences

A major hurdle in Alzheimer's disease research has been the low efficacy of results obtained from genetic models using mice when applied clinically to humans. The primary reason for this was the unidentified receptors for Aβ peptides. In analyses using cell morphology changes, particularly the presence or absence of contraction and the activity of lamellipodia around the cells as indicators, mouse-derived brain microvascular endothelial cell lines (bEnd.3) and mouse neuroblastoma/rat glioma cells (NG108-15) showed less cell contraction and lamellipodia ruffling activity due to Aβ40 and Aβ AD compared to human-derived cells (fetal kidney cell line (HEK), neuroblastoma cells (SH-SY5Y), umbilical vein endothelial cells (HUEhT-1)). The differences in species in the T1R2/T1R3 genes could be observed as differences in functional responses.

Additionally, both lactisole (Xu et al., 2004) and dichlorprop (Nakagita et al., 2019) have binding sites in the transmembrane region of T 1R3, and the amino acid sequences necessary for this binding are present only in humans and not in mice or rats (Xu et al., 2004; Winnig et al., 2005). From the above, it can be concluded that humanizing T1R GPCR is essential when creating mouse models of Alzheimer's disease.

For example, the following procedure can be followed:

1. Identify the target human gene (T1R3, T1R2, T1R1) or the genomic sequence of the gene, and design a targeting vector containing the human sequence adjacent to homologous arms matching the corresponding mouse gene locus.

2. Identify an appropriate mouse strain that can accept the transplantation of human genes or cells without rejection.

3. Use gene editing tools such as CRISPR-Cas9 to insert the human gene into the mouse genome, or introduce the targeting vector into mouse embryonic stem cells (ESC) by electroporation or transfection, and select positive clones using antibiotic resistance or fluorescent markers.

4. Screen positive clones by PCR, Southern blotting, or sequencing to confirm that the human sequence is correctly integrated into the mouse genome and eliminate off-target effects.

5. Inject the targeted ESCs into blastocysts of a compatible mouse strain and transplant them into pseudopregnant females to create chimeric mice.

6. Mate the chimeric mice with wild-type mice of the same strain and test the germline transmission of the humanized allele in their offspring by PCR or Southern blotting.

7. Establish a colony of heterozygous humanized mice and breed them to obtain homozygous humanized mice.

8. Characterize the expression and function of the human gene or genomic sequence in the humanized mice using appropriate assays and compare them with wild-type mice or other models.

Additionally, as a simple method, it is possible to use immunodeficient mice to transplant human neurons, human cardiac myocytes, and other cells.

(Implementation Example 28) Verification of the effect of the concentration ratio (Aβ42/40) of Aβ42 and Aβ40 in cerebrospinal fluid on VGSC currents

The concentration ratio of Aβ42 and Aβ40 in cerebrospinal fluid has been reported to be related to the severity of Alzheimer's disease symptoms. In the previous (Implementation Examples (25, 26), the concentration ratio of Aβ42/40 reported in healthy individuals (NDC, non-demented cohort/control) and in Alzheimer's disease patients with symptoms (AD-D) showed different effects on cell morphology and lamellipodia (membrane-like pseudopodia) morphology. In this implementation example, in addition to measuring changes in cell morphology, we investigated whether the effect of Aβ42/40 on VGSC currents differs between NDC and AD-D using an observation method for VGSC current changes.

Specifically, when Aβ42/40 was applied based on the concentration ratio in NDC, no significant change in VGSC current magnitude was observed (Figure 58B). However, when Aβ42/40 was applied based on the concentration ratio in AD-D, an increase in VGSC current was confirmed (Figure 58A-1). Compared to when Aβ1-42 and Aβ1-40 were individually applied, the degree of increase was substantial, suggesting that Aβ42/40 in AD-D significantly contributes to the expression of epilepsy-like symptoms observed in dementia patients. Following the enhancement of VGSC current by Aβ42/40 (AD-D), we administered β-alanine and observed its effects. From the experiments on cell/lamellipodia morphological changes using neuron-like differentiated SH-SY5Y cells, it has already been shown in this implementation example (Figure 53-2) that β-alanine enhances lamellipodia activity.

Furthermore, the enhancement of lamellipodia activity by β-alanine suggests a potential role in promoting the recovery of synaptic plasticity in AD-D. β-alanine tended to reverse the enhancement of VGSC current caused by Aβ42/40 (AD-D), decrease VGSC current magnitude, and restore it to the size before Aβ42/40 (AD-D) treatment. This experiment provides cellular-level evidence supporting the clinical observation that the concentration ratio of Aβ42 and Aβ40 is related to the severity of Alzheimer's disease symptoms (Figure 54B, 55, 58).

Moreover, this implies that in addition to using electrophysiological tests as an indicator of VGSC current enhancement, the ratio of Aβ42/40 contained in patients' cerebrospinal fluid or serum can be a convenient and effective evaluation method for assessing cognitive function progression, inhibiting disease progression, or screening for new drug candidates based on their effects on cell morphology/dynamics and neuronal process morphology/mobility.

In this implementation example, different Aβ42/40 ratios between NDC and AD-D demonstrate varying effects on cell morphology and lamellipodia morphology. Furthermore, these effects differ between human-derived cells and mouse/rat-derived cells. The experimental results revealed in this implementation example strongly suggest that the selectivity of ligands for T1R GPCRs is species-dependent. As previously mentioned, the response selectivity of T1R GPCR genes to amino acids and sweet substances differs between humans and mice/rats. Therefore, it is also believed that the response selectivity of Aβ peptides similarly varies in a species-dependent manner.

In this implementation example, we investigated the effect of Aβ42/40 (AD-D) on VGSC currents using differentiated NG108-15 cells with a neuronal-like phenotype (Figure 58C). Unlike human-derived cells, Aβ42/40 (AD-D) consistently reduced VGSC currents. Additionally, subsequent application of β-alanine did not cause any further changes. From these experimental results, it is evident that even if the Aβ42/40 (AD-D) concentration ratio measured in dementia patients can be replicated in mouse dementia models, the experimental system using human-type T1R GPCRs cannot be established in human-type dementia model mice (Figure 58).

Specifically, we used mouse/rat hybridoma cells NG108-15 differentiated into neuronal-like cells using forskolin and low-concentration FBS treatment, along with human-derived cells expressing human Nav1.5. We recorded VGSC currents using the whole-cell patch-clamp method and observed the effects of Aβ peptides (Figure 58A-1, B). The term "Aβ peptides" here refers to a mixture of Aβ40 and Aβ42. We used two types of mixtures similar to those used in the experiments on cell morphology and lamellipodia dynamics. One mixture corresponds to the Aβ42/40 ratio observed in cerebrospinal fluid from healthy individuals (NDC: non-demented control), and the other corresponds to the ratio observed in cerebrospinal fluid from Alzheimer's disease patients with cognitive impairment (ADD).

In Figure 58A-1, when Aβ42/40 (AD-D ratio) is applied, VGSC currents are enhanced compared to the pre-application state (n=6), and this increase is greater than when other Aβ peptides are applied individually. Interestingly, this effect qualitatively resembles the experiment where Aβ25-35 was applied after treatment with the actin depolymerizing agent latrunculin A (Figure 31). From this, it is suggested that the enhancement of VGSC currents due to the action of Aβ42/40 (AD-D ratio) observed in cerebrospinal fluid from Alzheimer's disease patients depends on changes in microtubules rather than microfilaments (actin). Specifically, the polymerization state of tubulin, not actin, is likely the main cause of VGSC current enhancement. Note that in (Figure 58), (•) represents the control, and (A) represents the effect after applying the Aβ peptide mixture. VGSC current recordings were performed using the whole-cell patch-clamp method, with a membrane potential fixed at -120 mV and 80 ms stimulus pulses applied. The pulses increased from -80 mV to 50 mV in 10 mV intervals. Control recordings were averaged based on the maximum VGSC current, and the effects of Aβ peptides were compared across multiple cells. The I-V plot shows average values with symbols, and error bars represent standard error.

In Figure 58A-2, when observing cell morphology, β-alanine activated lamellipodia, induced ruffling, and potentially enhanced cell motility and synaptic plasticity. When β-alanine was applied to VGSC currents enhanced by Aβ42/40 (AD-D ratio), the increase in VGSC current decreased and nearly returned to the control level (n=2). The bar graph control is set to 1, and the increase and recovery levels are shown as averages. The actual experimental measurements are indicated by horizontal lines. In Figure 58B, a peptide mixture corresponding to the Aβ41/40 ratio observed in healthy individuals was applied. Unlike AD-D, there was almost no change in VGSC currents (n=5). In (Figure 51BC), we observed the effect of Aβ42/40 (AD-D) that significantly enhanced VGSC currents in human cells (human T1R2/T1R3) when applied to VGSC currents expressed in mouse/rat hybridoma cells NG108-15. Unlike human cells expressing human-type T1R2/T1R3, mouse/rat-type T1R2/T1R3 resulted in decreased VGSC currents (n=3). These experiments in (Figure 58) demonstrate that NDC and AD-D exhibit effects similar to clinical symptoms, as proven by electrophysiological methods. Additionally, β-alanine was discovered to modify/recover the enhancement caused by Aβ42/40 (ADD ratio) and may be effective in treating epilepsy-like seizures observed during dementia. Finally, the differences between human and mouse/rat T1R2/T1R3 in their effects on VGSC currents were confirmed. This experimental result identifies differences in Aβ peptide effects due to species using electrophysiological methods (patch-clamp) in addition to measuring cell morphology changes.

Moreover, as shown in Figure 57, β-alanine differs from alanine in that the amino group of β-alanine is located at the β-carbon position. To explore the possibility that the structural specificity of β-alanine contributes to modifying/recovering the AD-DD ratio enhancement of Aβ42/40, we conducted similar experiments using carnosine, an amino acid formed by the binding of β-alanine (which also has an amino group at the β position) with histidine. However, the effects observed with β-alanine were not observed (Data not shown).

We also tested γ-aminobutyric acid (GABA), which has an amino group at the gamma (γ) position, similar to β-alanine, to investigate whether the absence of an amino group at the alpha (α) position, as seen in regular L-alanine, is crucial for β-alanine's effects. However, no effects similar to β-alanine were observed (Data not shown). Therefore, it can be said that β-alanine specifically has the ability to mitigate the detrimental effects of Aβ42/40 in Alzheimer's-type dementia compared to the substances mentioned above.

Furthermore, since the AD-DD ratio is influenced by the cleavage of Aβ1-42 and the relative increase in Aβ1-40, the peptide Isoleucyl-Alanine, a cleavage product of Aβ1-42, may play a role in improving the AD-DD ratio and could potentially exhibit effects similar to β-alanine.

Based on the above, β-alanine or structurally similar compounds could be considered as potential therapeutic candidates for conditions like Alzheimer's disease.

Now, in Figure 59, we've indicated the positions of various peptides used for creating anti-T1R3 antibodies on the crystal structure of T1R3 protein derived from medaka fish (as reported by Nuemket et al., 2017).

Comparing the amino acid sequences of each peptide, we confirmed that human T1R3 sequence number 5 (229-258 aa.) corresponds to medaka T1R3 sequence (232-258 aa.), and similarly, sequence number 6 (303-396 aa.) corresponds to (302-390 aa.), and sequence number 7 (400-570 aa.) corresponds to (394-460 aa., following the CRD).

(Figure 59A) shows the crystal structure of the heterodimer of T1R2/T1R3 (PDB 5X2M), specifically the ligand-binding domains in complex with L-glutamine. In Figure 59B, we focus on T1R3 alone, indicating the positions of the peptide sequences used as antigens with white bars. The 229-258 aa. region corresponds to LB2, the 303-396 aa. region corresponds to LB1, and the 400-570 aa. region includes both LB1 and LB2, along with the CRD.

< References for the claimed invention>

1. Agarwal et al. (2011) "Diagnostic Utility of CSF Tau and A in Dementia: A Meta-Analysis." International Journal of Alzheimer's Disease: 1-10. https://doi.org/10.4061/2011/503293.

2. Alzheimer et al. (1993) "Modal Gating of Na+ Channels as a Mechanism of Persistent Na+ Current in Pyramidal Neurons from Rat and Cat Sensorimotor Cortex." The Journal of Neuroscience 13(2): 660-73. https://doi.org/10.1523/JNEUROSCI.13-02-00660.1993.

3. Authier et al. (2009) "Animal Models of Chemotherapy-Evoked Painful Peripheral Neuropathies." Neurotherapeutics 6(4): 620-29. https://doi.org/10.1016/j.nurt.2009.07.003.

4. Bachmanov et al. (2016) "Genetics of Amino Acid Taste and Appetite." Advances in Nutrition: An International Review Journal 7(4): 806S-822S. https://doi.org/10.3945/an.115.011270.

5. Barnett et al. (2020) "Metabolic Changes in Synaptosomes in an Animal Model of Schizophrenia Revealed by 1H and 1H,13C NMR Spectroscopy." Metabolites 10 (2) : 79. https://doi.org/10.3390/metabo10020079.

6. Bayes-Genis et al. (2017) "Bloodstream Amyloid-Beta (1-40) Peptide, Cognition, and Outcomes in Heart Failure." Revista Espanola de Cardiologia (English Edition) 70(11): 924-32. https://doi.org/10.1016/j.rec.2017.02.021.

7. Bibl et al. (2007) "Validation of Amyloid-β Peptides in CSF Diagnosis of Neurodegenerative Dementias." Molecular Psychiatry 12(7): 671-80. https://doi.org/10.1038/sj.mp.4001967.

8. Brechet et al. (2008) "Protein Kinase CK2 Contributes to the Organization of Sodium Channels in Axonal Membranes by Regulating Their Interactions with Ankyrin G." Journal of Cell Biology 183(6): 1101-14. https://doi.

org/10.1083/jcb.200805169.

9. Brunet et al. (2020) "Cortical Circuit Dysfunction as a Potential Driver of Amyotrophic Lateral Sclerosis." Frontiers in Neuroscience 14: 363. https://doi.org/10.3389/fnins.2020.00363

10. Cantrell et al. (1996) "Muscarinic Modulation of Sodium Current by Activation of Protein Kinase C in Rat Hippocampal Neurons." Neuron 16(5): 1019-26. https://doi.org/10.1016/S0896-6273(00)80125-7.

11. Cantrell et al. (1997) "Dopaminergic Modulation of Sodium Current in Hippocampal Neurons via CAMP-Dependent Phosphorylation of Specific Sites in the Sodium Channel $\alpha$ Subunit." The Journal of Neuroscience 17(19): 7330. https://doi.org/10.1523/JNEUROSCI.17-19-07330.1997.

12. Carr et al. (2002) "Serotonin Receptor Activation Inhibits Sodium Current and Dendritic Excitability in Prefrontal Cortex via a Protein Kinase C-Dependent Mechanism." The Journal of Neuroscience 22(16): 6846. https://doi.org/10.1523/JNEUROSCI.22-16-06846.2002)

13. Carr et al. (2003) "Transmitter Modulation of Slow, Activity-Dependent Alterations in Sodium Channel Availability Endows Neurons with a Novel Form of Cellular Plasticity." Neuron 39(5): 793-806. https://doi.org/10.1016/S0896-6273(03)00531-2

14. Catterall (2012) "Voltage-Gated Sodium Channels at 60: Structure, Function and Pathophysiology." The Journal of Physiology 590(11): 2577-89. https://doi.org/10.1113/jphysiol.2011.224204.

15. Chen et al. (2018) "Suppression of Detyrosinated Microtubules Improves Cardiomyocyte Function in Human Heart Failure." Nature Medicine 24(8): 1225-33. https://doi.org/10.1038/s41591-018-0046-2.

16. Chifflet et al. (2012) "The Plasma Membrane Potential and the Organization of the Actin Cytoskeleton of Epithelial Cells." International Journal of Cell Biology 2012: 1-13. https://doi.org/10.1155/2012/121424.

17. Ciccone, et al. (2019) "Amyloid $\beta$-Induced Upregulation of Nav1.6 Underlies Neuronal Hyperactivity in Tg2576 Alzheimer's Disease Mouse Model." Scientific Reports 9(1): 13592. https://doi.org/10.1038/s41598-019-50018-1.

18. Dahlhamer et al. (2018) "Prevalence of Chronic Pain and High-Impact Chronic Pain Among Adults- United States, 2016." MMWR. Morbidity and Mortality Weekly Report 67(36): 1001-6. https://doi.org/10.15585/mmwr.mm6736a2.

19. De Jesus et al. (2015) "Atherosclerosis Exacerbates Arrhythmia Following Myocardial Infarction: Role of Myocardial Inflammation." Heart Rhythm 12(1): 169-78. https://doi.org/10.1016/j.hrthm.2014.10.007.

20. Field (2015) "Smell and Taste Dysfunction as Early Markers for Neurodegenerative and Neuropsychiatric Diseases." Journal of Alzheimer's Disease & Parkinsonism 05(1). https://doi.org/10.4172/2161-0460.1000186.

21. Ghatak et al. (2019) "Mechanisms of Hyperexcitability in Alzheimer's Disease HiPSC-Derived Neurons and Cerebral Organoids vs Isogenic Controls." ELife 8. https://doi.org/10.7554/eLife.50333.

22. Gonzalez-Burgos and Barrionuevo (2001) "Voltage-Gated Sodium Channels Shape Subthreshold EPSPs in Layer 5 Pyramidal Neurons From Rat Prefrontal Cortex." Journal of Neurophysiol. 86(4):1671-1684. https://doi.org/10.1152/jn.2001.86.4.1671.

23. Groemer et al. (2011) "Amyloid Precursor Protein Is Trafficked and Secreted via Synaptic Vesicles." PLoS ONE 6(4): e18754. https://doi.org/10.1371/journal .pone.0018754

24. Guvenc et al. (2010) "A Novel Explanation for the Cause of Atrial Fibrillation Seen in Atherosclerotic Coronary Artery Disease: 'Downstream Inflammation' Hypothesis." Medical Hypotheses 74(4): 665-67. https://doi.org/10.1016/j.mehy.2009.11.010.

25. Hampson et al. (1999) "Probing the Ligand-Binding Domain of the MGluR4 Subtype of Metabotropic Glutamate Receptor." Journal of Biological Chemistry 274(47): 33488-95. https://doi.org/10.1074/jbc.274.47.33488.

26. Hartley et al. (1999) "Protofibrillar Intermediates of Amyloid β-Protein Induce Acute Electrophysiological Changes and Progressive Neurotoxicity in Cortical Neurons." The Journal of Neuroscience 19(20): 8876-84. https://doi.org/10.1523/JNEUROSCI.19-20-08876.1999.

27. Heijmans and Joosten. (2020) "Mechanisms and Mode of Action of Spinal Cord Stimulation in Chronic Neuropathic Pain." Postgraduate Medicine, 1-5. https://doi.org/10.1080/00325481.2020.1769393.

28. Hille, Bertil. Ion Channels of Excitable Membranes. 3rd ed. Sunderland (Mass.) : Sinauer associates, 2001. http://lib.ugent.be/catalog/rug01:002054346.

29. Horvath et al. (2020) "Late Sodium Current Inhibitors as Potential Antiarrhythmic Agents." Frontiers in Pharmacology: 413. https://doi.org/10.3389/fphar.2020.00413.

30. Husted et al. (2017) "GPCR-Mediated Signaling of Metabolites." Cell Metabolism 25(4): 777-96. https://doi.org/10.1016/j.cmet.2017.03.008.

31. Huygen et al. (2019) "'Evidence-Based Interventional Pain Medicine According to Clinical Diagnoses': Update 2018." Pain Practice 19(6): 664-75. https://doi.org/10.1111/papr.12786.

32. Inoue et al. (2017) "The Prevalence and Impact of Chronic Neuropathic Pain on Daily and Social Life: A Nationwide Study in a Japanese Population." European Journal of Pain 21(4): 727-37. https://doi.org/10.1002/ejp.977.

33. Inyushin et al (2020) "On the Role of Platelet-Generated Amyloid Beta Peptides in Certain Amyloidosis Health Complications." Frontiers in Immunology 11: 571083. https://doi.org/10.3389/fimmu.2020.571083.

34. Jiang et al. (2005) Lactisole interacts with the transmembrane domains of human T1R3 to inhibit sweet taste. J. Biol. Chem. 280 (15), 15238-15246. doi:10.1074/jbc.M414287200)

35. Jiang et al. (2005) "Identification of the Cyclamate Interaction Site within the Transmembrane Domain of the Human Sweet Taste Receptor Subunit T1R3." Journal of Biological Chemistry 280(40): 34296-305. https://doi.org/10.1074/jbc.M505255200.

36. Kang et al. (2014) "Channel-Anchored Protein Kinase CK2 and Protein Phosphatase 1 Reciprocally Regulate KCNQ2-Containing M-Channels via Phosphorylation of Calmodulin." Journal of Biological Chemistry 289(16): 11536-44. https://doi.org/10.1074/jbc.M113.528497.

37. Kerr et al. (2015) "Detyrosinated Microtubules Modulate Mechanotransduction in Heart and Skeletal Muscle." Nature Communications 6(1): 8526. https://doi.org/10.1038/ncomms9526.

38. Kim et al. (2011) "Effects of Amyloid-β Peptides on Voltage-Gated L-Type CaV1.2 and CaV1.3 Ca2+ Channels." Molecules and Cells 32 (3) : 289-94. https://doi.org/10.1007/s10059-011-0075-x.

39. Kubo et al. (2002) "In vivo conversion of racemized beta-amyloid ([D-Ser 26]A beta 1-40) to truncated and toxic fragments ([D-Ser 26]A beta 25-35/40) and fragment presence in the brains of Alzheimer's patients." Journal of Neuroscience Research 70(3): 474-83. https://doi.org/10.1002/jnr.10391.

40. Kuo et al. (2000) "Elevated Aβ42 in Skeletal Muscle of Alzheimer Disease Patients Suggests Peripheral Alterations of AβPP Metabolism." The American Journal of Pathology 156(3):797-805. https://doi.org/10.1016/S0002-9440(10)64947-4.

41. Lanznaster, et al., "Aβ1-42 and Tau as Potential Biomarkers for Diagnosis and Prognosis of Amyotrophic Lateral Sclerosis." International Journal of Molecular Sciences 21(8): 2911. https://doi.org/10.3390/ijms21082911.

42. Lehmann et al. (2020) "Cerebrospinal Fluid A Beta 1-40 Peptides Increase in Alzheimer's Disease and Are Highly Correlated with Phospho-Tau in Control Individuals." Alzheimer's Research & Therapy 12 (1): 123. https://doi.org/10.1186/s13195-020-00696-1.

43. Li et al. (2016) "Amyloid Precursor Protein Modulates Nav1.6 Sodium Channel Currents through a Go-Coupled JNK Pathway." Scientific Reports 6(1). https://doi.org/10.1038/srep39320.

44. Li et al. (2002) "Human Receptors for Sweet and Umami Taste." Proceedings of the National Academy of Sciences 99(7): 4692-96. https://doi.org/10.1073/pnas.072090199.

45. Li. (2009)"T1R Receptors Mediate Mammalian Sweet and Umami Taste." The American Journal of Clinical Nutrition 90(3): 733S-737S. https://doi.org/10.3945/ajcn.2009.27462G.

46. Li et al. (2011) "Sweet Taste Receptor Gene Variation and Aspartame Taste in Primates and Other Species." Chemical Senses 36(5): 453-75. https://doi.org/10.1093/chemse/bjq145.

47. Li et al. (2016) "Amyloid Precursor Protein Modulates Nav1.6 Sodium Channel Currents through a Go-Coupled JNK Pathway." Scientific Reports 6(1). https://doi.org/10.1038/srep39320.

48. Li et al. (2018) "DRG Voltage-Gated Sodium Channel 1.7 Is Upregulated in Paclitaxel-Induced Neuropathy in Rats and in Humans with Neuropathic Pain." The Journal of Neuroscience 38(5): 1124-36. https://doi.org/10.1523/JNEUROSCI.0899-17.2017.

49. Lipowsky et al. (1996) Journal of Neurophysiology 76(4): 2181-91. https://doi.org/10.1152/jn.1996.76.4.2181;

50. Lowe et al. (2008) "Voltage-Gated Nav Channel Targeting in the Heart Requires an Ankyrin-G-Dependent Cellular Pathway." Journal of Cell Biology 180(1): 173-86. https://doi.org/10.1083/jcb.200710107

51. Lue et al. (1999) "Soluble Amyloid β Peptide Concentration as a Predictor of Synaptic Change in Alzheimer's Disease." The American Journal of Pathology 155(3): 853-62. https://doi.org/10.1016/S0002-9440(10)65184-X.

52. Ma et al. (1997) "Persistent Sodium Currents through Brain Sodium Channels Induced by G Protein By Subunits." Neuron 19(2): 443-52. https://doi.org/10.1016/S0896-6273(00)80952-6.

53. Maillet et al. (2015) "Characterization of the Binding Site of Aspartame in the Human Sweet Taste Receptor." Chemical Senses 40(8): 577-86. https://doi.org/10.1093/chemse/bjv045.

54. Mantegazza et al. (2005) Molecular Determinants for Modulation of Persistent Sodium Current by G-Protein βγ Subunits. Journal of Neuroscience 30(13) 3341-3349; DOI: 10.1523/JNEUROSCI.0104-05.

55. Martin et al. (2017) "Altered Learning, Memory, and Social Behavior in Type 1 Taste Receptor Subunit 3 Knock-out Mice Are Associated with Neuronal Dysfunction." Journal of Biological Chemistry 292(27): 11508-30. https://doi.org/10.1074/jbc.M116.773820.

56. Martinez et al. (1993) "Amino Acid Concentrations in Cerebrospinal Fluid and Serum in Alzheimer's Disease and Vascular Dementia." Journal of Neural Transmission - Parkinson's Disease and Dementia Section 6(1): 1-9. https://doi.org/10.1007/BF02252617.

57. Masubuchi et al. (2017) T1R3 homomeric sweet taste receptor regulates adipogenesis through Gas-mediated microtubules disassembly and Rho activation in 3T3-L1 cells. PLoS ONE 12(5): e0176841. https://doi.org/10.1371/journal.pone.0176841

58. Masuda et al. (2012) "Characterization of the Modes of Binding between Human Sweet Taste Receptor and Low-Molecular-Weight Sweet Compounds." Edited by Wolfgang Meyerhof. PLoS ONE 7(4): e35380. https://doi.org/10.1371/journal.pone.0035380.

59. Mattheisen et al. (2018) "Strong G-Protein-Mediated Inhibition of Sodium Channels." Cell Reports 23(9): 2770-81. https://doi.org/10.1016/j.celrep.2018.04.109.

60. Mailliet et al. (2009) "Phenoxy Herbicides and Fibrates Potently Inhibit the Human Chemosensory Receptor Subunit T1R3. Journal of Medicinal Chemistry 52, no. 21: 6931-35. https://doi.org/10.1021/jm900823s)

61. Mo et al. (2015) "Cerebrospinal Fluid β-Amyloid1-42 Levels in the Differential Diagnosis of Alzheimer's Disease-Systematic Review and Meta-Analysis." PLOS ONE 10(2): e0116802. https://doi.org/10.1371/journal. pone.0116802.

62. Nakagita et al. (2019) "Structural Insights into the Differences among Lactisole Derivatives in Inhibitory Mechanisms against the Human Sweet Taste Receptor." Edited by Maik Behrens. PLOS ONE 14(3): e0213552. https://doi.org/10.1371/journal.pone.0213552.

63. Neiers et al. (2016) In Sweeteners, edited by Jean-Michel Merillon and Kishan Gopal Ramawat, 1-20. Reference Series in Phytochemistry. Cham: Springer International Publishing, 2016. https://doi.org/10.1007/ 978-3-319-26478-3_2-1.

64. Newell-Litwa and Horwitz (2011) "Cell Migration: PKA and RhoA Set the Pace." Current Biology 21(15): R596-98. https://doi.org/10.1016/j.cub.2011.06.032.65. Nin et al.(2009)Membrane Potential Hyperpolarization Reorganizes Actin Cytoskeleton." Cell Motility and the Cytoskeleton 66, no. 12: 1087-99. https://doi.org/10.1002/cm.20416.

66. North et al. (2018) "Ectopic Spontaneous Afferent Activity and Neuropathic Pain." Neurosurgery 65(CN_suppl_1): 49-54. https://doi.org/10.1093/neuros/nyy119.67. O'Hara et al. (1993) The ligand-binding domain in metabotropic glutamate receptors is related to bacterial periplasmic binding proteins. Neuron 11(1):41-52. doi: 10.1016/0896-6273(93)90269-w. PMID: 8338667.

68. Padhi et al. (2009) "Clinical Pharmacokinetic and Pharmacodynamic Profile of Cinacalcet Hydrochloride." Clinical Pharmacokinetics 48(5): 303-11. https://doi.org/10.2165/00003088-200948050-00002.

69. Palop et al. (2016) "Network Abnormalities and Interneuron Dysfunction in Alzheimer Disease." Nature Reviews Neuroscience 17(12): 777-92. https://doi.org/10.1038/nrn.2016.141.

70. Palop and Jorge. (2009) "Epilepsy and Cognitive Impairments in Alzheimer Disease." Archives of Neurology 66(4): 435. https://doi.org/10.1001/archneurol.2009.15.

71. Palygin et al. (2008) "Regulation of Caveolar Cardiac Sodium Current by a Single G s $\alpha$ Histidine Residue." American Journal of Physiology-Heart and Circulatory Physiology 294, (4): H1693-99. https://doi.org/10.1152/ ajpheart.01337.2007.

72. Park (2004) "Recycling Endosomes Supply AMPA Receptors for LTP." Science 305(5692): 1972-75. https://doi. org/10.1126/science. 1102026.

73. Perry et al. (2016) "Muscle Atrophy in Patients with Type 2 Diabetes Mellitus: Roles of Inflammatory Pathways, Physical Activity and Exercise," 22:94-10974. Pianu et al. (2014) "The A$\beta$1-42 Peptide Regulates Microtubule Stability Independently of Tau." Journal of Cell Science 127(5): 1117. https://doi.org/10.1242/jcs.143750

75. Pradhan et al. (2017) "The Heterotrimeric G Protein G$\beta$1 Interacts with the Catalytic Subunit of Protein Phosphatase 1 and Modulates G Protein-Coupled Receptor Signaling in Platelets." Journal of Biological Chemistry 292(32): 13133-42. https://doi.org/10.1074/jbc. M117.796656

76. Quintao et al. (2019) "Pharmacological Treatment of Chemotherapy-Induced Neuropathic Pain: PPAR$\gamma$ Agonists as a Promising Tool." Frontiers in Neuroscience 13. https://doi.org/10.3389/fnins.2019.00907.

77. Roychowdhury and Rasenick (2008) "Submembraneous Microtubule Cytoskeleton: Regulation of Microtubule Assembly by Heterotrimeric G Proteins: G Proteins and Microtubule Assembly." FEBS Journal 275(19): 4654-63. https://doi.org/10.1111/j.1742-4658.2008.06614.x.

78. Saito (2019) "NanoTouch: Intracellular Recording Using Transmembrane Conductive Nanoparticles." Journal of Neurophysiology 122(5): 2016-26. https://doi.org/10.1152/jn.00359.2019.

79. Schappi et al. (2014) "Tubulin, Actin and Heterotrimeric G Proteins: Coordination of Signaling and Structure." Biochimica et Biophysica Acta (BBA) - Biomembranes 1838(2): 674-81. https://doi.org/10.1016/j.bba mem.2013.08.026.

80. Schwindt and Crill, (1995) "Amplification of Synaptic Current by Persistent Sodium Conductance in Apical Dendrite of Neocortical Neurons." Journal of Neurophysiology 74(5): 2220-24. https://doi.org/10.1152/jn.1995.74.5.2220.

81. Shcherbatko et al. (1999) "Voltage-Dependent Sodium Channel Function Is Regulated Through Membrane Mechanics." Biophysical Journal 77(4): 1945-59, https://doi.org/10.1016/50006-3495(99)77036-0.

82. Schell et al. (2014) "Glomerular Development - Shaping the Multi-Cellular Filtration Unit." Development of the Urogenital System & MTOR Signalling & Tight Junctions in Health and Disease 36: 39-49. https://doi.org/10.1016/j.semcdb.2014.07.016.

83. Shakoor et al. (2017) Alzheimer's: Learning from a Legacy of Bitter Setbacks. Endpoints News [online] Available at: https://endpts.com/special/alzheimers-2017/

84. Shibata et al. (2006) "Autonomic Regulation of Voltage-Gated Cardiac Ion Channels." Journal of Cardiovascular Electrophysiology 17(s1): S34-42. https://doi.org/10.1111/j.1540-8167.2006.00387.x.)

85. Smith et al. (1985) "Putative Amino Acid Transmitters in Lumbar Cerebrospinal Fluid of Patients with Histologically Verified Alzheimer's Dementia." Journal of Neurology, Neurosurgery & Psychiatry 48(5): 469-71. https://doi.org/10.1136/jnnp.48.5.469.

86. Sperling et al. (2009) "Amyloid Deposition Is Associated with Impaired Default Network Function in Older Persons without Dementia." Neuron 63 (2): 178-88. https://doi.org/10.1016/j.neuron.2009.07.003.

87. Stamatelopoulos et al. (2017) "Circulating Amyloid-Beta (1-40) Predicts Clinical Outcomes in Patients With Heart Failure." Revista Espanola de Cardiologia (English Edition) 70(11): 905-6. https://doi.org/10.1016/j.rec.2017.05.026.

88. Surmeier et al. (1992"Dopamine Receptor Subtypes Colocalize in Rat Striatonigral Neurons." Proceedings of the National Academy of Sciences 89(21): 10178-82. https://doi.org/10.1073/pnas.89.21.10178.

89. Temussi. (2012) "The Good Taste of Peptides: PEPTIDES TASTE." Journal of Peptide Science 18(2): 73-82. https://doi.org/10.1002/psc.1428.

90. Toda et al. (2018) "Positive/Negative Allosteric Modulation Switching in an Umami Taste Receptor (T1R1/T1R3) by a Natural Flavor Compound, Methional." Scientific Reports 8(1): 11796. https://doi.org/10.1038/s41598-018-30315-x.

91. Toda et al. (2021) "Evolution of the Primate Glutamate Taste Sensor from a Nucleotide Sensor." Current Biology 31(20): 4641-4649.e5. https://doi.org/10.1016/j.cub.2021.08.002.

92. Troncone et al. (2016) "A$\beta$ Amyloid Pathology Affects the Hearts of Patients With Alzheimer's Disease." Journal of the American College of Cardiology 68, no. 22 (December 2016): 2395-2407. https://doi.org/10.1016/j.jacc.2016.08.073.93. Tsuchiya, (2008) "Local Anesthetic Failure Associated with Inflammation: Verification of the Acidosis Mechanism and the Hypothetic Participation of Inflammatory Peroxynitrite." Journal of Inflammation Research 41. https://doi.org/10.2147/JIR.S3982.

94. Ueda et al., (2003) "Functional Interaction between T2R Taste Receptors and G-Protein $\alpha$ Subunits Expressed in Taste Receptor Cells." The Journal of Neuroscience 23(19): 7376. https://doi.org/10.1523/JNEUROSCI.23-19-07376.2003.

95. Vanderstichele et al. (2016) "Optimized Standard Operating Procedures for the Analysis of Cerebrospinal Fluid A$\beta$42 and the Ratios of A$\beta$ Isoforms Using Low Protein Binding Tubes." Journal of Alzheimer's Disease 53(3): 1121-32. https://doi.org/10.3233/JAD-160286.

96. Vetter et al. (2012) "Characterisation of Nav Types Endogenously Expressed in Human SH-SY5Y Neuroblastoma Cells." Biochemical Pharmacology 83 (11): 1562-71. https://doi.org/10.1016/j.bcp.2012.02.022.

97. Vitvitsky et al. (2012) "Na+ and K+ Ion Imbalances in Alzheimer's Disease." Biochimica et Biophysica Acta (BBA) - Molecular Basis of Disease 1822 (11): 1671-81. https://doi.org/10.1016/j.bbadis.2012.07.004.

98. Wang et al. (2017) "Distribution and Function of Voltage-Gated Sodium Channels in the Nervous System." Channels 11(6): 534-54. https://doi.org/10.1080/19336950.2017.1380758.

99. Wang et al. (2017) "Multiple Nav1.5 Isoforms Are Functionally Expressed in the Brain and Present Distinct Expression Patterns Compared with Cardiac Nav1.5." Molecular Medicine Reports 16(1): 719-29. https://doi.org/10.3892/mmr.2017.6654.

100. Wang et al. (2017) "Molecular Expression of Multiple Nav1.5 Splice Variants in the Frontal Lobe of the Human Brain." International Journal of Molecular Medicine. https://doi.org/10.3892/ijmm.2017.3286.

101. Wang et al. (2009) "Membrane Trauma and Na+ Leak from Nav1.6 Channels." American Journal of Physiology-Cell Physiology 297, no. 4: C823-34. https://doi.org/10.1152/ajpcell.00505.2008.

102. Williams et al. (1999) "Mechanisms and Consequences of Action Potential Burst Firing in Rat Neocortical Pyramidal Neurons." The Journal of Physiology 521(2): 467-82. https://doi.org/10.1111/j.1469-7793.1999.00467.x.

103. Williams et al. (1999) "Mechanisms and Consequences of Action Potential Burst Firing in Rat Neocortical Pyramidal Neurons." The Journal of Physiology 521(2): 467-82. https://doi.org/10.1111/j.1469-7793.1999.00467.x.

104. Waxman et al. (1999) "Sodium Channels and Pain." Proceedings of the National Academy of Sciences 96(14): 7635. https://doi.org/10.1073/pnas. 96.14.7635.

105. Xiao et al. (2008) "Chemotherapy-Evoked Neuropathic Pain: Abnormal Spontaneous Discharge in A-Fiber and C-Fiber Primary Afferent Neurons and Its Suppression by Acetyl-1-Carnitine:" Pain 135(3): 262-70. https://doi.org/10.1016/j.pain.2007.06.001.

106. Xu et al. (2004) "Different Functional Roles of T1R Subunits in the Heteromeric Taste Receptors." Proceedings of the National Academy of Sciences 101(39): 14258-63. https://doi.org/10.1073/pnas.0404384101.

107. Xu and Cooper. (2015) "An Ankyrin-G N-Terminal Gate and Protein Kinase CK2 Dually Regulate Binding of Voltage-Gated Sodium and KCNQ2/3 Potassium Channels." Journal of Biological Chemistry 290(27): 16619-32. https://doi.org/10.1074/jbc. M115.638932.

108. Yang et al. (2020) "G$\alpha$12/13 Signaling in Metabolic Diseases." Experimental & Molecular Medicine 52(6): 896-910. https://doi.org/10.1038/s12276-020-0454-5.

109. Zhao et al. (2003) "The Receptors for Mammalian Sweet and Umami Taste." Cell 115 (3): 255-66. https://doi.org/10.1016/50092-8674(03)00844-4.

110. Zhou, et al. (1998) "AnkyrinG Is Required for Clustering of Voltage-Gated Na Channels at Axon Initial Segments and for Normal Action Potential Firing. " Journal of Cell Biology 1(5): 1295-1304.

111. Kim, S., Rhim, H. "Effects of amyloid-b peptides on voltage-gated L-type CaV1.2 and CaV1.3 Ca2+ channels". Mol Cells 32, 289-294 (2011). https://doi.org/10.1007/s10059-011-0075-x

112. Thiel et al. (2017) Stability of gene expression in human T cells in different gravity environments is clustered in chromosomal region 11p15.4. NPJ Microgravity. 31;3:22. doi: 10.1038/s41526-017-0028-6. PMID: 28868355; PMCID: PMC5579209.

113. Fraser et a. (2004) T-lymphocyte invasiveness: control by voltage-gated Na+ channel activity, FEBS Letters, 569, doi: 10.1016/j.febslet.2004.05.063

114. Taylor et al. (2023) "Simple, Reliable Protocol for High-Yield Solubilization of Seedless Amyloid-$\beta$ Monomer". ACS Chemical Neuroscience 14 (1), 53-71DOI: 10.1021/acschemneuro.2c00411

115. Jiang et al. (2004) "The Cysteine-Rich Region of T1R3 Determines Responses to Intensely Sweet Proteins. " J. of Biol. Chem. 279(43): 45068-75. https://doi.org/10.1074/jbc.M406779200.

116. Masuda et al. (2013) "Five Amino Acid Residues in Cysteine-Rich Domain of Human T1R3 Were Involved in the Response for Sweet-Tasting Protein, Thaumatin. " Biochimie 95(7): 1502-5. https://doi.org/10.1016/j.biochi.2013.01.010.

117. Rasband, W.S., ImageJ, U. S. National Institutes of Health, Bethesda, Maryland, USA, https://imagej.nih.gov/ij/, 1997-2018. Schneider, C.A., Rasband, W.S., Eliceiri, K.W

118. Lewczuk et al. "Amyloid-β 42/40 Cerebrospinal Fluid Concentration Ratio in the Diagnostics of Alzheimer's Disease: Validation of Two Novel Assays". 1 Jan. 2015: 183 - 191.

119. Hata et al. (2019) Association Between Serum β-Alanine and Risk of Dementia. Am J Epidemiol. 1;188(9):1637-1645. doi: 10.1093/aje/kwz116. PMID: 31127276.120. Bibl et al. (2006) CSF amyloid-beta-peptides in Alzheimer's disease, dementia with Lewy bodies and Parkinson's disease dementia. Brain. 129(Pt 5):1177-87. doi: 10.1093/brain/awl063. Epub 2006 Apr 6. PMID: 16600985; PMCID: PMC5403412.121. Fandos et al. (2017) "Plasma amyloid β 42/40 ratios as biomarkers for amyloid β cerebral deposition in cognitively normal individuals". Alzheimers Dement (Amst). 8:179-187. doi: 10.1016/j.dadm.2017.07.004. PMID: 28948206; PMCID: PMC5602863.

122. Xu et al. (2004) "Different Functional Roles of T1R Subunits in the Heteromeric Taste Receptors. " Proceedings of the National Academy of Sciences 101(39): 14258-63. https://doi.org/10.1073/pnas.0404384101.

123. Winnig et al. (2005) "Valine 738 and lysine 735 in the fifth transmembrane domain of rTas1r3 mediate insensitivity towards lactisole of the rat sweet taste receptor. " BMC Neurosci. 6:22. doi: 10.1186/1471-2202-6-22. PMID: 15817126; PMCID: PMC1084349.

124. Nakagita et al. (2019) "Structural insights into the differences among lactisole derivatives in inhibitory mechanisms against the human sweet taste receptor". PLoS ONE 14(3): e0213552. https://doi.org/10.1371/journal.pone.0213552

125. Nuemket et al. (2017) "Structural Basis for Perception of Diverse Chemical Substances by T1r Taste Receptors. " Nature Communications 8(1). https://doi.org/10.1038/ncomms15530

## Claims

1. A ligand substance that specifically binds to Type I taste receptors present on the cell surface of neuronal or cardiac cells, controlling the activation current of voltage-dependent ion channels, wherein the ligand substance acts as an agonist or antagonist for Type I taste receptors.

2. The ligand substance according to claim 1, wherein the voltage-dependent ion channels comprise membrane potential-dependent sodium channels (VGSC) or membrane potential-dependent potassium channels (VGKC), and wherein the Type I taste receptors include T1R1, T1R2, or T1R3, as homodimers or heterodimers.

3. The ligand substance according to claim 2, wherein the ligand substance specifically binds to T1R2 or T1R3 and suppresses the activation current of the VGSC.

4. The ligand substance according to claim 3, wherein the ligand substance selectively binds to T1R3 and exerts an antagonist-like effect, thereby inhibiting the activation current of the VGSC, and wherein the ligand substance is selected from the group consisting of anti-human T1R3 antibody, lactisole, clofibrate, and dichlorprop.

5. The ligand substance according to claim 4, wherein the anti-human T1R3 antibody specifically binds to an epitope comprising a continuous sequence of at least 8 amino acids within the 229-258 amino acid region of the human T1R3 protein, and wherein the anti-human T1R3 antibody inhibits binding to this region by Aβ25-35 peptide, thereby suppressing the amplified activation current of the VGSC caused by Aβ25-35 peptide.

6. The ligand substance according to claim 4, wherein the anti-human T1R3 antibody specifically binds to an epitope comprising a continuous sequence of at least 8 amino acids within the 303-396 amino acid region of the human T1R3 protein, and wherein the anti-human T1R3 antibody inhibits binding to this region by Aβ1-40 peptide, thereby suppressing the amplified activation current of the VGSC caused by Aβ1-40 peptide.

7. The ligand substance according to claim 4, wherein the anti-human T1R3 antibody specifically binds to an epitope comprising at least eight consecutive amino acids within the 400-570 amino acid region of the human T1R3 protein, and wherein the anti-human T1R3 antibody inhibits binding to this region by Aβ1-42 peptide, thereby suppressing the amplified activation current of the VGSC induced by Aβ1-42 peptide.

8. The ligand substance according to claim 3, wherein the ligand substance selectively binds to T1R2 or T1R2/T1R3 and acts as an antagonist to suppress the activation current of the VGSC, and wherein the ligand substance is selected from the group consisting of saccharin, aspartame, sucralose, and a T1R2 or T1R2/T1R3 ligand selected from Gymnema tea extract.

9. The ligand substance according to claim 2, wherein the ligand substance specifically binds to T1R3, T1R2/T1R3, or T1R1/T1R3 and acts as an agonist to amplify the activation current of the VGSC.

10. The ligand substance according to claim 9, wherein the ligand substance is a sugar or sweet amino acid.

11. The ligand substance according to claim 10, wherein the ligand substance is selected from the group consisting of glucose, sucrose, fructose, glutamine, serine, and mixtures thereof.

12. The ligand substance according to claim 2, wherein the ligand substance specifically binds to T1R1 or T1R1/T1R3 and acts as an agonist to shift the VGSC activation current in the I-V curve to the left or delay inactivation, or the ligand substance is a T1R1 ligand that suppresses the VGKC activation current.

13. The ligand substance according to claim 12, wherein the ligand substance is selected from the group consisting of glutamic acid (L-Glu), monosodium glutamate (MSG), inosine monophosphate (IMP), guanosine monophosphate (GMP), and mixtures thereof.

14. A pharmaceutical composition for preventing and/or treating diseases resulting from the amplification of VGSC currents or the suppression of VGKC currents due to the binding of sweet-tasting amino acids or umami substances to T1R2/T1R3 or T1R1/T1R3 on the surface of nerve cells or cardiomyocytes, wherein the pharmaceutical composition contains a ligand that specifically binds to T1R2 or T1R3, including homodimers or heterodimers, and exerts an antagonist-like effect.

15. The pharmaceutical composition according to claim 14, wherein the pharmaceutical composition is used for preventing and/or treating diseases **characterized by** epileptic-like neuronal hyperactivity or neuropathic pain, including neurologic disorders and/or neuropathic pain syndromes.

16. The pharmaceutical composition according to claim 15, wherein the neuronal hyperactivity **characterized by** epileptic-like neuronal hyperactivity states include conditions such as Alzheimer's disease (AD), amyotrophic lateral sclerosis (ALS), Lewy body dementia, local brain neurologic symptoms of cerebrovascular disorders, epilepsy, Parkinson's disease, progressive supranuclear palsy, multisystem atrophy, local brain neurologic symptoms in regions damaged by traumatic brain injury, Huntington's disease, spinocerebellar degeneration, and multiple sclerosis.

17. The pharmaceutical composition according to claim 15, wherein the pharmaceutical composition is for treating neuropathic pain associated with neuronal hyperactivity based on VGSC current amplification, wherein the neuropathic pain may result from peripheral nerve damage due to chemotherapy, failed back surgery syndrome (FBSS), complex regional pain syndrome (CRPS), or diabetic polyneuropathy.

18. The pharmaceutical composition according to claim 14, wherein the ligand specifically binds to T1R3 and exerts an antagonist-like effect, wherein the ligand is selected from the group consisting of anti-human T1R3 antibodies, cinacalcet, NPS-2143, raloxifene, clofibrate, and structurally similar compounds.

19. The pharmaceutical composition according to claim 14, wherein the ligand specifically binds to T1R2 or T1R2/T1R3 and exerts an antagonist-like effect, wherein the ligand is selected from the group consisting of saccharin, aspartame, sucralose, Gymnema tea extract, and structurally similar compounds.

20. A pharmaceutical composition for treating and/or preventing a disease caused by neuronal hyperexcitability resulting from the binding of an Aβ peptide containing the amino acid sequence indicated by "sequence number 1" to Type I taste receptors on the surface of nerve cells, wherein the pharmaceutical composition specifically binds to T1R1, T1R2, or T1R3, or their homodimers or heterodimers, with a ligand that exerts an antagonist-like effect.

21. A pharmaceutical composition for treating and/or preventing a disease caused by neuronal hyperexcitability via T1R3 or T1R2/T1R3, mediated by any of the Aβ peptides selected from the group consisting of Aβ25-35, Aβ1-38, Aβ1-40, and Aβ1-42, wherein the pharmaceutical composition specifically binds to T1R2 or T1R3, or their homodimers or heterodimers, with a ligand that exerts an antagonist-like effect.

22. The pharmaceutical composition according to claim 21, wherein the disease is a neuronal disorder **characterized by** epileptic-like neuronal hyperactivity or neuropathic pain.

23. The pharmaceutical composition according to claim 22, wherein the epileptic -like neuronal hyperactivity disease includes conditions such as Alzheimer's disease (AD), amyotrophic lateral sclerosis (ALS), Lewy body dementia, local brain nerve symptoms of cerebrovascular disorders, epilepsy, Parkinson's disease, progressive supranuclear palsy, multisystem atrophy, local brain nerve symptoms in regions damaged by traumatic brain injury, Huntington's disease, spinocerebellar degeneration, and multiple sclerosis.

24. The pharmaceutical composition according to claim 22, wherein the neuropathic pain is associated with neural hyperactivity based on VGSC current amplification and includes peripheral nerve disorders caused by chemotherapy, failed back surgery syndrome (FBSS), complex regional pain syndrome (CRPS), and diabetic polyneuropathy.

25. The pharmaceutical composition according to claim 21, wherein the ligand specifically binds to T1R3 and exerts an antagonist-like effect, wherein said ligand is selected from the group consisting of anti-human T1R3 antibodies, cinacalcet, NPS-2143, the clofibrate, and structurally similar compounds.

26. The pharmaceutical composition according to claim 25, wherein the anti-human T1R3 antibody specifically binds to an epitope comprising at least eight consecutive amino acid residues in the 229-258 amino acid region of human T1R3, inhibiting binding to the Aβ25-35 peptide region and suppressing the amplified VGSC current induced by the Aβ25-35 peptide.

27. The pharmaceutical composition according to claim 25, wherein the anti-human T1R3 antibody specifically binds to an epitope comprising at least eight consecutive amino acid residues in the 303-396 amino acid region of human T1R3, inhibiting binding to the Aβ1-40 peptide region and suppressing the amplified VGSC current induced by the Aβ1-40 peptide.

28. The pharmaceutical composition according to claim 25, wherein the anti-human T1R3 antibody specifically binds to an epitope comprising at least eight consecutive amino acid residues in the 400-570 amino acid region of human T1R3, inhibiting binding to the Aβ1-42 peptide region and suppressing the amplified VGSC current induced by the Aβ1-42 peptide.

29. The pharmaceutical composition according to claim 20 or 21, wherein the ligand specifically binds to T1R2 or T1R2/T1R3 and exerts an antagonist-like effect, wherein the ligand is selected from the group consisting of saccharin, aspartame, sucralose, Gymnema tea extract, and structurally similar compounds.

30. A method for screening substances capable of controlling the amplification or inhibition of voltage-dependent ion channel activation currents induced via Type I taste receptors on the surface of neural cells or cardiac muscle cells, comprising the steps of:

Step (1) Culturing human-derived cells expressing voltage-dependent ion channels on the cell surface along with known, fluorescently labeled Type I taste receptor ligands;
Step (2) Administering test substances to the culture medium simultaneously with, before, or after step (1), with or without administration;
Step (3) Selecting the test substance as a candidate for controlling voltage-dependent ion channel currents on the surface of neural cells or cardiac muscle cells if a significant increase or decrease in fluorescence intensity on the cultured cell surface is observed or measured compared to when the test substance is not administered.

31. The method according to claim 30, wherein the control of the amplification or suppression of the activation current of the voltage-dependent ion channel is the amplification of VGSC current and/or the suppression of VGKC current, wherein the Type I taste receptor is T1R1, T1R2, or T1R3, or their homodimers or heterodimers, and wherein the cultured human-derived cells used in step (1) are cultured human nerve cells, cultured human cardiomyocytes, or human-derived cultured cells forcibly expressing VGSC and/or VGKC.

32. The method according to claim 30, wherein the Type I taste receptor is T1R2/T1R3, and the cultured human-derived cells used in step (1) are cultured human nerve cells or human-derived cultured cells forcibly expressing VGSC, and wherein the known Type I taste receptor ligand administered to the culture medium in step (1) is Aβ25-35 peptide.

33. A method for screening substances capable of controlling the amplification of VGSC currents and/or the inhibition of VGKC currents, wherein Aβ peptide or sweet-tasting amino acids or umami substances bind to Type I taste receptors on the surface of human nerve cells or cardiomyocytes, comprising the steps of:

   Step (1) Measuring VGSC and/or VGKC currents of cultured human nerve cells, cultured human cardiomyocytes, or human-derived cultured cells forcibly expressing VGSC and/or VGKC, which express Type I taste receptors on their surface, using the patch-clamp method (voltage-clamp method) to establish control values;
   Step (2) Administering Aβ peptide or sweet-tasting amino acids or umami substances to the culture medium of the cells, and measuring the VGSC and/or VGKC currents of the cells using the same method as in step (1), to measure the amplification of VGSC currents and/or the inhibition of VGKC currents caused by the binding of Aβ peptide or sweet-tasting amino acids or umami substances to Type I taste receptors;
   Step (3) Administering the test substance to the culture medium of the cells, and measuring the VGSC and/or VGKC currents of the cells using the same method as in step (1);
   Step (4) Evaluating the test substance as a candidate for controlling the amplification of VGSC currents and/or the inhibition of VGKC currents mediated by Type I taste receptors if the measured values of VGSC and/or VGKC currents in step (3) significantly inhibit the amplification of VGSC currents and/or the inhibition of VGKC currents measured in step (2).

34. A method for screening substances capable of controlling the amplification of VGSC currents and/or the inhibition of VGKC currents, wherein Aβ peptide or sweet-tasting amino acids or umami substances bind to Type I taste receptors on human nerve cells or cardiomyocytes, comprising the steps of:

   Step (1) Measuring spontaneous action potentials on the cell surface of cultured human nerve cells, cultured human cardiomyocytes, or human-derived cultured cells forcibly expressing VGSC and/or VGKC, which express Type I taste receptors on their surface, and/or recording their waveforms using the patch-clamp method (current-clamp mode), NanoTouch method, MEA method, or methods using fluorescent membrane potential-sensitive reagents or fluorescent intracellular calcium-sensitive reagents, to establish control values;
   Step (2) Administering Aβ peptide or sweet-tasting amino acids or umami substances to the culture medium of the cells, and measuring the action potentials induced by the binding of Aβ peptide or sweet-tasting amino acids or umami substances to Type I taste receptors and/or recording their waveforms using the same method as in step (1);
   Step (3) Administering the test substance to the culture medium of the cells, and measuring the action potentials induced by the administration of the test substance and/or recording their waveforms using the same method as in step (1);
   Step (4) Evaluating the test substance as a candidate for controlling the amplification of VGSC currents and/or the inhibition of VGKC currents mediated by Type I taste receptors if the measured frequency of action potentials or the shape of the waveforms in step (3) shows significant changes compared to the frequency of action potentials or the shape of the waveforms measured in step (2).

35. A method for screening compounds that modify/inhibit the activity of VGSC and VGKC via T1Rs in CSF by Aβ peptide or sweet-tasting amino acids or umami substances, comprising the steps of:

   Step (1) Measuring the impedance (resistance) values at both ends of a cell sheet cultured in a sheet form by acting Aβ peptide or sweet-tasting amino acids or umami substances on Type I taste receptors of human epithelial cells;
   Step (2) Measuring the impedance values when the test substance is applied to the cells;
   Step (3) Evaluating the test substance as a candidate compound capable of controlling the amplification of VGSC currents and/or the inhibition of VGKC currents mediated by Type I taste receptors of Aβ peptide or sweet-tasting amino acids or umami substances if a decrease in impedance values is observed due to the action of the test substance, indicating that the barrier function of the cell sheet has decreased.

36. A method for screening substances capable of controlling cytoskeletal modifications caused by the binding of Aβ peptide or sweet-tasting amino acids or umami substances to Type I taste receptors on human nerve cells, comprising the steps of:

Step (1) Culturing human nerve cells or human-derived cultured cells expressing Type I taste receptors on their surface in a medium containing fluorescent reagent, and measuring the intracellular calcium concentration using fluorescent reagents to establish control values;

Step (2) Administering Aβ peptide or sweet-tasting amino acids or umami substances to the culture medium of the cells, and measuring the increased intracellular calcium concentration caused by the binding of these substances to Type I taste receptors using the same method as in step (1);

Step (3) Administering the test substance to the culture medium of the cells, and measuring the intracellular calcium concentration using the same method as in step (1);

Step (4) Evaluating the test substance as a candidate for controlling cytoskeletal modifications mediated by Type I taste receptors of Aβ peptide or sweet-tasting amino acids or umami substances if the intracellular calcium concentration measured in step (3) is significantly decreased compared to the calcium concentration measured in step (2).

37. A method for screening candidate compounds for the active ingredients of a pharmaceutical composition to prevent and/or treat diseases caused by the amplification of VGSC currents and/or the inhibition of VGKC currents due to the binding of Aβ peptide or sweet-tasting amino acids or umami substances to Type I taste receptors on human nerve cells or cardiomyocytes, comprising the steps of:

Step (1) Measuring VGSC and/or VGKC currents of cultured human nerve cells, cultured human cardiomyocytes, or human-derived cultured cells forcibly expressing VGSC and/or VGKC, which express Type I taste receptors on their surface, using the patch-clamp method (voltage-clamp method) to establish control values;

Step (2) Administering Aβ peptide or sweet-tasting amino acids or umami substances to the culture medium, and measuring VGSC and/or VGKC currents of the cells using the same method as in step (1) to measure the amplification of VGSC currents and/or the inhibition of VGKC currents from the control values;

Step (3) Administering the test substance to the culture medium of the cells, and measuring VGSC and/or VGKC currents of the cells using the same method as in step (1);

Step (4) Evaluating the test substance as a substance capable of controlling the amplification of VGSC currents and/or the inhibition of VGKC currents mediated by Type I taste receptors of Aβ peptide or sweet-tasting amino acids or umami substances in human nerve cells or cardiomyocytes, and selecting the test substance as a candidate compound for the active ingredients of the pharmaceutical composition if the measured values of VGSC and/or VGKC currents in step (3) significantly inhibit the amplification of VGSC currents and/or the inhibition of VGKC currents measured in step (2).

38. A method for screening candidate compounds as effective ingredients in pharmaceutical compositions for preventing and/or treating diseases resulting from the amplification of voltage-gated sodium channels (VGSC) or inhibition of VGKC currents due to the binding of Aβ peptide or sweet-tasting amino acids or umami substances to Type I taste receptors in human neurons or cardiomyocytes, comprising the steps of:

Step (1) Establishing control values by measuring the action potentials or recording their waveform widths in cultured human neurons, cultured human cardiomyocytes, or cultured human-derived cells in which Type I taste receptors are expressed on the cell surface and can spontaneously fire action potentials or be stimulated externally using the patch-clamp method (current-clamp mode), NanoTouch method, or MEA methods, wherein VGSC and/or VGKC are forcibly expressed;

Step (2) Administering Aβ peptide or sweet-tasting amino acids or umami substances to the cell culture medium of the cells described in step (1) and measuring the induced action potentials or recording their waveform widths using the same method as in step (1), wherein the Aβ peptide or sweet-tasting amino acids or umami substances bind to Type I taste receptors;

Step (3) Administering a test substance to the cell culture medium from step (2) and measuring the induced action potentials or recording their waveform widths using the same method as in step (1);

Step (4) Evaluating the test substance as a substance capable of controlling the amplification of VGSC current and/or the suppression of VGKC current via the Type I taste receptor for Aβ peptide or sweet-tasting amino acids

or umami substances in human nerve cells or cardiomyocytes, and selecting the test substance as a candidate compound for the active ingredient of the pharmaceutical composition if the measurement value of the frequency of action potential occurrence or the shape of the waveform width measured in step (3) shows a significant change compared to the frequency of action potential occurrence or the shape of the waveform width measured in step (2).

39. A method for screening candidate compounds as effective ingredients in pharmaceutical compositions for preventing and/or treating diseases resulting from the amplification of voltage-gated sodium channel (VGSC) currents due to the binding of Aβ peptides, wherein the method comprises observing changes in the area or volume of cultured human neurons or humanized mouse neurons.

40. A method for screening candidate compounds as effective ingredients in pharmaceutical compositions, wherein the VGSC currents were enhanced by acting on Type I taste receptors of human neurons or humanized experimental animal-derived cells with the Aβ42/40 ratio observed in cerebrospinal fluid from Alzheimer's disease (AD) patients, and subsequently observing a decrease in VGSC current enhancement upon exposure to the test substance, and evaluating the candidate substance for preventing and/or treating diseases resulting from the amplification of voltage-gated sodium channel (VGSC) currents due to the binding of Aβ peptides.

41. A method for evaluating cell barrier function (degree of cell-cell adhesion) mediated by T1Rs due to differences in the Aβ42/40 ratio in CSF or serum, comprising the steps of:

Step (1) Measuring the impedance (resistance) values at both ends of a cell sheet cultured in a sheet form of human vascular endothelial cells;
Step (2) Administering Aβ peptide or sweet-tasting amino acids or umami substances, as test substances, to the Type I taste receptors of the cells, and measuring the impedance values of the cell sheet at that time;
Step (3) Evaluating whether a decrease in impedance values occurs due to the action of the test substance, and determining whether there is a pathological effect on the barrier function of the cell sheet.

[Figure 1]

Expression of Tas1R3 in HEK293 cells

[Figure 2]

[Figure 3]

[Figure 4]

[Figure 5]

[Figure 6]

[Figure 7]

[Figure 8]

T1R3 Antibody pre-treated HEK Nav1.5

[Figure 9]

Ab25-35 on SH-SY5Y
EC50=86.8nM
Hill slope=1.33

[Figure 10]

Cell capacitance

VGSC Current

[Figure 11]

[Figure 12]

| Cell name | HEK Nav1.5 | | | SH-SY5Y |
|---|---|---|---|---|
| Codition | Current Density (pA/pF) | | | |
| Control | 14.4 | 39.6 | 14.7 | 58.0 |
| Low Aβ25-35 | 18.8 | 60.2 | 30.4 | 70.0 |
| High Aβ25-35 | 13.1 | 46.5 | 27.5 | |
| Inserted | 40.9 | 409.4 | 145.0 | 104.6 |
| Retrieved | 52.2 | 113.9 | 44.9 | |

[Figure 13]

[Figure 14]

(A)

(B)

[Figure 15]

[Figure 16]

[Figure 17]

(A)

(B)

360ms

(C)

10mM MSG on 30uM TBB treated HEK Nav1.5

30uM TBB

+ 10mM MSG

8sec intervals

[Figure 18]

Control >TBB > MSG
HEK Nav1.5

10mM MSG

20uM TBB

Control
20uM TBB
10mM MSG

Control

20uM TBB

10mM MSG

● Control
▲ Control
△ 20uM TBB
○ 20uM TBB
▲ 10mM MSG
● 10mM MSG

[Figure 19]

(A)

● Control

○2.4uM Taxol

(B)

● Control

○2.4uM Taxol

(C)

1uM Taxol

D

[Figure 20]

(A)

Control

(B)

5uM NPS-2143

[Figure 21]

(A)

IKv

INa

(B)

IKv

Cinacalcet

INa

EP 4 537 845 A1

[Figure 22]

(A)

(B)

pp-80mV

20uM Cinacalcet          20uM Cinacalcet

pp-120mV

Row Number

[Figure 23]

(A)

Control
2uM Cyt D
+ 20uM Cinacalcet

Control      2uM Cyt D      10uM Cinacalcet

(B)

6uM Cinacalcet

Control

20ms

7.5uM Colchicine present

6uM Cinacalcet

EP 4 537 845 A1

[Figure 24]

[Figure 25]

[Figure 26]

[Figure 27]

[Figure 28]

(A)

(B)

| GTE | Vh diff | K diff | I/Imax % |
|---|---|---|---|
| SH-SY5Y | -4.44 | 2.69 | 62.6 |
| NG108-15 | -7.54 | -3.43 | 65.6 |
| Nav1.5 | -7.87 | 1.18 | 54 |
| 10ul/mL GTE | | | |

[Figure 29]

[Figure 30]

[Figure 31]

[Figure 32]

[Figure 33A]

[Figure 33B]

[Figure 34]

[Figure 35]

[Figure 36]

[Figure 37]

[Figure 38]

[Figure 39]

[Figure 40]

[Figure 41]

(A)

T1R1 ligand MSG2.5 on T1R1 KO Nav1.5 HEK cell

T1R1 KO

● Control
○ 5mM MSG2.5

(B)

T1R1 ligand MSG2.5 action preserved in T1R3 KO Nav1.5 HEK cell

T1R3 KO

● Control
○ 5mM MSG2.5

(C)

T1R1 KO

● Control
○ 5mM MSG2.5

[Figure 42]

| Enhancement of VGSC current / Pathway to nerve hyperactivity |
|---|

Amyloid β peptide, L-Gln, Lactate etc.

↓

Tas1R3 homodier, or Tas1R2/Tas1R3 heterodimer

↓

Neuropathic Pain Caused by Chemotherapeutic Agents, like Paclitaxel, for Cancer Treatment → Enhancement of VGSC current Nerve hyperactivity ← Increased Stimulation of Nerves Due to Injury or Invasion (Nociceptive Pain)

↓

- Reduces threshold for action potential generation. (More prone to action potential generation).
- Bursting activity developed (Single stimulus leads to multiple, continuous action potential repetitions).

Consequence: Seizure, epilepsy, nociceptive and neuropathic pain.
→ Touching triggers bursting or tonic action potentials. In a healthy state, one stimulus results in a single action potential. However, in the pathological state, a single stimulus leads to a burst of action potentials.
→ Epilepsy-like symptoms, detected in Alzheimer's disease patients, induce neuro-hyperactivity, eventually leading to nerve cell death.

[Figure 43]

Control

Aβ25-35

10 R.V.U.
50 sec

10 R.V.U.
5 sec

[Figure 44]

(A) Aβ1-38

● Control
○ Aβ1-38

Control  Aβ1-38

(B) Aβ1-40

● Control
○ Aβ1-40

Control  Aβ1-40

[Figure 45]

(A) Aβ1-40

10nM  20nM

(B) Aβ1-42

10nM  20nM

[Figure 46]

[Figure 47]

[Figure 48]

[Figure 49]

[Figure 50]

[Figure 51]

A

Nav1.6/Kv1.2 HEK

- Control
- 1ul SuM Ab1-42
- NobusBio AntiR3

B

Nav1.6/Kv1.2 HEK

- Control
- 25nM Ab1-42
- 31ng/ml Thaumanin

C

Nav1.6/Kv1.2 HEK

- Control
- 25ug/ml Thaumatin
- 50ug/ml Thaumatin

[Figure 52]

A

Nav1.5

- Control
- Aβ1-42
- Anti T1R3 (400-570)

B

T1R2 miRNA Aβ1-40

- Control
- Aβ1-40

[Figure 53-1]

Control (T=0)          120uM Aspartame (T=225sec)

[Figure 53-2]

A

B

Control    7.5uM β-alanine ————————————→

5min. Intervals, From left to right

[Figure 54]

Control    Aβ1-40
A1    T=0    T=30min.

Nav1.5 HEK

A2    T=0    T=17mn.

HUEhT-1

Control    AβAD
B1    T=0    T=20min.

T=0    T=36min.
B2

[Figure 55]

Nav1.5 HEK

Control  T=0  Aβ ND  T=15min

[Figure 56]

B.end-3

Control  T=0  Aβ AD  T=18min.

Control  T=0  Aβ ND  T=22min.

[Figure 57]

A

A$\beta$1-40 -Ile-Ala → A$\beta$1-42

B

L-Isoleucyl-L-alanine

Beta Alanine

GABA

Carnosine

[Figure 58]

[Figure 59]

**EP 4 537 845 A1**

## INTERNATIONAL SEARCH REPORT

International application No.

**PCT/JP2023/021634**

### A. CLASSIFICATION OF SUBJECT MATTER

*A61K 45/00*(2006.01)i; *A61K 31/192*(2006.01)i; *A61K 31/198*(2006.01)i; *A61K 31/216*(2006.01)i; *A61K 31/428*(2006.01)i;
*A61K 31/70*(2006.01)i; *A61K 31/7004*(2006.01)i; *A61K 31/7016*(2006.01)i; *A61K 31/7076*(2006.01)i;
*A61K 31/708*(2006.01)i; *A61K 38/05*(2006.01)i; *A61K 39/395*(2006.01)i; *A61P 9/00*(2006.01)i; *A61P 21/02*(2006.01)i;
*A61P 25/00*(2006.01)i; *A61P 25/04*(2006.01)i; *A61P 25/08*(2006.01)i; *A61P 25/14*(2006.01)i; *A61P 25/16*(2006.01)i;
*A61P 25/28*(2006.01)i; *A61P 43/00*(2006.01)i; *C07K 16/28*(2006.01)i; *C12Q 1/02*(2006.01)i; *G01N 33/15*(2006.01)i
FI: A61K45/00; A61K31/192; A61K31/198; A61K31/216; A61K31/428; A61K31/70; A61K31/7004; A61K31/7016;
A61K31/7076; A61K31/708; A61K38/05; A61K39/395 N; A61P9/00; A61P21/02; A61P25/00; A61P25/04; A61P25/08
ZNA; A61P25/14; A61P25/16; A61P25/28; A61P43/00 111; C07K16/28; C12Q1/02; G01N33/15 Z

According to International Patent Classification (IPC) or to both national classification and IPC

### B. FIELDS SEARCHED

Minimum documentation searched (classification system followed by classification symbols)

A61K45/00; A61K31/192; A61K31/198; A61K31/216; A61K31/428; A61K31/70; A61K31/7004; A61K31/7016;
A61K31/7076; A61K31/708; A61K38/05; A61K39/395; A61P9/00; A61P21/02; A61P25/00; A61P25/04; A61P25/08;
A61P25/14; A61P25/16; A61P25/28; A61P43/00; C07K16/28; C12Q1/02; G01N33/15

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| X | JP 2005-505262 A (THE REGENTS OF THE UNIVERSITY OF CALIFORNIA) 24 February 2005 (2005-02-24)<br>in particular, claims | 1-13 |
| X | KHARATMAL, S. B. et al. Comparative evaluation of in vitro and in vivo high glucose-induced alterations in voltage-gated tetrodotoxin-resistant sodium channel: effects attenuated by sodium channel blockers. Neuroscience. 2015, vol. 305, pp. 183-196<br>in particular, abstract | 1-2, 9-11 |

☑ Further documents are listed in the continuation of Box C.  ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **28 August 2023** | **05 September 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

114

**INTERNATIONAL SEARCH REPORT**

International application No.

**PCT/JP2023/021634**

**C.   DOCUMENTS CONSIDERED TO BE RELEVANT**

| Category* | Citation of document, with indication, where appropriate, of the relevant passages | Relevant to claim No. |
|---|---|---|
| X | MISONOU, Hiroaki et al. Regulation of ion channel localization and phosphorylation by neuronal activity. NATURE NEUROSCIENCE. 2004, vol. 7, no. 7, pp. 711-718<br>in particular, abstract, p. 715, right column, 2nd-4th paragraphs, fig. 7 | 1-2, 9, 12-13 |
| X | WANG, Xi et al. Elevated neuronal excitability due to modulation of the voltage-gated sodium channel Nav1.6 by Abeta1-42. Front. Neurosci. 2016, vol. 10, article 94, pp. 1-9<br>in particular, abstract, p. 4, right column, 2nd paragraph to p. 5, right column, 1st paragraph, fig. 3 | 1-2, 9 |

Form PCT/ISA/210 (second sheet) (January 2015)

**INTERNATIONAL SEARCH REPORT**

International application No.

**PCT/JP2023/021634**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

    The "form of an Annex C/ST.25 text file" is replaced with the "form of ST.26."

Form PCT/ISA/210 (continuation of first sheet) (January 2015)

**INTERNATIONAL SEARCH REPORT**

International application No.

**PCT/JP2023/021634**

| Box No. III      Observations where unity of invention is lacking (Continuation of item 3 of first sheet) |
|---|

This International Searching Authority found multiple inventions in this international application, as follows:

(Invention 1) Claims 1-13

Document 1 (particularly, claims) discloses an antibody that specifically binds to an isolated taste receptor containing a T1R3 polypeptide, wherein the antibody can specifically bind to a taste receptor containing T1R1 and T1R3, and discloses saccharin, aspartame, sucrose, fructose, glutamine, serine, glutamic acid, and the like as taste ligands that bind to said receptor.

Accordingly, claims 1-13 lack novelty in light of document 1, and thus do not have a special technical feature. Thus, claims 1-13 are classified as invention 1.

(Invention 2) Claims 14-19

Claims 14-19 cannot be said to have a special technical feature identical or corresponding to that of claim 1 classified as invention 1.

In addition, claims 14-19 are not dependent on claim 1 classified as invention 1.

Furthermore, claims 14-19 are not substantially identical to or similarly closely related to any of the claims classified as invention 1.

Therefore, claims 14-19 cannot be classified as invention 1.

In addition, claims 14-19 have the special technical feature of a "pharmaceutical composition for the prevention and/or treatment of diseases caused by amplification of current of VGSC and/or suppression of current of VGKC caused by binding of sweetening amino acids or umami substances to T1R2/T1R3 or T1R1/T1R3 on the surfaces of nerve cells or myocardial cells, wherein the pharmaceutical composition specifically binds to T1R2 or T1R3, or a homodimer or heterodimer thereof and contains ligands having an antagonist-like effect as an active ingredient," and are thus classified as invention 2.

(Invention 3) Claim 20

Claim 20 cannot be said to have a special technical feature identical or corresponding to that of claim 1 classified as invention 1 or those of claims 14-19 classified as invention 2.

In addition, claim 20 is not dependent on any of claims 1 and 14-19.

Furthermore, claim 20 is not substantially identical to or similarly closely related to any of the claims classified as invention 1 or 2.

Therefore, claim 20 cannot be classified as either invention 1 or invention 2.

In addition, claim 20 has the special technical feature of a "pharmaceutical composition for the treatment and/or prevention of diseases caused by the excessive stimulation of nerve cells caused by the binding of Aβ peptides containing the amino acid sequence represented by "SEQ ID NO: 1" to TypeI taste receptors on the surfaces of nerve cells, wherein the pharmaceutical composition specifically binds to T1R1, T1R2, or T1R3, or a homodimer or heterodimer thereof and contains ligands having an antagonist-like effect as an active ingredient," and is thus classified as invention 3.

(Invention 4) Claims 21-29

Claims 21-29 cannot be said to have a special technical feature identical or corresponding to that of claim 1 classified as invention 1 or those of claims 14-20 classified as any of inventions 2-3.

Furthermore, claims 21-29 are not dependent on any of claims 1 and 14-20.

In addition, claims 21-29 are not substantially identical to or similarly closely related to any of the claims classified as inventions 1-3.

Therefore, claims 21-29 cannot be classified as any of inventions 1-3.

In addition, claims 21-29 have the special technical feature of a "pharmaceutical composition for the treatment and/or prevention of diseases caused by the excessive stimulation of nerve cells mediated by T1R3 or T1R2/T1R3 by any one Aβ peptide selected from an Aβ25-35 peptide, an Aβ1-38 peptide, an Aβ1-40 peptide, and an Aβ1-42 peptide, wherein the pharmaceutical composition specifically binds to T1R2 or T1R3, or a homodimer or heterodimer thereof and contains ligands having an antagonist-like effect as an active ingredient," and are thus classified as invention 4.

(Invention 5) Claims 30-32

Claims 30-32 cannot be said to have a special technical feature identical or corresponding to that of claim 1 classified as invention 1 or those of claims 14-29 classified as any of inventions 2-4.

Furthermore, claims 30-32 are not dependent on any of claims 1 and 14-29.

Form PCT/ISA/210 (continuation of first sheet) (January 2015)

**INTERNATIONAL SEARCH REPORT**

| International application No. |
| --- |
| **PCT/JP2023/021634** |

| Box No. III    Observations where unity of invention is lacking (Continuation of item 3 of first sheet) |
| --- |

In addition, claims 30-32 are not substantially identical to or similarly closely related to any of the claims classified as inventions 1-4.

Therefore, claims 30-32 cannot be classified as any of inventions 1-4.

In addition, claims 30-32 have the special technical feature of a "method for screening a substance capable of controlling the amplification or suppression of the activation current of a voltage-gated ion channel caused by TypeI taste receptors on the surfaces of nerve cells or myocardial cells, the method comprising the following steps (1)-(3): (1) a step for introducing and culturing an existing TypeI taste receptor ligand fluorescently labeled in a culture medium of a cultured human-derived cell expressing a voltage-gated ion channel together with the TypeI taste receptor on the cell surface; (2) a step for introducing a substance to be tested simultaneously with or before or after step (1) or without introducing the substance into the culture medium to culture the substance of each case; and (3) a step for selecting the substance to be tested as a candidate for a substance capable of controlling the current of the voltage-gated ion channel on the surfaces of nerve cells or myocardial cells when it can be observed or measured that the substance to be tested has been significantly amplified or decreased by comparing the case where the fluorescent intensity of the surfaces of the cultured cells when the substance to be tested has been introduced with the case where the substance to be tested has not been introduced," and are thus classified as invention 5.

(Invention 6) Claim 33

Claim 33 cannot be said to have a special technical feature identical or corresponding to that of claim 1 classified as invention 1 or those of claims 14-32 classified as inventions 2-5.

In addition, claim 33 is not dependent on any of claims 1 and 14-32.

Furthermore, claim 33 is not substantially identical to or similarly closely related to any of the claims classified as inventions 1-5.

Thus, claim 33 cannot be classified as any of inventions 1-5.

In addition, claim 33 has the special technical feature of a "method for screening a substance capable of controlling the action of amplifying the current of VGSC and/or suppressing the current of VGKC caused by binding of Aβ peptides, sweetening amino acids, or umami substances to TypeI taste receptors on the surfaces of human nerve cells or myocardial cells, the method comprising the following steps (1)-(4): (1) a step for measuring, by using a patch clamp method (membrane potential fixing method), the current of VGSC and/or VGKC of cultured human nerve cells, cultured human myocardial cells, which express TypeI taste receptors on the cell surfaces, or human-derived cultured cells forcibly expressing VGSC and/or VGKC to establish a control value; (2) a step for introducing the Aβ peptides, the sweetening amino acids, or the umami substances to the culture medium of the cells, measuring the VGSC and/or VGKC current of the cells by using the same method as in step (1), and measuring the amplification amount of the current of VGSC and/or the suppression amount of the current of VGKC caused by the binding of the Aβ peptides, the sweetening amino acids, or the umami substances to the TypeI taste receptors; (3) a step for subsequently introducing a substance to be tested to the culture medium of the cells and then measuring the current of VGSC and/or VGKC of the cells by using the same method as step (1); and (4) a step for evaluating the substance to be tested as a substance capable of controlling the action of amplifying the current of VGSC and/or suppressing the current of VGKC of the Aβ peptides, the sweetening amino acids, or the umami substances in human nerve cells or myocardial cells mediated by the TypeI taste receptors when the measured values of the current of VGSC and/or VGKC measured in step (3) show values that significantly inhibit either or both of the amplification amount of the current of VGSC and/or the suppression amount of the current of VGKC measured in step (2)," and is thus classified as invention 6.

(Invention 7) Claim 34

Claim 34 cannot be said to have a special technical feature identical or corresponding to that of claim 1 classified as invention 1 or those of claims 14-33 classified as any of inventions 2-6.

In addition, claim 34 is not dependent on any of claims 1 and 14-33.

Furthermore, claim 34 is not substantially identical to or similarly closely related to any of the claims classified as inventions 1-6.   Thus, claim 34 cannot be classified as any of inventions 1-6.

In addition, claim 34 has the special technical feature of a "method for screening a substance capable of controlling the amplification of the current of VGSC and/or the suppression of the current of VGKC caused by the binding of Aβ peptides, sweetening amino acids, or umami substances to TypeI taste receptors of human nerve cells or myocardial cells, the method comprising the following steps (1)-(4): (1) a step for measuring the spontaneous action potentials of the surfaces of cultured human nerve cells, cultured human myocardial cells, or human-derived cultured cells forcibly expressing VGSC and/or VGKC which are cells expressing TypeI taste receptors on the surfaces of the cells or firing the action potentials spontaneously or by external stimulation by means of a patch clamp method (current clamp mode), a NanoTouch method, an MEA method, or a method using a fluorescent membrane potential sensitive reagent or a fluorescent intracellular calcium sensitive reagent and/or recording the

Form PCT/ISA/210 (continuation of first sheet) (January 2015)

| International application No. |
| --- |
| **PCT/JP2023/021634** |

**Box No. III      Observations where unity of invention is lacking (Continuation of item 3 of first sheet)**

waveform thereof to establish a control value; (2) a step for introducing the Aβ peptides, sweetening amino acids, or umami substances to the culture medium of the cells, and measuring, by using the same method as in step (1), the action potentials induced by the binding of the Aβ peptides, the sweetening amino acids, or the umami substances to the TypeI taste receptors and/or record the waveform thereof; (3) a step for subsequently introducing the substance to be tested to the culture medium in step (2), and measuring, by using the same method as in step (1), the action potentials induced by the introduction of the substance to be tested and/or recording the waveform thereof; and (4) a step for evaluating the substance to be tested as a candidate of substances capable of controlling the amplification of the current of VGSC and/or the suppression of the current of VGKC of the Aβ peptides, the sweetening amino acids, or the umami substances in human nerve cells or myocardial cells mediated by the TypeI taste receptors when the measured values of the frequency of the onset of the action potentials measured in step (3) or the shape of the waveform shows a significant change compared to the measured values of the frequency of the onset of the action potentials measured in step (2) or the shape of the waveform," and is thus classified as invention 7.

(Invention 8) Claim 35
    Claim 35 cannot be said to have a special technical feature identical or corresponding to that of claim 1 classified as invention 1 or those of claims 14-34 classified as inventions 2-7.
    In addition, claim 35 is not dependent on any of claims 1 and 14-34.
    Furthermore, claim 35 is not substantially identical to or similarly closely related to any of the claims classified as inventions 1-7.
    Thus, claim 35 cannot be classified as any of inventions 1-7.
    In addition, claim 35 has the special technical feature of a "method for screening a compound that modifies/inhibits the action of Aβ peptides, sweetening amino acids, or umami substances on VGSC and VGKC activity mediated by T1Rs in CSF, the method comprising (1) a step for acting Aβ peptides, sweetening amino acids, or umami substances on TypeI taste receptors of human epithelial cells to culture the cells in sheet-like form and measure the impedance (resistance) values of both ends of the cell sheet; (2) a step for subsequently measuring impedance values when a substance to be tested is made to act on the cells; and (3) a step for evaluating that the barrier function of the cell sheet has decreased when a decrease in the impedance value is observed due to the action of the substance to be tested, and selecting said substance to be tested as a candidate compound capable of controlling the amplification of the current of VGSC and/or the suppression of the current of VGKC of the Aβ peptides, the sweetening amino acids, or the umami substances mediated by the TypeI taste receptors," and is thus classified as invention 8.

(Invention 9) Claim 36
    Claim 36 cannot be said to have a special technical feature identical or corresponding to that of claim 1 classified as invention 1 or those of claims 14-35 classified as inventions 2-8.
    In addition, claim 36 is not dependent on any of claims 1 and 14-35.
    Furthermore, claim 36 is not substantially identical to or similarly closely related to any of the claims classified as inventions 1-8.
    Thus, claim 36 cannot be classified as any of inventions 1-8.
    In addition, claim 36 has the special technical feature of a "method for screening a substance capable of controlling the modification of a cytoskeleton produced by binding of Aβ peptides, sweetening amino acids, or umami substances to TypeI taste receptors of human nerve cells, the method comprising the following steps (1)-(4): (1) a step for culturing cultured human nerve cells or human-derived cultured cells expressing TypeI taste receptors in a culture medium containing a fluorescent reagent, and measuring a calcium concentration in the cells by a calcium measurement method in cells to establish a control value; (2) a step for introducing the Aβ peptides, sweetening amino acids, or umami substances to the culture medium of the cells and measuring, by using the same method as in step (1), a calcium concentration in the cells increased by the binding of the substance to the TypeI taste receptor; (3) a step for subsequently introducing a substance to be tested to the culture medium in step (2) and measuring a calcium concentration in the cells by using the same method as in step (1); and (4) a step for evaluating the substance to be tested as a substance for activating the TypeI taste receptors of the nerve cells to cause a change in the calcium concentration ((Ca2+)i) in the cells and selecting the substance to be tested as a candidate substance capable of controlling the modification of the cytoskeleton mediated by the TypeI taste receptors caused by the Aβ peptides, sweetening amino acids, or umami substances in human nerve cells when the calcium concentration in the cells measured in step (3) is significantly reduced compared to the calcium concentration measured in step (2)," and is thus classified as invention 9.

(Invention 10) Claim 37

Form PCT/ISA/210 (continuation of first sheet) (January 2015)

**INTERNATIONAL SEARCH REPORT**

International application No.

PCT/JP2023/021634

| Box No. III | Observations where unity of invention is lacking (Continuation of item 3 of first sheet) |
|---|---|

Claim 37 cannot be said to have a special technical feature identical or corresponding to that of claim 1 classified as invention 1 or those of claims 14-36 classified as inventions 2-9.

In addition, claim 37 is not dependent on any of claims 1 and 14-36.

Furthermore, claim 37 is not substantially identical to or similarly closely related to any of the claims classified as inventions 1-9.

Thus, claim 37 cannot be classified as any of inventions 1-9.

In addition, claim 37 has the special technical feature of a "method for screening a candidate compound of an active ingredient of a pharmaceutical composition for the prevention and/or treatment of diseases caused by the action of amplifying the current of VGSC and/or suppressing the current of VGKC caused by binding of Aβ peptides, sweetening amino acids, or umami substances to TypeI taste receptors of human nerve cells or myocardial cells, the method comprising the following steps (1)-(4): (1) a step for measuring, by using a patch clamp method (membrane potential fixing method), the current of VGSC and/or VGKC of cultured human nerve cells, cultured human myocardial cells, which express TypeI taste receptors on the cell surfaces, or human-derived cultured cells forcibly expressing VGSC and/or VGKC to establish a control value; (2) a step for introducing the Aβ peptides, the sweetening amino acids, or the umami substances to the culture medium of the cells, measuring the VGSC and/or VGKC current of the cells by using the same method as in step (1), and measuring the amplification amount of the current of VGSC and/or the suppression amount of the current of VGKC from the control value; (3) a step for subsequently introducing a substance to be tested to the culture medium of the cells and then measuring the current of VGSC and/or VGKC of the cells by using the same method as in step (1); and (4) a step for evaluating the substance to be tested as a substance capable of controlling the action of amplifying the current of VGSC and/or suppressing the current of VGKC of the Aβ peptides, the sweetening amino acids, or the umami substances in human nerve cells or myocardial cells mediated by the TypeI taste receptors, and selecting the substance to be tested as a candidate compound of an active ingredient of the pharmaceutical composition when the measured values of the current of VGSC and/or VGKC measured in step (3) show values that significantly inhibit either or both of the amplification amount of the current of VGSC and/or the suppression amount of the current of VGKC measured in step (2)," and is thus classified as invention 10.

(Invention 11) Claims 38-39

Claims 38-39 cannot be said to have a special technical feature identical or corresponding to that of claim 1 classified as invention 1 or those of claims 14-37 classified as any of inventions 2-10.

Furthermore, claims 38-39 are not dependent on any of claims 1 and 14-37.

In addition, claims 38-39 are not substantially identical to or similarly closely related to any of the claims classified as inventions 1-10.

Therefore, claims 38-39 cannot be classified as any of inventions 1-10.

In addition, claim 38-39 have the special technical feature of a "method for screening a candidate compound of an active ingredient of a pharmaceutical composition for the prevention and/or treatment of diseases caused by the action of amplifying the current of VGSC and/or suppressing the current of VGKC caused by binding of Aβ peptides, sweetening amino acids, or umami substances to TypeI taste receptors of human nerve cells or myocardial cells, the method comprising the following steps (1)-(4): (1) a step for measuring the action potentials of the surfaces of cultured human nerve cells, cultured human myocardial cells, or human-derived cultured cells forcibly expressing VGSC and/or VGKC which are cells expressing TypeI taste receptors on the surfaces of the cells and capable of firing the action potentials spontaneously or by external stimulation by means of a patch clamp method (current clamp mode), a NanoTouch method, or an MEA method, and/or recording the width of waveform thereof to establish a control value; (2) a step for introducing the Aβ peptides, sweetening amino acids, or umami substances to the culture medium of the cells, measuring, by using the same method as in step (1), the action potentials induced by the binding of the Aβ peptides, the sweetening amino acids, or the umami substances to the TypeI taste receptors and/or recording the width of waveform thereof; (3) a step for subsequently introducing the substance to be tested to the culture medium in step (2), and measuring, by using the same method as in step (1), the action potentials induced by the introduction of the substance to be tested, and/or recording the width of waveform thereof; and (4) a step for evaluating the substance to be tested as a substances capable of controlling the amplification of the current of VGSC and/or the suppression of the current of VGKC of the Aβ peptides, the sweetening amino acids, or the umami substances in human nerve cells or myocardial cells mediated by the TypeI taste receptors and selecting the substance to be tested as a candidate compound of an active ingredient of the pharmaceutical composition when the measured values of the frequency of the onset of the action potentials measured in step (3) or the shape of the width of waveform shows a significant change compared to the measured values of the frequency of the onset of the action potentials measured in step (2) or the shape of the width of waveform," and are thus classified as invention 11.

(Invention 12) Claim 40

Form PCT/ISA/210 (continuation of first sheet) (January 2015)

**INTERNATIONAL SEARCH REPORT**

International application No.

**PCT/JP2023/021634**

| Box No. III Observations where unity of invention is lacking (Continuation of item 3 of first sheet) |
|---|

Claim 40 cannot be said to have a special technical feature identical or corresponding to that of claim 1 classified as invention 1 or those of claims 14-39 classified as inventions 2-11.

In addition, claim 40 is not dependent on any of claims 1 and 14-39.

Furthermore, claim 40 is not substantially identical to or similarly closely related to any of the claims classified as inventions 1-11.

Thus, claim 40 cannot be classified as any of inventions 1-11.

In addition, claim 40 has the special technical feature of a "method for screening an active ingredient of a pharmaceutical composition, the method comprising a step for evaluating a substance to be tested as a candidate compound of the active ingredient of the pharmaceutical composition for the prevention and/or treatment of diseases caused by the amplification of the current of VGSC caused by binding of Aβ peptides when it is observed that the current of VGSC enhanced by acting Aβ42/40 ratio (AD-D) observed in the cerebrospinal fluid of an AD patient on TypeI taste receptors of human nerve cells or humanized experimental animal-derived cells has a decrease in the increment of the current of VGSC by the acted substance to be tested," and is thus classified as invention 12.

(Invention 13) Claim 41

Claim 41 cannot be said to have a special technical feature identical or corresponding to that of claim 1 classified as invention 1 or those of claims 14-40 classified as inventions 2-12.

In addition, claim 41 is not dependent on any of claims 1 and 14-40.

Furthermore, claim 41 is not substantially identical to or similarly closely related to any of the claims classified as inventions 1-12.

Thus, claim 41 cannot be classified as any of inventions 1-12.

In addition, claim 41 has the special technical feature of a "method of evaluating cell barrier function (degree of cell adhesion) mediated by T1Rs due to the difference of the ratio of Aβ42/40 contained in CSF or serum, the method comprising: (1) a step for measuring impedance (resistance) values at both ends of a cell sheet cultured in a human vascular epithelial cell sheet; (2) a step for subsequently acting a substance to be tested such as Aβ peptides, sweetening amino acids, or umami substances on the TypeI taste receptors of the cells to measure the impedance values of the cell sheet at that time; (3) a step for evaluating whether or not a decrease in impedance values is caused by the action of the substance to be tested, and evaluating whether or not the barrier function of the cell sheet has a pathological action," and is thus classified as invention 13.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **claims 1-13**

**Remark on Protest**  ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (January 2015)

INTERNATIONAL SEARCH REPORT
Information on patent family members

International application No.

**PCT/JP2023/021634**

| Patent document cited in search report | | | Publication date (day/month/year) | Patent family member(s) | | | Publication date (day/month/year) |
|---|---|---|---|---|---|---|---|
| JP | 2005-505262 | A | 24 February 2005 | WO | 2003/004992 | A2 | |
| | | | | claims | | | |
| | | | | US | 2003/0040045 | A1 | |
| | | | | US | 2003/0166137 | A1 | |
| | | | | US | 2005/0260599 | A1 | |
| | | | | US | 2008/0318251 | A1 | |
| | | | | US | 2009/0098580 | A1 | |
| | | | | US | 2011/0151483 | A1 | |
| | | | | US | 2011/0244514 | A1 | |
| | | | | US | 2014/0024043 | A1 | |
| | | | | EP | 1585954 | A1 | |
| | | | | EP | 2381256 | A1 | |

Form PCT/ISA/210 (patent family annex) (January 2015)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018199334 A

- WO 2019208828 A

**Non-patent literature cited in the description**

- **GHATAK et al.** *ELife*, 2019, vol. 8, https://doi.org/10.7554/eLife.50333
- **LI et al.** *Proceedings of the National Academy of Sciences*, 2002, vol. 99 (7), 4692-96, https://doi.org/10.1073/pnas.072090199
- **LI et al.** *The Journal of Neuroscience*, 2018, vol. 38 (5), 1124-36, https://doi.org/10.1523/JNEUROSCI.0899-17.2017
- **MAILLET et al.** *Chemical Senses*, 2015, vol. 40 (8), 577-86, https://doi.org/10.1093/chemse/bjv045
- **MARTIN et al.** *Journal of Biological Chemistry*, 2017, vol. 292 (27), 11508-30, https://doi.org/10.1074/jbc.M116.773820
- **MARTINEZ et al.** *Journal of Neural Transmission - Parkinson's Disease and Dementia Section*, 1993, vol. 6 (1), 1-9, https://doi.org/10.1007/BF02252617
- **MATTHEISEN et al.** *Cell Reports*, May 2018, vol. 23 (9), 2770-81, https://doi.org/10.1016/j.celrep.2018.04.109
- **VETTER et al.** *Biochemical Pharmacology*, 2012, vol. 83 (11), 1562-71, https://doi.org/10.1016/j.bcp.2012.02.022
- **XU et al.** *Journal of Biological Chemistry*, 03 July 2015, vol. 290 (27), 16619-32, https://doi.org/10.1074/jbc.M115.638932
- **PARK et al.** *Science*, 2004, vol. 24 (5692), 1972-5
- **SANEMATSU et al.** *J. of Biol. Chem.*, 2014, vol. 289 (37), 25711-20
- **AGARWAL et al.** Diagnostic Utility of CSF Tau and A in Dementia: A Meta-Analysis. *International Journal of Alzheimer's Disease*, 2011, 1-10, https://doi.org/10.4061/2011/503293
- **ALZHEIMER et al.** Modal Gating of Na+ Channels as a Mechanism of Persistent Na+ Current in Pyramidal Neurons from Rat and Cat Sensorimotor Cortex. *The Journal of Neuroscience*, 1993, vol. 13 (2), 660-73, https://doi.org/10.1523/JNEUROSCI.13-02-00660.1993

- **AUTHIER et al.** Animal Models of Chemotherapy-Evoked Painful Peripheral Neuropathies. *Neurotherapeutics*, 2009, vol. 6 (4), 620-29, https://doi.org/10.1016/j.nurt.2009.07.003
- **BACHMANOV et al.** Genetics of Amino Acid Taste and Appetite.. *Advances in Nutrition: An International Review Journal*, 2016, vol. 7 (4), 806S-822S, https://doi.org/10.3945/an.115.011270
- **BARNETT et al.** Metabolic Changes in Synaptosomes in an Animal Model of Schizophrenia Revealed by 1H and 1H,13C NMR Spectroscopy. *Metabolites*, 2020, vol. 10 (2), 79, https://doi.org/10.3390/metabo10020079
- **BAYES-GENIS et al.** Bloodstream Amyloid-Beta (1-40) Peptide, Cognition, and Outcomes in Heart Failure. *Revista Espanola de Cardiologia (English Edition)*, 2017, vol. 70 (11), 924-32, https://doi.org/10.1016/j.rec.2017.02.021
- **BIBL et al.** Validation of Amyloid-β Peptides in CSF Diagnosis of Neurodegenerative Dementias. *Molecular Psychiatry*, 2007, vol. 12 (7), 671-80, https://doi.org/10.1038/sj.mp.4001967
- **BRECHET et al.** Protein Kinase CK2 Contributes to the Organization of Sodium Channels in Axonal Membranes by Regulating Their Interactions with Ankyrin G.. *Journal of Cell Biology*, 2008, vol. 183 (6), 1101-14, https://doi.org/10.1083/jcb.200805169
- **BRUNET et al.** Cortical Circuit Dysfunction as a Potential Driver of Amyotrophic Lateral Sclerosis. *Frontiers in Neuroscience*, 2020, vol. 14, 363, https://doi.org/10.3389/fnins.2020.00363
- **CANTRELL et al.** Muscarinic Modulation of Sodium Current by Activation of Protein Kinase C in Rat Hippocampal Neurons.. *Neuron*, 1996, vol. 16 (5), 1019-26, https://doi.org/10.1016/S0896-6273(00)80125-7
- **CANTRELL et al.** Dopaminergic Modulation of Sodium Current in Hippocampal Neurons via CAMP-Dependent Phosphorylation of Specific Sites in the Sodium Channel α Subunit. *The Journal of Neuroscience*, 1997, vol. 17 (19), 7330, https://doi.org/10.1523/JNEUROSCI.17-19-07330.1997

- **CARR et al.** Serotonin Receptor Activation Inhibits Sodium Current and Dendritic Excitability in Prefrontal Cortex via a Protein Kinase C-Dependent Mechanism. *The Journal of Neuroscience*, 2002, vol. 22 (16), 6846, https://doi.org/10.1523/JNEUROSCI.22-16-06846.2002
- **CARR et al.** Transmitter Modulation of Slow, Activity-Dependent Alterations in Sodium Channel Availability Endows Neurons with a Novel Form of Cellular Plasticity. *Neuron*, 2003, vol. 39 (5), 793-806, https://doi.org/10.1016/S0896-6273(03)00531-2
- **CATTERALL**. Voltage-Gated Sodium Channels at 60: Structure, Function and Pathophysiology. *The Journal of Physiology*, 2012, vol. 590 (11), 2577-89, https://doi.org/10.1113/jphysiol.2011.224204
- **CHEN et al.** Suppression of Detyrosinated Microtubules Improves Cardiomyocyte Function in Human Heart Failure. *Nature Medicine*, 2018, vol. 24 (8), 1225-33, https://doi.org/10.1038/s41591-018-0046-2
- **CHIFFLET et al.** The Plasma Membrane Potential and the Organization of the Actin Cytoskeleton of Epithelial Cells.. *International Journal of Cell Biology*, 2012, vol. 2012, 1-13, https://doi.org/10.1155/2012/121424
- **CICCONE et al.** myloid β-Induced Upregulation of Nav1.6 Underlies Neuronal Hyperactivity in Tg2576 Alzheimer's Disease Mouse Model.. *Scientific Reports*, 2019, vol. 9 (1), 13592, https://doi.org/10.1038/s41598-019-50018-1
- **DAHLHAMER et al.** Prevalence of Chronic Pain and High-Impact Chronic Pain Among Adults- United States, 2016. *MMWR. Morbidity and Mortality Weekly Report*, 2018, vol. 67 (36), 1001-6, https://doi.org/10.15585/mmwr.mm6736a2
- **DE JESUS et al.** Atherosclerosis Exacerbates Arrhythmia Following Myocardial Infarction: Role of Myocardial Inflammation. *Heart Rhythm*, 2015, vol. 12 (1), 169-78, https://doi.org/10.1016/j.hrthm.2014.10.007
- **FIELD**. Smell and Taste Dysfunction as Early Markers for Neurodegenerative and Neuropsychiatric Diseases. *Journal of Alzheimer's Disease & Parkinsonism*, 2015, vol. 05 (1), https://doi.org/10.4172/2161-0460.1000186
- **GHATAK et al.** Mechanisms of Hyperexcitability in Alzheimer's Disease HiPSC-Derived Neurons and Cerebral Organoids vs Isogenic Controls. *ELife*, 2019, vol. 8, https://doi.org/10.7554/eLife.50333
- **GONZALEZ-BURGOS** ; **BARRIONUEVO**. Voltage-Gated Sodium Channels Shape Subthreshold EPSPs in Layer 5 Pyramidal Neurons From Rat Prefrontal Cortex. *Journal of Neurophysiol.*, 2001, vol. 86 (4), 1671-1684, https://doi.org/10.1152/jn.2001.86.4.1671
- **GROEMER et al.** Amyloid Precursor Protein Is Trafficked and Secreted via Synaptic Vesicles. *PLoS ONE*, 2011, vol. 6 (4), e18754, https://doi.org/10.1371/journal .pone.0018754
- **GUVENC et al.** A Novel Explanation for the Cause of Atrial Fibrillation Seen in Atherosclerotic Coronary Artery Disease: 'Downstream Inflammation' Hypothesis. *Medical Hypotheses*, 2010, vol. 74 (4), 665-67, https://doi.org/10.1016/j.mehy.2009.11.010
- **HAMPSON et al.** Probing the Ligand-Binding Domain of the MGluR4 Subtype of Metabotropic Glutamate Receptor.. *Journal of Biological Chemistry*, 1999, vol. 274 (47), 33488-95, https://doi.org/10.1074/jbc.274.47.33488
- **HARTLEY et al.** Protofibrillar Intermediates of Amyloid β-Protein Induce Acute Electrophysiological Changes and Progressive Neurotoxicity in Cortical Neurons. *The Journal of Neuroscience*, 1999, vol. 19 (20), 8876-84, https://doi.org/10.1523/JNEUROSCI.19-20-08876.1999
- **HEIJMANS** ; **JOOSTEN**. Mechanisms and Mode of Action of Spinal Cord Stimulation in Chronic Neuropathic Pain. *Postgraduate Medicine*, 2020, 1-5, https://doi.org/10.1080/00325481.2020.1769393
- **HILLE, BERTIL**. Ion Channels of Excitable Membranes. Sinauer associates, 2001
- **HORVATH et al.** Late Sodium Current Inhibitors as Potential Antiarrhythmic Agents. *Frontiers in Pharmacology*, 2020, vol. 413, https://doi.org/10.3389/fphar.2020.00413
- **HUSTED et al.** GPCR-Mediated Signaling of Metabolites. *Cell Metabolism*, 2017, vol. 25 (4), 777-96, https://doi.org/10.1016/j.cmet.2017.03.008
- **HUYGEN et al.** Evidence-Based Interventional Pain Medicine According to Clinical Diagnoses': Update 2018. *Pain Practice*, 2019, vol. 19 (6), 664-75, https://doi.org/10.1111/papr.12786
- **INOUE et al.** The Prevalence and Impact of Chronic Neuropathic Pain on Daily and Social Life: A Nationwide Study in a Japanese Population. *European Journal of Pain*, 2017, vol. 21 (4), 727-37, https://doi.org/10.1002/ejp.977
- **INYUSHIN et al.** On the Role of Platelet-Generated Amyloid Beta Peptides in Certain Amyloidosis Health Complications. *Frontiers in Immunology*, 2020, vol. 11, 571083, https://doi.org/10.3389/fimmu.2020.571083
- **JIANG et al.** Lactisole interacts with the transmembrane domains of human T1R3 to inhibit sweet taste.. *J. Biol. Chem.*, 2005, vol. 280 (15), 15238-15246
- **JIANG et al.** Identification of the Cyclamate Interaction Site within the Transmembrane Domain of the Human Sweet Taste Receptor Subunit T1R3.. *Journal of Biological Chemistry*, 2005, vol. 280 (40), 34296-305, https://doi.org/10.1074/jbc.M505255200

- **KANG et al.** Channel-Anchored Protein Kinase CK2 and Protein Phosphatase 1 Reciprocally Regulate KCNQ2-Containing M-Channels via Phosphorylation of Calmodulin.. *Journal of Biological Chemistry*, 2014, vol. 289 (16), 11536-44, https://doi.org/10.1074/jbc.M113.528497

- **KERR et al.** Detyrosinated Microtubules Modulate Mechanotransduction in Heart and Skeletal Muscle.. *Nature Communications*, 2015, vol. 6 (1), 8526, https://doi.org/10.1038/ncomms9526

- **KIM et al.** Effects of Amyloid-β Peptides on Voltage-Gated L-Type CaV1.2 and CaV1.3 Ca2+ Channels. *Molecules and Cells*, 2011, vol. 32 (3), 289-94, https://doi.org/10.1007/s10059-011-0075-x

- **KUBO et al.** In vivo conversion of racemized beta-amyloid ([D-Ser 26]A beta 1-40) to truncated and toxic fragments ([D-Ser 26]A beta 25-35/40) and fragment presence in the brains of Alzheimer's patients.. *Journal of Neuroscience Research*, 2002, vol. 70 (3), 474-83, https://doi.org/10.1002/jnr.10391

- **KUO et al.** Elevated Aβ42 in Skeletal Muscle of Alzheimer Disease Patients Suggests Peripheral Alterations of AβPP Metabolism. *The American Journal of Pathology*, 2000, vol. 156 (3), 797-805, https://doi.org/10.1016/S0002-9440(10)64947-4

- **LANZNASTER et al.** Aβ1-42 and Tau as Potential Biomarkers for Diagnosis and Prognosis of Amyotrophic Lateral Sclerosis. *International Journal of Molecular Sciences*, vol. 21 (8), 2911, https://doi.org/10.3390/ijms21082911

- **LEHMANN et al.** Cerebrospinal Fluid A Beta 1-40 Peptides Increase in Alzheimer's Disease and Are Highly Correlated with Phospho-Tau in Control Individuals. *Alzheimer's Research & Therapy*, 2020, vol. 12 (1), 123, https://doi.org/10.1186/s13195-020-00696-1

- **LI et al.** Amyloid Precursor Protein Modulates Nav1.6 Sodium Channel Currents through a Go-Coupled JNK Pathway. *Scientific Reports*, 2016, vol. 6 (1), https://doi.org/10.1038/srep39320

- **LI et al.** Human Receptors for Sweet and Umami Taste.. *Proceedings of the National Academy of Sciences*, 2002, vol. 99 (7), 4692-96, https://doi.org/10.1073/pnas.072090199

- **LI**. T1R Receptors Mediate Mammalian Sweet and Umami Taste. *The American Journal of Clinical Nutrition*, 2009, vol. 90 (3), 733S-737S, https://doi.org/10.3945/ajcn.2009.27462G

- **LI et al.** Sweet Taste Receptor Gene Variation and Aspartame Taste in Primates and Other Species. *Chemical Senses*, 2011, vol. 36 (5), 453-75, https://doi.org/10.1093/chemse/bjq145

- **LI et al.** DRG Voltage-Gated Sodium Channel 1.7 Is Upregulated in Paclitaxel-Induced Neuropathy in Rats and in Humans with Neuropathic Pain.. *The Journal of Neuroscience*, 2018, vol. 38 (5), 1124-36, https://doi.org/10.1523/JNEUROSCI.0899-17.2017

- **LIPOWSKY et al.** *Journal of Neurophysiology*, 1996, vol. 76 (4), 2181-91, https://doi.org/10.1152/jn.1996.76.4.2181

- **LOWE et al.** Voltage-Gated Nav Channel Targeting in the Heart Requires an Ankyrin-G-Dependent Cellular Pathway.. *Journal of Cell Biology*, 2008, vol. 180 (1), 173-86, https://doi.org/10.1083/jcb.200710107

- **LUE et al.** Soluble Amyloid β Peptide Concentration as a Predictor of Synaptic Change in Alzheimer's Disease.. *The American Journal of Pathology*, 1999, vol. 155 (3), 853-62, https://doi.org/10.1016/S0002-9440(10)65184-X

- **MA et al.** Persistent Sodium Currents through Brain Sodium Channels Induced by G Protein By Subunits. *Neuron*, 1997, vol. 19 (2), 443-52, https://doi.org/10.1016/S0896-6273(00)80952-6

- **MAILLET et al.** Characterization of the Binding Site of Aspartame in the Human Sweet Taste Receptor.. *Chemical Senses*, 2015, vol. 40 (8), 577-86, https://doi.org/10.1093/chemse/bjv045

- **MANTEGAZZA et al.** Molecular Determinants for Modulation of Persistent Sodium Current by G-Protein βγ Subunits.. *Journal of Neuroscience*, 2005, vol. 30 (13), 3341-3349

- **MARTIN et al.** Altered Learning, Memory, and Social Behavior in Type 1 Taste Receptor Subunit 3 Knockout Mice Are Associated with Neuronal Dysfunction.. *Journal of Biological Chemistry*, 2017, vol. 292 (27), 11508-30, https://doi.org/10.1074/jbc.M116.773820

- **MARTINEZ et al.** Amino Acid Concentrations in Cerebrospinal Fluid and Serum in Alzheimer's Disease and Vascular Dementia. *Journal of Neural Transmission - Parkinson's Disease and Dementia Section*, 1993, vol. 6 (1), 1-9, https://doi.org/10.1007/BF02252617

- **MASUBUCHI et al.** T1R3 homomeric sweet taste receptor regulates adipogenesis through Gas-mediated microtubules disassembly and Rho activation in 3T3-L1 cells. *PLoS ONE*, 2017, vol. 12 (5), e0176841, https://doi.org/10.1371/journal.pone.0176841

- Characterization of the Modes of Binding between Human Sweet Taste Receptor and Low-Molecular-Weight Sweet Compounds. **MASUDA et al.** PLoS ONE. 2012, vol. 7, e35380

- **MATTHEISEN et al.** Strong G-Protein-Mediated Inhibition of Sodium Channels. *Cell Reports*, 2018, vol. 23 (9), 2770-81, https://doi.org/10.1016/j.celrep.2018.04.109

- **MAILLIET et al.** Phenoxy Herbicides and Fibrates Potently Inhibit the Human Chemosensory Receptor Subunit T1R3. *Journal of Medicinal Chemistry*, 2009, vol. 52 (21), 6931-35, https://doi.org/10.1021/jm900823s
- **MO et al.** Cerebrospinal Fluid β-Amyloid1-42 Levels in the Differential Diagnosis of Alzheimer's Disease-Systematic Review and Meta-Analysis. *PLOS ONE*, 2015, vol. 10 (2), e0116802, https://doi.org/10.1371/-journal.pone.0116802
- Structural Insights into the Differences among Lactisole Derivatives in Inhibitory Mechanisms against the Human Sweet Taste Receptor. **NAKAGITA et al.** PLOS ONE. 2019, vol. 14, e0213552
- Sweeteners. **NEIERS et al.** Reference Series in Phytochemistry. Springer International Publishing, 2016, 1-20
- **NEWELL-LITWA** ; **HORWITZ**. Cell Migration: PKA and RhoA Set the Pace. *Current Biology*, 2011, vol. 21 (15), R596-98, https://doi.org/10.1016/j.cub.2011.06.032.65
- **NIN et al.** Membrane Potential Hyperpolarization Reorganizes Actin Cytoskeleton.. *Cell Motility and the Cytoskeleton*, 2009, vol. 66 (12), 1087-99, https://doi.org/10.1002/cm.20416
- **NORTH et al.** Ectopic Spontaneous Afferent Activity and Neuropathic Pain.. *Neurosurgery*, 2018, vol. 65 (1), 49-54, https://doi.org/10.1093/neuros/nyy119.67
- **O'HARA et al.** The ligand-binding domain in metabotropic glutamate receptors is related to bacterial periplasmic binding proteins. *Neuron*, 1993, vol. 11 (1), 41-52
- **PADHI et al.** Clinical Pharmacokinetic and Pharmacodynamic Profile of Cinacalcet Hydrochloride. *Clinical Pharmacokinetics*, 2009, vol. 48 (5), 303-11, https://doi.org/10.2165/00003088-200948050-00002
- **PALOP et al.** Network Abnormalities and Interneuron Dysfunction in Alzheimer Disease.. *Nature Reviews Neuroscience*, 2016, vol. 17 (12), 777-92, https://doi.org/10.1038/nrn.2016.141
- **PALOP** ; **JORGE**. Epilepsy and Cognitive Impairments in Alzheimer Disease. *Archives of Neurology*, 2009, vol. 66 (4), 435, https://doi.org/10.1001/archneurol.2009.15
- **PALYGIN et al.** Regulation of Caveolar Cardiac Sodium Current by a Single G s α Histidine Residue.. *American Journal of Physiology-Heart and Circulatory Physiology*, 2008, vol. 294 (4), H1693-99, https://doi.org/10.1152/ajpheart.01337.2007
- **PARK**. Recycling Endosomes Supply AMPA Receptors for LTP.. *Science*, 2004, vol. 305 (5692), 1972-75, https://doi.org/10.1126/science. 1102026
- **PERRY et al.** *Muscle Atrophy in Patients with Type 2 Diabetes Mellitus: Roles of Inflammatory Pathways, Physical Activity and Exercise*, 2016, vol. 22, 94-10974
- **PIANU et al.** The Aβ1-42 Peptide Regulates Microtubule Stability Independently of Tau. *Journal of Cell Science*, 2014, vol. 127 (5), 1117, https://doi.org/10.1242/jcs.143750
- **PRADHAN et al.** The Heterotrimeric G Protein Gβ1 Interacts with the Catalytic Subunit of Protein Phosphatase 1 and Modulates G Protein-Coupled Receptor Signaling in Platelets.. *Journal of Biological Chemistry*, 2017, vol. 292 (32), 13133-42, https://doi.org/10.1074/jbc. M117.796656
- **QUINTAO et al.** Pharmacological Treatment of Chemotherapy-Induced Neuropathic Pain: PPARγ Agonists as a Promising Tool. *Frontiers in Neuroscience*, 2019, vol. 13, https://doi.org/10.3389/fnins.2019.00907
- **ROYCHOWDHURY** ; **RASENICK**. Submembraneous Microtubule Cytoskeleton: Regulation of Microtubule Assembly by Heterotrimeric G Proteins: G Proteins and Microtubule Assembly. *FEBS Journal*, 2008, vol. 275 (19), 4654-63, https://doi.org/10.1111/j.1742-4658.2008.06614.x
- **SAITO**. NanoTouch: Intracellular Recording Using Transmembrane Conductive Nanoparticles.. *Journal of Neurophysiology*, 2019, vol. 122 (5), 2016-26, https://doi.org/10.1152/jn.00359.2019
- **SCHAPPI et al.** Tubulin, Actin and Heterotrimeric G Proteins: Coordination of Signaling and Structure.. *Biochimica et Biophysica Acta (BBA) - Biomembranes*, 2014, vol. 1838 (2), 674-81, https://doi.org/10.1016/j.bbamem.2013.08.026
- **SCHWINDT** ; **CRILL**. Amplification of Synaptic Current by Persistent Sodium Conductance in Apical Dendrite of Neocortical Neurons. *Journal of Neurophysiology*, 1995, vol. 74 (5), 2220-24, https://doi.org/10.1152/jn.1995.74.5.2220
- **SHCHERBATKO et al.** Voltage-Dependent Sodium Channel Function Is Regulated Through Membrane Mechanics. *Biophysical Journal*, 1999, vol. 77 (4), 1945-59, https://doi.org/10.1016/50006-3495(99)77036-0
- **SCHELL et al.** Glomerular Development - Shaping the Multi-Cellular Filtration Unit.. *Development of the Urogenital System & MTOR Signalling & Tight Junctions in Health and Disease*, 2014, vol. 36, 39-49, https://doi.org/10.1016/j.semcdb.2014.07.016
- **SHAKOOR et al.** Alzheimer's: Learning from a Legacy of Bitter Setbacks. *Endpoints News*, 2017, https://endpts.com/special/alzheimers-2017
- **SHIBATA et al.** Autonomic Regulation of Voltage-Gated Cardiac Ion Channels. *Journal of Cardiovascular Electrophysiology*, 2006, vol. 17 (s1), S34-42, https://doi.org/10.1111/j.1540-8167.2006.00387.x

- **SMITH et al.** Putative Amino Acid Transmitters in Lumbar Cerebrospinal Fluid of Patients with Histologically Verified Alzheimer's Dementia. *Journal of Neurology, Neurosurgery & Psychiatry*, 1985, vol. 48 (5), 469-71, https://doi.org/10.1136/jnnp.48.5.469
- **SPERLING et al.** Amyloid Deposition Is Associated with Impaired Default Network Function in Older Persons without Dementia. *Neuron*, 2009, vol. 63 (2), 178-88, https://doi.org/10.1016/j.neuron.2009.07.003
- **STAMATELOPOULOS et al.** Circulating Amyloid-Beta (1-40) Predicts Clinical Outcomes in Patients With Heart Failure. *Revista Espanola de Cardiologia (English Edition)*, 2017, vol. 70 (11), 905-6, https://doi.org/10.1016/j.rec.2017.05.026
- **SURMEIER et al.** Dopamine Receptor Subtypes Colocalize in Rat Striatonigral Neurons. *Proceedings of the National Academy of Sciences*, 1992, vol. 89 (21), 10178-82, https://doi.org/10.1073/pnas.89.21.10178
- **TEMUSSI**. The Good Taste of Peptides: PEPTIDES TASTE.. *Journal of Peptide Science*, 2012, vol. 18 (2), 73-82, https://doi.org/10.1002/psc.1428
- **TODA et al.** Positive/Negative Allosteric Modulation Switching in an Umami Taste Receptor (T1R1/T1R3) by a Natural Flavor Compound, Methional. *Scientific Reports*, 2018, vol. 8 (1), 11796, https://doi.org/10.1038/s41598-018-30315-x
- **TODA et al.** Evolution of the Primate Glutamate Taste Sensor from a Nucleotide Sensor.. *Current Biology*, 2021, vol. 31 (20), 4641-4649, https://doi.org/10.1016/j.cub.2021.08.002
- **TRONCONE et al.** Aβ Amyloid Pathology Affects the Hearts of Patients With Alzheimer's Disease. *Journal of the American College of Cardiology*, December 2016, vol. 68 (22), 2395-2407, https://doi.org/10.1016/j.jacc.2016.08.073.93
- **TSUCHIYA**. Local Anesthetic Failure Associated with Inflammation: Verification of the Acidosis Mechanism and the Hypothetic Participation of Inflammatory Peroxynitrite. *Journal of Inflammation Research*, 2008, vol. 41, https://doi.org/10.2147/JIR.S3982
- **UEDA et al.** Functional Interaction between T2R Taste Receptors and G-Protein α Subunits Expressed in Taste Receptor Cells. *The Journal of Neuroscience*, 2003, vol. 23 (19), 7376, https://doi.org/10.1523/JNEUROSCI.23-19-07376.2003
- **VANDERSTICHELE et al.** Optimized Standard Operating Procedures for the Analysis of Cerebrospinal Fluid Aβ42 and the Ratios of Aβ Isoforms Using Low Protein Binding Tubes.. *Journal of Alzheimer's Disease*, 2016, vol. 53 (3), 1121-32, https://doi.org/10.3233/JAD-160286
- **VETTER et al.** Characterisation of Nav Types Endogenously Expressed in Human SH-SY5Y Neuroblastoma Cells.. *Biochemical Pharmacology*, 2012, vol. 83 (11), 1562-71, https://doi.org/10.1016/j.bcp.2012.02.022
- **VITVITSKY et al.** Na+ and K+ Ion Imbalances in Alzheimer's Disease.. *Biochimica et Biophysica Acta (BBA) - Molecular Basis of Disease*, 2012, vol. 1822 (11), 1671-81, https://doi.org/10.1016/j.bbadis.2012.07.004
- **WANG et al.** Distribution and Function of Voltage-Gated Sodium Channels in the Nervous System. *Channels*, 2017, vol. 11 (6), 534-54, https://doi.org/10.1080/19336950.2017.1380758
- **WANG et al.** Multiple Nav1.5 Isoforms Are Functionally Expressed in the Brain and Present Distinct Expression Patterns Compared with Cardiac Nav1.5. *Molecular Medicine Reports*, 2017, vol. 16 (1), 719-29, https://doi.org/10.3892/mmr.2017.6654
- **WANG et al.** Molecular Expression of Multiple Nav1.5 Splice Variants in the Frontal Lobe of the Human Brain. *International Journal of Molecular Medicine*, 2017, https://doi.org/10.3892/ijmm.2017.3286
- **WANG et al.** Membrane Trauma and Na+ Leak from Nav1.6 Channels.. *American Journal of Physiology-Cell Physiology*, 2009, vol. 297 (4), C823-34, https://doi.org/10.1152/ajpcell.00505.2008
- **WILLIAMS et al.** Mechanisms and Consequences of Action Potential Burst Firing in Rat Neocortical Pyramidal Neurons. *The Journal of Physiology*, 1999, vol. 521 (2), 467-82, https://doi.org/10.1111/j.1469-7793.1999.00467.x
- **WAXMAN et al.** Sodium Channels and Pain. *Proceedings of the National Academy of Sciences*, 1999, vol. 96 (14), 7635, https://doi.org/10.1073/pnas. 96.14.7635
- **XIAO et al.** Chemotherapy-Evoked Neuropathic Pain: Abnormal Spontaneous Discharge in A-Fiber and C-Fiber Primary Afferent Neurons and Its Suppression by Acetyl-1-Carnitine. *Pain*, 2008, vol. 135 (3), 262-70, https://doi.org/10.1016/j.pain.2007.06.001
- **XU et al.** Different Functional Roles of T1R Subunits in the Heteromeric Taste Receptors.. *Proceedings of the National Academy of Sciences*, 2004, vol. 101 (39), 14258-63, https://doi.org/10.1073/pnas.0404384101
- **XU ; COOPER**. An Ankyrin-G N-Terminal Gate and Protein Kinase CK2 Dually Regulate Binding of Voltage-Gated Sodium and KCNQ2/3 Potassium Channels. *Journal of Biological Chemistry*, 2015, vol. 290 (27), 16619-32, https://doi.org/10.1074/jbc.M115.638932
- **YANG et al.** Gα12/13 Signaling in Metabolic Diseases. *Experimental & Molecular Medicine*, 2020, vol. 52 (6), 896-910, https://doi.org/10.1038/s12276-020-0454-5

- **ZHAO et al.** The Receptors for Mammalian Sweet and Umami Taste.. *Cell*, 2003, vol. 115 (3), 255-66, https://doi.org/10.1016/50092-8674(03)00844-4
- **ZHOU et al.** AnkyrinG Is Required for Clustering of Voltage-Gated Na Channels at Axon Initial Segments and for Normal Action Potential Firing.. *Journal of Cell Biology*, 1998, vol. 1 (5), 1295-1304
- **KIM, S.** ; **RHIM, H.** Effects of amyloid-b peptides on voltage-gated L-type CaV1.2 and CaV1.3 Ca2+ channels. *Mol Cells*, 2011, vol. 32, 289-294, https://doi.org/10.1007/s10059-011-0075-x
- **THIEL et al.** Stability of gene expression in human T cells in different gravity environments is clustered in chromosomal region 11p15.4. *NPJ Microgravity*, 2017, vol. 31 (3), 22
- **FRASER**. T-lymphocyte invasiveness: control by voltage-gated Na+ channel activity. *FEBS Letters*, 2004, 569
- **TAYLOR et al.** Simple, Reliable Protocol for High-Yield Solubilization of Seedless Amyloid-β Monomer. *ACS Chemical Neuroscience*, 2023, vol. 14 (1), 53-71
- **JIANG et al.** The Cysteine-Rich Region of T1R3 Determines Responses to Intensely Sweet Proteins.. *J. of Biol. Chem.*, 2004, vol. 279 (43), 45068-75, https://doi.org/10.1074/jbc.M406779200
- **MASUDA et al.** Five Amino Acid Residues in Cysteine-Rich Domain of Human T1R3 Were Involved in the Response for Sweet-Tasting Protein, Thaumatin. *Biochimie*, 2013, vol. 95 (7), 1502-5, https://doi.org/10.1016/j.biochi.2013.01.010
- **RASBAND, W.S.** ImageJ. U. S. National Institutes of Health, 1997-2018
- **SCHNEIDER, C.A., RASBAND, W.S., ELICEIRI, K.W LEWCZUK**. *Amyloid-β 42/40 Cerebrospinal Fluid Concentration Ratio in the Diagnostics of Alzheimer's Disease: Validation of Two Novel Assays*, 01 January 2015, 183-191
- **HATA et al.** Association Between Serum β-Alanine and Risk of Dementia. *Am J Epidemiol. 1*, 2019, vol. 188 (9), 1637-1645
- **BIBL et al.** CSF amyloid-beta-peptides in Alzheimer's disease, dementia with Lewy bodies and Parkinson's disease dementia. *Brain*, 06 April 2006, vol. 129 (5), 1177-87
- **FANDOS et al.** Plasma amyloid β 42/40 ratios as biomarkers for amyloid β cerebral deposition in cognitively normal individuals. *Alzheimers Dement (Amst).*, 2017, vol. 8, 179-187
- **WINNIG et al.** Valine 738 and lysine 735 in the fifth transmembrane domain of rTas1r3 mediate insensitivity towards lactisole of the rat sweet taste receptor.. *BMC Neurosci.*, 2005, vol. 6, 22
- **NAKAGITA et al.** Structural insights into the differences among lactisole derivatives in inhibitory mechanisms against the human sweet taste receptor. *PLoS ONE*, 2019, vol. 14 (3), e0213552, https://doi.org/10.1371/journal.pone.0213552
- **NUEMKET et al.** Structural Basis for Perception of Diverse Chemical Substances by T1r Taste Receptors.. *Nature Communications*, 2017, vol. 8 (1), https://doi.org/10.1038/ncomms15530